(19) Europäisches Patentamt
European Patent Office
Office européen des brevets

(11) EP 1 565 467 B1

(12) **FASCICULE DE BREVET EUROPEEN**

(45) Date de publication et mention de la délivrance du brevet:
**20.05.2009 Bulletin 2009/21**

(21) Numéro de dépôt: **03799611.3**

(22) Date de dépôt: **12.11.2003**

(51) Int Cl.:
***C07D 471/04*** *(2006.01)* ***C07D 401/14*** *(2006.01)*
***A61K 31/506*** *(2006.01)* ***A61P 19/00*** *(2006.01)*
***A61P 9/00*** *(2006.01)* ***A61P 35/00*** *(2006.01)*
***A61P 27/02*** *(2006.01)* ***A61P 13/00*** *(2006.01)*

(86) Numéro de dépôt international:
**PCT/FR2003/003349**

(87) Numéro de publication internationale:
**WO 2004/048375 (10.06.2004 Gazette 2004/24)**

(54) **NOUVEAUX DERIVES ANTAGONISTES DU RECEPTEUR DE LA VITRONECTINE LEUR PROCEDE DE PREPARATION, LEUR APPLICATION COMME MEDICAMENTS ET LES COMPOSITIONS PHARMACEUTIQUES LES RENFERMANT**

NEUE ANTAGONISTEN FÜR DEN VITRONECTINREZEPTOR, VERFAHREN ZU DEREN HERSTELLUNG, DEREN VERWENDUNG ALS ARZNEIMITTEL UND PHARMAZEUTISCHE ZUSAMMENSETZUNG, DIE DIESE ENTHALTEN

NOVEL VITRONECTIN RECEPTOR ANTAGONIST DERIVATIVES, METHOD FOR PREPARING SAME, USE THEREOF AS MEDICINES AND PHARMACEUTICAL COMPOSITIONS CONTAINING SAME

(84) Etats contractants désignés:
**AT BE BG CH CY CZ DE DK EE ES FI FR GB GR HU IE IT LI LU MC NL PT RO SE SI SK TR**
Etats d'extension désignés:
**LT LV MK**

(30) Priorité: **19.11.2002 FR 0214429**

(43) Date de publication de la demande:
**24.08.2005 Bulletin 2005/34**

(60) Demande divisionnaire:
**09003174.1**

(73) Titulaire: **Galapagos SAS**
**93230 Romainville (FR)**

(72) Inventeurs:
- **RUXER, Jean-Marie**
  **F-92130 Issy les Moulineaux (FR)**
- **LEFRANCOIS, Jean-Michel**
  **F-93390 Livry Gargan (FR)**
- **HECKMANN, Bertrand**
  **F-91440 Bures sur Yvette (FR)**

(74) Mandataire: **Lord, Hilton David et al**
**Marks & Clerk LLP**
**90 Long Acre**
**London**
**WC2E 9RA (GB)**

(56) Documents cités:
**EP-A- 0 820 991** **EP-A- 0 933 367**
**WO-A-96/00574**

- **HENRY, C.; MOITESSIER, N.; CHAPLEUR, Y.: MINI REVIEWS IN MEDICINAL CHEMISTRY, vol. 2, 2002, pages 531-542, XP0001180444**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet de nouveaux dérivés antagonistes du récepteur de la vitronectine, leur procédé de préparation, leur application comme médicaments et les compositions pharmaceutiques les renfermant.

L'invention a pour objet les composés de formule (I) :

(I)

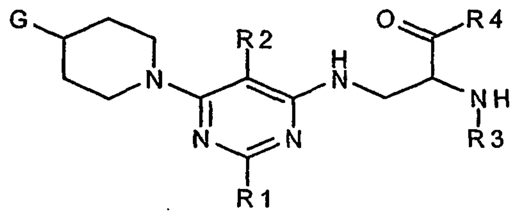

dans laquelle $R_1$, $R_2$, $R_3$, $R_4$ et G ont les significations indiquées plus bas ainsi que leurs sels physiologiquement acceptables. Les composés de formule (I) sont des composés ayant une activité pharmacologique et sont donc utilisables à titre de médicaments. Ce sont des antagonistes du récepteur de la vitronectine et des inhibiteurs de l'adhésion cellulaire et ils inhibent la résorption osseuse médiée par les ostéoclastes. Ils sont donc utiles pour le traitement thérapeutique ou prophylactique de maladies qui sont causées au moins en partie par une augmentation non désirée de la résorption osseuse, par exemple l'ostéoporose. L'invention de plus a pour objet les procédés de préparation des composés de formule (I), leur application, en particulier à titre de médicament et les compositions pharmaceutiques les renfermant.

L'os est constamment soumis à un processus dynamique qui inclut la résorption osseuse et la formation osseuse. Ces processus sont médiés via des cellules spécialisées. La formation osseuse est le résultat du dépôt d'une matrice minérale par les ostéoblastes et la résorption osseuse est le résultat de la dissolution de cette matrice osseuse par les ostéoclastes. La majorité des désordres au niveau de l'os sont basés sur un équilibre perturbé entre la formation osseuse et la résorption osseuse. L'ostéoporose est caractérisée par une perte sèche de cette matrice osseuse. Un ostéoclaste mature activé·résorbe l'os après adhésion à la matrice osseuse via la sécrétion d'enzyme protéolytique, et de protons à l'intérieur de la zone d'adhésion, aboutissant à des dépressions ou des creusements de la surface de l'os qui apparaissent au moment où l'ostéoclaste se détache de l'os.

Des études ont montré que la fixation de l'ostéoclaste sur l'os est médiée par des récepteurs : les intégrines. Les intégrines sont une superfamille de récepteurs médiant les processus d'adhésion cellule/cellule et plus particulièrement cellule/matrice, incluant notamment $\alpha_{IIb}\beta_3$ comme récepteur des plaquettes sanguines (fibrinogène) et $\alpha_v\beta_3$ comme récepteur de la vitronectine. Les peptides contenant le motif RGD ainsi que les anticorps anti $\alpha_v\beta_3$ sont connus pour leur capacité d'inhibition de la résorption de la dentine et d'empêchement de l'adhésion des ostéoclastes sur les matrices minéralisées (Horton et al. Exp. Cell. Res. (1991), 195, 368). Le peptide Echistatine, isolé du venin de serpent contenant également un motif RGD et décrit comme inhibiteur de l'adhésion des ostéoclastes à l'os est un puissant inhibiteur de la résorption osseuse dans les tissus en culture in vitro (Sato et al. J. Cell. Biol. (1990), 111, 1713) et in vivo chez le rat (Fisher et al. Endocrinology (1993), 132, 1411).

Le récepteur $\alpha_v\beta_3$ est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés de formule (I) selon l'invention.

En effet, les récepteurs $\alpha_v\beta_3$, exprimés au niveau de la membrane des ostéoclastes sont à la base du processus d'adhésion/résorption, contribuent à l'organisation du cytosquelette cellulaire, et sont impliqués dans l'ostéoporose. Les récepteurs $\alpha_v\beta_3$ exprimés au niveau des cellules du muscle lisse de l'aorte, stimulent leur migration vers la néointima, ce qui entraîne la formation de l'artériosclérose et la survenue de resténose post-angioplastique (Brown et al., cardiovascular Res. (1994), 28, 1815). Les cellules endothéliales secrètent des facteurs de croissance qui sont mitogènes pour l'endothélium et peuvent contribuer à la formation de nouveaux vaisseaux sanguins (Angiogenèse).

Les antagonistes de l'intégrine $\alpha_v\beta_3$ peuvent ainsi entraîner une régression des tumeurs cancéreuses en induisant l'apoptose des vaisseaux sanguins angiogéniques. (Brook et al. Cell (1994) 79, 1157).

Cheresh et al (Science 1995, 270, 1500) ont décrit des anticorps anti-$\alpha_v\beta_3$ ou des antagonistes du récepteur $\alpha_v\beta_3$ qui inhibent le processus d'angiogénèse induit par bFGF dans l'oeil de rat, une propriété qui peut être utilisée pour le traitement des rétinopathies, notamment du diabétique.

La demande de brevet WO-A-94/12181 décrit des systèmes aromatiques ou non aromatiques substitués et WO-A-94/08577 décrit des hétérocycles substitués comme antagonistes du récepteur de fibrinogène et inhibiteurs de l'agrégation plaquettaire. EP-A-528586 et EP-A-528587 décrivent des dérivés de la phénylalanine substitués par un aminoalkyle ou un hétérocycle et WO-A-95/32710 décrivent des dérivés aryliques comme inhibiteurs de la résorption osseuse par les ostéoclastes. WO-A-96/00574 décrit des benzodiazépines et WO-A-96/00730 décrit des composés

inhibiteurs du récepteur fibrinogène, en particulier benzodiazépines qui sont liés à un cycle à 5 chaînons azoté comme antagonistes du récepteur de la vitronectine. WO9800395, WO99/32457 et WO99/37621 décrivent des antagonistes du récepteur de la vitronectine dérivés de la tyrosine. EP0820991 revendique des dérivés de cycloalkyle comme antagonistes du récepteur de la vitronectine.

D'autres investigations ont permis de montrer que les dérivés de formule (I) présentent une forte activité comme antagonistes du récepteur de la vitronectine et de la résorption osseuse médiée via les ostéoclastes.

L'invention a pour objet les composés de formule (I) :

(I) 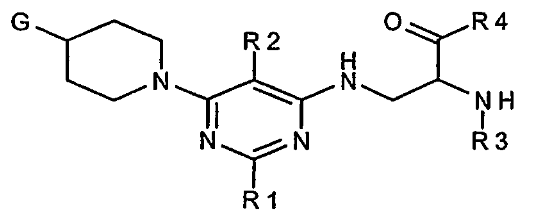

sous toutes leurs formes isomères, seules ou en mélange, ainsi que leurs sels d'addition physiologiquement acceptables, dans laquelle

- **G** représente :

  $R^7R^8N$-C (=$NR^6$) -NH-CO-
  Het-NH-CO-;
  Het-NH-$CH_2$-,
  Het-,

  Het représentant un système monocyclique ou polycyclique, chaque cycle étant constitué de 4 à 10 chaînons aromatiques ou non aromatiques, le cycle ou au moins l'un des cycles renfermant de 1 à 4 atome d'azote, substitué ou non substitué par un ou plusieurs groupements $R_9$;
- **$R^1$** représente un atome d'hydrogène; un groupement ($C_5$-$C_{14}$)-aryle; ($C_5$-$C_{14}$)-aryl-($C_1$-$C_4$)-alkyle-; un radical amino non substitué ou monosubstitué ou disubstitué par un radical alkyle et/ou un radical acyle renfermant de 1 à 4 atomes de carbone;
- **$R^2$** représente un atome d'hydrogène; un atome d'halogène; un groupement nitro; un radical alkyle renfermant de 1 à 4 atomes de carbones; un radical amino non substitué ou monosubstitué ou disubstitué par un radical alkyle et/ou un radical acyle renfermant de 1 à 4 atomes de carbone; un groupement -$(CH_2)_{0-2}$-$CO_2R^5$; ou un groupement -$(CH_2)_{0-2}$-$OR^5$;
- **$R^3$** représente :

  - un atome d'hydrogène
  - un radical -$CO_2R^5$,
  - un radical -$SO_2R^5$ ou
  - un système monocyclique ou polycyclique, chaque cycle étant constitué de 4 à 10 chaînons aromatiques ou non aromatiques, le cycle ou au moins l'un des cycles renfermant de 1 à 4 hétéroatomes choisis parmi N, O ou S, substitué ou non substitué par un ou plusieurs radicaux $R_9$,

- **$R^4$** représente OH; ($C_1$-$C_8$)-alkoxy-; ($C_5$-$C_{14}$)-aryl- ($C_1$-$C_4$)-alkyloxy-; ($C_5$-$C_{14}$)-aryloxy-; ($C_3$-$C_{12}$)-cycloalkyloxy; ($C_3$-$C_{12}$)-cycloalkyl-($C_1$-$C_4$)-alkyloxy-; ($C_1$-$C_7$)-alkylcarbonyloxy-($C_1$-$C_4$)-alkyloxy-; ($C_5$-$C_{14}$)-aryl-($C_1$-$C_4$)-alkylcarbonyloxy-($C_1$-$C_4$) alkyloxy-; ($C_1$-$C_8$)dialkylaminocarbonylméthyloxy-; ($C_5$-$C_{14}$)-aryl-($C_1$-$C_4$)-dialkylaminocarbonylméthyloxy-; un radical amino non substitué ou monosubstitué ou disubstitué par un radical ($C_1$-$C_4$)-alkyle et/ou ($C_5$-$C_{14}$)-aryle et/ou ($C_5$-$C_{14}$)-aryl-($C_1$-$C_4$)-alkyle- et/ou un radical ($C_1$-$C_5$)-acyle; ou bien le reste d'un aminoacide D ou L;
- **$R^5$** représente ($C_1$-$C_8$)-alkyle; ($C_5$-$C_{14}$)-aryle; ($C_5$-$C_{14}$)-aryl-($C_1$-$C_4$)-alkyle-; ($C_3$-$C_{12}$)-cycloalkyle ou ($C_3$-$C_{12}$)-cycloalkyl-($C_1$-$C_4$)-alkyle-, bicycloalkyle-($C_1$-$C_4$)-alkyle-, tricycloalkyle-($C_1$-$C_4$)-alkyle-, les dits radicaux aryles, alkyles, cycloalkyles, bicycloalkyles et tricycloalkyles étant non substitués ou substitués par un ou plusieurs groupements choisis $R^9$;
- **$R^6$** représente un atome d'hydrogène; un groupement hydroxyle; nitro, ($C_1$-$C_6$)-alkyl-O-CO-; ou ($C_1$-$C_6$)-alkyl-O-CO-O-;

- **R⁷** et **R⁸,** indépendamment l'un de l'autre représentent un atome d'hydrogène ou un radical ($C_1$-$C_6$)-alkyle non substitué ou substitué par $R_9$;
- **R⁹** représente halogène; amino; nitro; hydroxyle, ($C_1$-$C_4$)-alkyloxy-; ($C_1$-$C_4$)-alkylthio-; carboxy; ($C_1$-$C_4$)-alkyloxycarbonyle-; ($C_1$-$C_8$)-alkyle non substitué ou substitué par un ou plusieurs atomes d'halogène, ($C_5$-$C_{14}$)-aryle , ($C_5$-$C_{14}$)-aryl-($C_1$-$C_4$)-alkyle-.

Tous les radicaux qui peuvent se retrouver plusieurs fois dans les composés de formule (I), comme par exemple le radical $R_9$, sont indépendants les uns des autres et peuvent être identiques ou différents.
Les radicaux alkyles peuvent être linéaires ou ramifiés. Ceci s'applique également lorsqu'ils portent un substituant ou lorsqu'ils sont inclus dans des groupements tels que par exemple alkoxy, alkoxycarbonyle ou aralkyle.
Par ($C_1$-$C_8$)-alkyle on entend les radicaux méthyle, éthyle, propyle, butyle, pentyle, hexyle, heptyle, octyle, nonyle, les n-isomères de ces radicaux, isopropyle, isobutyle, isopentyle, néopentyle, isohexyle, 3-méthylepentyle, 2,3,4-triméthylhexyle, sec-butyle, tert-butyle, tert-pentyle. Parmi les radicaux préférés on peut citer les groupements ($C_1$-$C_4$)-alkyles tels que méthyle, éthyle, n-propyle, isopropyle, n-butyle, isobutyle, et tert-butyle. De préférence alkyle représente méthyle ou éthyle.
Les radicaux cycloalkyles peuvent être monocycliques, bicycliques ou tricycliques. Il s'agit par exemple des radicaux cyclopropyle, cyclobutyle, cyclopentyle, cyclohexyle, cycloheptyle, cyclooctyle, cyclononyle, cyclodécyle, cycloundécyle, cyclododécyle, cyclotétradécyle ou cyclooctadécyle qui le cas échéant peuvent être substitués par exemple par un alkyle renfermant de 1 à 4 atomes de carbone. Comme radicaux cycloalkyles substitués, on peut citer le 4-méthylecyclo-hexyle et le 2,3-diméthylecyclo-hexyle.
Les radicaux bicycloalkyles et tricycloalkyles peuvent être non substitués ou substitués en toute position, par exemple par un ou plusieurs groupes oxo et/ou 1 ou plusieurs groupes alkyles identiques ou différents tels que méthyle ou isopropyle et de préférence méthyle. La liaison de jonction du radical bi ou tricyclique peut se situer en toutes positions de la molécule. La liaison peut se situer au niveau de l'atome de carbone ponté ou d'un des autres atomes de carbone. Cette liaison peut présenter également toute position du point de vue de la stéréochimie, par exemple exo ou endo. Comme exemple de radicaux bicycloalkyles ou tricycloalkyles, on peut citer le camphanyle, le bornyle, l'adamantyle tel que le 1-adamantyle ou le 2-adamantyle, le caranyle, l'épiisobornyle, l'épibornyle, le norbornyle ou le norpinanyle.
Par halogène on entend fluor, chlore, brome ou iode.
Par le terme ($C_5$-$C_{14}$)-aryle on entend :

- soit les radicaux ($C_5$-$C_{14}$)-aryle hétérocycliques (= ($C_5$-$C_{14}$)-hétéroaryle), dans lesquels un ou plusieurs atomes de carbone du cycle sont remplacés par un hétéroatome tel que azote, oxygène ou soufre,
- soit les radicaux ($C_6$-$C_{14}$)-aryle carbocyclique.

Parmi les radicaux($C_6$-$C_{14}$)-aryle carbocycliques, on peut citer le phényle, le naphtyle, le biphénylyle, l'anthryle ou le fluorényle et tout particulièrement le 1-naphtyle, le 2-naphtyle et le phényle.
Sauf indication contraire, les radicaux aryles, en particulier phényle, peuvent être non substitués ou substitués par un ou plusieurs radicaux identiques ou différents choisis parmi ($C_1$-$C_8$)-alkyle, en particulier ($C_1$-$C_4$)-alkyle, hydroxyle, ($C_1$-$C_8$)-alkyloxy, ($C_1$-$C_8$)-alkylthio, halogène tel que fluor, chlore et brome, nitro, amino, ($C_1$-$C_4$)-alkylamino, di-($C_1$-$C_4$)-alkylamino, trifluorométhyle, méthylènedioxy, cyano, aminocarbonyle, ($C_1$-$C_4$)-alkylaminocarbonyle, di-($C_1$-$C_4$)-alkylaminocarbonyle, carboxy, ($C_1$-$C_4$)-alkoxycarbonyle, phényle, phénoxy, benzyle et benzyloxy.
Dans le cas du phényle monosubstitué, le substituant peut être situé en position 2, 3 ou 4, et de préférence en position 3 ou 4. Dans le cas où le phényle est di-substitué, les substituants peuvent être en position 2, 3 ou 2, 4 ou 2, 5 ou 2, 6 ou 3, 4 ou 3, 5. De préférence, dans les phényles di-substitués, les deux substituants sont en position 3,4. Lorsque ce phényle est tri-substitué les positions sont les suivantes : 2, 3, 4 ou 2, 3, 5 ou 2, 3, 6 ou 2, 4, 5 ou 2, 4, 6 ou 3, 4, 5. De la même manière, les radicaux naphtyles ou d'autres radicaux aryles peuvent être substitués en toute position, par exemple le radical 1-naphtyle en position 2-, 3-, 4-, 5-, 6-, 7-, et 8 et le radical 2-naphtyle en position 1-, 3-, 4-, 5-, 6-, et 7.
Le groupement (C5-C14)-aryle peut représenter également un système aromatique monocyclique ou polycyclique dans lequel 1, 2, 3, ou 4 atomes de carbone du cycle sont remplacés par des hétéroatomes, en particulier identiques ou différents du groupe constitué de l'azote, l'oxygène et le soufre. Parmi les groupements (C5-C14)-aryle hétérocycliques (= (C5-C14)-hétéroaryle) on peut citer les groupements 2-pyridyle, 3-pyridyle, 4-pyridyle, pyrrolyle, furyle, thiényle, imidazolyle, pyrazolyle, oxazolyle, isoxazolyle, thiazolyle, isothiazolyle, tétrazolyle, pyridyle, pyrazinyle, pyrimidinyle, indolyle, isoindolyle, indazolyle, phtalazinyle, quinolyle, isoquinolyle, quinoxalinyle, quinazolinyle, cinnolinyle, -carbolinyle, ou encore des dérivés benzo-condensés, cyclopenta-, cyclohexa-, ou cyclohepta- condensés de ces radicaux. Le système hétérocyclique peut être substitué par les mêmes substituants cités plus haut pour le système carbocyclique.
Les atomes de carbone optiquement actifs contenus dans les composés de formule (I) peuvent indépendamment les uns des autres présenter la configuration R ou la configuration S.

Les composés de formule (I) peuvent être sous la forme d'énantiomères purs ou de diastéréoisomères purs ou sous la forme d'un mélange d'énantiomères, par exemple sous forme de racémates ou de mélanges de diastéréoisomères.

La présente invention a donc pour objet les énantiomères purs, les mélanges de ces énantiomères, les diastéréoisomères purs et les mélanges de ces diastéréoisomères.

L'invention comprend les mélanges de deux ou plus de deux stéréoisomères de formule (I) et tous les rapports de ces stéréoisomères au sein des dits mélanges.

Les composés de formule (I) peuvent le cas échéant être présent sous forme d'isomères E ou d'isomères Z. L'invention a donc pour objet les isomères E purs, les isomères Z purs et les mélanges E/Z selon un rapport quelconque.

L'invention comprend également toutes les formes tautomères des composés de formule (I), par exemple en ce qui concerne la forme représentée par la formule (I), avec G = $R_7R_8N-C(=NR_6)-NH-CO-$ on considère la forme dans laquelle l'acylguanidine est présente sous la forme d'un groupe $-CO-N=C(NR_6)-NR_7R_8$, et toutes les autres formes qui diffèrent par la position différente de l'atome d'hydrogène.

Les diastéréoisomères, incluant les isomères E/Z peuvent être séparés en isomères individuels, par exemple par chromatographie. Les racémates peuvent être séparés en deux énantiomères par des méthodes courantes telles que la chromatographie en phase chirale ou par des méthodes de résolution.

Les sels physiologiquement acceptables des composés de formule (I) sont en particulier des sels pharmaceutiquement utilisables ou non toxiques ou physiologiquement utilisables.

Lorsque les composés de formule (I) renferment un groupe acide tel que l'acide carboxylique, il s'agit par exemple des sels de métaux alcalins ou alcalino terreux tels que les sels de sodium, de potassium, de magnésium, de calcium, et également les sels formés avec des ions ammonium quaternaires physiologiquement acceptables et les sels d'addition avec les acides tel que l'ammoniac et des amines organiques physiologiquement acceptables telles que par exemple la triéthylamine, l'éthanolamine ou le tris-(2-hydroxyéthyle)amine.

Lorsque les composés de formule (I) contiennent un groupe basique, ils peuvent former un sel d'addition avec les acides par exemple avec les acides inorganiques tels que l'acide chlorhydrique, sulfurique, phosphorique ou avec les acides organiques carboxyliques tels que l'acide acétique, trifluoroacétique, citrique, benzoique, maléique, fumarique, tartarique, méthanesulfonique ou para toluène sulfonique.

Les composés de formule (I) qui comportent un groupe basique et un groupe acide, tel que par exemple guanidino et carboxylique, peuvent être présents sous forme de Zwiterions (bétaînes), qui sont également inclus dans la présente invention.

Le cas échéant un anion $Q^-$ physiologiquement acceptable peut être contenu dans les composés de formule (I) renfermant un groupement ammonium chargé. Il s'agit de préférence d'un anion monovalent ou d'un équivalent d'anion polyvalent d'un acide organique ou inorganique non toxique, physiologiquement acceptable et en particulier pharmaceutiquement acceptable, par exemple l'anion ou un équivalent d'anion d'un des acides cités plus haut utile pour la formation des sels d'addition. $Q^-$ peut être par exemple un des anions (ou équivalent d'anion) d'un groupe choisi parmi chlore, sulfate, phosphate, acétate, trifluoroacétate, citrate, benzoate, maléate, fumarate, tartrate, méthanesulfonate et para-toluènesulfonate.

Les sels des composés de formule (I) peuvent être obtenus par les méthodes ordinaires connues de l'homme du métier, par exemple en combinant un composé de formule (I) avec un acide organique ou inorganique ou une base dans un solvant ou un dispersant ou à partir d'un autre sel par échange de cation ou d'anion.

L'invention inclut également tous les sels des composés de formule (I) qui, à cause de leur faible acceptabilité physiologique, ne sont pas directement utilisables comme médicament, mais sont utilisables comme produits intermédiaires pour mettre en oeuvre des modifications chimiques ultérieures au niveau des composés de formule (I) ou comme produits de départ pour la préparation de sels physiologiquement acceptables.

La présente invention inclut également tous les solvates des composés de formule (I) par exemples les hydrates, les solvates formés avec les alcools, et tous les dérivés des composés de formule (I), par exemple les esters, prodrogues et autres dérivés physiologiquement acceptables, ainsi que les métabolites des composés de formule (I).

L'invention a plus particulièrement pour objet les composés de formule (I) dans laquelle G représente un groupement Het, Het-NHCO-, ou Het-NH-$CH_2$- dans lequel Het représente :

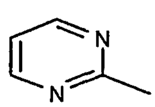
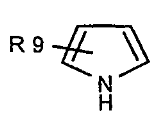
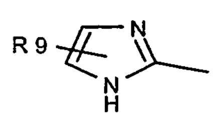

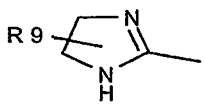
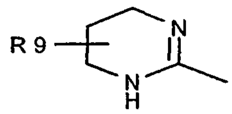
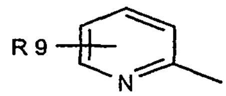

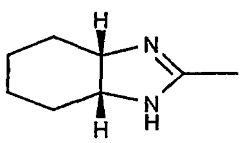
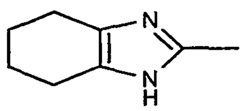
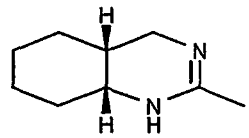

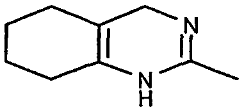
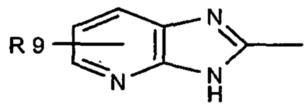
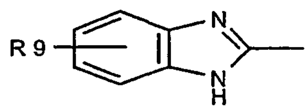

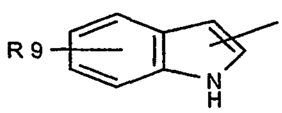
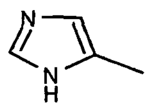
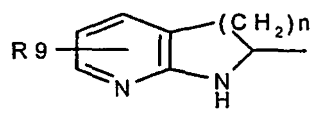

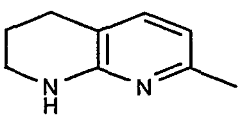
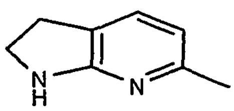
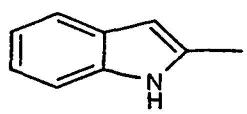
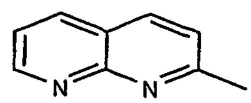

L'invention a plus particulièrement pour objet les composés de formule (I) tels que définis plus haut dans laquelle $R_3$ est :

- un hétérocycle choisi parmi

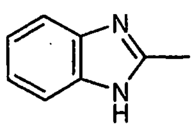
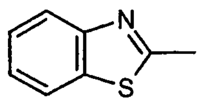
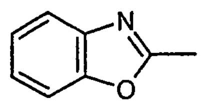

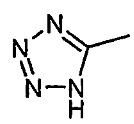
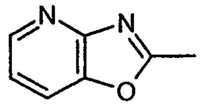
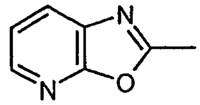

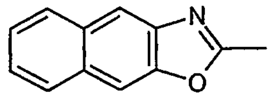

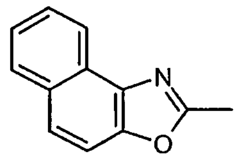

ainsi que leurs sels d'addition pharmaceutiquement acceptables.

L'invention a plus particulièrement pour objet les composés de formule (I) tels que définis plus haut dans laquelle $R_3$ est un groupement benzyloxycarbonyle, ainsi que leurs sels d'addition pharmaceutiquement acceptables.
L'invention a plus particulièrement pour objet les composés de formule (I) tels que définis plus haut dans laquelle $R_2$ est un hydrogène, un radical alkyle renfermant de 1 à 4 atomes de carbones, et tout particulièrement méthyle et éthyle, ou un atome de fluor ainsi que leurs sels d'addition pharmaceutiquement acceptables.
L'invention a plus particulièrement pour objet les composés de formule (I) tels que définis plus haut dans laquelle :

- G représente :

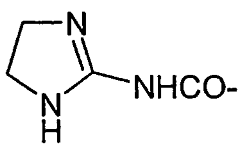

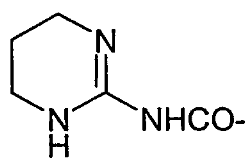

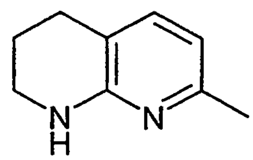

ainsi que leurs sels d'addition pharmaceutiquement acceptables.

L'invention a plus particulièrement pour objet les composés de formule (I) telle que définie plus haut dans laquelle

- G représente :

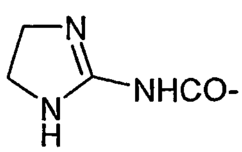

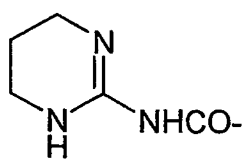

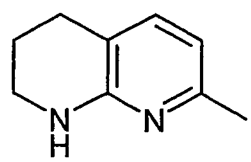

$R_1$ représente un atome d'hydrogène
$R_2$ représente un atome d'hydrogène, un atome de fluor, un radical méthyle ou un radical éthyle,
$R_3$ représente un groupement benzyloxycarbonyl
$R_4$ représente un groupement hydroxy ou ($C_1$-$C_4$)-alkyloxy,

Ainsi que les sels d'addition pharmaceutiquement acceptables.
L'invention a particulièrement pour objet les composés de formule (I) dont les noms suivent :

- 3-[[5-éthyl-6-[4-(1,2,3,4-tétrahydro-1,8-naphthyridin-7-yl)-1-pipéridinyl]-4-pyrimidinyl]amino]-N-[(phénylméthoxy) carbonyl]alanine

- 3-[[5-éthyl-6-[4-[(1,2,3,4,5,6-hexahydro-2-pyrimidinyl)iminocarbonyl]-1-pipéridinyl]-4-pyrimidinyl]amino]-N-[(phénylméthoxy)carbonyl] alanine

- 3-[[6-[4-(1,2,3,4-tetrahydro-1,8-naphthyridin-7-yl)-1-pipéridinyl]-5-méthyl-4-pyrimidinyl]amino]-N-[(phénylméthoxy) carbonyl]alanine

- 3-[[6-[4-[(1,2,3,4,5,6-hexahydro-2-pyrimidinyl)iminocarbonyl]-1-pipéridinyl]-5-méthyl-4-pyrimidinyl]amino]-N-[(phénylméthoxy)carbonyl]alanine
- 3-[[5-éthyl-6-[4-(1,2,3,4-tétrahydro-1,8-naphthyridin-7-yl)-1-pipéridinyl]-4-pyrimidinyl]amino]-N-[(phénylméthoxy) carbonyl]alaninate d'éthyle
- 3-[[6-[4-(1,2,3,4-tetrahydro-1,8-naphthyridin-7-yl)-1-pipéridinyl]-5-méthyl-4-pyrimidinyl]amino]-N-[(phénylméthoxy) carbonyl]alaninate d'isopropyle
- 3-[[5-éthyl-6-[4-(1,2,3,4-tétrahydro-1,8-naphthyridin-7-yl)-1-pipéridinyl]-4-pyrimidinyl]amino]-N-[(phénylméthoxy) carbonyl]alaninate de (1,1-diméthyléthyle)
- 3-[[5-méthyl-6-[4-(1,2,3,4-tétrahydro-1,8-naphthyridin-7-yl)-1-pipéridinyl]-4-pyrimidinyl]amino]-N-[(phénylméthoxy) carbonyl]alaninate de (1,1-diméthyléthyle)
- 3-[[5-éthyl-6-[4-(1,2,3,4-tétrahydro-1,8-naphthyridin-7-yl)-1-pipéridinyl]-4-pyrimidinyl]amino]-N-(1-naphtalènesulfonyl)alaninate de (1,1-diméthyléthyle)

sous la configuration (R) ou la configuration (S) ou leurs mélanges, ainsi que leurs sels d'addition.

La présente invention a également pour objet un procédé de préparation des composés de formule (I). Les composés peuvent généralement être préparés, par exemple au cours d'une synthèse convergente par couplage de deux ou plusieurs fragments qui peuvent être dérivés par rétrosynthèse des composés de formule (I). Afin d'éviter que les groupes fonctionnels puissent mener à des réactions indésirables ou secondaires au cours de chaque étape de synthèse, il peut être avantageux ou nécessaire au cours de la synthèse des composés de formule (I), d'introduire les groupes fonctionnels sous forme de précurseurs qui sont par la suite convertis en groupes fonctionnels désirés ou de bloquer temporairement ces groupes fonctionnels en mettant en oeuvre une stratégie de groupe protecteur appropriée à la synthèse qui est connue par l'homme de l'art (Greene, Wuts protective Group in Organic Synthesis, Wiley 1991).

Ainsi les composés de formule (I) peuvent être préparés, selon le schéma suivant :

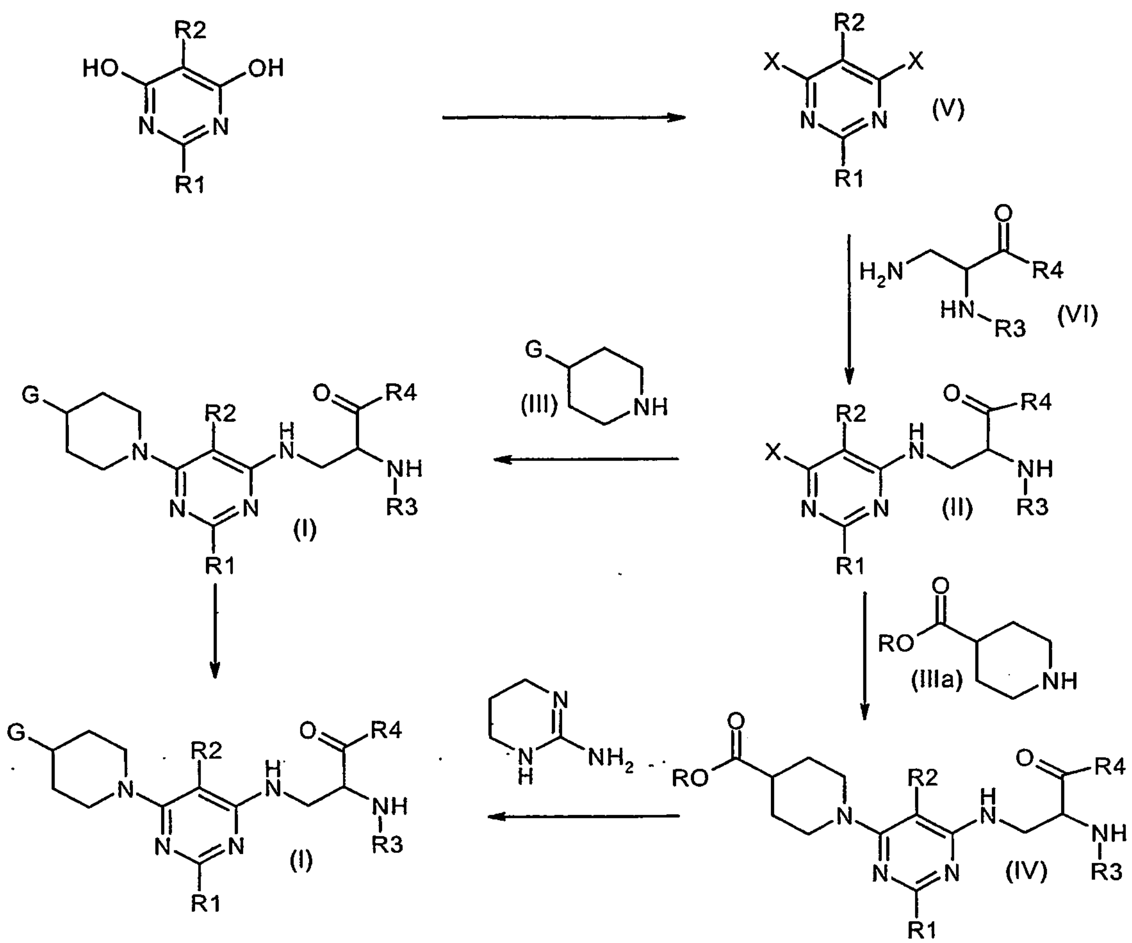

L'invention a donc pour objet un procédé de préparation des composés de formule (I) dans lequel : on fait réagir un composé de formule (II)

(II)

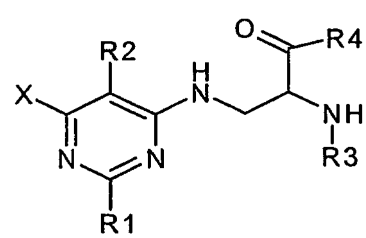

dans laquelle $R^1$, $R^2$, $R^3$ et $R^4$ sont tels que définis précédemment,

a) avec un composé de formule (III)

(III)

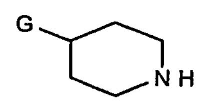

dans laquelle G est telle que définie précédemment en présence d'une base ou un réactif de couplage de métal de transition
b) puis le composé de formule (I) est soumis, éventuellement au clivage de la fonction $R^3$-NH- pour régénérer l'amine libre, suivi de la condensation de radicaux $R^3$ de structure $-CO_2-R^5$ ou $-SO_2-R^5$, et/ou le cas échéant à

l'hydrolyse et éventuellement à l'estérification ou à l'amidification et/ou à la salification.

A titre de variante, l'invention a également pour objet un procédé de préparation des composés de formule (I) dans lequel

a) on fait réagir un composé de formule (II) telle que définie précédemment avec un composé de formule (IIIa) :

(IIIa)

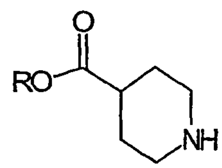

afin d'obtenir le composé intermédiaire de formule (IV) :

(IV)

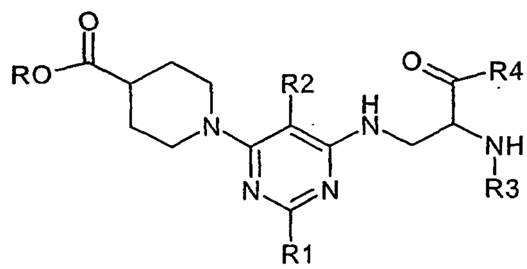

b) puis on fait réagir un composé de formule Het-$NH_2$, afin d'obtenir les composés de formule (I) avec G représentant un groupement Het-NHCO-,
c) puis le composé de formule (I) obtenu est soumis, éventuellement au clivage de la fonction $R^3$-NH- pour régénérer l'amine libre, suivi de la condensation de radicaux $R^3$ de structure -$CO_2$-$R^5$ ou -$SO_2$-$R^5$, et/ou le cas échéant, à l'estérification ou à l'amidification et/ou à la salification.

Le couplage d'un composé de formule (II) avec un dérivé de pipéridine de formule (III) ou (IIIa) peut s'effectuer en présence d'une base forte encombrée au reflux. En particulier on utilise de la diisopropyléthylamine. La réaction peut varier de 4 à 12 heures au reflux.
Au niveau du composé de formule (IV), lorsque OR est un hydroxyle, donc si une guanidine de formule (Het-$NH_2$) réagit avec un acide carboxylique de formule (IV), alors l'acide carboxylique est d'abord activé.
L'activation peut être effectuée par exemple avec le dicyclohexylcarbodiimide (DCCI) ou avec le O-((cyano (éthoxycarbonyl)-méthylène)amino)-1,1,3,3-tétraméthyluronium tétrafluoroborate (TOTU; Kônig et al, Proc. 21st Europ. Peptide Symp. 1990 (Eds Giralt, Andreu, Escom, Leiden 1991, p. 243) ou d'autres agents activants courants en synthèse peptidique.
Outre les guanidines libres de formule (Het-$NH_2$), les sels de guanidine peuvent également être mis en oeuvre dans la réaction avec les composés de formules (IV), les guanidines libres étant formés in situ ou par une étape séparée au moyen d'une base.
La réaction d'un dérivé activé d'acide carboxylique de formule (IV) avec la guanidine (ou dérivé) de formule (Het-$NH_2$) est de préférence effectuée d'une manière connue en soi dans un solvant organique protique ou aprotique, mais inerte. Dans ce cas on utilise des solvants tels que le méthanol, l'isopropanol, le tert-butanol, le diméthylformamide, le dichlorométhane ou le tétrahydrofurane à des températures allant de 0°C à la température de reflux de ces solvants, notamment lors de la réaction des esters méthyliques ou éthyliques (OR est un méthoxy ou un éthoxy) avec les guanidines.
Les réactions des composés de formule (IV) avec les guanidines libres sont avantageusement mises en oeuvre dans un solvant aprotique inerte tel que le diméthylformamide, le dichlorométhane, le tétrahydrofurane, le diméthoxyéthane, ou le dioxane, le cas échéant en additionnant une base telle que par exemple le tert-butoxide de potassium, le méthoxide de sodium ou une base organique telle que la N-méthylmorpholine. Cependant, l'eau peut également être utilisée comme solvant dans les réactions des composés de formule (IV) avec les guanidines de formule (Het-$NH_2$), par exemple en utilisant une base telle que l'hydroxyde de sodium.

Le mélange réactionnel est ensuite traité et si désiré le produit réactionnel est purifié selon les méthodes connues de l'homme du métier.

Les groupes protecteurs éventuellement présents dans les composés de formule (I) obtenus à partir des composés de formule (IV) avec les amines ou guanidines de formule Het-$NH_2$ ou à partir des composés de formule (II) avec les composés de formule (III) sont ensuite éliminés par des procédés classiques; par exemple, les groupes tert-butyl ester sont convertis en acide carboxylique par traitement avec de l'acide trifluoroacétique, les groupes benzyles sont éliminés par hydrogénation ou encore les groupes fluorénylméthoxycarbonyle sont éliminés en présence d'amine secondaire et d'autres réactions sont mises en oeuvre par des procédés standards, par exemple par des réactions d'acylation.

Les réactions d'hydrolyse afin d'obtenir un dérivé d'acide ($COR^4$ = $CO_2H$), d'estérification afin d'obtenir un ester ou une prodrogue (particulièrement $COR^4$ = alkyloxycarbonyle ou aryloxycarbonyle à partir de l'acide correspondant) ou d'amidification ($COR^4$ = aminocarbonyle mono ou disubstitué à partir de l'acide correspondant) s'effectuent selon les méthodes usuelles connues de l'homme du métier.

Notamment l'hydrolyse s'effectue en milieu acide, en présence d'acide trifluoracétique par exemple, dans un solvant organique halogéné comme le dichlorométhane par exemple.

Si nécessaire, la conversion en des sels physiologiquement acceptables s'effectue par des procédés connus de l'homme du métier.

Les composés de départs de formule (II) peuvent être préparés selon des procédés décrits dans la littérature ou encore sont accessibles par analogie. La préparation des composés de formule (II) est illustrée dans le schéma décrit plus haut, étant entendu que la présente invention n'est pas restreinte à ces synthèses ou ces produits de départ. Il n'y a pas de difficulté majeure pour l'homme du métier de prévoir des modifications des synthèses décrites dans notre demande pour la préparation d'autres composés de formule (II) selon l'invention.

L'invention a donc pour objet le procédé de préparation des composés de formule (II) caractérisé en ce qu'on fait réagir un composé de formule (V) :

(V) 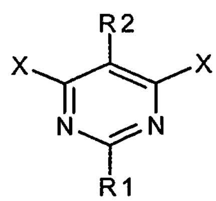

dans laquelle $R^1$ et $R^2$ sont tels que définis précédemment, et X représente un halogène, de préférence chlore avec un composé de formule (VI) :

(VI) 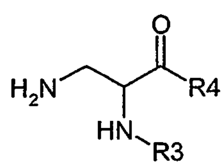

dans laquelle $R^3$ et $R^4$ sont tels que définis précédemment, en présence d'une base forte.

En général, on utilise une base forte encombrée telle que la diisopropyléthylamine dans des conditions réactionnelles connues de l'homme du métier dans la mise en oeuvre de substitution nucléophile. De préférence on opère en présence de diméthylformamide et à la température de reflux. Par ailleurs le groupement COR4 représentera de préférence un groupement ester encombré tel que le groupement tertbutyloxycarbonyle.

Selon une autre variante de l'invention, les produits de formule (I) peuvent être également préparés selon le schéma suivant :

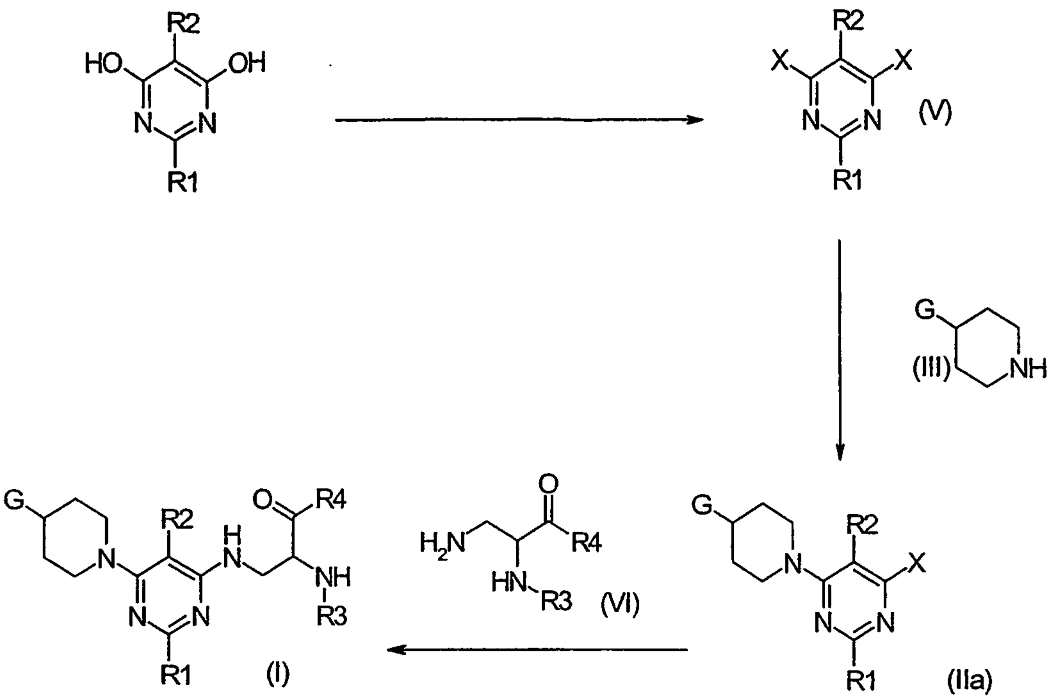

Selon l'invention, le procédé de préparation des produits de formule (I) consiste dans

a) la réaction d'un produit de formule (IIa)

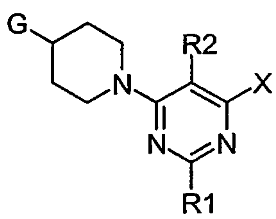

(IIa)

dans laquelle $R^1$, $R^2$, G et X sont tels que définis précédemment, avec un produit de formule (VI)

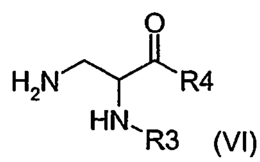

(VI)

dans laquelle $R^3$ et $R^4$ sont définis comme précédemment, soit en présence d'une base forte, soit par catalyse au palladium,
b) puis le produit de formule (I) est soumis, éventuellement au clivage de la fonction $R^3$-NH- pour régénérer l'amine libre, suivi de la condensation de radicaux $R^3$ de structure $-CO_2-R^5$ ou $-SO_2-R^5$, et/ou le cas échéant à l'hydrolyse et éventuellement à l'estérification ou à l'amidification et/ou à la salification.

La réaction de la pyrimidine de formule (IIa) avec l'amine de formule générale (VI) s'effectue dans des conditions analogues aux conditions décrites précédemment pour la réaction d'une pyrimidine de formule (V) avec l'amine de formule générale (VI). Notamment, il est possible d'opérer dans la diisopropyléthylamine dans un solvant organique tel qu'un amide (diméthylacétamide, diméthylformamide par exemple), à une température comprise entre 90°C et la température de reflux du mélange réactionnel. Il est également possible d'opérer par catalyse au palladium (par exemple tris(dibenzylidèneacétone) dipalladium) en présence de fluorure de césium, à la température de reflux du mélange réactionnel. Il est entendu que les fonctions pouvant interférer avec la réaction sont protégées. La protection et la libération de ces fonctions s'effectue selon les méthodes habituelles qui n'altèrent pas le reste de la molécule.

La condensation de radicaux $R^3$ de structure $-CO_2-R^5$ ou $-SO_2-R^5$ sur l'amine libre, et l'hydrolyse s'effectuent comme décrit précédemment.

Les dérivés de pyrimidine de formule (IIa) peuvent être préparés par action d'un produit de formule (III)

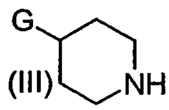

dans laquelle G est défini comme précédemment, sur un dérivé dihalogéné de la pyrimidine de formule (V)

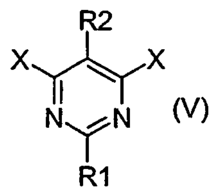

dans laquelle $R^1$, $R^2$ et X sont définis comme précédemment.

La réaction s'effectue avantageusement en présence d'une base forte encombrée, à la température de reflux du mélange réactionnel. On opère dans les conditions décrites ci-après dans les exemples et notamment en présence d'une amine encombrée comme la diisopropyléthylamine, dans un amide comme le diméthylacétamide par exemple. Il est entendu que les fonctions pouvant interférer avec la réaction sont protégées. La protection et la libération de ces fonctions s'effectue selon les méthodes habituelles qui n'altèrent pas le reste de la molécule.

Les composés de formule (I) sont des composés ayant une activité pharmacologique et peuvent ainsi être utilisés à titre de médicaments notamment dans le traitement ou la prévention des maladies de l'os, des maladies tumorales ainsi que des désordres cardiovasculaires.

La présente invention a donc pour objet les composés de formule (I) et/ou leurs sels physiologiquement acceptables à titre de médicament.

Les composés de formule (I) ainsi que leurs sels physiologiquement acceptables et leurs prodrugs peuvent être administrés aux animaux, de préférence aux mammifères et en particulier aux êtres humains comme médicaments à titre thérapeutique ou prophylactique.

Ils peuvent être administrés tels quels ou en mélange avec un ou plusieurs autres composés de formule (I) ou encore sous la forme d'une préparation pharmaceutique (composition pharmaceutique) qui permet une administration entérale ou parentérale et qui renferme à titre de composé actif une dose efficace d'au moins un composé de formule (I) et/ou ses sels physiologiquement acceptables ainsi que des supports et/ou additifs courants et pharmaceutiquement inertes.

Les compositions pharmaceutiques selon l'invention permettent une administration entérale ou parentérale qui renferment à titre de composé actif une dose efficace d'au moins un composé de formule (I) et/ou ses sels physiologiquement acceptables ainsi qu'un ou plusieurs supports pharmaceutiquement inertes, et/ou un ou plusieurs additifs usuels.

L'invention a donc pour objet les compositions pharmaceutiques renfermant un composé de formule (I) tel que défini précédemment ainsi qu'un ou plusieurs excipients.

Les médicaments peuvent être administrés oralement, par exemple sous forme de pilule, de comprimés, de comprimés enrobés, de pelliculés, de granules, de gélules et capsules molles, de solutions, de sirops, d'émulsion, de suspension ou de mélange d'aérosol.

L'administration peut cependant être effectuée par voie rectale, par exemple sous forme de suppositoire, par voie parentérale, par exemple sous forme de solutions injectables ou d'infusions, de microcapsules ou d'implants, par voie percutanée, par exemple sous la forme de pommade, de solutions, de pigments ou de colorants, par voie transdermique sous forme de patchs ou par d'autres voies telles que sous la forme d'aérosol ou de spray nasal.

Les compositions pharmaceutiques selon l'invention sont préparées selon des méthodes connues en soi, des supports organiques ou inorganiques, pharmaceutiquement inertes étant additionnés aux composés de formule (I) et/ou

leurs sels physiologiquement acceptables.

Pour la production de pilule, de comprimés, de comprimés enrobés et de capsule en gélatine dure, il est possible d'utiliser par exemple, du lactose, de l'amidon de mais ou ses dérivés, du talc, de l'acide stéarique ou ses sels, etc. Les supports convenables pour des capsules en gélatine molle ou pour les suppositoires sont par exemple les graisses, les cires les polyols semi-solides ou liquides, les huiles naturelles ou modifiées etc. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc. Les supports convenables pour les microcapsules ou les implants sont par exemple les copolymères d'acide glyoxylique et d'acide lactique. Les préparations pharmaceutiques contiennent normalement de 0,5 % à 90 % en poids de composés de formule (I) et/ou leurs sels physiologiquement acceptables.

En plus des principes actifs et des supports, les préparations pharmaceutiques peuvent contenir des additifs tels que par exemple des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants.

Elles peuvent également contenir deux ou plusieurs composés de formule (I) et/ou leurs sels physiologiquement acceptables. En outre, en plus d'au moins un ou plusieurs composés de formule (I) et/ou leurs sels physiologiquement acceptables, ils peuvent contenir au moins un ou plusieurs autres principes actifs utilisables à titre thérapeutique ou prophylactique.

Les préparations pharmaceutiques (compositions pharmaceutiques) renferment normalement de 0,2 à 500 mg, et de préférence de 1 à 200 mg de composé de formule (I) et/ou leurs sels physiologiquement acceptables et/ou leurs prodrugs.

Les composés de formule (I) sont tout particulièrement des antagonistes des récepteurs de la Vitronectine et sont capables ainsi par exemple d'inhiber l'adhésion des ostéoclastes sur la surface de l'os et ainsi la résorption osseuse par les ostéoclastes.

L'action des composés de formule (I) peut être démontrée par exemple dans un test dans lequel l'inhibition de la liaison de la vitronectine aux cellules qui contiennent le récepteur de la vitronectine est déterminée. Des précisions sur ce test sont données plus bas. Comme antagonistes du récepteur de la vitronectine, les composés de formule (I) et leurs sels physiologiquement acceptables conviennent en général pour le traitement ou la prévention de maladies liées aux interactions entre les récepteurs de la vitronectine et leurs ligands, dans les processus d'interaction cellule-cellule ou cellule-matrice ou qui peuvent être influencés par l'inhibition des interactions de ce type, pour soulager ou guérir lorsqu'une inhibition des interactions de ce type est désirée. Comme on l'a expliqué au début, une telle interaction joue un rôle important dans la résorption osseuse, dans l'angiogénèse ou dans la prolifération des cellules du muscle vasculaire lisse.

Les maladies de l'os dont le traitement ou la prévention nécessitent l'emploi des composés de formule (I), sont notamment l'ostéoporose, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, et la maladie de Paget. En outre les composés de formule (I) peuvent être utilisés pour soulager, empêcher ou traiter les désordres de l'os qui sont causés par les traitements, par les glucocorticoïdes, les thérapies liées à la prise de stéroïdes ou de corticostéroïdes ou par les déficiences d'hormones sexuelles mâles ou femelles.

Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes. A côté de cette utilisation comme inhibiteur de la résorption osseuse médiée via les ostéoclastes, les composés de formule (I) et leurs sels physiologiquement acceptables sont utilisés comme inhibiteurs de la croissance tumorale ou des métastases cancéreuses, dans le traitement des désordres inflammatoires, pour le traitement ou la prévention des désordres cardiovasculaires, telles que l'artériosclérose ou la resténose, ou le traitement ou la prévention de néphropathie ou rétinopathie tel que par exemple la rétinopathie diabétique.

Les composés selon l'invention peuvent posséder également une activité vis-à-vis d'autres intégrines qui interagissent avec leur ligand via la séquence tripeptidique RGD ($\alpha_v\beta_1$, $\alpha_v\beta_5$, $\alpha_{IIb}\beta_3$), leur conférant des propriétés pharmacologiques utilisables pour traiter les pathologies associées à ces récepteurs.

Cette activité vis-à-vis des intégrines rend ainsi les composés de formule (I) utilisables dans la prévention ou le traitement de nombreuses maladies telles que celles mentionnées plus haut ou dans la revue de Dermot Cox DN§P 8(4) mai 1995, 197-205 dont le contenu est intégré dans la présente demande.

La présente invention a donc plus particulièrement pour objet un composé de formule (I) et/ou ses sels physiologiquement acceptables tel que défini plus haut à titre de médicament ayant une activité antagoniste du récepteur de la vitronectine.

La présente invention a donc plus particulièrement pour objet un composé de formule (I) et/ou ses sels physiologiquement acceptables et/ou ses prodrugs tel que défini plus haut à titre de médicament ayant une activité inhibitrice

de la résorption osseuse ou pour le traitement ou la prévention de l'ostéoporose.

La présente invention a donc plus particulièrement pour objet un composé de formule (I) et/ou ses sels physiologiquement acceptables tel que défini plus haut à titre de médicament ayant une activité inhibitrice de la croissance tumorale ou des métastases cancéreuses.

La présente invention a donc plus particulièrement pour objet un composé de formule (I) et/ou ses sels physiologiquement acceptables tel que défini plus haut à titre de médicament ayant une activité anti-inflammatoire ou pour le traitement ou la prévention des désordres cardiovasculaires, la resténose, l'artériosclérose, les néphropathies ou rétinopathies.

La présente invention a également pour objet l'utilisation des composés de formule (I) et/ou leurs sels physiologiquement acceptables tels que définis plus haut pour la préparation de médicaments destinés à la prévention ou au traitement de l'ostéoporose.

La présente invention a également pour objet l'utilisation des composés de formule (I) et/ou leurs sels physiologiquement acceptables tels que définis plus haut pour la préparation de médicaments destinés à inhiber la croissance tumorale ou les métastases cancéreuses.

La présente invention a également pour objet l'utilisation des composés de formule (I) et/ou leurs sels physiologiquement acceptables tels que définis plus haut pour la préparation de médicaments destinés à la prévention ou au traitement des désordres cardiovasculaires, de la resténose, de l'artériosclérose, des néphropathies ou des rétinopathies.

Lorsqu'on utilise les composés de formule (I), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.

Dans le cas d'une administration par voie orale, la dose quotidienne varie en général de 0,01 à 100 mg/kg et de préférence de 0,1 à 50 mg/kg, en particulier de 0,1 à 5 mg/kg. Par exemple pour un adulte de 75 kg on pourra envisager une dose quotidienne variant de 0,3 à 0,5 mg/kg.

Dans le cas d'une administration par voie intraveineuse, la dose quotidienne varie approximativement de 0,01 à 100 mg/kg et de préférence de 0,05 à 10 mg/kg.

La dose quotidienne peut être divisée, en particulier dans le cas de l'administration de grande quantité de principe actif, en plusieurs, par exemple 2, 3 ou 4 parts. Le cas échéant, en fonction du comportement individuel, il peut être nécessaire d'administrer les différentes doses de manière croissante ou décroissante. Mis à part l'utilisation des composés de formule (I) comme médicaments, on peut également envisager leur utilisation comme véhicule ou support de composés actifs afin de transporter ces composés actifs de manière spécifique vers un site d'action (Drug targeting, voir Targeted Drug Delivery, R. C. Juliano, Handbook of Experimental Pharmacology, Vol 100, Ed. Born, G. V. R. et al, Springer Verlag). Les composés actifs qui peuvent être transportés sont en particulier ceux utilisés pour le traitement ou la prévention des maladies citées plus haut.

Les composés de formule (I) et leurs sels peuvent également être employés comme agent de diagnostique, par exemple pour des méthodes in vitro ou comme auxiliaire dans des études biochimiques dans lesquelles on désire bloquer le récepteur de la vitronectine ou influencer les interactions cellules-cellules ou cellules-matrices. Ils peuvent en outre être utilisés comme intermédiaire pour la préparation d'autres composés, en particulier d'autres agents actifs, qui sont accessibles à partir des composés de formule (I), par exemple par modification ou introduction de radicaux ou de groupes fonctionnels.

## Exemples

Les produits ont été identifiés par spectre de masse (MS), infrarouge (IR) et/ou spectre RMN. Les composés, qui ont été purifiés par chromatographie en utilisant un éluant qui contient par exemple de l'acide acétique ou trifluoroacétique, et qui ensuite sont séchés ou dans lesquels lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.

## Abréviations/noms chimiques éventuellement employés :

AcOEt : acétate d'éthyle; EDCI : chlorhydrate de 1-(3-diméthylaminopropyl)-3-éthyl-carbodiimide; DMF : diméthylformamide; DIPEA : Diisopropyléthylamine; MeOH : méthanol; TEA : triéthylamine; TFA : acide trifluoroacétique; THF : tétrahydrofurane; MCPBA : acide métachloroperoxybenzoique; DBU : 1,8-diazabicyclo[5.4.0] undec-7-ene; APTS : acide paratoluènesulfonique; DPPA : diphénylphosphorylazide; DMSO : diméthylsulfoxyde; Pd/C Palladium sur charbon; Boc : terbutoxycarbonyl; CBz : benzyloxycarbonyl; DCC 1,3-dicyclohexylcarbodiimide; TMSBr : bromotriméthylsilane; TMSI : iodure de triméthylsilane.

IR : Infrarouge; RMN : Résonnance Magnétique Nucléaire; SM : Spectre de Masse; ESP : Electrospray mode positif; ep. : Épaulement; F : fort; s : singulet; d : doublet; t : triplet; q : quadruplet; quint : quintuplet; 1 : large; m : multiplet ou massif; J : constante de couplage; Rf : facteur de rétention (chromatographie).

Il est entendu que dans les exemples qui suivent les produits des exemples 1 à 5 sont de forme racémique, les produits des exemples 6 à 9, 11, et 13 à 41 ainsi que leurs esters précurseurs sont de forme (S) sur le centre asymétrique de la 3-amino alanine et les exemples 10 et 12 et le cas échéant leurs esters précurseurs sont de forme (R) sur le centre asymétrique de la 3-amino alanine.

**Préparation 1**

Synthèse de la 4,6-dichloro-5-éthyl-pyrimidine (Composé de formule (V).

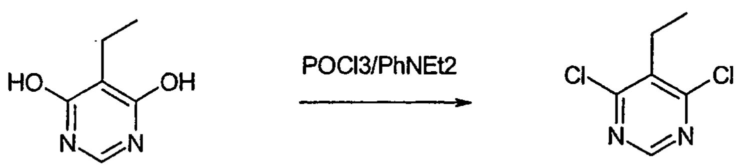


Un mélange de 5 g (35,7 mmoles) de 5-éthyl-4,6-dihydroxypyrimidine (commercialisée par Aldrich) dans 30 ml d'oxychlorure de phosphore est porté au reflux durant 1 heure. Après retour à température ambiante, on ajoute goutte à goutte un mélange de 4 ml de N,N-diéthylaniline dans 10 ml d'oxychlorure de phosphore et porte le tout au reflux pendant 4 heures. Après retour à température ambiante, le milieu réactionnel est versé sur un mélange de glace et d'eau. On extrait à l'acétate d'éthyle, lave les phases organiques avec de l'acide chlorhydrique 2N, sèche sur sulfate de magnésium et évapore à sec sous vide. On obtient 6 g (Rdt = 95 %) d'une huile marron de produit attendu utilisée telle quelle pour la suite.

**CCM :** Rf = 0,5 (silicagel, éluant : dichlorométhane-méthanol 90-10).

**Exemple 1**

**3-[[5-éthyl-6-[4-(1,2,3,4-tétrahydro-1,8-naphthyridin-7-yl)-1-pipéridinyl]-4-pyrimidinyl]amino]-N-[(phénylméthoxy) carbonyl]alanine, bis(trifluoroacetate)**

**Etape a)**

Synthèse du 3-[(6-chloro-5-éthyl-4-pyrimidinyl)amino]-N-[(phénylméthoxy)carbonyl] alaninate de (1,1-diméthyléthyle).

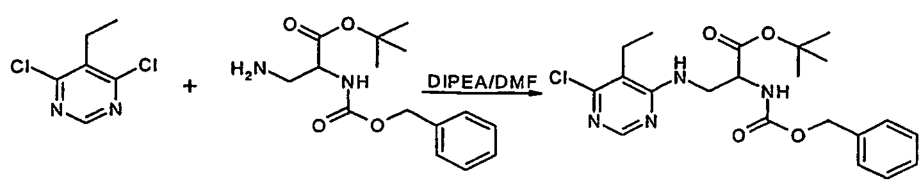


A un mélange de 3,8 g (21 mmoles) de 4,6-dichloro-5-éthylpyrimidine et de 4,4 g (15 mmoles) de 3-amino-N-[(phénylméthoxy)carbonyl]alaninate de (1,1-diméthyléthyle) (préparé selon J. Med. Chem.(2001), 44(8), 1158-1176) dans 50 ml de diméthylformamide, on additionne 10 ml de diisopropyléthylamine puis chauffe ce mélange à 120°C pendant 6 heures. On élimine alors la diméthylformamide sous vide et reprend le résidu par un mélange d'acétate d'éthyle, d'eau et une solution saturée de bicarbonate de sodium. La phase organique est décantée puis séchée sur sulfate de magnésium et le solvant éliminé par évaporation sous vide. Le résidu est chromatographié sur silicagel en éluant avec un gradient d'heptane 100 % jusqu'à heptane-acétate d'éthyle 50-50. On obtient 4,7 g (Rdt = 70 %) de produit attendu sous forme d'une huile.

**CCM :** Rf = 0,2 (silicagel, éluant : heptane-acétate d'éthyle 70-30)

**IR (CHCl3) :** 3411 (NH); 1718 (C=O); 1571; 1498 cm-1 (Hétérocycle+Aromatique+Amide)
**1H-RMN (DMSO-d6) :** δ 1,02 (t, 3H, $CH_2$-$\underline{CH_3}$); 1,30 (s, 9H, tBu); 2,57 (q, 2H, $\underline{CH_2}$-$CH_3$); 3,74 (m, 2H, NH-$\underline{CH_2}$-CH-NH); 4,29 (ql, 1H, NH-$CH_2$-$\underline{CH}$-NH); 5,00 et 5,06 (syst. AB, 2H, O-$\underline{CH_2}$-Ph); 7,22 (tl, 1H, $\underline{NH}$-$CH_2$-CH-NH); 7,34 (m, 5H, Ph); 7,64 (dl, 1H, NH-$CH_2$-CH-$\underline{NH}$); 8,16 ppm (s, 1H, N=$\underline{CH}$-N)
**HPLC/SM :** (tr = 26 min) : 435 (MH+); 379 (MH-tBu+).

**Etape b)**

Synthèsedu3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

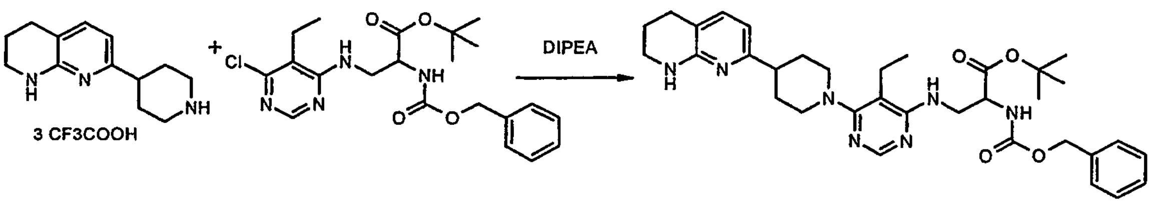


Un mélange de 1,68 g (3 mmoles) de 1,2,3,4-tétrahydro-7-(4-pipéridinyl)-1,8-naphthyridine, tris(trifluoroacetate) (préparé selon les brevets EP1065207 ou WO 0078317) et de 7,5 g (14,1 mmoles équivalents base) d'aminométhyl polystyrène (Polymer Labs 1,88 mmoles/g) dans 200 ml de solution de dichlorométhane-méthanol 50-50 est agité à température ambiante durant 1 heure. Le mélange est filtré, la résine lavée avec du méthanol et du dichlorométhane et le filtrat concentré à sec sous vide donnant 630 mg d'amine libre. On rajoute à ce résidu 651 mg (1,5 mmoles) de 3-[(6-chloro-5-ethyl-4-pyrimidinyl)amino]-N-[(phénylméthoxy) carbonyl]alaninate de (1,1-diméthyléthyle) et 2 ml de diisopropyléthylamine et porte au reflux durant 8 heures. Le milieu réactionnel est évaporé à sec sous vide et le résidu est repris par de l'acétate d'éthyle, de l'eau et une solution saturée de bicarbonate de sodium. La phase organique est séparée, séchée sur sulfate de magnésium et le solvant évaporé sous vide. Le résidu est chromatographié sur silicagel en éluant successivement avec du dichlorométhane pur, un mélange de dichlorométhane-méthanol 90-10 puis un mélange de dichlorométhane-méthanol-eau-acide acétique 90-10-1-1. On obtient 115 mg (Rdt = 12 %) de produit attendu sous forme d'huile. On récupère également 280 mg (43 %) de composé chloré de départ.
**CCM :** Rf = 0,55 (alumine, éluant : heptane-acétate d'éthyle 50-50).
**IR (CHCl3) :** 3438 (NH 1717 (C=O); 1583; 1500 cm-1 (Hétérocycle+Aromatique+Amide).
**1H-RMN (DMSO-d6) :** δ 1,06 (t, 3H, $CH_2$-$\underline{CH_3}$); 1,31 (s, 9H, tBu); 1,77 (m, 2H, $CH_2$-$\underline{CH_2}$-$CH_2$-NH); 1,78 (m, 4H, $\underline{CH_2}$-CH-$\underline{CH_2}$); 2,43 (q, 2H, $\underline{CH_2}$-$CH_3$); 2,51 (m, 1H, $CH_2$-$\underline{CH}$-$CH_2$); 2,61 (m, 2H, $\underline{CH_2}$-$CH_2$-$CH_2$-NH); 2,82 et 3,44 (2m, 4H, $\underline{CH_2}$-$CH_2$-N-$CH_2$-$\underline{CH_2}$); 3,23 (m, 2H, $CH_2$-$CH_2$-$\underline{CH_2}$-NH); 3,72 (m, 2H, NH-$\underline{CH_2}$-CH-NH); 4,23 (m, 1H, NH-$CH_2$-$\underline{CH}$-NH); 5,03 (dl, 2H, $\underline{CH_2}$-Ph); 6,22 (m, 1H, $CH_2$-$CH_2$-$CH_2$-$\underline{NH}$); 6,30 et 7,05 (2m, 2H, H naphthyridine); 6,35 (m, 1H, $\underline{NH}$-$CH_2$-CH-NH); 7,34 (m, 5H, Ph); 7,60 (m, 1H, NH-$CH_2$-CH-$\underline{NH}$); 8,11 ppm (N=$\underline{CH}$-N).
**HPLC/SM :** (tr = 14 min) : 616 (MH+).

**Etape c)**

Synthèse de la 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

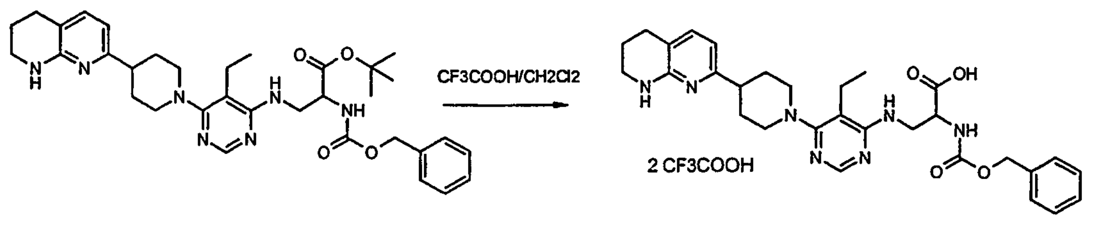

On agite 110 mg (0,18 mmoles) de 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle) dans 5 ml de dichlorométhane avec 1 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le milieu réactionnel à sec sous vide. Le résidu est solubilisé dans le minimum de dichlorométhane avec un peu de méthanol puis versé sur de l'éther diisopropylique. Le précipité est filtré. On obtient 108 mg (Rdt = 76 %) de produit attendu sous forme d'un solide amorphe.
**CCM :** Rf = 0,33 (silicagel, éluant : dichlorométhane-méthanol-eau-acide acétique 90-10-1-1)
**IR ($CHCl_3$)** : 1677 (C=O); 1626; 1586; 1500 cm-1 (Hétérocycle+Aromatique+Amide).
**1H-RMN (DMSO-d6) :** δ 1,07 (t, 3H, CH2-CH3); 1,77 et 1,94 (2m, 4H, N-CH2-CH2-CH-); 1,84 (m, 2H, NH-CH2-CH2-CH2-); 2,45 (q, 2H, CH2-CH3); 2,75 (t, 2H, NH-CH2-CH2-CH2-); 2,85 (t, 1H, N-CH2-CH2-CH-); 2,98 et 3,53 (2m, 4H, N-CH2-CH2-CH-); 3,43 (m, 2H, N-CH2-CH2-CH2-); 3,61 et 3,85 (2m, 2H, NH-CH2-CH-NH); 4,32 (q, 1H, NH-CH2-CH-NH); 4,99 et 5,04 (syst AB, 2H, O-CH2-Ph); 6,67 (d, 1H, H naphthyridine); 7,22 (sl, 1H, NH-CH2-CH-NH); 7,35 (m, 5H, Ph); 7,60 (d, 1H, NH-CH2-CH-NH); 7,64 (d, 1H, H naphthyridine), 8,26 (s, 1H, N=CH-N); 8,29 ppm (sl, 1H, NH-CH2-CH2-CH2-).
**HPLC/SM :** (tr = 8,0 min) : 560 (MH+); 427 (MH-Naphthyridine+); 280 (M+2H++)
**Microanalyse :**

| | |
|---|---|
| Théorie | C=51,84 %; H=4,99 %; N=12,45 %; |
| Trouvé | C=52,0 %; H=5,2 %; N=12,4 %; |

**Exemple 2**

**3-[[5-éthyl-6-[4-[(1,2,3,4,5,6-hexahydro-2-pyrimidinyl)iminocarbonyl]-1-pipéridinyl]-4-pyrimidinyl]amino]-N-[(phénylméthoxy)carbonyl] alanine.**

**Etape a)**

Synthèse du 3-[[5-éthyl-6-[4-(méthoxycarbonyl)-1-pipéridinyl]-4-pyrimidinyl]amino]-N-[(phénylméthoxy)carbonyl]alaninate de (1,1-diméthyléthyle).

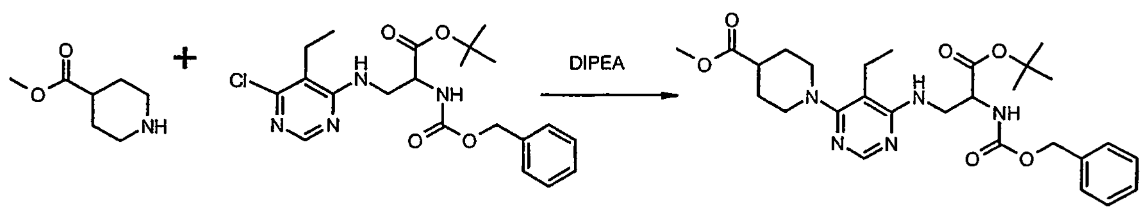


Un mélange de 1,2 g (2,8 mmoles) de 3-[(6-chloro-5-éthyl-4-pyrimidinyl)amino]-N-[(phényl méthoxy)carbonyl] alaninate de (1,1-diméthyléthyle), de 5 ml de 4-pipéridinylcarboxylate de méthyle et de 1 ml d'isopropyléthylamine est chauffé entre 110 et 120°C pendant 4 heures. Après refroidissement à température ambiante, le milieu réactionnel est repris par de l'acétate d'éthyle, de l'eau et une solution saturée de bicarbonate de sodium. La phase organique est séparée, séchée sur sulfate de magnésium puis concentrée à sec sous vide. Le résidu est chromatographié sur silicagel avec un gradient d'heptane pur à l'acétate d'éthyle pur. On obtient 260 mg (Rdt = 17 %) de produit attendu sous forme d'une huile.
**CCM :** Rf = 0,36 (silicagel, éluant : heptane-acétate d'éthyle 50-50).
**IR ($CHCl_3$) :** 3422 (NH); 1725 (C=O); 1582; 1499 cm-1 (Hétérocycle+Aromatique+Amide)
**1H-RMN (DMSO-d6) :** δ 1,05 (t, 3H, CH2-CH3); 1,30 (s, 9H, tBu); 1,67 et 1,88 (2m, 4H, N-CH2-CH2-CH); 2,40 (q, 2H, CH2-CH3); 2,50 (m, 1H, N-CH2-CH2-CH); 2,81 et 3,33 (2m, 4H, N-CH2-CH2-CH); 3,62 (s, 3H, O-CH3); 3,71 (m, 2H, NH-CH2-CH-NH); 4,22 (q, 1H, NH-CH2-CH-NH); 5,02 (s, 2H, O-CH2-Ph); 6,36 (t, 1H, NH-CH2-CH-NH); 7,34 (m, 5H, Ph); 7,64 (d, 1H, NH-CH2-CH-NH); 8,10 ppm (s, 1H, N=CH-N).
**HPLC/SM** : (tr=12min) : 564 (MNa+); 542 (MH+); 486 (MH-tBu+).

**Etape b)**

Synthèse de la 3-[[5-éthyl-6-[4-[(1,2,3,4,5,6-hexahydro-2-pyrimidinyl)iminocarbonyl]-1-pipéridinyl]-4-pyrimidinyl] amino]-N-[(phénylméthoxy)carbonyl] alanine.

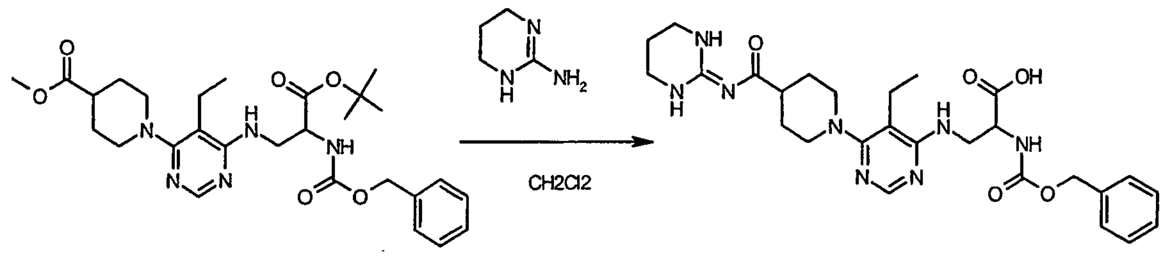


Un mélange de 250 mg (0,46 mmoles) de 3-[[5-éthyl-6-[4-(méthoxycarbonyl)-1-pipéridinyl]-4-pyrimidinyl]amino]-N-[(phénylméthoxy)carbonyl]alaninate de (1,1-diméthyléthyle) et de 200 mg (2,0 mmoles) de 2-amino-1,4,5,6-tétrahydropyrimidine (préparée selon R. F. Evans J. Chem. Soc. 1964, 2450-2455) dans 15 ml de dichlorométhane est agitée pendant 24 heures à température ambiante. Après évaporation à sec sous vide, le résidu est chromatographié sur silicagel en éluant avec un gradient de dichlorométhane pur jusqu'au mélange dichlorométhane-méthanol-eau-acide acétique 85-15-2-2. Après évaporation du solvant des fractions intéressantes, le résidu est repris dans le minimum d'un mélange de méthanoldichlorométhane 50-50 et l'acide précipité par l'addition d'éther éthylique. Après filtration et séchage sous vide on obtient 18 mg (Rdt = 7 %) de produit attendu sous forme d'un solide amorphe.

**CCM :** Rf = 0,5 (silicagel, éluant : dichlorométhane-méthanol-acide acétique 70-20-10)

**IR ($CHCl_3$)** : 3287 (OH/NH); 1700; 1600 (C=O); 1582; 1499 cm-1 (Hétérocycle+ Aromatique+Amide).

**1H-RMN (DMSO-d6) :** δ 1,05 (t, 3H, CH2-CH3); 1,67 et 1,86 (2m,4H, N-CH2-CH2-CH-); 1,80 (m, 2H, NH-CH2-CH2-CH2-NH); 2,26 (m, 1H, N-CH2-CH2-CH-); 2,38 (m, 2H, CH2-CH3); 2,78 et 3,33 (2m, 4H, N-CH2-CH2-CH-); 3,24 (m, 4H, NH-CH2-CH2-CH2-NH); 3,42 et 3,75 (2m, 2H, NH-CH2-CH-NH); 4,08 (m, 1H, NH-CH2-CH-NH); 4,95 et 5,00 (syst AB, 2H, O-CH2-Ph); 6,10 (tl, 1H, NH-CH2-CH-NH); 6,74 (sl, 1H, NH-CH2-CH-NH); 7,30 (m, 5H, Ph); 8,05 (s, 1H, N=CH-N); 8,55 ppm (sl, 2H, NH-CH2-CH2-CH2-NH).

**HPLC/SM** : (tr = 6min) : 553 (MH+); 364 (MH-tBu-PhCH2+); 320 (MH-tBu-Z+).

**Exemple 3**

**3-[[6-[4-(1,2,3,4-tetrahydro-1,8-naphthyridin-7-yl)-1-pipéridinyl]-5-méthyl-4-pyrimidinyl]amino]-N-[(phénylméthoxy) carbonyl]alanine**

**Stade a)**

Synthèse du 3-[(6-chloro-5-méthyl-4-pyrimidinyl)amino]-N-[(phénylméthoxy)carbonyl] alaninate de (1,1-diméthyléthyle).

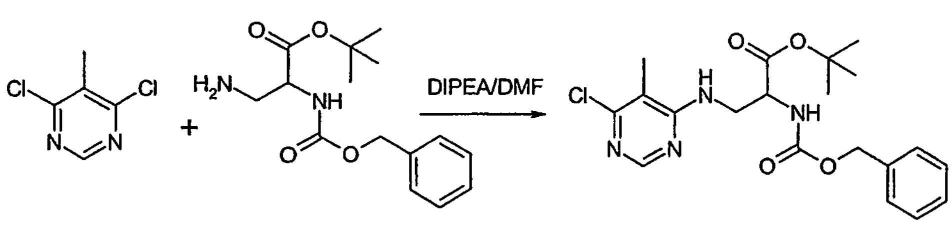


On chauffe à 120°C un mélange de 325 mg (2 mmoles) de 4,6-dichloro-5-méthyl-pyrimidine (commercialisée par SPECS), de 600 mg (2 mmoles) de 3-amino-N-[(phénylméthoxy)carbonyl] alaninate de (1,1-diméthyléthyle) (préparé selon J. Med. Chem. (2001), 44(8), 1158-1176) dans 3ml de diméthylformamide et 3 ml de diisopropyléthylamine pendant

une nuit. On concentre le milieu réactionnel à sec sous vide et reprend le résidu par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant évaporé sous vide. Le résidu est chromatographié sur silicagel en éluant avec un gradient d'heptane (100 %) jusqu'à un mélange d'heptane-acétate d'éthyle (50-50). On obtient 450 mg (Rdt = 53 %) de produit attendu sous forme d'une huile.

**CCM :** Rf = 0,5 (silicagel, éluant : acétate d'éthyleheptane(25-75)

**1H-RMN (CDCl3) :** δ 1,48 (s, 9H, tBu); 2,08 (s, 3H, CH3); 3,80 et 3,97 (2m, 2H, NH-CH2-CH-NH); 4,50 (m, 1H, NH-CH2-CH-NH); 5,08 et 5,15 (syst AB, 2H, O-CH2-Ph); 5,86 (dl, 1H, NH-CH2-CH-NH); 6,12 (sl, 1H, NH-CH2-CH-NH); 7,35 (m, 5H, Ph); 8,30 ppm (s, 1H, N=CH-N).

**SM (FAB) :** 421 (MH+); 365 (MH-tBu).

**Etape b)**

Synthèse du 3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alaninate de (1,1-diméthyléthyle).

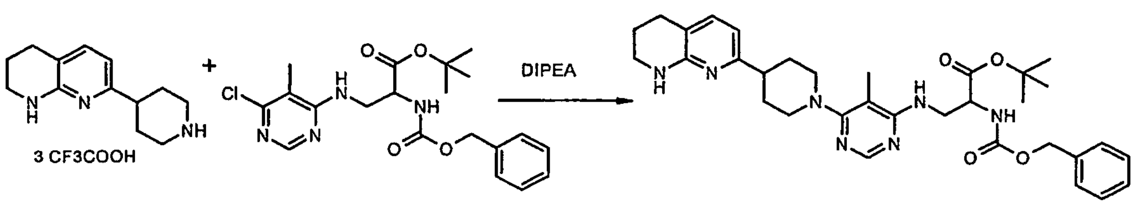


Un mélange de 370 mg (0,88 mmoles) de 3-[(6-chloro-5-méthyl-4-pyrimidinyl)amino]-N-[(phénylméthoxy)carbonyl] alaninate de (1,1-diméthyléthyle) et de 1,0 g (1,79 mmoles) de 1,2,3,4-tétrahydro-7-(4-pipéridinyl)-1,8-naphthyridine, tris (trifluoroacétate) (préparé selon les brevets EP1065207 ou WO 0078317) dans 1 ml de diisopropyléthylamine est chauffé à 120°C pendant 2 heures. On ajoute alors 10 ml de xylène et porte le milieu au reflux durant 4 heures. Le milieu réactionnel est repris par un mélange d'eau, d'acétate d'éthyle et d'une solution saturée de bicarbonate de sodium. La phase organique est décantée et la phase acqueuse est réextraite par de l'acétate d'éthyle. Les phases organiques regroupées sont séchées sur sulfate de magnésium et évaporées à sec sous vide et le résidu est chromatographié sur silicagel avec un gradient d'acétate d'éthyle 100 % jusqu'à un mélange d'acétate d'éthyle-méthanol-triéthylamine- dichlorométhane 50-10-10-50. On obtient 32 mg (Rdt = 6 %) de produit attendu et récupère 200 mg (54 %) de produit chloré de départ.

**CCM :** Rf = 0,6 (silicagel, éluant : acétate d'éthyledichlorométhane-méthanol 50-40-10)

**1H-RMN ($CDCl_3$) :** δ 1,49 (s, 9H, tBu); 1,97 (m, 2H, NH-CH2-CH2-CH2); 2,01 (s, 3H, CH3); 2,18 (m, 4H, N-CH2-CH2-CH); 2,79 (m, 2H, NH-CH2-CH2-CH2); 2,98 (m, 1H, N-CH2-CH2-CH); 3,39 et 3,89 (2m, 4H, N-CH2-CH2-CH); 3,52 (m, 2H, NH-CH2-CH2-CH2); 3,77 et 4,09 (2m, 2H, NH-CH2-CH-NH); 4,47 (m, 1H, NH-CH2-CH-NH); 5,13 (sl, 2H, O-CH2-Ph); 5,92 (sl, 1H, NH-CH2-CH-NH); 6,47 (dl, 1H, H naphthyridine); 7,37 (m, 5H, Ph); 7,38 (m, 1H, H naphthyridine); 8,41; 8,68 et 14,80 ppm (3sl, 3H, N=CH-N et mobiles).

**SM :** 602 (MH+); 546 (MH-tBu+).

**Etape c)**

Synthèse de la 3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alanine.

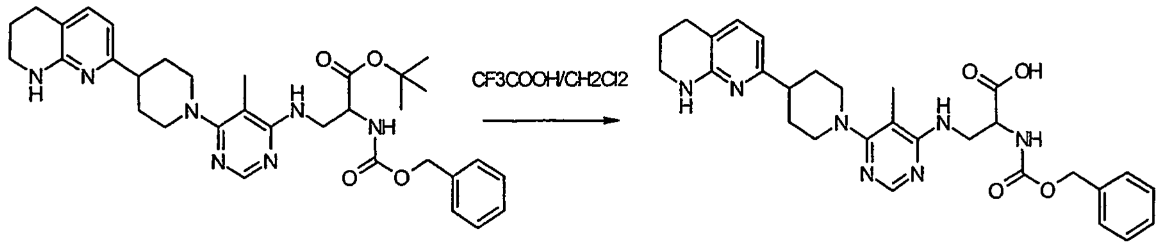


On agite à température ambiante un mélange de 38 mg (0,06 mmoles) de 3-[[6-[4-(1,2,3,4-tetrahydro-1,8-naphthyridin-7-yl)-1-pipéridinyl]-5-méthyl-4-pyrimidinyl]amino]-N-[(phényl méthoxy)carbonyl]alaninate de (1,1-diméthyléthyle) et de 2 ml d'acide trifluoroacétique dans 10 ml de dichlorométhane pendant 3 heures. On additionne alors 5 ml de toluène et évapore à sec le mélange. On obtient 26 mg (Rdt = 76 %) de produit attendu sous forme d'un solide amorphe.

**CCM :** Rf = 0,8 (silicagel, éluant : dichlorométhane-méthanol-eau-acide acétique 90-10-1-1).

**1H-RMN (DMSO-d6) :** δ 1,84 (m, 2H, NH-CH2-CH2-CH2); 1,92 (s, 3H, CH3); 1,75 et 1,91 (2m, 4H, N-CH2-CH2-CH); 2,74 (m, 2H, NH-CH2-CH2-CH2); 2,84 (m, 1H, N-CH2-CH2-CH); 2,92 et 3,67 (2m, 4H, N-CH2-CH2-CH); 3,44 (m, 2H, NH-CH2-CH2-CH2); 3,63 et 3,82 (2m, 2H, NH-CH2-CH-NH); 4,29 (m, 1H, NH-CH2-CH-NH); 5,02 (sl, 2H, O-CH2-Ph); 6,68 (d, 1H, H naphthyridine); 7,20 à 7,40 (m, 5H, Ph); 7,64 (dl, 1H, NH-CH2-CH-NH); 7,65 (d, 1H, H naphthyridine); 7,82 et 8,21 ppm (2sl, 3H, N=CH-N et mobiles).

**HPLC/SM :** (tr = 7min) 546 (MH+); 273 (M+2H++).

## Exemple 4

**3-[[6-[4-[(1,2,3,4,5,6-hexahydro-2-pyrimidinyl)iminocarbonyl] -1-pipéridinyl]-5-méthyl-4-pyrimidinyl]amino]-N-[(phénylméthoxy)carbonyl]alanine**

**Etape a)**

Synthèse du 3-[[6-[4-(méthoxycarbonyl)-1-pipéridinyl]-5-méthyl-4-pyrimidinyl]amino]-N-[(phénylméthoxy)carbonyl]alaninate de (1,1-diméthyléthyle).

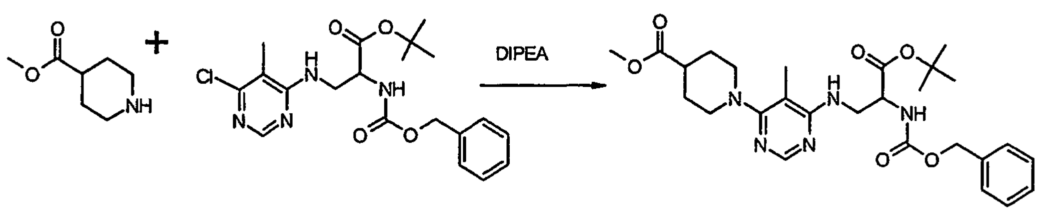


On chauffe au reflux un mélange de 80 mg (0,19 mmoles) de 3-[(6-chloro-5-méthyl-4-pyrimidinyl)amino]-N-[(phénylméthoxy) carbonyl] alaninate de (1,1-diméthyléthyle) et 3 ml de 4-pipéridinylcarboxylate de méthyle pendant 3 heures. Le milieu réactionnel est repris après refroidissement par de l'eau, de l'acétate d'éthyle et une solution saturée de bicarbonate de sodium. La phase organique est séparée et la phase acqueuse réextraite par de l'acétate d'éthyle. Les phases organiques regroupées sont séchées sur sulfate de magnésium puis le solvant éliminé sous vide. Le résidu est chromatographié sur silicagel en éluant avec un gradient d'heptane (100 %) jusqu'à de l'heptane-acétate d'éthyle (50-50). On obtient 25 mg (Rdt = 25 %) du produit attendu sous forme d'huile.

**CCM :** Rf = 0,3 (silicagel, éluant : acétate d'éthyle-heptane 50-50)

**IR ($CHCl_3$) :** 3421 (NH); 1724 (C=O); 1585; 1501 cm-1 (C=C, C=N)

**1H-RMN (DMSO-d6) :** δ 1,31 (s, 9H, tBu); 1,68 (ql, 2H, CH2-CH(C=O)); 1,87 (m, 2H, CH2-CH(C=O)); 1,87 (s, 3H, CH3-C=); 2,51 (masqué, 1H, -CH2-CH-CH2-); 2,80 (tl, 2H, -N-(CH2)2-(CH2)2-); 3,44 (dl, 2H, -N-(CH2)2-(CH2) 2-); 3,62 (s, 3H, CH3-O); 3,71 (m, 2H, NH-CH2-CH-NH-); 4,22 (m, 1H, NH-CH2-CH-NH-); 5,03 (sl, 2H, O-CH2-Ph); 6,35 sl, 1H, NH-CH2-CH-NH-); 7,34 (m, 5H, Ph); 7,63 (dl, 1H, NH-CH2-CH-NH-); 8,08 ppm (s, 1H, N=CH-N).

**HPLC/SM :** (tr = 17min) 528 (MH+); 472 (MH-tBu+)

**Etape b)**

Synthèse de la 3-[[6-[4-[(1,2,3,4,5,6-hexahydro-2-pyrimidinyl)iminocarbonyl]-1-pipéridinyl]-5-méthyl-4-pyrimidinyl]amino]-N-[(phénylméthoxy)carbonyl]alanine.

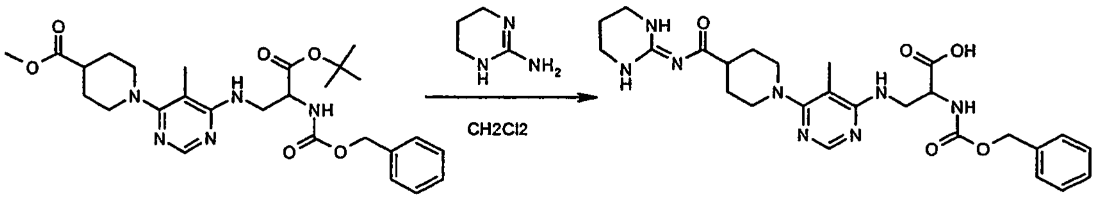


On agite à température ambiante un mélange de 100 mg (0,19 mmoles) de 3-[[6-[4-(méthoxycarbonyl)-1-pipéridinyl]-5-méthyl-4-pyrimidinyl]amino]-N-[(phénylméthoxy) carbonyl]alaninate de (1,1-diméthyléthyle) et de 150 mg (1,5 mmoles) de 2-amino-1,4,5,6-tétrahydropyrimidine (préparée selon R. F. Evans J. Chem. Soc. 1964, 2450-2455) dans 10 ml de dichlorométhane pendant 5 heures. On évapore à sec sous vide le milieu réactionnel et chromatographie le résidu sur silicagel en éluant avec un mélange dichlorométhane-methanol-eau-acide acétique 85-15-2-2.

On obtient 10 mg (Rdt = 10 %) de produit attendu sous forme d'un solide amorphe.

**CCM :** Rf = 0,4 (silicagel, éluant : dichlorométhane-méthanol-eau-acide acétique 85-15-2-2)

**1H-RMN (DMSO-d6) :** δ 1,64 et 1,74 (2m, 4H, N-CH2-CH2-CH); 1,77 (m, 2H, NH-CH2-CH2-CH2-NH); 1,85 (s, 3H, CH3); 2,11 (m, 1H, N-CH2-CH2-CH); 3,17 (m, 4H, NH-CH2-CH2-CH2-NH); 2,68 et 3,42 (2m, 4H, N-CH2-CH2-CH); 3,30 et 3,62 (2m, 2H, HN-CH2-CH-NH); 3,73 (m, 1H, N-CH2-CH-NH); 5,00 (m, 2H, O-CH2-Ph); 6,54 (dl, 1H, NH-CH2-CH-NH); 6,77 (sl, 1H, NH-CH2-CH-NH); 7,33 (m, 5H, Ph); 8,04 ppm (s, 1H, N=CH-N).

**HPLC/SM** : (tr = 1,9min) 1077 (2MH+); 539 (MH+); 440 (MH-aminotetrahydro pyrimidine+)

## Exemple 5

**3-[[5-éthyl-6-[4-(1,2,3,4-tétrahydro-1,8-naphthyridin-7-yl)-1-pipéridinyl]-4-pyrimidinyl]axnino]-N-[(phénylméthoxy)carbonyl]alaninate d'éthyle, dichlorhydrate**

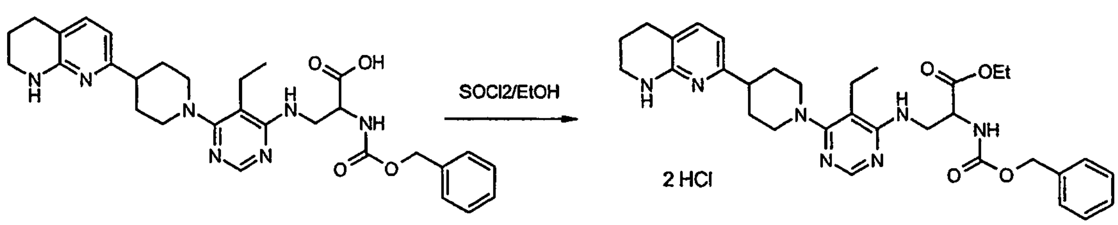


A 4 ml d'éthanol refroidi à -12°C sous atmosphère d'azote on ajoute 160 µl (2,19 mmoles) de chlorure de thionyle et agite le mélange pendant 30 minutes. On additionne alors une solution de 354 mg (0,45 mmoles) de 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate) dans 13 ml d'éthanol. Le mélange est agité 30 minutes à -12°C puis on laisse revenir à température ambiante et enfin chauffe à 40°C pendant 4 heures sous azote. On évapore le milieu réactionnel à sec sous vide et cristallise le résidu dans un mélange d'éther diisopropylique et de pentane et filtre le solide. On obtient 284 mg (Rdt = 95 %) de produit attendu sous forme d'un solide beige amorphe.

**CCM :** Rf = 0,4 (silicagel, éluant : dichlorométhane-méthanol-eau-acide acétique 90-10-1-1)

**IR ($CHCl_3$) :** 3400-3000(OH, NH); 1719(C=O); 1654; 1623; 1580; 1504 cm-1 (C=C, C=N, aromatique)

**1H-RMN (DMSO-d6) :** δ 1,09 (t, 3H, CH2-CH3); 1,14 (t, 3H, O-CH2-CH3); 1,85 (m, 2H, NH-CH2-CH2-CH2); 1,85 et 2,00 (2m, 4H, N-CH2-CH2-CH); 2,48 (m, 2H, CH2-CH3); 2,75 (t, 2H, NH-CH2-CH2-CH2); 2,92 (m, 1H, N-CH2-CH2-CH); 3,03 et 3,57 (2m, 4H, N-CH2-CH2-CH); 3,44 (t, 2H, N-CH2-CH2-CH2); 3,76 et 3,87 (2m, 2H, NH-CH2-CH-NH); 4,07 (q, 2H, O-CH2-CH3); 4,39 (q, 1H, NH-CH2-CH-NH); 5,03 (s, 2H, O-CH2-Ph); 6,62 (d, 1H, H naphthyridine); 6,86 (sl, 1H, NH-CH2-CH-NH); 7,30 (m, 5H, Ph); 7,33 (sl, 1H, NH-CH2-CH-NH); 7,58 (d, 1H, H naphthyridine); 8,20 ppm (s, 1H, N=CH-N).

**HPLC/SM :** (tr = 6,2min) 588 (MH+); 454 (MH-COOCH2Ph+); 437 (MH-NHCOOCH2Ph+).

**Exemple 6**

Synthèse de la 4,6-dihydroxy-5-méthyl-pyrimidine.

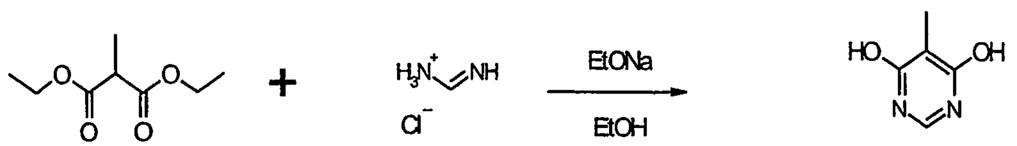


A une solution de 7,5 g (94 mmoles) de chlorhydrate de formamidine dans 250 ml d'éthanol refroidie à 0°C on ajoute 102 ml (282 mmoles) d'une solution d'éthylate de sodium à 21% dans l'éthanol et agite le mélange pendant 30 minutes; on additionne alors une solution de 13 ml (94 mmoles) de méthyl malonate de diéthyle dans 50 ml d'éthanol et agite une nuit à température ambiante. On évapore le mélange réactionnel à sec sous pression réduite (2 kPa) et reprend le résidu par de l'acétate d'éthyle, de l'eau et de l'acide acétique pour amener le pH à 6. Le précipité est filtré puis lavé successivement par de l'eau, de l'isopropanol, de l'éther diéthylique et enfin du pentane. On obtient 7 g (Rdt = 60%) de produit attendu sous forme d'un solide beige.

**CCM :** Rf = 0,20 (silicagel, éluant : acétate d'éthyledichlorométhane-méthanol 50-40-10)

**1H-RMN (DMSO d6) :** δ ppm 1,73(s, 3H, C-$CH_3$); 7,88 (s,N=CH-N).

**SM :** 127 (MH+); 125 (M-H-).

Synthèse de la 4,6-dibromo-5-méthyl-pyrimidine.

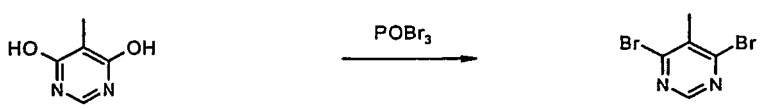


Un mélange de 6 g (47,6 mmoles) de 5-méthyl-4,6-dihydroxypyrimidine dans 18 g d'oxybromure de phosphore est porté à 200°C durant 3 heures. Après retour à température ambiante, le mélange réactionnel est repris par un mélange d'eau glacée de bicarbonate de sodium et extrait à l'acétate d'éthyle, lave les phases organiques avec de l'eau, sèche sur sulfate de magnésium et évapore à sec sous pression réduite (2 kPa). On obtient 9 g (Rdt = 75 %) d'un solide beige.

**CCM :** Rf = 0,27 (silicagel, éluant : dichlorométhanepentane 50-50)

**1H-RMN (CDCl3) :** δ ppm 2,58 (s, 3H, C-$CH_3$); 8,51 (s,N=CH-N).

**SM :** 253-255 (MH+).

Synthèse du de 6-[4-(1,2,3,4-tetrahydro-1,8-naphthyridin-7-yl)-1-pipéridinyl]-5-méthyl-4-bromo-pyrimidine :

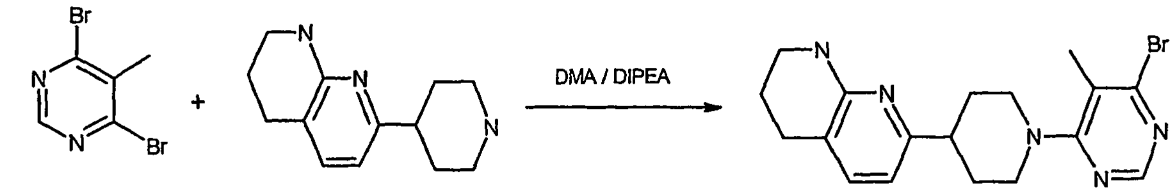


Dans un monocol contenant 8 g (36,8 mmoles) de 4-(1,2,3,4-tetrahydro-1,8-naphthyridin-7-yl)-1-pipéridine libérée de son sel, on ajoute 70 ml diméthylacétamide, 7,56 g (30 mmoles) de 4,6-dibromo-5-méthyl-pyrimidine mis en solution dans 40 ml de diméthylacétamide et 14 ml de diisopropyléthylamine. Ce mélange est chauffé à 140˚C pendant 4 heures puis concentré à sec sous pression réduite (2 kPa). Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant évaporé sous pression réduite (2 kPa). Le solide obtenu est lavé plusieurs fois à l'éther diisopropylique puis au pentane, on obtient 7.7 g du produit attendu sous la forme d'une poudre marron clair. Le filtrat est concentré à sec puis chromatographié sur silicagel en éluant avec un gradient d'acétate d'éthyle (100 %) puis d'acétate d'éthyle- méthanol (95-5). On obtient 533 mg de produit attendu sous la forme d'une poudre jaune. (Rdt global = 70 %)

Préparation de la naphtyridine sous forme d'amine libre :

22 g de naphtyridine est déplacée de son sel par 6 équivalents massiques de résine basique amberlyst A21 (résine de type R-$NMe_2$) dans un mélange $CH_2Cl_2$ / MeOH /AcOEt 1/1/1 sous agitation pendant 30 minutes. On lave préalablement la résine et on la laisse gonfler 20 minutes dans ce mélange de solvant. Cette opération doit être répétée 3 fois pour que le déplacement du sel soit total. Après filtration de la résine et évaporation des solvants, on obtient 8 g (36,8 mmoles) de naphtyridine libre.

**CCM :** Rf = 0,33 (silicagel, éluant : acétate d'éthyle (100%)).

**1H-RMN (DMSO-d6) :** δ 1,7 à 1,85 (m, 6H, NH-CH2-CH2-CH2, N-CH2-CH2-CH-CH2); 2,23 (s, 3H, CH3-); 2,60 (m, 3H, CH2-CH-CH2, NH-CH2-CH2-CH2); 3,23 (m, 2H, NH-CH2-CH2-CH2); 2,97 et 3,92 (2m, 4H, CH2-CH2-N-CH2-CH2); 6,3 et 7,05 (2d, 2H, CH=CH naphthyridine); 8,28 (s, 1H, N=CH-N).

**SM (FAB) :** 388 (M) ; 389 (MH+)

Synthèse du 3-[[5-méthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

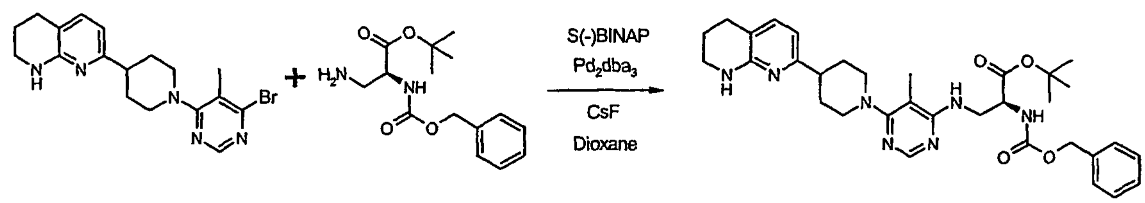


On chauffe au reflux un mélange de 620 mg (1,6 mmoles) de 4-bromo-5-méthyl-6-[4-(1,2,3,4-tétrahydro-1,8-naphthyridin-7-yl)-1-pipéndinyl]-pyrimidine, de 565 mg (1,92 mmoles) de 3-amino-N-[(phénylméthoxy)carbonyl]alaninate de (1,1-diméthyléthyle) (préparé selon J. Med. Chem. (2001), 44(8), 1158-1176), de 340 mg (2,25 mmoles) de fluorure de césium, de 73 mg (0,08 mmoles) de tris(dibenzylidèneacétone) dipalladium(0), de 100 mg (0,16 mmoles) de S(-)2,2'-bis(diphényl-phosphino)-1,1'-binaphtyl dans 50 ml de dioxane pendant 5 heures. On refroidit puis ajoute à nouveau 280 mg (0,96 mmoles) de 3-amino-N-[(phénylméthoxy)carbonyl]alaninate de (1,1-diméthyléthyle), 340 mg (2,25 mmoles) de fluorure de césium, 73 mg (0,08 mmoles) de tris(dibenzylidèneacétone) dipalladium(0),et 100 mg (0,16 mmoles) de S(-)2,2'-bis(diphényl-phosphino)-1,1'-binaphtyl et chauffe au reflux pendant 5 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié une première fois sur silicagel en éluant avec un gradient d'acétate d'éthyle-chlorure de méthylène-triéthylamine-méthanol de 50-50-0-0 à 50-50-2-2. Le produit obtenu est chromatographié une deuxième fois sur alumine en éluant avec un mélange d'heptanechlorure de méthylène 50-50 puis à l'acétate d'éthyle-éther diisopropylique 50-50. On obtient 360 mg (Rdt = 37%) de produit attendu sous forme de solide amorphe blanc.

**CCM :** Rf = 0,20 (silicagel, éluant : chlorure de méthylène-méthanol 95-5).

**IR (CHCl3) :** 3435 (NH); 1717 (C=O); 1583,1555,1501 cm-1 (Hétérocycle+Aromatique+Amide).

**1H-RMN (CDCl3) :** δ 1,47 (s, 9H, tBu); 1,94 (s, 3H, C-$\underline{CH_3}$); 1, 75 à 2, 05 (m, 6H, $CH_2$-$\underline{CH_2}$-$CH_2$-NH et $\underline{CH_2}$-CH-$\underline{CH_2}$); 2, 60 à 2,77 (m, 3H, $CH_2$-$\underline{CH}$-$CH_2$ et $\underline{CH_2}$-$CH_2$-$CH_2$-NH); 2,93 et 3,90 (2m, 4H, $CH_2$-$\underline{CH_2}$-N-$\underline{CH_2}$-$CH_2$); 3,42 (m, 2H, $CH_2$-$CH_2$-$\underline{CH_2}$-NH); 3,66 (m, 2H, NH-$\underline{CH_2}$-CH-NH); 4,45 (m, 1H, NH-$CH_2$-$\underline{CH}$-NH); 4,95, 5,25 et 6,20 (3m, 3H $CH_2$-$CH_2$-$CH_2$-$\underline{NH}$ NH-$CH_2$-CH-$\underline{NH}$, $\underline{NH}$-$CH_2$-CH-NH); 5,12 (s, 2H, $\underline{CH_2}$-Ph); 7,35 (m, 5H, Ph); 8,29 ppm (s,N=$\underline{CH}$-N).

**SM :** 602 (MH+); 546 (MH-tBu+); 412 (MH-COOCH2Ph+).

**[$\alpha_D$]** (0,625 % EtOH) : -4,5°

Synthèse de la 3-[[5-méthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

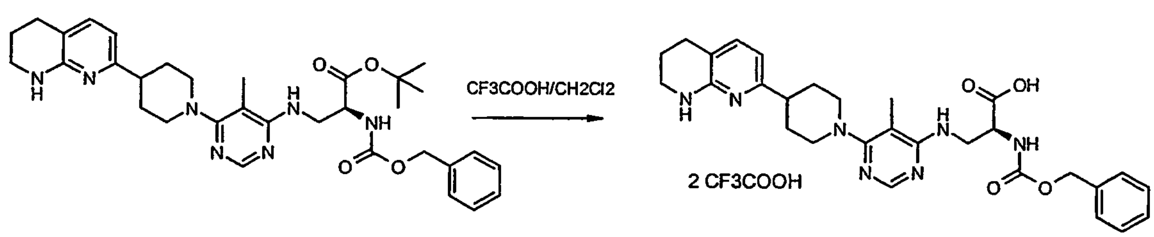


On agite 350 mg (0,58 mmoles) de 3-[[5-méthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle) dans 15 ml de dichlorométhane avec 3 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 330 mg (Rdt = 73% exprimé en ditrifluoroacétate) de produit attendu sous forme d'un solide blanc.

**CCM :** Rf = 0,44 (silicagel, éluant : dichlorométhane-méthanol-eau-acide acétique 90-10-1-1)

1H-RMN (CDCl3) : δ 1,96 (s, 3H, C-$CH_3$); 1,80 à 2,05 (m, 6H, $CH_2$-$CH_2$-$CH_2$-NH et $CH_2$-CH-$CH_2$); 2,76 (t, 2H, NH-CH2-CH2-CH2-); 2,96 (t, 1H, N-CH2-CH2-CH-); 3,21 et 3,83 (2m, 4H, N-CH2-CH2-CH); 3,50 (m, 2H, N-CH2-CH2-CH2-); 3,83 et 4,05 (2m, 2H, NH-CH2-CH-NH); 4,54 (q, 1H, NH-CH2-CH-NH); 5,50 (s, 2H, O-CH2-Ph); 6,40 (d, 1H, H naphthyridine); 6,55 (dl, 1H, NH); 7,28 (m, 5H, Ph); 7,45 (ml, 1H, NH); 8,22 (d, 1H, H naphthyridine), 8,22 (s, 1H, N=CH-N); 9,62 ppm (sl, 1H).

**SM :** 546 (MH+); 412 (MH-COOCH2Ph+); 544- (M-H-); 436- (544-OCH2Ph-); 1089- (2M-H-)

**[$\alpha_D$]** (0,60% MeOH) : -14,0.

<u>**Exemple 7**</u>

Préparation de la naphtyridine sous forme d'amine libre :

22 g de naphtyridine est déplacée de son sel par 6 équivalents massiques de résine basique amberlyst A21 (résine de type R-$NMe_2$) dans un mélange $CH_2Cl_2$ / MeOH /AcOEt 1/1/1 sous agitation pendant 30 minutes. On lave préalablement la résine et on la laisse gonfler 20 minutes dans ce mélange de solvant. Cette opération doit être répétée 3 fois pour que le déplacement du sel soit total. Après filtration de la résine et évaporation des solvants, on obtient 8 g (36,8 mmoles) de naphtyridine libre.

**CCM :** Rf = 0,33 [silicagel, éluant : acétate d'éthyle (100%)]

**1H-RMN (DMSO-d6) :** δ 1,7 à 1,85 (m, 6H, NH-CH2-<u>CH2</u>-CH2, N-CH2-<u>CH2</u>-CH-<u>CH2</u>) ;2,23 (s, 3H, CH3-); 2,60 (m, 3H, CH2-<u>CH</u>-CH2, NH-CH2-CH2-<u>CH2</u>); 3,23 (m, 2H, NH-CH2-CH2-<u>CH2</u>); 2,97 et 3,92 (2m, 4H, CH2-<u>CH2</u>-N-<u>CH2</u>-CH2); 6,3 et 7,05 (2d, 2H, <u>CH</u>=<u>CH</u> naphthyridine); 8,28 (s, 1H, N=<u>CH</u>-N).

**SM (FAB) :** (tr = 0,50 min et 2,80 min): 388(M); 389(MH+)

Synthèse du N-(alpha)-Z-L,2-3-diaminopropionate d'éthyle.

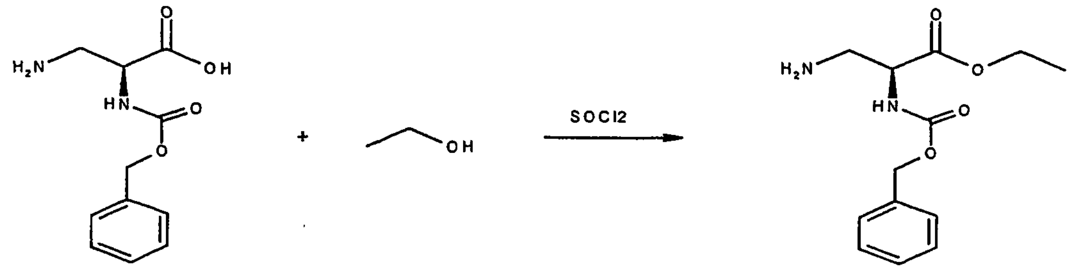


Dans un mono col contenant 26 ml d'éthanol absolu, on ajoute goutte à goutte et à 0°C, 12 ml (168 mmoles) de chlorure de thionyle. Ce mélange est agité pendant une vingtaine de minutes à TA puis on y ajoute par petites quantités 2 g (8,4 mmoles) d'acide N-(alpha)-Z-L,2-3-diaminopropionique, un trouble blanc apparaît.

Le mélange réactionnel est alors porté au reflux (78°C) pendant 2 heures (après quelques minutes de chauffage, la solution devient limpide).

Après refroidissement la solution est concentrée à sec, on obtient un liquide jaune auquel on ajoute de l'éther isopropylique. Le produit attendu prend en masse, on ôte alors le surnageant et le résidu est repris par un mélange d'eau, d'acétate d'éthyle et d'une solution de bicarbonate de sodium saturée. La phase organique est séparée et la phase aqueuse de nouveau extraite à l'acétate d'éthyle. Les phases organiques regroupées sont séchées sur sulfate de magnésium puis le solvant évaporé sous vide. On obtient 1,4 g d'une huile jaune pâle, il s'agit de l'ester sous la forme d'amine libre (Rendement = 63 %).

**CCM :** Rf = 0,41 [ Silicagel, éluant : chlorure de méthylène-méthanol (85-15)et 2% d'eau-acide acétique(1-1).

**1H-RMN (CDCl3) :** δ 1,33 (t, 3H, CH3-CH2-O); 2,53 (m, 2H, NH2-CH2-CH) 3,15 (m, 2H, NH2-CH2-CH); 4,22 (q, 2H, CH3-CH2-O); 4,42 (m, 1H, NH2-CH2-CH-NH); 5,85 (m,1H, CH-NH-CO); 5,15 (s, 2H, O-CH2-Ph); 7,35 (m, 5H, Ph)

**SM (FAB) :** (tr = 0,51 min et 1,2 min) : 267(MH+); 533(2MH+) **$[\alpha]_D$ (CHCl3) =** +6,336

Synthèse du 3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alaninate d'éthyle.

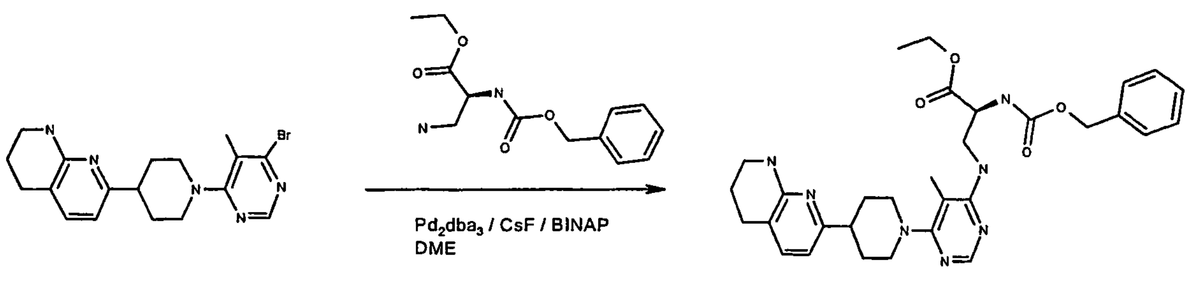


Un mélange de 1,9 g (4,9 mmoles) de 6-[4-(1,2,3,4-tetrahydro-1,8-naphthyridin-7-yl)-1-pipéridinyl]-5-méthyl-4-bromo-pyrimidine et de 1,62 g (6,08.mmoles) de 3-amino-N-[(phénylméthoxy)carbonyl] alaninate d'éthyle, en présence de 1,06 g (6,86 mmoles) de fluorure de cesium, de 310 mg (490 μmoles)de (2,2'-bis(diphénylphosphino)-1,1'-binaphthyl et de 230 mg (245 μmoles) de tris(dibenzylidèneacétone) dipalladium(o) dans 40 ml de 1,2-diméthoxyéthane est chauffé au reflux pendant 4 heures. Le mélange réactionnel est ensuite ramené à température pour l'ajout de 230 mg (245 μmoles) de tris(dibenzylidèneacétone)dipalladium(o), puis on porte de nouveau au reflux pendant 16 heures.

Après refroidissement la solution est concentrée à sec puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous vide. Le résidu est chromatographié sur silicagel par l'acétate d'éthyle 100%. On obtient 1,15 g de produit attendu sous la forme d'une huile jaune pâle (rendement = 40%)

**CCM :** Rf = 0.16 (silicagel éluant : acétate d'éthyle 100 %

**1H-RMN (CDCl3) :** δ 1,25 (t, 3H, CH3-CH2-O); 1,80 à 2,05 (m,

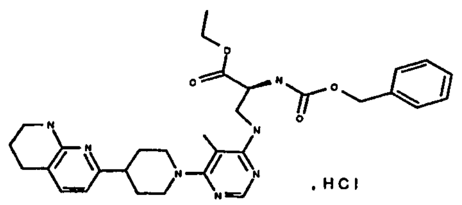

M W =610.15
M F =C31H39N7O4,HCl

9H, NH-CH2-CH2-CH2, N-CH2-CH2-CH-CH2, CH3-C=C-); 2,72 (m, 3H, CH2-CH-CH2, NH-CH2-CH2-CH2); 2,95 et 3,93 (2m, 4H, CH2-CH2-N-CH2-CH2); 3,45 (m, 2H, NH-CH2-CH2-CH2); 3,68 (d, 2H, NH-CH2-CH-NH) 4,23 (t, 2H, CH3-CH2-O) , 4,45 (m, 1H, NH-CH2-CH-NH); 5,10 et 5,17 (syst AB, 2H, O-CH2-Ph); -6,41 et 7,18 (2d, 2H, CH=CH naphthyridine); 7,35 (m, 5H, Ph); 8,27 (s, 1H, N=CH-N).

**HPLC/SM :** (tr = 0,48 min et 2,9 min): 574 (MH+); 440 [MH - Z+]; 287 [MH - (tBu - Z+)]

$[\alpha]_D$ (CHCl3) = + 0,788

Formation du Chlorhydrate:

1,15 g de 3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alaninate d'éthyle sont solubilisés dans un minimum de chlorure de méthylène. Cette solution est coulée sur de l'éther éthylique, la solution doit rester limpide. On ajoute ensuite goutte à goutte, sous agitation, 2,65 ml d'HCl 2N dilué dans un peu d'éther éthylique. Le chlorhydrate prend en masse, on ôte le surnageant, puis le résidu est cristallisé dans l'éther isopropylique. On obtient alors un solide qui est filtrée, rincée à l'éther puis au pentane. Après séchage, on obtient 890 mg d'une poudre jaune pâle. (Rendement = 73%)

## Exemple 8

Préparation de la naphtyridine sous forme d'amine libre :

22 g de naphtyridine est déplacée de son sel par 6 équivalents massiques de résine basique amberlyst A21 (résine de type R-$NMe_2$) dans un mélange $CH_2Cl_2$ / MeOH /AcOEt 1/1/1 sous agitation pendant 30 minutes. On lave préalablement la résine et on la laisse gonfler 20 minutes dans ce mélange de solvant. Cette opération doit être répétée 3 fois pour que le déplacement du sel soit total. Après filtration de la résine et évaporation des solvants, on obtient 8 g (36,8 mmoles) de naphtyridine libre.

**CCM :** Rf = 0,33 [silicagel, éluant : acétate d'éthyle (100%)]

**1H-RMN (DMSO-d6) :** $\delta$ 1,7 à 1,85 (m, 6H, NH-CH2-CH2-CH2, N-CH2-CH2-CH-CH2) ;2,23 (s, 3H, CH3-); 2,60 (m, 3H, CH2-CH-CH2, NH-CH2-CH2-CH2); 3,23 (m, 2H, NH-CH2-CH2-CH2); 2,97 et 3,92 (2m, 4H, CH2-CH2-N-CH2-CH2); 6,3 et 7,05 (2d, 2H, CH=CH naphthyridine); 8,28 (s, 1H, N=CH-N).

**SM (FAB) :** (tr=0,50 min et 2,80 min): 388(M); 389(MH+)

Synthèse du N-(alpha)-Z-L,2-3-diaminopropionate d'isopropyle.

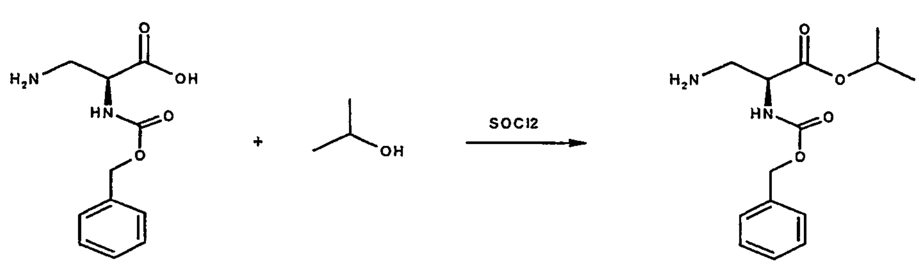


Dans un monocol contenant 125 ml d'isopropanol, on ajoute goutte à goutte et à 0°C, 46 ml (840 mmoles) de chlorure de thionyle. Ce mélange est agité pendant une vingtaine de minutes à TA puis on y ajoute par petites quantités 10 g (42 mmoles) d'acide N-(alpha)-Z-L,2-3-diamino-propionique, un trouble blanc apparaît.

Le mélange réactionnel est alors porté au reflux (78°C) pendant 2 heures (après quelques minutes de chauffage, la solution devient limpide).

Après refroidissement la solution est concentrée à sec, on obtient un liquide jaune auquel on ajoute de l'éther isopropylique. Le produit attendu prend en masse, on ôte alors le surnageant et le résidu est repris par un mélange d'eau, d'acétate d'éthyle et d'une solution de bicarbonate de sodium saturée. La phase organique est séparée et la phase aqueuse de nouveau extraite à l'acétate d'éthyle. Les phases organiques regroupées sont séchées sur sulfate de magnésium puis le solvant évaporé sous vide. On obtient 7,4 g d'une poudre blanche, il s'agit de l'ester sous la forme d'amine libre (Rendement = 42 %).

**CCM :** Rf = 0.5 Silicagel, éluant : chlorure de méthylène-méthanol (85-15)et 2 % d'eau-acide acétique(1-1).

**1H-RMN (CDCl3) :** δ 1,25 (d, 6H, CH3-CH-CH3); 1,60 (m, 2H, NH2-CH2-CH) 3,10 (m, 2H, NH2-CH2-CH); 4,35 (m, 1H, NH2-CH2-CH-NH) 5,10 (m, 1H, CH3-CH-CH3); 5,15 (s, 2H, O-CH2-Ph) ;5,2 (m,1H, CH-NH-CO), 7,37 (m, 5H, Ph)

**SM (FAB) :** (tr = 0,52 min et 2,09 min) : 281(MH+) ; 239(MH-ipr+)

**[α]$_D$ (CHCl3) = +15,02**

Synthèse du 3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alaninate d'isopropyle.

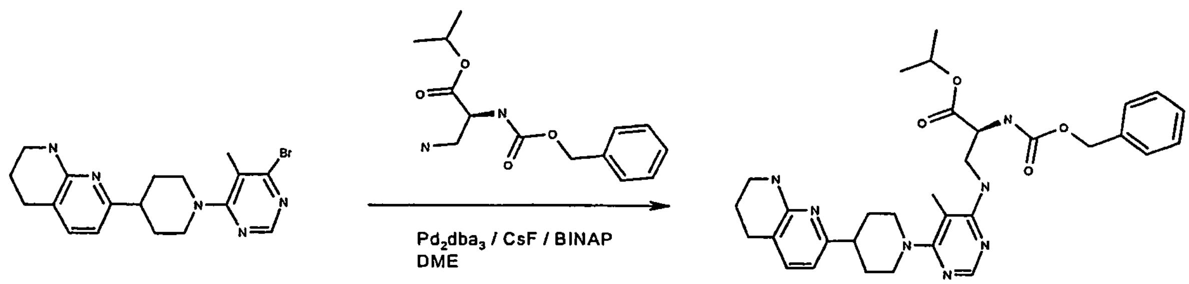


Un mélange de 1,5 g (3,86 mmoles) de 6-[4-(1,2,3,4-tetrahydro-1,8-naphthyridin-7-yl)-1-pipéridinyl]-5-méthyl-4-bromo-pyrimidine et de 1,30 g (4,63.mmoles) de 3-amino-N-[(phénylméthoxy)carbonyl] alaninate d'isopropyle, en présence de 825 mg (5,41 mmoles) de fluorure de cesium, de 241 mg (386 μmoles)de (2,2'-bis(diphénylphôsphino)-1,1'-binaphthyl et de 180 mg (193 μmoles) de tris(dibenzylidèneacétone) dipalladium(o) dans 40 ml de 1,2-diméthoxyéthane est chauffé au reflux pendant 5 heures.et 30 minutes.Le mélange réactionnel est ensuite ramené à température pour l'ajout de 180 mg (245 μmoles) de tris(dibenzylidèneacétone) dipalladium(o), puis on porte de nouveau au reflux pendant 16 heures.

Le lendemain, on chauffe encore 9 heures après avoir ajouté 180 mg (193 μmoles) de tris(dibenzylidèneacétone) dipalladium(o).Après refroidissement la solution est concentrée à sec puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous vide. Le résidu est chromatographié sur silicagel par l'acétate d'éthyle 100%. On obtient 1,52 g de produit attendu sous la forme de cristaux jaune pâle (rendement = 50%)

**CCM :** Rf = 0.18 (silicagel éluant : acétate d'éthyle 100 %

**1H-RMM (CDCl3) :** δ 1,27 (d, 6H, CH3-CH-CH3); 1,83 à 2,05 (m, 9H, NH-CH2-CH2-CH2, N-CH2-CH2-CH-CH2, CH3-C=C-); 2,72 (m, 3H, CH2-CH-CH2, NH-CH2-CH2-CH2); 2,95 et 3,93 (2m, 4H, CH2-CH2-N-CH2-CH2); 3,45 (m, 2H, NH-CH2-CH2-CH2); 3,68 (d, 2H, NH-CH2-CH-NH), 4,50 (m, 1H, NH-CH2-CH-NH); 5,07 (m, 1H, CH3-CH-CH3); 5,12 (s, 2H, O-CH2-Ph) ; 6,42 et 7,18 (2d, 2H, CH=CH naphthyridine); 7,35 (m, 5H, Ph); 8,28 (s, 1H, N=CH-N).

**HPLC/SM :** (tr = 0,48 min et 3,03 min): 588 (MH+); 454 [MH - Z+] ; 412 [MH - (ipr - Z)+]

**[α]$_D$** (CHCl3) = + 0,7655

Formation du Chlorhydrate :

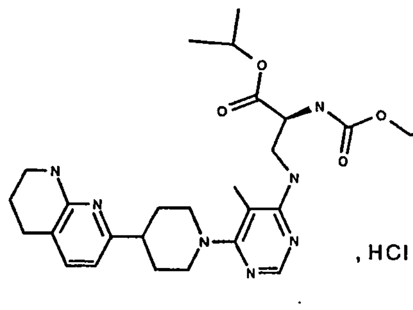


3.8gde3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alaninate d'isopropyle sont solubilisés dans 20 ml de chlorure de méthylène. Cette solution est coulée sur de l'éther éthylique, la solution doit rester limpide. On ajoute ensuite goutte à goutte, sous agitation, 3,23 ml d'HCl 2N dilué dans un peu d'éther éthylique. Le chlorhydrate prend en masse, on ôte le surnageant, puis le résidu est cristallisé dans l'éther isopropylique. On obtient alors un solide qui est filtrée, rincée à l'éther puis au pentane. Après séchage, on obtient 3.44 g d'une poudre blanche. (Rendement = 85%)

## Exemple 9

Synthèse de la 4,6-dibromo-5-éthyl-pyrimidine

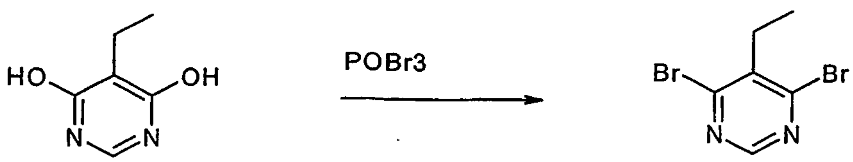


Un mélange de 2,8 g (20 mmoles) de 5-éthyl-4,6-dihydroxypyrimidine (commercialisée par Aldrich) et de 8,85 g (31 mmoles) d'oxybromure de phosphore est porté à 160°C durant 1 heure. Après retour à température ambiante, le mélange réactionnel est versé sur un mélange de glace, de solution saturée de bicarbonate de sodium et d'acétate d'éthyle. On décante, lave la phase organique avec une solution saturée de bicarbonate de sodium, sèche sur sulfate de magnésium et le solvant est éliminé par évaporation sous pression réduite (2 kPa). Le résidu est chromatographié sur silicagel en éluant avec un gradient d'heptane 100 % jusqu'à heptane-acétate d'éthyle 50-50. On obtient 3,92 g (Rdt = 74 %) d'un solide marron clair de produit attendu utilisé telle quelle pour la suite.

**CCM :** Rif 0,5 (silicagel, éluant : heptane-acétate d'éthyle 90-10)

**IR ($CHCl_3$) :** 1538; 1503 (C=C, C=N)

**1H-RMN (DMSO-d6) :** δ 1,16 (t, 3H, $CH_2$-$\underline{CH_3}$); 2,86 (q, 2H, $\underline{CH_2}$-$CH_3$); 8,63 ppm (s, 1H, N=CH-N)

**SM :** 266 (mu+); 249 (MH-CH3+); 184 (MH-HBr+).

Synthèse 4-bromo-5-éthyl-6-[4-(1,2,3,4-tétrahydro-1,8-naphthyridin-7-yl)-1-pipéridinyl]-pyrimidine

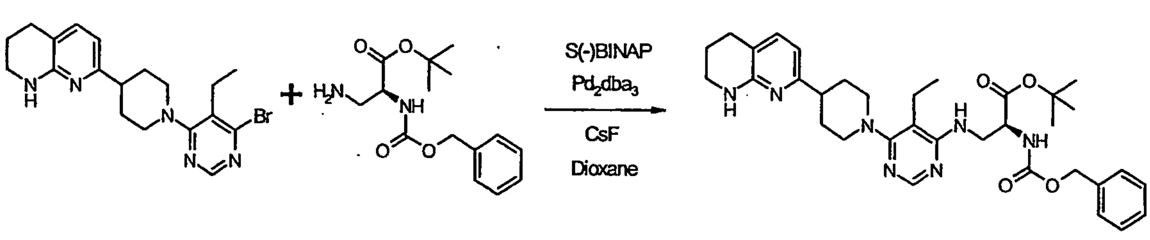

Un mélange de 3,67 g (6,45 mmoles) de 1,2,3,4-tétrahydro-7-(4-pipéridinyl)-1,8-naphthyridine, tris(trifluoroacetate) (préparé selon les brevets EP1065207 ou WO 0078317) et de 25 g de résine Amberlyst A-21 (origine Fluka 06424 préalablement lavée avec la solution dichlorométhane-acétate d'éthyleméthanol 1-1-1) dans 150 ml de solution de dichlorométhane-acétate d'éthyle-méthanol 1-1-1 est agité à température ambiante durant 1 heure. Le mélange est filtré, la résine lavée avec la solution ternaire. Le filtrat obtenu est agité à température ambiante durant 1 heure en présence de 25 g de résine Amberlyst A-21 traitée comme précédemment; cette opération est renouvelée une troisième fois. Le filtrat ainsi obtenu est concentré à sec sous pression réduite (2 kPa) donnant 1,4 g d'amine libre. On rajoute à ce résidu 2,06 g (7,74 mmoles) de 4,6-dibromo-5-éthyl-pyrimidine, 200 ml de diméthylacétamide et 5 ml de diisopropyléthylamine et porte à 100°C durant 3,5 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'heptane 100 % jusqu'à heptane-acétate d'éthyle 50-50. On obtient 1,5 g (Rdt = 58 %) de produit attendu sous forme d'un solide beige.

CCM : Rf = 0,5 (alumine, éluant : heptane-acétate d'éthyle 50-50)

**IR (CHCl3) :** 3440 (NH); 1596, 1586, 1547, 1508, 1480 cm-1 (Hétérocycle+Aromatique)

**1H-RMN (DMSO-d6) :** δ 1,23 (t, 3H, $CH_2$-$CH_3$); 1,75 (m, 6H, $CH_2$-$CH_2$-$CH_2$-NH, $CH_2$-CH-$CH_2$ ); 2,67 (m, 5H, $CH_2$-$CH_3$, $CH_2$-CH-$CH_2$, $CH_2$-$CH_2$-$CH_2$-NH); 3,02 (t, 2H, $CH_2$-$CH_2$-N); 3,23 (m, 2H, $CH_2$-$CH_2$-$CH_2$-NH); 3,90 (d, 2H, $CH_2$-$CH_2$-N); 6,20 (s, 1H, $CH_2$-$CH_2$-$CH_2$-NH); 6,30 et 7,04 (2d, 2H, H naphthyridine); 8,27 ppm (s,N=CH-N).

**SM :** 403 (MH+).

Synthèsedu3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

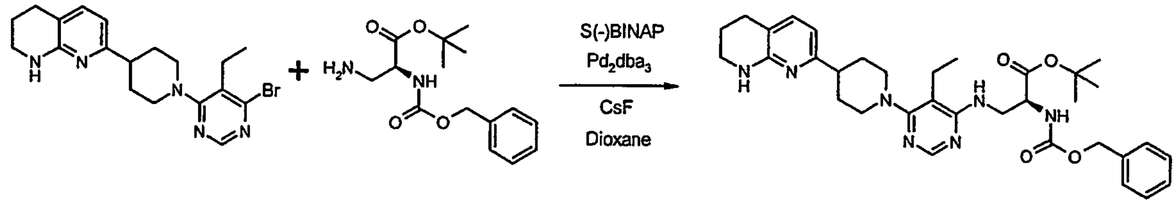


On chauffe au reflux un mélange de 317 mg (0,79 mmoles) de 4-bromo-5-éthyl-6-[4-(1,2,3,4-tétrahydro-1,8-naphthyridin-7-yl)-1-pipéridinyl]-pyrimidine, de 279 mg (0,95 mmoles) de 3-amino-N-[(phénylméthoxy)carbonyl]alaninate de (1,1-diméthyléthyle) (préparé selon J. Med. Chem. (2001), 44(8), 1158-1176), de 168 mg (1,11 mmoles) de fluorure de césium, de 36 mg (0,04 mmoles) de tris(dibenzylidèneacétone)dipalladium(0), de 49 mg (0,08 mmoles) de S(-)2,2'-bis(diphényl-phosphino)-1,1'-binaphtyl dans 11 ml de dioxane pendant 21 heures. On refroidit puis ajoute à nouveau 140 g (0,48 moles) de 3-amino-N-[(phénylméthoxy)carbonyllalaninate de (1,1-diméthyléthyle), 168 mg (1,11 mmoles) de fluorure de césium, 36 mg (0,04 mmoles) de tris(dibenzylidèneacétone)dipalladium(0),et 49 mg (0,08 mmoles) de S(-)2,2'-bis(diphényl-phosphino)-1,1'-binaphtyl dans 2 ml de dioxane et chauffe au reflux pendant 3,5 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'heptane-acétate d'éthyle-methanol 50-50-0, 25-75-0, 0-100-0, et enfin 0-100-3. On obtient 400 mg (Rdt = 83%) de produit attendu sous forme d'huile.

**CCM :** Rf = 0,55 (silicagel, éluant : acétate d'éthyleméthanol-triéthylamine 95-2-2). **IR (CHCl3) :** 3435 (NH); 1717 (C=O); 1583,1555,1501 cm-1 (Hétérocycle+Aromatique+Amide).

**1H-RMN (DMSO-d6) :** δ 1,06 (t, 3H, $CH_2$-$CH_3$); 1,31 (s, 9H, tBu); 1,75 (m, 2H, $CH_2$-$CH_2$-$CH_2$-NH); 1,77 (m, 4H, $CH_2$-CH-$CH_2$); 2,46 (m, 2H, $CH_2$-$CH_3$); 2,53 (m, 1H, $CH_2$-CH-$CH_2$); 2,62 (t, 2H, $CH_2$-$CH_2$-$CH_2$-NH); 2,83 et 3,44 (2m, 4H, $CH_2$-$CH_2$-N-$CH_2$-$CH_2$); 3,24 (m, 2H, $CH_2$-$CH_2$-$CH_2$-NH); 3,72 (m, 2H, NH-$CH_2$-CH-NH); 4,23 (q, 1H, NH-$CH_2$-CH-NH); 5,02 (s, 2H, $CH_2$-Ph); 6,24 (s, 1H, $CH_2$-$CH_2$-$CH_2$-NH); 6,30 et 7,06 (2d, 2H, H naphthyridine); 6,36 (m, 1H, NH-$CH_2$-CH-NH); 7,34 (m, 5H, Ph); 7,62 (d, 1H, NH-$CH_2$-CH-NH); 8,11 ppm (s,N=CH-N).

**SM :** 616 (MH+); 560 (MH-tBu+); 426 (MH-COOCH2Ph+).

**[$\alpha_D$] (1 % CHCl3) :** +2.4°

**[$\alpha_D$] (1,05 % EtOH) :** -6,1°

Synthèse de la 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacétate).

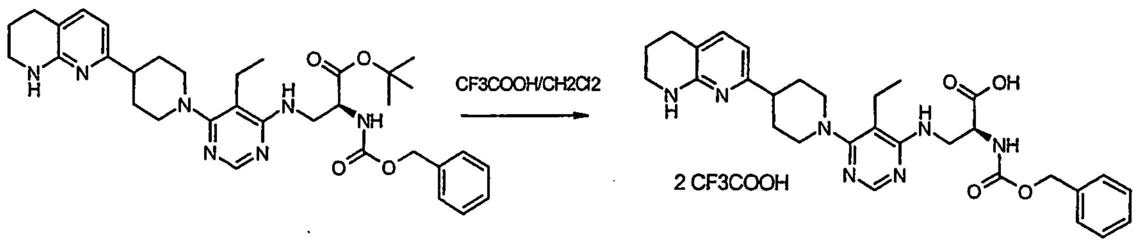


On agite 475 mg (0,77 mmoles) de 3-[[5-éthyl-6-[4-(1,2,3,4-tétrahydro-1,8-naphthyridin-7-yl)-1-pipéridinyl]-4-pyrimidinyl]aminol-N-[(phénylméthoxy)carbonyl]alaninate de (1,1-diméthyléthyle) dans 15 ml de dichlorométhane avec 3,5 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 368 mg (Rdt = 61 % exprimé en ditrifluoroacétate) de produit attendu sous forme d'un solide amorphe.

**CCM :** Rf = 0,33 (silicagel, éluant : dichlorométhane-méthanol-eau-acide acétique 90-10-1-1)

**IR ($CHCl_3$) :** 1677 (C=O); 1625; 1587; 1492 cm-1 (Système conjugué+Aromatique).

**1H-RMN (DMSO-d6) :** δ 1,04 (t, 3H, CH2-CH3); 1,77 et 1,90 (2m, 4H, N-CH2-CH2-CH-); 1,81 (m, 2H, NH-CH2-CH2-CH2-); 2,45 (q, 2H, CH2-CH3); 2,73 (t, 2H, NH-CH2-CH2-CH2-); 2,78 (t, 1H, N-CH2-CH2-CH-); 2,87 et 3,48 (2m, 4H, N-CH2-CH2-CH-); 3,23 (m, 2H, N-CH2-CH2-CH2-); 3,58 et 3,83 (2m, 2H, NH-CH2-CH-NH); 4,28 (q, 1H, NH-CH2-CH-NH); 5,01 (syst AB, 2H, O-CH2-Ph); 6,65 (d, 1H, H naphthyridine); 6,55 (sl, 1H, NH-CH2-CH-NH); 7,33 (m, 5H, Ph); 7,68 (d, 1H, NH-CH2-CH-NH); 7,58 (d, 1H, H naphthyridine); 8,15 (s, 1H, N=CH-N); 6,24 ppm (sl, 1H, NH-CH2-CH2-CH2-).

**SM :** 560 (MH+); 426 (MH-COOCH2Ph+); 558- (M-H-); 450- (558-OCH2Ph-); 1117 - (2M-H-)

**[$\alpha_D$] (1 % CHCl3)** :+2,4°

## Exemple 10

N-[(phénylméthoxy)carbonyl]alaninate de (1,1 diméthyléthyle)

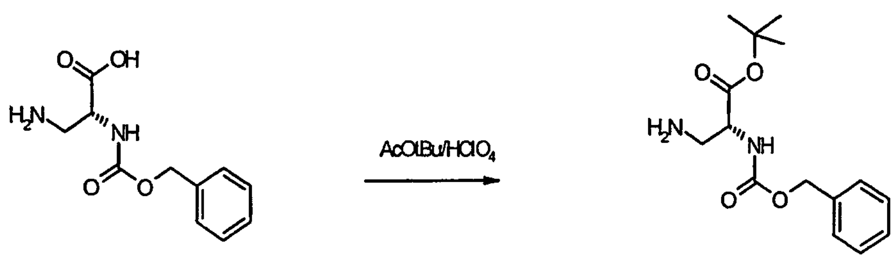


A 480 mg (2 mmoles) d'acide N-[(phénylméthoxy)carbonyl] alanique dans 15 ml d'acétate de terbutyle on ajoute goutte à goutte 1 ml d'acide perchlorique. Le mélange biphasique est agité 24 heures à température ambiante. Le mélange est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'heptane-acétate d'éthyle-méthanol 100-0-0, 0-100-0, et enfin 0-95-05. On obtient 50 mg (Rdt = 10 %) de produit attendu sous forme d'huile.

**CCM :** Rf = 0,20 (silicagel, éluant : dichlorométhane-méthanol-eau-acide acétique 90-10-1-1)

**[$\alpha_D$] (1,25 % CHCl3) :** -9,0°

Synthèsedu3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate d'(1,1 diméthyléthyle).

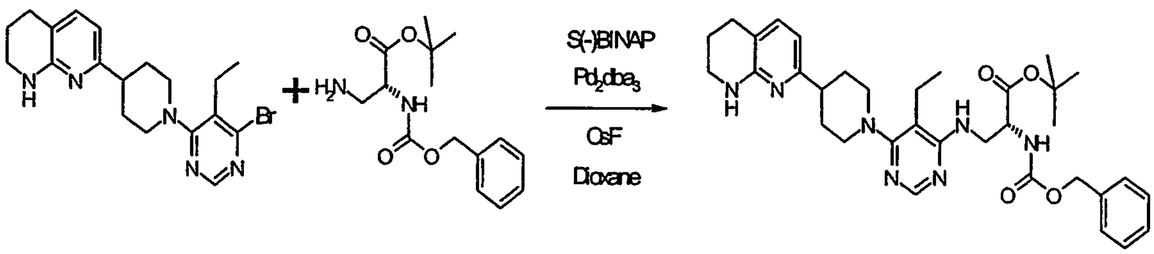


On chauffe au reflux un mélange de 200 mg (0,5 mmole) de 4-bromo-5-éthyl-6-[4-(1,2,3,4-tétrahydro-1,8-naphthyridin-7-yl)-1-pipéridinyl]-pyrimidine, de 50 mg (0,17 mmole) de (D) 3-amino-N-[(phénylméthoxy)carbonyl]alaninate d'(1,1 diméthyléthyle),de 40 mg (0,26 mmole) de fluorure de césium, de 15 mg (0,016 mmole) de tris(dibenzylidèneacétone)dipalladium(0), de 20 mg (0,032 mmole) de 2,2'-bis(diphényl-phosphino)-1,1'-binaphtyl dans 20 ml de dioxane pendant 5 heures. On refroidit puis ajoute à nouveau 15 mg (0,016 mmole) de tris(dibenzylidèneacétone)dipalladium (0) et chauffe au reflux pendant 15 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié une première fois sur alumine en éluant avec un gradient d'acétate d'éthyle-ether diisopropylique-chlorure de méthylène 50-50-50, 10-50-50, et enfin 10-20-70 puis sur silicagel en éluant avec de l'acétate d'éthyle. On obtient 40 mg (Rdt = 38 %) de produit attendu sous forme d'huile.

**CCM :** Rf = 0,25 (silicagel, éluant : acétate d'éthyle).

**SM :** 616 (MH+) ; 560 (MH-tBu+).

[$\alpha_D$] (1,0 % **CHCl3) :** -2,4

Synthèse de la 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

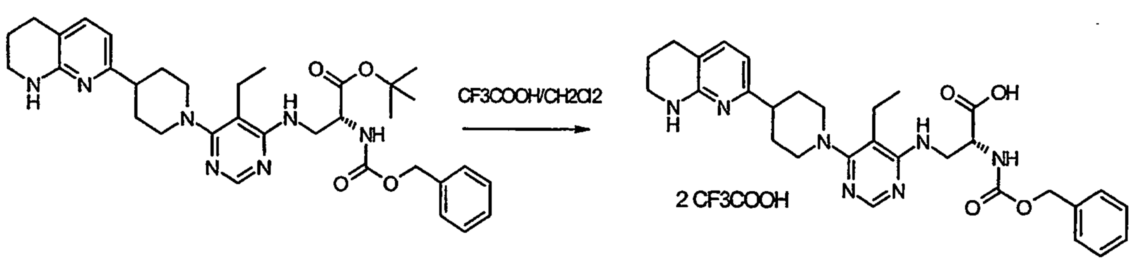


On agite 40 mg (0,77 mmoles) de 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle) dans 10 ml de dichlorométhane avec 1 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est chromatographié sur silicagel en éluant avec un mélange CH2Cl2-MeOH-H2O-AcOH 90-10-1-1. On obtient 25 mg (Rdt = 48 % exprimé en ditrifluoroacétate) de produit attendu sous forme d'un solide amorphe.

**CCM :** Rf = 0,10 (silicagel, éluant : dichlorométhane-méthanol-eau-acide acétique 90-10-1-1)

**SM :** 560 (MH+); 426 (MH-COOCH2Ph+); 558- (M-H-); 450- (558-OCH2Ph-); 1117- (2M-H-)

**[$\alpha_D$] (1,75 % CHCl3) :** -6,0

**Exemple 11**

3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate d'éthyle, dichlorhydrate

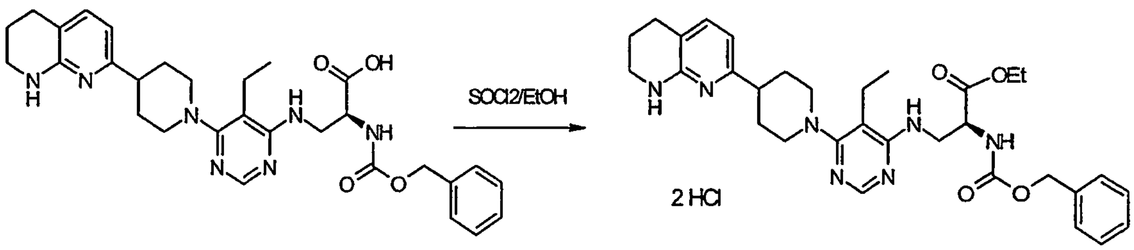


A 5 ml d'éthanol refroidi à -12°C sous atmosphère d'azote on ajoute 160 µl (2,19 mmoles) de chlorure de thionyle et agite le mélange pendant 30 minutes. On additionne alors une solution de 354 mg (0,45 mmoles) de 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis (trifluoroacetate) dans 13 ml d'éthanol. Le mélange est agité 30 minutes à -12°C puis on laisse revenir à température ambiante et enfin chauffe à 40°C pendant 4 heures sous azote. On évapore le mélange réactionnel à sec sous pression réduite (2 kPa) et cristallise le résidu dans un mélange d'éther diisopropylique et de pentane et filtre le solide. On obtient 284 mg (Rdt = 95 %) de produit attendu sous forme d'un solide beige amorphe.

**CCM :** Rf = 0,4 (silicagel, éluant : dichlorométhane-méthanol-eau-acide acétique 90-10-1-1)

**IR ($CHCl_3$)** : 3400-3000(OH, NH); 1719(C=O); 1654; 1623; 1580; 1504 cm-1(C=C, C=N, aromatique)

**1H-RMN (DMSO-d6) :** δ 1,09 (t, 3H, CH2-CH3); 1,14 (t, 3H, O-CH2-CH3); 1,85 (m, 2H, NH-CH2-CH2-CH2); 1,85 et 2,00 (2m, 4H, N-CH2-CH2-CH); 2,48 (m, 2H, CH2-CH3); 2,75 (t, 2H, NH-CH2-CH2-CH2); 2,92 (m, 1H, N-CH2-CH2-CH); 3,03 et 3,57 (2m, 4H, N-CH2-CH2-CH); 3,44 (t, 2H, N-CH2-CH2-CH2); 3,76 et 3,87 (2m, 2H, NH-CH2-CH-NH); 4,07 (q, 2H, O-CH2-CH3); 4,39 (q, 1H, NH-CH2-CH-NH); 5,03 (s, 2H, O-CH2-Ph); 6,62 (d, 1H, H naphthyridine); 6,86 (sl, 1H, NH-CH2-CH-NH); 7,30 (m, 5H, Ph); 7,33 (s1, 1H, NH-CH2-CH-NH); 7,58 (d, 1H, H naphthyridine); 8,20 ppm (s, 1H, N=CH-N).

**SM :** 588 (MH+) ; 454 (MH-COOCH2Ph+); 426 (454-C2H4+).

**[αD] (1 % CHCl3) :** +4

**Exemple 12**

N-[(phénylméthoxy)carbonyl]alaninate d'éthyle

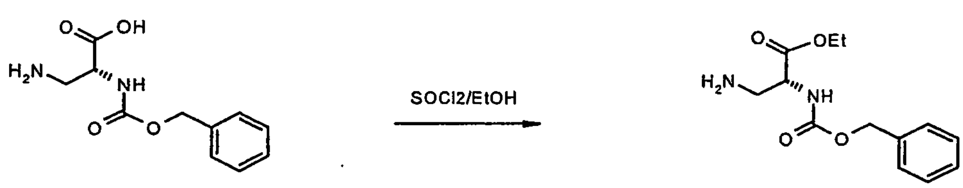


A 12 ml d'éthanol refroidi à 0°C sous atmosphère d'azote on ajoute 6 ml (63 mmoles) de chlorure de thionyle et agite le mélange pendant 20 minutes à température ambiante. On additionne alors une solution de 950 mg (4 mmoles) d'acide N-[(phénylméthoxy)carbonyl]alanique dans 5 ml d'éthanol. Le mélange est agité au reflux pendant 1 heure sous azote. On évapore le mélange réactionnel à sec sous pression réduite (2 kPa) et cristallise le résidu dans un mélange d'éther diisopropylique et de pentane et filtre le solide. Le solide obtenu est repris par de l'acétate d'éthyle, de l'eau et une solution saturée de bicarbonate de sodium. On obtient 535 mg (Rdt = 50 %) de produit attendu sous forme d'une huile incolore.

**CCM :** Rf = 0,4 (silicagel, éluant : dichlorométhane-méthanol-eau-acide acétique 90-10-1-1)

**HPLC/SM :** (tr = 0,5 et 1,4 min) 267 (MH+).
**[αD] (1,0 % CHCl3) :** -11,2

Synthèsedu3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate d'éthyle.

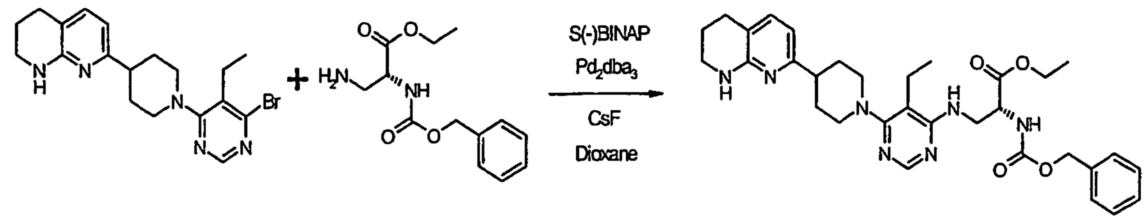


On chauffe au reflux un mélange de 320 mg (0,8 mmole) de 4-bromo-5-éthyl-6-[4-(1,2,3,4-tétrahydro-1,8-naphthyridin-7-yl)-1-pipéridinyl]-pyrimidine, de 215 mg (1,04 mmole) de (D) 3-amino-N-[(phénylméthoxy)carbonyl]alaninate d'éthyle, de 190 mg (1,25 mmoles) de fluorure de césium, de 37 mg (0,04 mmole) de tris (dibenzylidèneacétone) dipalladium(0), de 50 mg (0,08 mmole) de S(-)2,2'-bis(diphényl-phosphino)-1,1'-binaphtyl dans 15 ml de dioxane pendant 5 heures. On refroidit puis ajoute à nouveau 37 mg (0,04 mmole) de tris(dibenzylidèneacétone)dipalladium(0) et chauffe au reflux pendant 7 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un gradient d'acétate d'éthyle-ether diisopropylique-chlorure de méthylène 50-50-50, 10-50-50, et enfin 10-20-70. On obtient 260 mg (Rdt = 55%) de produit attendu sous forme d'huile.
**CCM :** Rf = 0,25 (silicagel, éluant : acétate d'éthyle).
**1H-RMN (CDCl3) :** δ 1,16 (t, 3H, CH2-CH3); 1,27 (t, 3H, O-CH2-CH3); 1,85 à 2,00 (m, 6H, N-CH2-CH2-CH et NH-CH2-CH2-CH2); 2,46 (m, 2H, CH2-CH3); 2,75 (t, 3H, NH-CH2-CH2-CH2 et
N-CH2-CH2-CH); 3,89 (t, 2H, N-CH2-CH2-CH2); 3,47 et 3,61 (2m, 4H, N-CH2-CH2-CH); 3,00 et 3,98 (2m, 2H, NH-CH2-CH-NH); 4,22 (m, 2H, O-CH2-CH3); 4,54 (m, 1H, NH-CH2-CH-NH); 5,13 (s, 2H, O-CH2-Ph); 6,43 (d, 1H, H naphthyridine); 7,22 (d, 1H, H naphthyridine); 7,35 (m, 5H, Ph); 8,27 ppm (s, 1H, N=CH-N).
**SM :** 588 (MH+); 454 (MH-COOCH2Ph+).
**[αD] (1,3 % CHCl3) :** -6,0

## Exemple 13

Synthèse du de 6-[4-(1,2,3,4-tetrahydro-1,8-naphthyridin-7-yl)-1-pipéridinyl]-5-éthyl-4-chloro-pyrimidine :

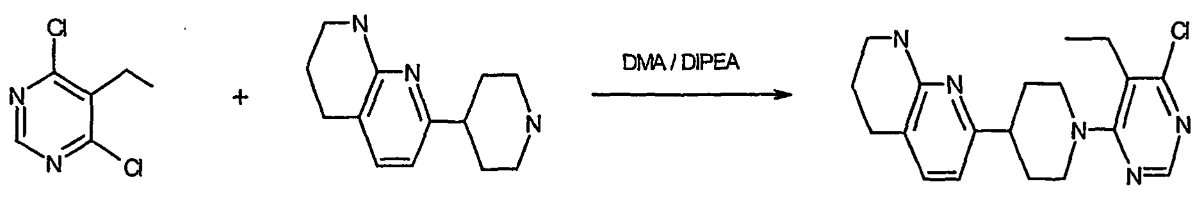


Dans un monocol contenant 12,5 g (57,5 mmoles) de 4-(1,2,3,4-tetrahydro-1,8-naphthyridin-7-yl)-1-pipéridine libérée de son sel, on ajoute 80 ml diméthylacétamide, 11,8 g (30 mmoles) de 4,6-dichloro-5-éthyl-pyrimidine mis en solution dans 40 ml de diméthylacétamide et 24 ml de diisopropyléthylamine. Ce mélange est chauffé à 140°C pendant 5 heures puis concentré à sec sous vide. Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant évaporé

sous vide pour donner 15,5 g d'une huile orangée. Cette huile est solubilisée dans un minimum de chlorure de méthylène et précipitée dans l'éther isopropylique, on obtient 9 g du produit attendu sous la forme d'une poudre beige. Le filtrat est concentré à sec puis chromatographié sur silicagel en éluant avec un gradient de cyclohexane (100 %), d'acétate d'éthyle-cyclohexane (50-50) puis d'acétate d'éthyle-cyclohexane (70-30).

On obtient 3 g de produit attendu sous la forme d'une poudre jaune pâle. (Rdt global = 58 %)

Préparation de la naphtyridine sous forme d'amine libre :

28,5 g de naphtyridine est déplacée de son sel par 6 équivalents massiques de résine basique amberlyst A21 (résine de type R-$NMe_2$) dans un mélange $CH_2Cl_2$ / MeOH /AcOEt 1/1/1 sous agitation pendant 30 minutes. On lave préalablement la résine et on la laisse gonfler 20 minutes dans ce mélange de solvant.

Cette opération doit être répétée 3 fois pour que le déplacement du sel soit total.

Après filtration de la résine et évaporation des solvants, on obtient 12,5 g (57,5 mmoles) de naphtyridine libre.

**CCM :** Rf = 0,32 [silicagel, éluant : acétate d'éthyle (100 %)]

**1H-RMN ()** : δ 1,32 (t, 3H, <u>CH3</u>-CH2); 1,81 à 2,05 (m, 6H, NH-CH2-<u>CH2</u>-CH2, N-CH2-<u>CH2</u>-CH-<u>CH2</u>); 2,70 (m, 5H, CH2-<u>CH</u>-CH2, NH-CH2-CH2-<u>CH2</u>, CH3-<u>CH2</u>); 3,43 (m, 2H, NH-CH2-CH2-<u>CH2</u>); 3.05 et 3,97 (2m, 4H, CH2-<u>CH2</u>-N-<u>CH2</u>-CH2); 6,4 et 7,12 (2d, 2H, <u>CH</u>=<u>CH</u> naphthyridine); 8,37 (s, 1H, N=<u>CH</u>-N).

**HPLC / SM :** (tr = 0,51 min et 2,93 min): 358 (MH+)

Synthèsedu3-[[6-[4-(1,2,3,4-tétrahydro-1,8-naphthyridin-7-yl)-1-pipéridinyl]-5-éthyl-4-pyrimidinyl]amino]-N-[(phénylméthoxy)carbonyl]alaninate d'isopropyle.

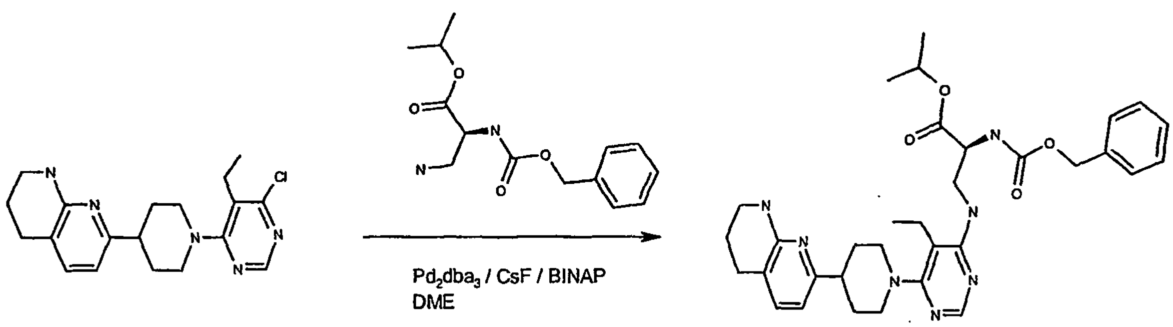


Un mélange de 1 g (2,79 mmoles) de 6-[4-(1,2,3,4-tetrahydro-1,8-naphthyridin-7-yl)-1-pipéridinyl]-5-éthyl-4-chloro-pyrimidine et de 940 mg (3,35.mmoles) de 3-amino-N-[(phénylméthoxy)carbonyl] alaninate d'isopropyle, en présence de 594 mg (3,91 mmoles) de fluorure de cesium, de 174 mg (279 μmoles) de (2,2'-bis(diphénylphosphino)-1,1'-binaphthyl et de 130 mg (140 μmoles) de tris(dibenzylidèneacétone) dipalladium(o) dans 35 ml de 1,2-diméthoxyéthane est chauffé au reflux pendant 5 heures. Le mélange réactionnel est ensuite ramené à température pour l'ajout de 180 mg (140 μmoles) de tris(dibenzylidèneacétone)dipalladium(o), puis on porte de nouveau au reflux pendant 16 heures.

Après refroidissement la solution est concentrée à sec puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous vide. Le résidu est chromatographié sur silicagel par l'acétate d'éthyle 100 %. On obtient 1,07 g de produit attendu sous la forme de cristaux jaune pâle (rendement = 64 %)

**CCM :** Rf = 0.20 (silicagel éluant : acétate d'éthyle 100 %

**1H-RMN (CDCl3) :** δ 1,18 (t, 3H, <u>CH3</u>-CH2); 1.25 (d, 6H, CH3-CH-CH3); 1,83 à 2,05 (m, 6H, NH-CH2-<u>CH2</u>-CH2, N-CH2-<u>CH2</u>-CH-<u>CH2</u>); 2,45 (q, 2H, CH3-<u>CH2</u>); 2,72 (m, 3H, CH2-<u>CH</u>-CH2, NH-CH2-CH2-<u>CH2</u>); 2,97 et 3,93 (2m, 4H, CH2-<u>CH2</u>-N-<u>CH2</u>-CH2); 3,45 (m, 2H, NH-<u>CH2</u>-CH2-CH2); 3,60 (d, 2H, NH-<u>CH2</u>-CH-NH), 4,50 (m, 1H, NH-CH2-<u>CH</u>-NH); 4,97 à 5,20 (m, 3H, CH3-<u>CH</u>-CH3, O-<u>CH2</u>-Ph); 6,43 et 7,18 (2d, 2H, <u>CH</u>=<u>CH</u> naphthyridine); 7,35 (m, 5H, Ph); 8,28 (s, 1H, N=<u>CH</u>-N).

**HPLC/SM :** (tr = 0,50 min et 3,14 min) : 602 (MH+); 468 [MH - Z+]; 426 [MH - (ipr - Z) +]

**$[\alpha]_D$ (CHCl3) =** + 2,175

Formation du Chlorhydrate :

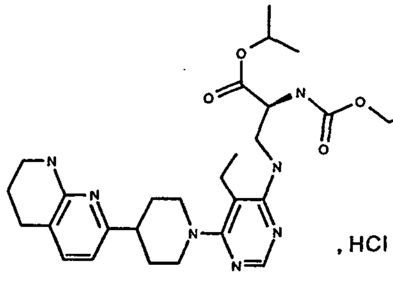


M W =638.22
M F =C33H43N7O4,HCl

3,2 g (5.3 mmoles) de 3-[[6-[4-(1,2,3,4-tetrahydro-1,8-naphthyridin-7-yl)-1-pipéridinyl]-5-éthyl-4-pyrimidinyl] amino]-N-[(phénylméthoxy)carbonyl]alaninate d'isopropyle sont solubilisés dans un minimum de chlorure de méthylène. Cette solution est coulée sur de l'éther éthylique, la solution doit rester limpide. On ajoute ensuite goutte à goutte, sous agitation, 2,65 ml d'HCl 2N dans l'éther éthylique. Le chlorhydrate prend en masse, on ôte le surnageant, puis le résidu est recristallisé dans l'éther isopropylique. On obtient alors un solide qui est filtrée, rincée à l'éther puis au pentane. Après séchage, on obtient 3,5 g d'une poudre blanc-cassé. (Rendement = quantitatif)

## Exemple 14

Synthèse du 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-4-methylpentanoate de terbutyle.

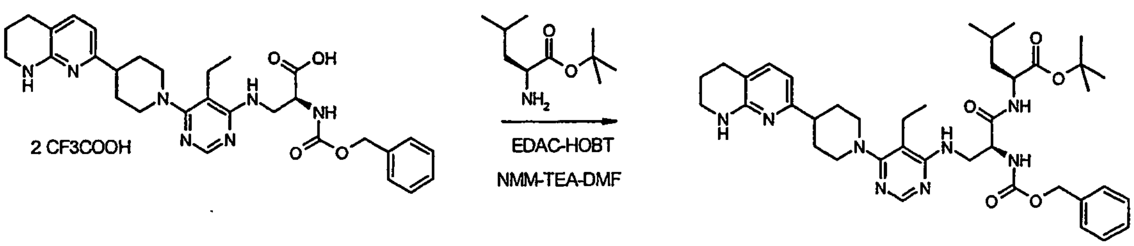


On agite 80 mg (0,15 mmole) de 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, 45 mg (0,20 mmole) de chlorhydrate de L-leucinate de terbutyle, 22 mg (0,16 mmole) de 1-hydoxy benzotriazole, 30 mg (0,16 mmole) de chlorhydrate de 1-[3-(diméthylamino) propyl]-3-ethyl carbodiimide, 0,050 ml (0,45 mmole) de N-methhylmorpholine et de 0,070 ml (0,50 mmole) de triéthylamine dans 5 ml de diméthylformamide pendant 24 heures à température ambiante. Le mélange réactionnel est évaporé à sec sous pression réduite (2 kPa) et le résidu est repris par de l'acétate d'éthyle et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'acétate d'éthyle-chlorure de méthylène-méthanol de 50-50-0 à 50-45-5. On obtient 60 mg (Rdt = 55 %) de produit attendu sous forme d'un solide blanc.

**CCM :** Rf = 0,66 (silicagel, éluant : dichlorométhane-méthanol-eau-acide acétique 85-15-2-2)

**1H-RMN (CDCl3) :** δ 0,91 (t, 3H, CH2-CH3); 1,16 (d, 6H, CHCH2CH(CH3)2); 1,28 (tl, 2H, CHCH2CH(CH3) 2); 1,41 à 1,57 (m, 1H, CHCH2CH(CH3)2); 1,48 (s, 9H, tBu); 1,80 à 2,05 (m, 6H, $CH_2$-$CH_2$-$CH_2$-NH, $CH_2$-CH-$CH_2$); 2,49 (m, 2H, $CH_2$-$CH_3$); 2,67 (tt, 1H, $CH_2$-CH-$CH_2$); 2,73 (t, 2H, $CH_2$-$CH_2$-$CH_2$-NH); 2,98 et 3,66 (ql et m, 4H, $CH_2$-$CH_2$-N-$CH_2$-$CH_2$); 3,43 (m, 2H, $CH_2$-$CH_2$-$CH_2$-NH); 3,70 et 4,16 (2m, 2H, NH-$CH_2$-CH-NH); 4,32 (m, 1H, NH-$CH_2$-CH-NH); 4,49 (m, 1H, CONH-CHCH2CH(CH3)2)-CO); 5,13 (m, 2H, $CH_2$-Ph); 5,42, 6,83 et 7,92 (3H, Hmobiles); 6,43 et 7,15 (2d, 2H, H naphthyridine); 7,37 (m, 5H, Ph); 8,2 ppm (s, 1H, N=CH-N).

**SM :** 729 (MH+).

Synthèse de l'acide 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-4-méthylpentanoique.

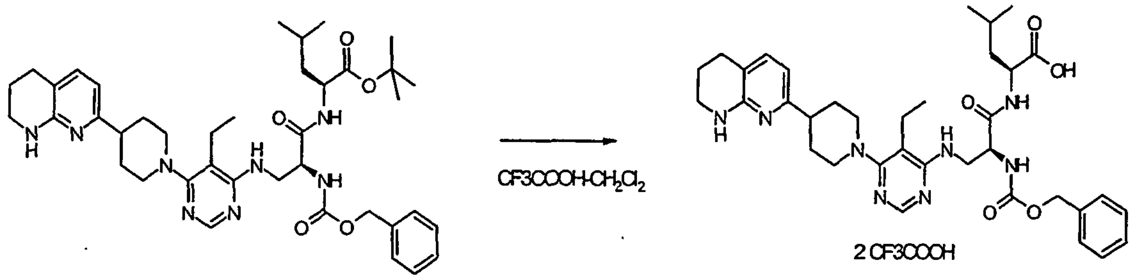


On agite 60 mg (0,083 mmoles) de 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-4-methylpentanoate de terbutyle dans 5 ml de dichlorométhane avec 0,5 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 55 mg (Rdt = 74 % exprimé en ditrifluoroacétate) de produit attendu sous forme d'un solide blanc.

**CCM :** Rf = 0,52 (silicagel, éluant : dichlorométhane-méthanol-eau-acide acétique 85-15-2-2)

**SM :** 673 (MH+).

**Exemple 15**

Synthèse de la 4,6-dihydroxy-5-propyl-pyrimidine.

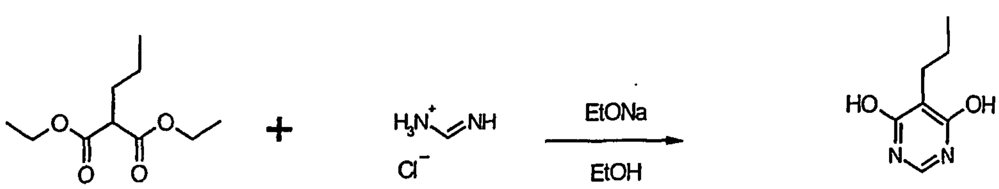


A une solution de 7,5 g (94 mmoles) de chlorhydrate de formamidine dans 200 ml d'éthanol refroidie à 0°C on ajoute 102 ml (282 mmoles) d'une solution d'éthylate de sodium à 21 % dans l'éthanol et agite le mélange pendant 30 minutes; on additionne alors une solution de 19,5 ml (94 mmoles) de propyl malonate de diéthyle dans 50 ml d'éthanol et agite une nuit à température ambiante. On évapore le mélange réactionnel à sec sous pression réduite (2 kPa). Le résidu est repris par 100 ml d'une solution saturée en chlorure de sodium et extrait par 800 et 200 ml de n-butanol. les phases organiques sont séchées sur sulfate de sodium, filtrées et évaporées à sec sous pression réduite (2 kPa). On obtient 7,3 g (Rdt = 50 %) de produit attendu sous forme d'une solide beige.

**1H-RMN (DMSO d6) :** $\delta$ 0,85(t, 3H,$CH_2$-$CH_2$-$\underline{CH_3}$); 1,39(sext, 2H, $CH_2$-$\underline{CH_2}$-$CH_3$); 2,23(t, 2H, $\underline{CH_2}$-$CH_2$-$CH_3$ ); 7,85 ppm (s,N=CH-N).

**SM :** 155 (MH+); 159 (M-H-).

Synthèse de la 4,6-dichloro-5-propyl-pyrimidine

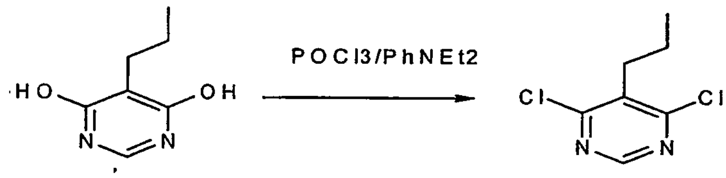


Un mélange de 3 g (19,5 mmoles) de 5-propyl-4,6-dihydroxy-pyrimidine dans 20 ml d'oxychlorure de phosphore est porté au reflux durant 1 heure. Après retour à température ambiante, on ajoute goutte à goutte un mélange de 3 ml de N,N-diéthylaniline dans 10 ml d'oxychlorure de phosphore et porte le tout au reflux pendant 4 heures. Après retour à température ambiante, le mélange réactionnel est versé sur un mélange de glace et d'eau puis on ajoute doucement du bicarbonate de sodium jusqu'à pH basique. On extrait à l'acétate d'éthyle, sèche sur sulfate de magnésium et évapore à sec sous pression réduite (2 kPa). Le résidu est chromatographié sur alumine en éluant avec un gradient d'heptane-chlorure de méthylène 100-0 à 80-20. On obtient 2 g (Rdt = 54 %) de produit attendu sous forme d'un solide bleuté.
**CCM :** Rf = 0,35 (silicagel, éluant : heptane-acétate d'éthyle 90-10).

Synthèse de la 6-[4-(1,2,3,4-tetrahydro-1,8-naphthyridin-7-yl)-1-pipéridinyl]-5-propyl-4-chloro-pyrimidine :

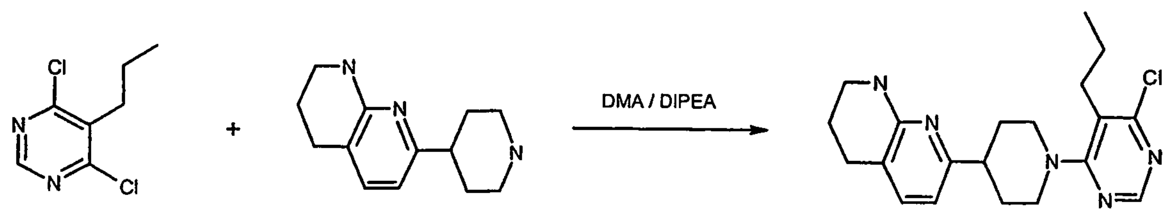


Dans un monocol contenant 1,6 g (7,4 mmoles) de 4-(1,2,3,4-tetrahydro-1,8-naphthyridin-7-yl)-1-pipéridine libérée de son sel, on ajoute 50 ml diméthylacétamide, 2 g (10,5 mmoles) de 4,6-dichloro-5-propyl-pyrimidine et 4 ml de diisopropyléthylamine. Ce mélange est chauffé à 140°C pendant 5 heures puis concentré à sec sous pression réduite (2 kPa). Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant évaporé sous pression réduite (2 kPa). Le résidu est chromatographié sur silicagel en éluant avec un gradient d'heptane-chlorure de méthylène- acétate d'éthyle de 50-50-0 à 0-0-100. On obtient 875mg de produit attendu sous la forme d'un solide amorphe. (Rdt = 50 %)
**CCM :** Rf = 0,25 [silicagel, éluant : acétate d'éthyle (100 %)]
**1H-RMN (CDCl3) :** δ 1,02(t, 3H,C-$CH_2$-$CH_2$-$CH_3$); 1,72(sext, 2H, C-$CH_2$-$CH_2$-$CH_3$); 1,81 à 2,07 (m, 6H, NH-CH2-CH2-CH2, N-CH2-CH2-CH-CH2); 2,64(t, 2H, C-$CH_2$-$CH_2$-$CH_3$ ); 2,73 (m, 3H, CH2-CH-CH2, NH-CH2-CH2-CH2); 3,46 (t, 2H, NH-CH2-CH2-CH2); 3,06 et 3,96 (m et d, 4H, CH2-CH2-N-CH2-CH2); 5,00 (m, 2H, NH-CH2-CH2-CH2); 6,40 et 7,14 (2d, 2H, CH=CH naphthyridine); 8,38 (s, 1H, N=CH-N).
**SM :** 372,374 (MH+).

Synthèse du 3-[[5-propyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

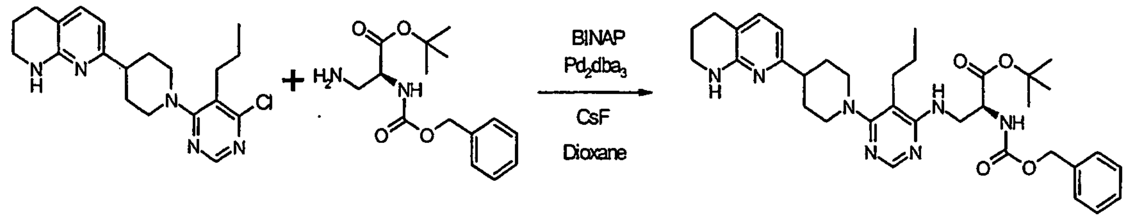


On chauffe au reflux un mélange de 600 mg (1,62 mmoles) de 4-chloro-5-propyl-6-[4-(1,2,3,4-tétrahydro-1,8-naphthyridin-7-yl)-1-pipéridinyl]-pyrimidine, de 590mg (2 mmoles) de 3-amino-N-[(phénylméthoxy)carbonyl]alaninate de (1,1-diméthyléthyle) (préparé selon J. Med. Chem.(2001), 44(8), 1158-1176), de 380 mg (2,51 mmoles) de fluorure de césium, de 76 mg (0,083 mmole) de tris(dibenzylidèneacétone)dipalladium(0), de 102 mg (0,163 mmole) de 2,2'-bis (diphényl-phosphino)-1,1'-binaphtyl dans 50 ml de dioxane pendant 20 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un gradient de chlorure de méthylène-ether diisopropylique- acétate d'éthyle-methanol de 50-50-0-0 à 0-50-50-0 puis 0-0-100-0 et enfin 0-0-90-10. On obtient 620 mg (Rdt = 61 %) de produit attendu sous forme de solide amorphe jaune.

**CCM :** Rf = 0,12 (silicagel, éluant : acétate d'éthyle).

**1H-RMN (CDCl3) :** δ 0,90(t, 2H, C-$CH_2$-$CH_2$-$CH_3$); 1,01(t, 3H,C-$CH_2$-$CH_2$-$CH_3$); 1,48 (s, 9H, tBu); 1,81 à 2,07 (m, 6H, NH-CH2-CH2-CH2, N-CH2-CH2-CH-CH2); 2,39(t, 2H, C-$CH_2$-$CH_2$-$CH_3$ ); 2,65 (tl, 1H, CH2-CH-CH2); 2,73 (t, 2H, NH-CH2-CH2-CH2); 2,97 et 3,55 (tl et dl, 4H, CH2-CH2-N-CH2-CH2); 3,44 (m, 2H, NH-CH2-CH2-CH2); 3,85 et 3,93 (2m, 2H, NH-$CH_2$-CH-NH); 4,46 (m, 1H, NH-$CH_2$-CH-NH); 5,14 (s, 2H, $CH_2$-Ph); 6,16 (d, 1H $CH_2$-$CH_2$-$CH_2$-NH); 6,44 (d, 1H, H naphthyridine); 7,15 (d, 1H, H naphthyridine), 7,37 (m, 5H, CH2Ph); 8,29 (s, 1H, N=CH-N);

**SM :** 630 (MH+); 574 (MH-tBu+); 440 (MH-COOCH2Ph+).

Synthèsedela3-[[5-propyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

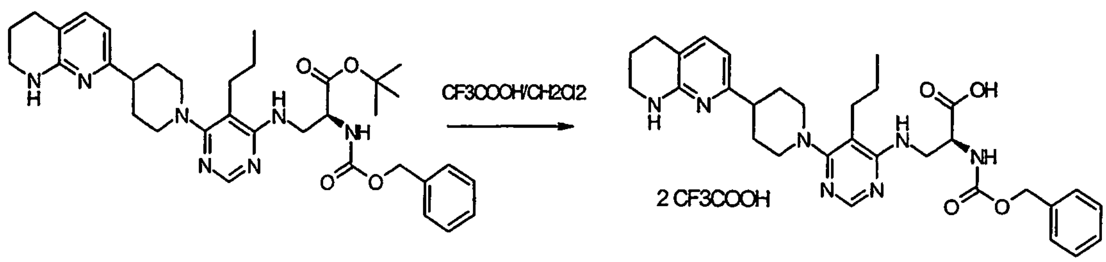


On agite 610 mg (0,97 mmole) de 3-[[5-propyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle) dans 50 ml de dichlorométhane avec 5 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 400 mg (Rdt = 51 % exprimé en ditrifluoroacétate) de produit attendu sous forme d'un solide beige.

**CCM :** Rf = 0,40 (silicagel, éluant : dichlorométhane-méthanol-eau-acide acétique 85-15-2-2)

**1H-RMN (DMSO d6) :** δ 0,93(t, 3H,C-$CH_2$-$CH_2$-$CH_3$); 1,48(m, 2H, C-$CH_2$-$CH_2$-$CH_3$); 1,67 à 2,00(m, 6H, $CH_2$-$CH_2$-$CH_2$-NH et $CH_2$-CH-$CH_2$); 2,40(t, 2H, C-$CH_2$-$CH_2$-$CH_3$); 2,65 à 2,96 (m, 5H, NH-CH2-CH2-CH2-, N-CH2-CH2-CH-, N-CH2-CH2-CH-); 3,32 à 3,51 (m, 4H, N-CH2-CH2-CH- et NH-CH2-CH2-CH2-); 3,56 et 3,84 (2m, 2H, NH-CH2-CH-NH); 4,31 (q, 1H, NH-CH2-CH-NH); 5,02 (s, 2H, O-CH2-Ph); 6,68 (d, 1H, H naphthyridine); 7,33 (m, 5H, Ph); 7,61 (m, 1H, H naphthyridine); 8,20 (s, 1H, N=CH-N).

**SM :** 574 (MH+); 440 (MH-COOCH2Ph+); 572- (M-H-); 464- (572-OCH2Ph-); 1145- (2M-H-)

**[$\alpha_D$]** (1,20 **% CHCl3) :** -17,7.

**Exemple 16**

Synthèse de la 4,6-dihydroxy-5-isobutyl-pyrimidine.

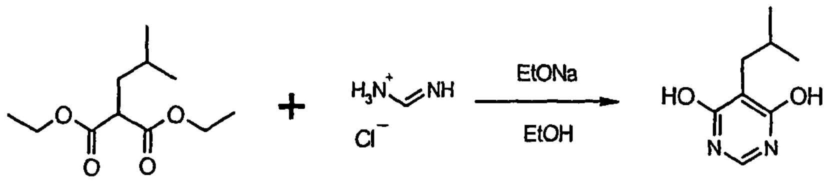


A une solution de 7,5 g (94 mmoles) de chlorhydrate de formamidine dans 200 ml d'éthanol refroidie à 0°C on ajoute 102 ml (282 mmoles) d'une solution d'éthylate de sodium à 21 % dans l'éthanol et agite le mélange pendant 30 minutes; on additionne alors une solution de 20,6 ml (94 mmoles) de propyl malonate de diéthyle dans 50 ml d'éthanol et agite une nuit à température ambiante. On évapore le mélange réactionnel à sec sous pression réduite (2 kPa). Le résidu est repris par 100 ml d'une solution saturée en chlorure de sodium et extrait par 800 et 200 ml de n-butanol. les phases organiques sont séchées sur sulfate de sodium, filtrées et évaporées à sec sous pression réduite (2 kPa). On obtient 13,7 g (Rdt = 86 %) de produit attendu sous forme d'une solide beige.

**CCM :** Rf = 0,53 (silicagel, éluant : dichlorométhane-méthanol-eau-acide acétique 85-15-2-2)

**1H-RMN (DMSO d6) :** δ 0,81(d, 6H,$CH_2$-CH-$(CH_3)_2$); 1,85 (m, 1H, -$CH_2$-CH-$(CH_3)_2$); 2, 13 (d, 2H, -$CH_2$-CH-$(CH_3)_2$); 7, 81 ppm (s,N=CH-N).

**SM :** 169 (MH+) ; 167 (M-H-).

Synthèse de la 4,6-dichloro-5-isobutyl-pyrimidine

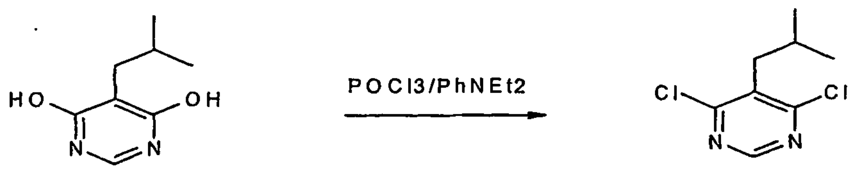


Un mélange de 3 g (17,9 mmoles) de 5-isobutyl-4,6-dihydroxy-pyrimidine dans 20 ml d'oxychlorure de phosphore est porté au reflux durant 1 heure. Après retour à température ambiante, on ajoute goutte à goutte un mélange de 3 ml de N,N-diéthylaniline dans 10 ml d'oxychlorure de phosphore et porte le tout au reflux pendant 4 heures. Après retour à température ambiante, le mélange réactionnel est versé sur un mélange de glace et d'eau puis on ajoute doucement du bicarbonate de sodium jusqu'à pH basique. On extrait à l'acétate d'éthyle, sèche sur sulfate de magnésium et évapore à sec sous pression réduite (2 kPa). Le résidu est chromatographié sur alumine en éluant avec un gradient d'heptane-chlorure de méthylène 100-0 à 80-20. On obtient 0,9 g (Rdt = 25 %) d'une huile marron.

**CCM :** Rf = 0,25 (silicagel, éluant : heptane-acétate d'éthyle 95-5).

Synthèse du de 6-[4-(1,2,3,4-tetrahydro-1,8-naphthyridin-7-yl)-1-pipéridinyl]-5-isobutyl-4-chloro-pyrimidine :

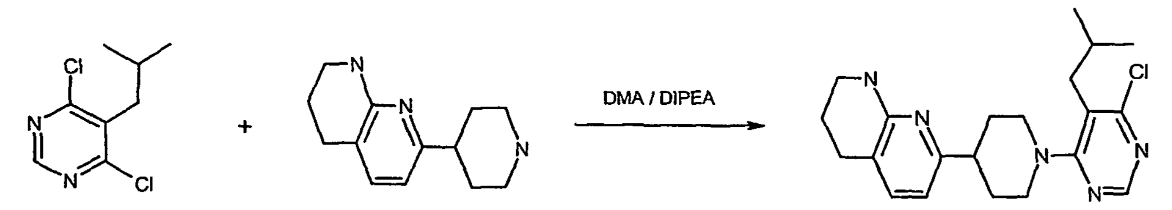


Dans un monocol contenant 0,95 g (4,4 mmoles) de 4-(1,2,3,4-tetrahydro-1,8-naphthyridin-7-yl)-1-pipéridine libérée de son sel, on ajoute 25 ml diméthylacétamide, 0,9 g (4,4 mmoles) de 4,6-dichloro-5-isobutyl-pyrimidine et 2 ml de diisopropyléthylamine. Ce mélange est chauffé à 120°C pendant 5 heures puis concentré à sec sous pression réduite (2 kPa). Le résidu obtenu est repris par un mélange d'eau, d'acétate d'éthyle et d'une solution de bicarbonate de sodium saturée. La phase organique est séparée et la phase acqueuse réextraite par de l'acétate d'éthyle. Les phases organiques regroupées sont séchées sur sulfate de magnésium puis le solvant évaporé sous pression réduite (2 kPa). Le résidu est chromatographié sur silicagel en éluant avec un gradient d'heptane-chlorure de méthylène- acétate d'éthyle de 50-50-0 à 0-0-100. On obtient 1 g de produit attendu sous la forme d'un solide beige. (Rdt = 60 %)

**CCM :** Rf = 0,25 [silicagel, éluant : acétate d'éthyle (100 %)]

**1H-RMN (CDCl3) :** δ 0,89(d, 6H,$CH_2$-CH-$(CH_3)_2$); 1,77 à 2,12 (m, 7H, NH-CH2-CH2-CH2, N-CH2-CH2-CH-CH2 et $CH_2$-CH-$(CH_3)_2$); 2,66 (d, 2H, -$CH_2$-CH-$(CH_3)_2$); 2,73 (t, 2H, NH-CH2-CH2-CH2); 2,61 à 2,77 (1H, masqué, CH2-CH-CH2); 3,44 (m, 2H, NH-CH2-CH2-CH2); 3,03 et 3,87 (t et d, 4H, CH2-CH2-N-CH2-CH2); 5,11 (m, 2H, NH-CH2-CH2-CH2); 6,41 et 7,15 (2d, 2H, CH=CH naphthyridine); 8,40 (s, 1H, N=CH-N).

**SM :** 386,388 (MH+).

Synthèsedu3-[[5-isobutyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

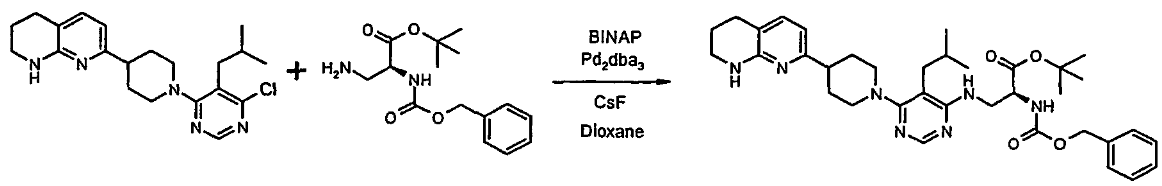


.

On chauffe au reflux un mélange de 450 mg (1,17 mmoles) de 4-chloro-5-isobutyl-6-[4-(1,2,3,4-tétrahydro-1,8-naphthyridin-7-yl)-1-pipéridinyl]-pyrimidine, de 413mg (1,4 mmoles) de 3-amino-N-[(phénylméthoxy)carbonyl]alaninate de (1,1-diméthyléthyle) (préparé selon J. Med. Chem.(2001), 44(8), 1158-1176), de 275 mg (1,81 mmoles) de fluorure de césium, de 55 mg (0,060 mmole) de tris (dibenzylidèneacétone)dipalladium(0), de 75 mg (0,12 mmole) de 2,2'-bis(diphényl-phosphino)-1,1'-binaphtyl dans 35 ml de dioxane pendant 20 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un gradient de chlorure de méthylène-ether diisopropylique- acétate d'éthyle-methanol de 50-50-0-0 à 0-50-50-0 puis 0-0-100-0 et enfin 0-0-90-10. On obtient 630 mg (Rdt = 83 %) de produit attendu sous forme de solide amorphe jaune.

**CCM :** Rf = 0,12 (silicagel, éluant : acétate d'éthyle).

**1H-RMN (CDCl3) :** δ 0,91(d, 6H,$CH_2$-CH-$(CH_3)_2$); 1,49 (s, 9H, tBu); 1,77 à 2,07 (m, 7H, -$CH_2$-CH-$(CH_3)_2$), NH-CH2-CH2-CH2 et N-CH2-CH2-CH-CH2); 2,32(d, 2H, -$CH_2$-CH-$(CH_3)_2$); 2,65 (tl, 1H, CH2-CH-CH2) ; 2,73 (t, 2H, NH-CH2-CH2-CH2) ; 2,95 (tl, 2H CH2-CH2-N-CH2-CH2); 3,44 (m 4H, CH2-CH2-N-CH2-CH2 et NH-CH2-CH2-CH2); 3,85 et 3,92 (2m, 2H, NH-$CH_2$-CH-NH) ; 4,45 (m, 1H, NH-$CH_2$-CH-NH); 5,14 (s, 2H, $CH_2$-Ph); 6,12 (d, 1H $CH_2$-$CH_2$-$CH_2$-NH); 6,43 (d, 1H, H naphthyridine); 7,16 (d, 1H, H naphthyridine), 7,37 (m, 5H, CH2Ph); 8,31 (s, 1H, N=CH-N);

**SM :** 644 (MH+); 588 (MH-tBu+); 454 (MH-COOCH2Ph+).

Synthèse de la 3-[[5-isobutyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacétate).

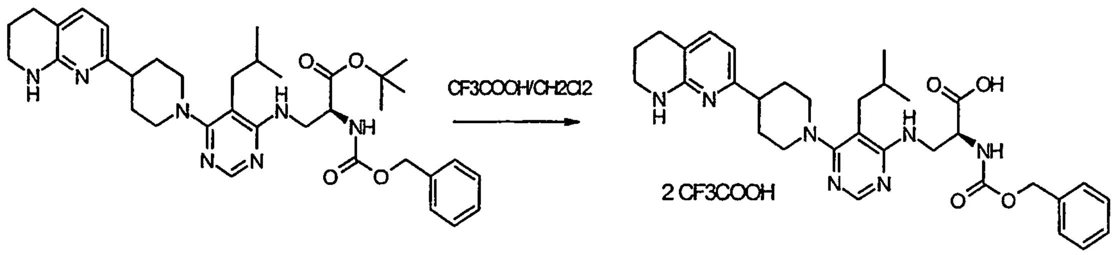


On agite 620 mg (0,96 mmole) de 3-[[5-isobutyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle) dans 50 ml de dichlorométhane avec 5 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 525 mg (Rdt = 67 % exprimé en ditrifluoroacétate) de produit attendu sous forme d'un solide beige.

**CCM :** Rf = 0,40 (silicagel, éluant : dichlorométhane-méthanol-eau-acide acétique 85-15-2-2)

**1H-RMN (DMSO d6) :** 0,83(d, 6H, $CH_2$-CH-$(CH_3)_2$); 1,60 à 2,05 (m, 7H, -$CH_2$-CH-$(CH_3)_2$), NH-CH2-CH2-CH2 et N-CH2-CH2-CH-CH2); 2,35(m, 2H, -$CH_2$-CH-$(CH_3)_2$); 2,65 à 2,87 (m, 3H, CH2-CH-CH2 et NH-CH2-CH2-CH2); 2,91 (tl, 2H CH2-CH2-N-CH2-CH2); 3,42 (m 4H, CH2-CH2-N-CH2-CH2 et NH-CH2-CH2-CH2); 3,57 et 3,84 (2m, 2H, NH-$CH_2$-CH-NH); 4,32 (m, 1H, NH-$CH_2$-CH-NH); 5,0 (s, 2H, $CH_2$-Ph); 6,68 (d, 1H, H naphthyridine); 7,37 (m, 5H, CH2Ph); 7,66 (d, 1H, H naphthyridine); 7,59 (d, 1H $CH_2$-$CH_2$-$CH_2$-NH); 8,27 (s, 1H, N=CH-N).

**SM :** 588 (MH+); 454 (MH-COOCH2Ph+); 586- (M-H-); 478- (586-OCH2Ph-); 1173- (2M-H-)

**[$\alpha_D$] (0,75 % CHCl3)** : +1,0.

**Exemple 17**

Synthèse de la 5-méthoxy-4,6-dihydroxy-pyrimidine :

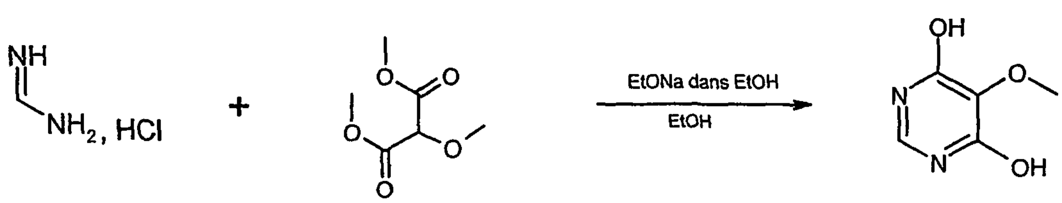


Dans un monocol contenant 30 ml d'éthanol absolu et 90 ml d'une solution d'éthylate de sodium de concentration à 1,7 mol/l dans l'éthanol, on ajoute à 0°C et par petites quantités 5 g (6,2 mmoles)de chlorhydrate de formamidine. On laisse agiter à température ambiante pendant une vingtaine de minutes, puis on ajoute goutte à goutte 8,5 ml de méthoxymalonate de diméthyle. On maintient l'agitation pendant 16 heures. Le mélange réactionnel est ensuite acidifié avec de l'acide acétique pur jusqu'à un pH entre 4 et 5, puis concentré à sec en présence de cyclohexane. Le brut obtenu est chromatographié sur silicagel après empâtage en éluant avec un mélange de chlorure de méthylène-méthanol (85-15)et 2 % d'eau-acide acétique(1-1). On obtient 9.5 g du produit attendu contena t une faible quantité de sels d'acétate de sodium.

**CCM :** Rf = 0 [Silicagel, éluant : chlorure de méthylène-méthanol (85-15)et 2 % d'eau-acide acétique(1-1).

**1H-RMN (MeOD) :** $\delta$ 3,67 (s, CH3-O); 7,73 (s, 1H, N=CH-N)

2,00 (s, 3H, CH3-COO-)

**HPLC / SM :** (tr = 0,5 min)

Synthèse de la 4,6-dichloro-5-méthoxy-pyrimidine :

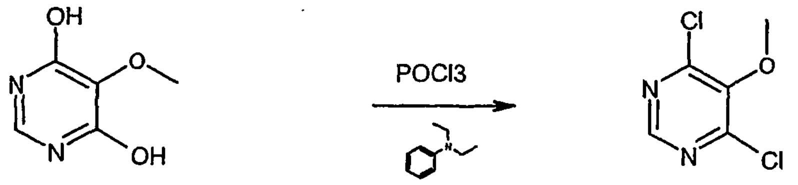


Un mélange de 5 g (35.2 mmoles) de 5-méthoxy-4,6-dihydroxypyrimidine et 40 ml d'oxychlorure de phosphore, est porté au reflux pendant une heure. Après retour à température ambiante, on ajoute goutte à goutte un mélange de 4.8 ml de N,N-diéthylaniline et 18 ml d'oxychlorure de phosphore.

L'ensemble est de nouveau porté au reflux pendant 4 heures 30 minutes. Après refroidissement, le mélange réactionnel est versé lentement sur un mélange de glace et d'eau puis neutralisé par une solution saturée de bicarbonate de sodium. Cette phase aqueuse est extraite à l'acétate d'éthyle. Les phases organiques regroupées sont séchées sur du sulfate de magnésium et évaporées à sec sous vide.

Le résidu obtenu est purifié sur silice en éluant avec un mélange de cyclohexane et d'acétate (95-5).

On obtient 2.5 g (Rdt = 40 %) de produit attendu sous forme d'une poudre blanche.

**CCM :** Rf = 0,48 [Silicagel, éluant : cyclohexane-acétate d'éthyle(80-20)]

**1H-RMN (MeOD)** : δ 4,00 (s, CH3-O); 8,55 (s, 1H, N=CH-N)

**HPLC/SM :** tr = 1,3 min

Synthèse de la 6-[4-(1,2,3,4-tetrahydro-1,8-naphthyridin-7-yl)-1-pipéridinyl]-5-méthoxy-4-chloro-pyrimidine :

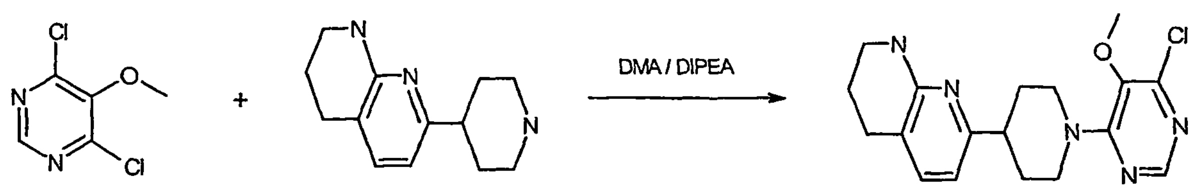


Dans un monocol contenant 800 mg (3,68 mmoles) de 4-(1,2,3,4-tetrahydro-1,8-naphthyridin-7-yl)-1-pipéridine libérée de son sel, on ajoute 2.2 g (12,3 mmoles) de 4,6-dichloro-5-méthoxy-pyrimidine mis en solution dans 25 ml de diméthylacétamide et 3640 µl de diisopropyléthylamine. Ce mélange est chauffé à 130°C pendant 2 heures puis concentré à sec sous vide. Le résidu obtenu est repris par un mélange d'eau, d'acétate d'éthyle et d'une solution de bicarbonate de sodium saturée. La phase organique est séparée et la phase acqueuse réextraite par de l'acétate d'éthyle. Les phases organiques regroupées sont séchées sur sulfate de magnésium puis le solvant évaporé sous vide. Le résidu est chromatographié sur alumine en éluant avec un mélange de cyclohexane et d'acétate (80-20). On obtient 900 mg (Rdt = 68 %) de produit attendu sous forme d'une poudre jaune.

Préparation de la naphtyridine sous forme d'amine libre :

2.4 g de naphtyridine est déplacée de son sel par 6 équivalents massiques de résine basique amberlyst A21 (résine de type R-$NMe_2$ ) dans un mélange $CH_2Cl_2$ / MeOH /AcOEt 1/1/1 sous agitation pendant 30 minutes. On lave préalablement la résine et on la laisse gonfler 20 minutes dans ce mélange de solvant. Cette opération doit être répétée 3 fois pour que le déplacement du sel soit total. Après filtration de la résine et évaporation des solvants, on obtient 800 mg (3.68 mmoles) de naphtyridine libre. (Rdt = 88 %).

**CCM :** Rf = 0,4 [alumine, éluant : acétate d'éthyle cyclohexane (30-70)]

**1H-RMN (MeOD) :** δ 1,75 à 1,95 (m, 6H, NH-CH2-CH2-CH2, N-CH2-CH2-CH-CH2); 2,70 (t, 1H, CH2-CH-CH2); 2,8 (m, 2H, NH-CH2-CH2-CH2); 3,15 et 3,75 (2m, 4H, CH2-CH2-N-CH2-CH2); 3,75 (s, CH3-O); 6,4 et 7,15 (2d, 2H, CH=CH naphthyridine); 8,1 (s, 1H, N=CH-N).

**HPLC / SM :** (tr = 0,53 min et 2,56 min): 359(M); 360(MH+); 361 (M+2H++).

Synthèsedu3-[[6-[4-(1,2,3,4-tetrahydro-1,8-naphthyridin-7-yl)-1-pipéridinyl]-5-méthoxy-4-pyrimidinyl]amino]-N-[(phénylméthoxy)carbonyl]alaninate de (1,1-diméthyléthyle).

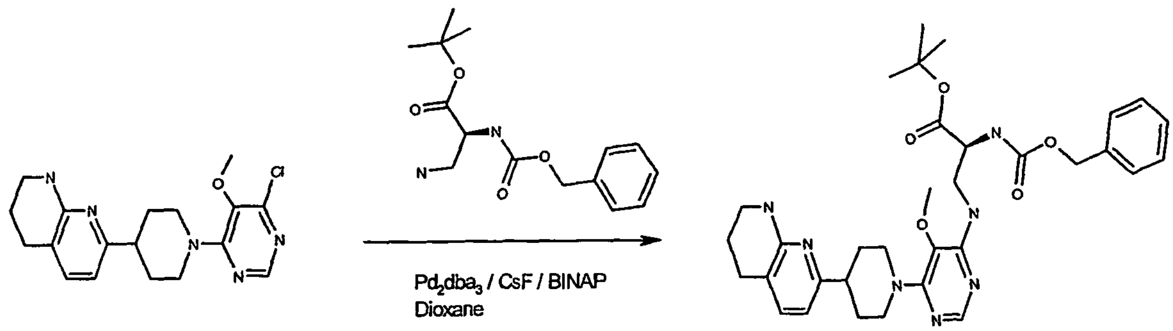


Un mélange de 300 mg (0, 83 mmoles) de 6-[4-(1,2,3,4-tetrahydro-1,8-naphthyridin-7-yl)-1-pipéridinyl]-5-méthoxy-4-chloro-pyrimidine et de 295 mg (1 mmole) de 3-amino-N-[(phénylméthoxy)carbonyl] alaninate de (1,1-diméthyléthyle) (préparé selon J. Med. Chem. (2001), 44(8), 1158-1176), ( en présence de 177 mg (1,17 mmoles) de fluorure de cesium, de 52 mg (83 µmoles) de (2,2'-bis(diphénylphosphino)-1,1'-binaphthyl et de 40 mg (42 µmoles) de tris-dibenzylidèneacétone)dipalladium(o), dans 10 ml de dioxane est chauffé au reflux pendant 3 heures 30 minutes. Le mélange réactionnel est ensuite ramené à température. On ajoute alors 0,5 mmoles de 3-amino-N-[(phénylméthoxy) carbonyl] alaninate de (1,1-diméthyléthyle) et 1.17 mmoles de fluorure de cesium, 83 µmoles de (2,2'-bis(diphénylphosphino)-1,1'-binaphthyl et 42 µmoles de tris-dibenzylidèneacétone)dipalladium(o), puis le mélange réactionnel est porté au reflux pendant encore 8 heures.

Après refroidissement la solution est concentrée à sec puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous vide. Le résidu est chromatographié sur alumine avec un gradient d'éther isopropylique et d'acétate d'éthyle (70-30, 60-40 , 50-50) en terminant par l'acétate d'éthyle 100 %. Les fractions contenant le produit attendu sont rassemblées pour une seconde purification sur silicagel avec un gradient d'acétate d'éthyle 100 %. On obtient le produit attendu sous la forme d'une huile jaune pâle qui est repris dans un mélange éther isopropylique et pentane pour donner 220 mg (Rendement = 43 %) d'une poudre blanche.

**CCM :** Rf = 0.3 (alumine éluant : éther isopropyliqueacétate d'éthyle 60-40)

**1H-RMN (MeOD) :** δ 1,46 (s, 9H, tBu); 1,80 (m, 2H, NH-CH2-CH2-CH2); 1,90 (m, 4H, N-CH2-CH2-CH-CH2); 2,65 (m, 1H, CH2-CH-CH2); 2,69 (t, 2H, NH-CH2-CH2-CH2); 2,95 et 3,78 (2m, 4H, CH2-CH2-N-CH2-CH2); 3,38 (t, 2H, NH-CH2-CH2-CH2); 3,59 (s, CH3-O); 4,35 (m, 1H, NH-CH2-CH-NH); 4.45 (d, 2H, NH-CH2-CH-NH); 5,03 et 5,12 (syst AB, 2H, O-CH2-Ph); 6,38 et 7,15 (2d, 2H, CH=CH naphthyridine); 7,32 (m, 5H, Ph); 7,90 (s, 1H, N=CH-N).

**HPLC/SM :** (tr = 3,4 min) : 618 (MH+) ;562 (MH -tBu); 428 [MH - (tBu-Z+)]

Synthèse de l'acide correspondant :

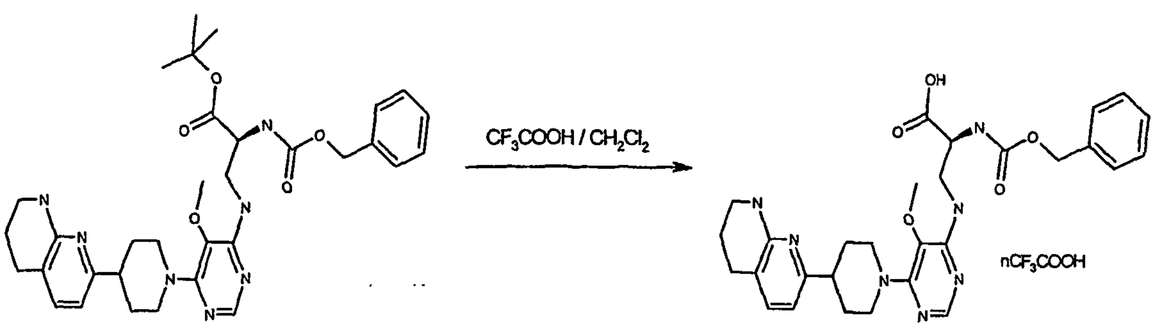


On agite à température ambiante un mélange de 200 mg (0,32 mmoles) de 3-[[5-methoxy-6-[4-(1,2,3,4-tetrahydro-1,8-naphthyridin-7-yl)-1-pipéridinyl]-5-méthyl-4-pyrimidinyl]amino]-N-[(phényl méthoxy)carbonyl]alaninate de (1,1-diméthyléthyle) et de 1.5 ml d'acide trifluoroacétique dans 15 ml de dichlorométhane pendant 8 heures. On additionne alors du toluène et évapore à sec le mélange. On obtient 350 mg d'une huile jaune, cette huile est repris dans un minimum de chlorure de méthylène et précipité dans l'éther isopropylique, on obtient ainsi le sel de trifuoroacétate impur. On renouvelle la précipitation dans l'éther jusqu'à purification (controlée en CCM).On obtient finalement 156 mg du produit attendu (Rdt = 71 %) sous la forme d'une poudre blanche

**CCM :** Rf = 0,4 (silicagel, éluant : dichlorométhane-méthanol-eau-acide acétique 90-10-1-1).

**1H-RMN (MeOD) :** δ 1,85 (m, 2H, NH-CH2-CH2-CH2); 2,00 (m, 4H, N-CH2-CH2-CH-CH2); 2,83 (t, 2H, NH-CH2-CH2-CH2); 3,90 (m, 1H, CH2-CH-CH2); 3,12 et 3.72 (2m, 4H, CH2-CH2-N-CH2-CH2); 3,52 (t, 2H, NH-CH2-CH2-CH2); 3.62 (s, 3H, CH3-O); 4,52 (m, 2H, NH-CH2-CH-NH); 4,60 (m, 1H, NH-CH2-CH-NH); 5.06 et 5,10 (syst AB, 2H, O-CH2-Ph); -6,65 et 7,62 (2d, 2H, CH=CH naphthyridine); 7,43 (m, 5H, Ph); 8,00 (s, 1H, N=CH-N).

**HPLC/SM :** (tr = 0,5 min et 2,75 min) : 562 (MH+); 428 [MH - (tBu-Z+)]; 428

**Exemple 18**

Synthèse de la 4,6-dibromo-5-fluoro-pyrimidine.

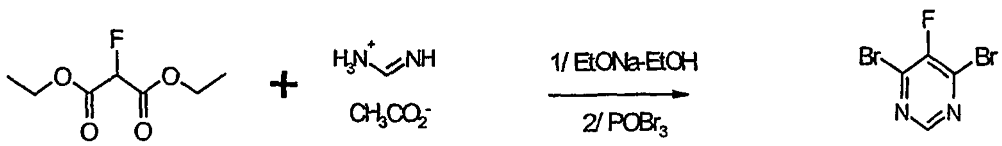


1/ A une solution de 5,83 g (56 mmoles) d'acétate de formamidine dans 300 ml d'éthanol refroidie à 0˚C on ajoute 60,7 ml (168 mmoles) d'une solution d'éthylate de sodium à 21 % dans l'éthanol et agite le mélange pendant 30 minutes; on additionne alors une solution de 10 g (56 mmoles) de fluoro malonate de diéthyle dans 25 ml d'éthanol et agite une nuit à température ambiante. Le mélange est refroidi à 0˚C puis on ajoute 17,85 ml d'acide chlorhydrique concentré pour amener le pH à 6. Le précipité est filtré puis lavé successivement par de l'eau, de l'isopropanol, de l'éther diéthylique et enfin du pentane. On obtient 14,2 g (théorie = 7,3 g) de produit attendu et de sels minéraux utilisé tel quel pour la suite.

2/ Un mélange de 14,2 g (56 mmoles) de 5-fluoro-4,6-dihydroxy-pyrimidine dans 21 g d'oxybromure de phosphore est porté à 200˚C durant 3 heures. Après retour à température ambiante, le mélange réactionnel est repris par un mélange d'eau glacée de bicarbonate de sodium et extrait à l'acétate d'éthyle, lave les phases organiques avec de l'eau, sèche sur sulfate de magnésium et évapore à sec sous pression réduite (2 kPa). Le résidu est chromatographié sur silicagel en éluant avec un gradient de chlorure de méthylène-heptane de 0-100 à 100-0. On obtient 850 mg (Rdt = 06 %) de produit attendu sous forme de solide beige utilisé tel quel pour la suite.

**CCM :** Rf = 0,50 (silicagel, éluant : dichlorométhane-heptane 50-50)

Synthèse 4-bromo-5-fluoro-6-[4-(1,2,3,4-tétrahydro-1,8-naphthyridin-7-yl)-1-pipéridinyl]-pyrimidine

Un mélange de 840 mg (1,5 mmoles) de 1,2,3,4-tétrahydro-7-(4-pipéridinyl)-1,8-naphthyridine, tris(trifluoroacetate) (préparé selon les brevets EP1065207 ou WO 0078317) et de 5 g de résine Amberlyst A-21 (origine Fluka 06424 préalablement lavée avec la solution dichlorométhane-acétate d'éthyle-méthanol 1-1-1) dans 100 ml de solution de dichlorométhane-acétate d'éthyle-méthanol 1-1-1 est agité à température ambiante durant 1 heure. Le mélange est filtré, la résine lavée avec la solutin ternaire. Le filtrat obtenu est agité à température ambiante durant 1 heure en présence de 5 g de résine Amberlyst A-21 traitée comme précédemment. Le filtrat ainsi obtenu est concentré à sec sous pression réduite (2 kPa) donnant 320 mg d'amine libre. On rajoute à ce résidu 384 mg (1,5 mmoles) de 4,6-dibromo-5-fluoro-pyrimidine, 6 ml de diméthylacétamide et 1,5 ml de diisopropyléthylamine et porte à 130˚C durant 1,5 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec heptane-acétate d'éthyle 50-50. On obtient 300 mg (Rdt = 52 %) de produit attendu sous forme d'un solide amorphe.

**CCM :** Rf = 0,15 (silicagel, éluant : heptane-acétate d'éthyle 50-50).

**IR (CHCl3) :** 3440 (NH); 1573, 1542, 1503 cm-1 (Hétérocycle).

**1H-RMN (CDCl3) :** δ 1,82, 1,92 et 2,02(dt, t et d 6H, $CH_2$-$CH_2$-$CH_2$-NH et $CH_2$-CH-$CH_2$); 2,72 (t, 2H, $CH_2$-$CH_2$-$CH_2$-NH); 2,80 (tl, 1H, $CH_2$-CH-$CH_2$); 3,13 et 4,68 (t1 et d, 4H, $CH_2$-$CH_2$-N-$CH_2$-$CH_2$); 3,42 (m, 2H,

$CH_2$-$CH_2$-$CH_2$-NH); 5,00 (m, 1H NH); 6,37 (d, 1H, naphthyridine); 7,14 (d, 1H, naphthyridine); 8,11 ppm (s,N=CH-N).

**SM :** 392 (MH+).

Synthèse du 3-[[5-fluoro-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

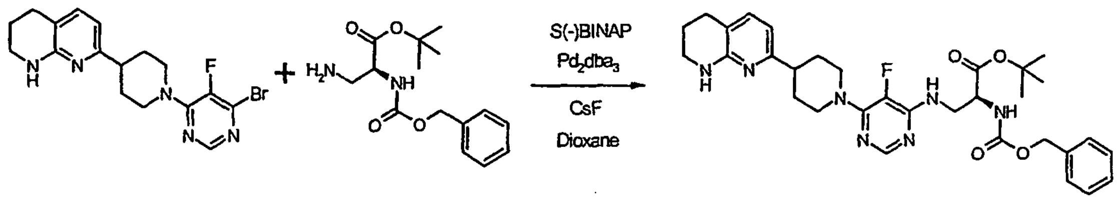


On chauffe au reflux un mélange de 280 mg (0,72 mmole) de 4-bromo-5-fluoro-6-[4-(1,2,3,4-tétrahydro-1,8-naphthyridin-7-yl)-1-pipéridinyl]-pyrimidine, de 253 mg (0,86 mmole) de 3-amino-N-[(phénylméthoxy)carbonyl]alaninate de (1,1-diméthyléthyle) (préparé selon J. Med. Chem.(2001), 44(8), 1158-1176), de 152 mg (1,00 mmole) de fluorure de césium, de 33 mg (0,036 mmole) de tris(dibenzylidèneacétone)dipalladium(0), de 45 mg (0,072 mmole) de 2,2'-bis (diphényl-phosphino)-1,1'-binaphtyl dans 50 ml de dioxane pendant 6 heures. On refroidit puis ajoute à nouveau 125 mg (0,43 mmole) de 3-amino-N-[(phénylméthoxy)carbonyl]alaninate de (1,1-diméthyléthyle), 152 mg (1,0 mmole) de fluorure de césium, 33 mg (0,036 mmole) de tris(dibenzylidèneacétone)dipalladium(0), et 45 mg (0,072 mmole) de 2,2'-bis(diphényl-phosphino)-1,1'-binaphtyl et chauffe au reflux pendant 4 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'heptane-acétate d'éthyle-methanol de 100-0-0 à 0-100-0 et enfin 0-95-5. Le produit obtenu est chromatographié une deuxième fois sur alumine en éluant avec un mélange d'heptane-chlorure de méthylène 50-50 puis à l'acétate d'éthyle-ether diisopropylique 50-50. On obtient 300 mg (Rdt = 70 %) de produit attendu sous forme d'une huile jaune.

**CCM :** Rf = 0,35 (silicagel, éluant : acétate d'éthyle.

**IR (CHCl3)** : 3439 (NH); 1718 (C=O); 1609,1503 cm-1 (Hétérocycle+Aromatique+Amide).

**1H-RMN (CDCl3) :** δ 1,47 (s, 9H, tBu); 1,70 à 2,05(m, 6H, $CH_2$-$CH_2$-$CH_2$-NH et $CH_2$-CH-$CH_2$); 2,73 (t, 2H, $CH_2$-$CH_2$-$CH_2$-NH); 2,80 (tl, 1H, $CH_2$-CH-$CH_2$); 3,01 et 3,90 (tl et m, 4H, $CH_2$-$CH_2$-N-$CH_2$-$CH_2$); 3,43 (m, 2H, $CH_2$-$CH_2$-$CH_2$-NH); 4,42 (m, 1H, NH-$CH_2$-CH-NH); 4,51 (dl, 2H, NH-$CH_2$-CH-NH); 5,11 (m, 1H NH); 5,14 (s, 2H, $CH_2$-Ph); 6,12 (m, 1H $CH_2$-$CH_2$-$CH_2$-NH ); 6,37 (d, 1H, naphthyridine); 7,16 (d, 1H, naphthyridine); 7,35 (m, 5H, Ph); 7,98 ppm (s,N=CH-N).

**SM :** 606 (MH+); 550 (MH-tBu+); 416 (550-COOCH2Ph+), 604-[(M-H)]-, 650- [(604+HCOOH)]-, 496- [(604-OCH2Ph)]-.

**[$\alpha_D$] (1,0 % CHCl3) :** +4,4

Synthèsedela3-[[5-fluoro-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

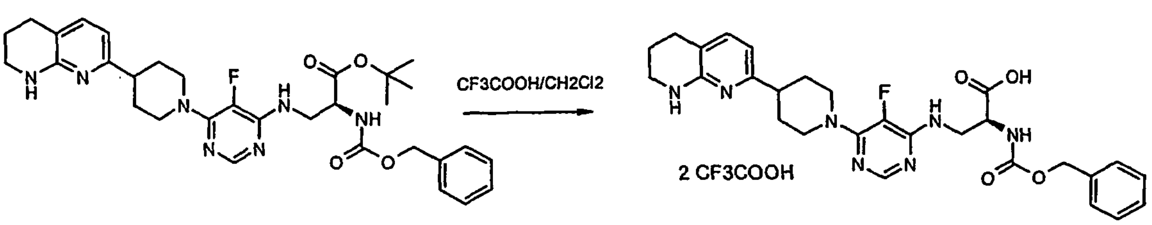


On agite 290 mg (0,48 mmole) de 3-[[5-fluoro-6-[4-(1,2,3,4-tétrahydro-1,8-naphthyridin-7-yl)-1-pipéridinyl]-4-pyrimidinyl]amino]-N-[(phénylméthoxy)carbonyl] alaninate de (1,1-diméthyléthyle) dans 40 ml de dichlorométhane avec

4 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 310 mg (Rdt=83% exprimé en ditrifluoroacétate) de produit attendu sous forme d'un solide blanc.

**CCM :** Rf = 0,44 (silicagel, éluant : dichlorométhane-méthanol-eau-acide acétique 90-10-1-1)

**SM :** 550 (MH+); 416 (MH-COOCH2Ph+); 548- (M-H-); 440- (548-OCH2Ph-); 1097- (2M-H-)

**[$\alpha_D$] (0,30 % MeOH) :** -9,3.

**Exemple 19**

Synthèse du 3-[5-éthyl-6-[4-(1,2,3,4-tétrahydro-1,8-naphthyridin-7-yl)-1-pipéridinyl]-4-pyrimidinyl]amino]alaninate de (1,1-diméthyléthyle).

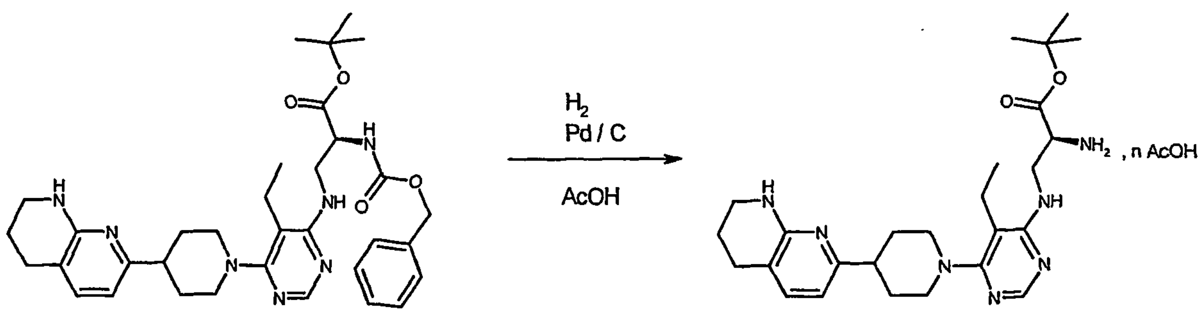


Dans un monocol contenant 3 g (4.87 mmoles) de 3-[[6-[4-(1,2,3,4-tetrahydro-1,8-naphthyridin-7-yl)-1-pipéridinyl]-5-éthyl-4-pyrimidinyl]amino]-N-[(phénylméthoxy) carbonyl]alanine, on charge 150 ml d'acide acétique 100 % et

300 mg de palladium activé sur charbon (5-10 %). Ce mélange est purgé sous pression réduite (2 kPa) puis on le laisse agiter à température ambiante et sous pression atmosphérique d'hydrogène pendant 22 heures.

Le milieu hétérogène obtenu est filtré sur clarcel. Le filtrat est concentré à sec puis repris par un mélange d'eau, d'acétate d'éthyle et d'une solution de bicarbonate de sodium saturée. La phase organique est extraite à l'acétate d'éthyle et séchée sur sulfate de magnésium puis le solvant est évaporé sous pression réduite (2 kPa). On obtient 1,95 g d'une huile jaune pâle.

CCM : Rf = 0,65 (silicagel, éluant : acétate d'éthyle-méthanol-triéthylamine 90-5-5)

**1H-RMN (CDCl3) :** $\delta$ 1,09 (t, 3H, $CH_2$-$CH_3$); 1,35 (s, 9H, tBu); 1,75 (m, 6H, $CH_2$-CH-$CH_2$ et $CH_2$-$CH_2$-$CH_2$-NH); 2,44 (m, 2H, $CH_2$-$CH_3$); 2,60 (t, 2H, $CH_2$-$CH_2$-$CH_2$-NH); 2,81 (m, 2H, $CH_2$-CH-$CH_2$ et NH-$CH_2$-CH-NH); 3,23 (m, 2H, $CH_2$-$CH_2$-$CH_2$-NH); 3,41 (m, 4H, $CH_2$-$CH_2$-N-$CH_2$-$CH_2$); 3,42 et 3,57 (2m, 2H, NH-$CH_2$-CH-NH); 6,25 (m, 2H, $CH_2$-$CH_2$-$CH_2$-NH et NH-$CH_2$-CH-NH); 6,30 et 7,04 (2d, 2H, H naphthyridine); 8,08 ppm (s, 1H, N=CH-N).

**IR (CHCl3) :** 3439 (NH); 1726 (C=O); 1580, 1502 cm-1 (heterocycle).

**SM :** $482^+$ $[MH]^+$; $426^+$ $[MH^+ - tBu]^+$; $339^+$ $[MH^+ - CH_2CH(NH_2)CO_2tBu]^+$; $241.7^+$ $[M+2H]^2$

**[$\alpha_D$] :** -3 (1 % CHCl3)

Synthèse du 3-[[5-éthyl-6-[4-(1,2,3,4-tétrahydro-1,8-naphthyridin-7-yl)-1-pipéridinyl]-4-pyrimidinyl]amino]-N-[(adamantylméthoxy)carbonyl]alaninate de (1,1-diméthyléthyle).

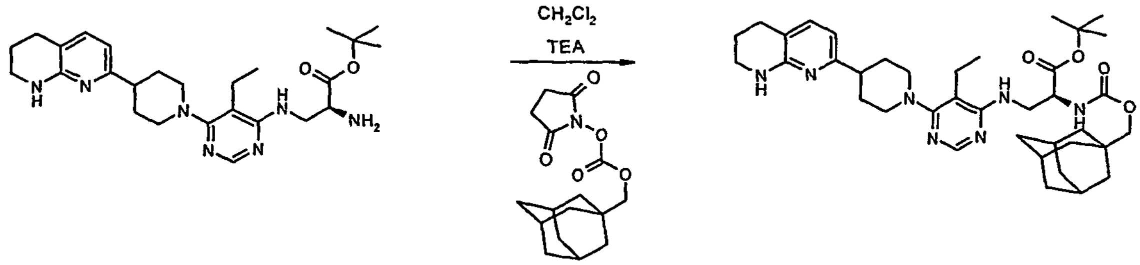


Un mélange de 239 mg (0,49 mmoles) de 3-[5-éthyl-6-[4-(1,2,3,4-tétrahydro-1,8-naphthyridin-7-yl)-1-pipéridinyl]-4-pyrimidinyl]amino] alaninate de (1,1-diméthyléthyle), 152 mg (0,49 mmoles) de N-adamantylméthoxycarbonyloxysuccinimide et de 0,104 ml (0,75 mmoles) de triéthylamine dans 50 ml de chlorure de méthylène est agité durant 5 heures à température ambiante. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient de chlorure de méthylène 100 % jusqu'à chlorure de méthylène-méthanol 90-10. On obtient 77 mg (Rdt = 17 %) de produit attendu sous forme d'un solide beige.

**CCM :** Rf = 0,45 (silicagel, éluant : chlorure de méthylène-méthanol-acide acétique-eau 90-10-1-1).

**IR (CHCl3) :** 3437 (NH); 1711 (C=O); 1583, 1556, 1501 cm-1 (Hétérocycle)

**1H-RMN (DMSO-d6) :** δ 1,08 (t, 3H, $CH_2$-$\underline{CH_3}$); 1,45 à 1,92 (m, 15H, adamantyl); 1,32 (s, 9H, tBu); 1,77 (m, 6H, $\underline{CH_2}$-CH-$\underline{CH_2}$ et $CH_2$-$\underline{CH_2}$-$CH_2$-NH); 2,44 (q, 2H, $\underline{CH_2}$-$CH_3$); 2,50 (m, 1H, $CH_2$-$\underline{CH}$-$CH_2$); 2,61 (t, 2H, $\underline{CH_2}$-$CH_2$-$CH_2$-NH); 2,82 et 3,44 (2m, 4H, $CH_2$-$\underline{CH_2}$-N-$\underline{CH_2}$-$CH_2$); 3,22 (m, 2H, $CH_2$-$CH_2$-$\underline{CH_2}$-NH); 3,56 (s, 2H, O-$\underline{CH_2}$adam); 3,66 (m, 2H, NH-$\underline{CH_2}$-CH-NH); 4,17 (q, 1H, NH-$CH_2$-$\underline{CH}$-NH); 6,24 (s, 1H, $CH_2$-$CH_2$-$CH_2$-$\underline{NH}$); 6,30 et 7,05 (2d, 2H, H naphthyridine); 6,37 (t, 1H, $\underline{NH}$-$CH_2$-CH-NH); 7,44 (d, 1H, NH-$CH_2$-CH-$\underline{NH}$); 8,10 ppm (s,N=$\underline{CH}$-N).

**SM :** 674 (MH+); 618 (MH-tBu+); 426 (618-COOCH2adam+); 337,7 (M2H++); 718- (M-H-HCOOH); 672- (M-H).

**[α]$_D$ :** +2,5 (1% CHCl3).

Synthèsedu ß-[[5-éthyl-6-[4-(1,2,3,4-tétrahydro-1,8-naphthyridin-7-yl)-1-pipéridinyl]-4-pyrimidinyl]amino]-N-[(adamantylméthoxy)carbonyl]alanine

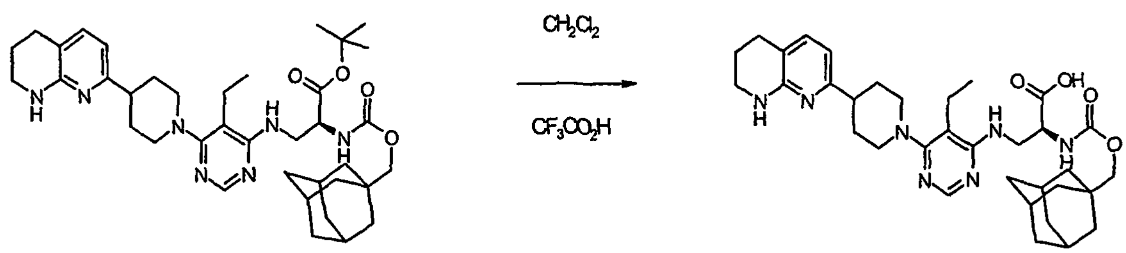


On agite 130 mg (0,19 mmoles) de 3-[[5-éthyl-6-[4-(1,2,3,4-tétrahydro-1,8-naphthyridin-7-yl)-1-pipéridinyl]-4-pyrimidinyl]amino]-N-[(adamantylméthoxy)carbonyl]alaninate de (1,1-diméthyléthyle) dans 20 ml de dichlorométhane avec 2 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 368 mg (Rdt = 61 % exprimé en ditrifluoroacétate) de produit attendu sous forme d'un solide amorphe.

**CCM :** Rf = 0,35 (silicagel, éluant : dichlorométhane-méthanol-eau-acide acétique 90-10-1-1)

**IR (CHCl3)** : 3401 (NH); 1668 (C=O); 1581, 1492 cm-1 (Hétérocycle)

**1H-RMN (DMSO-d6) :** δ 1,08 (t, 3H, $CH_2$-$\underline{CH_3}$); 1,45 à 1,92 (m, 15H, adamantyl); 1,76 (m, 6H, $\underline{CH_2}$-CH-$\underline{CH_2}$, $CH_2$-$\underline{CH_2}$-$CH_2$-NH); 2,43 (q, 2H, $\underline{CH_2}$-$CH_3$); 2,50 (m, 1H, $CH_2$-$\underline{CH}$-$CH_2$); 2,60 (t, 2H, $\underline{CH_2}$-$CH_2$-$CH_2$-NH); 2,82 et 3,45 (2m, 4H, $CH_2$-$\underline{CH_2}$-N-$\underline{CH_2}$-$CH_2$); 3,23 (m, 2H, $CH_2$-$CH_2$-$\underline{CH_2}$-NH); 3,49 et 3,58 (m, 2H, O-$\underline{CH_2}$-adam); 3,56 et 3,76 (m, 2H, NH-$\underline{CH_2}$-CH-NH); 4,18 (m, 1H, NH-$CH_2$-$\underline{CH}$-NH); 6,26 (s, 1H, $CH_2$-$CH_2$-$CH_2$-$\underline{NH}$); 6,30 et 7,05 (2d, 2H, H naphthyridine); 6,44 (m, 1H, NH-$CH_2$-CH-NH); 7,25 (d, 1H, NH-$CH_2$-CH-NH); 8,11 ppm (s,N=CH-N).

**SM :** 618 (MH+); 426 (MH-COOCH2Ph+); 1235 (2MH+); 309,8 (M2H++); 616- (M-H-)

**[$\alpha_D$] :** (0,7 % CH30H) : -2,8

## Exemple 20

Synthèse du 3-[5-éthyl-6-[4-(1,2,3,4-tétrahydro-1,8-naphthyridin-7-yl)-1-pipéridinyl]-4-pyrimidinyl]amino]alaninate de (1,1-diméthyléthyle).

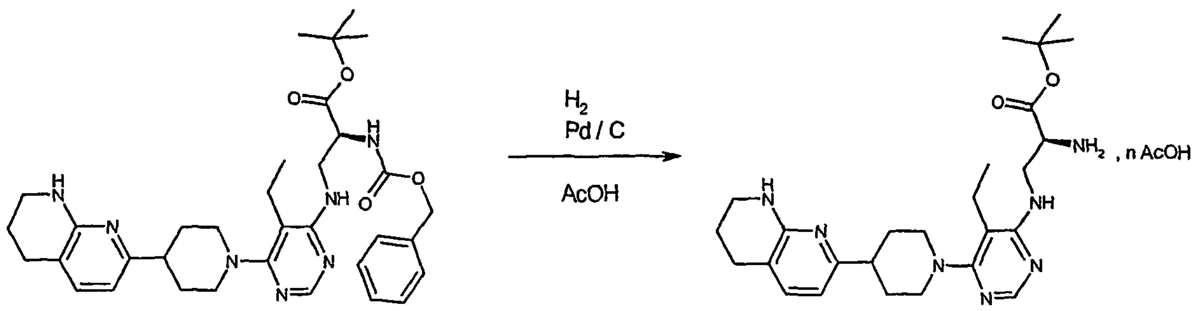


Dans un monocol contenant 3 g (4.87 mmoles)de 3-[[6-[4-(1,2,3,4-tetrahydro-1,8-naphthyridin-7-yl)-1-pipéridinyl]-5-éthyl-4-pyrimidinyl]amino]-N-[(phénylméthoxy)carbonyl]alanine, on charge 150 ml d'acide acétique 100 % et

300 mg de palladium activé sur charbon (5-10 %). Ce mélange est est purgé sous pression réduite (2 kPa) puis on le laisse agiter à TA sous pression atmosphérique d'hydrogène pendant 22 heures.

Le milieu hétérogène obtenu est filtré sur clarcel. Le filtrat est concentré à sec sous pression réduite (2 kPa) puis repris par un mélange d'eau, d'acétate d'éthyle et d'une solution de bicarbonate de sodium saturée. La phase organique est extraite à l'acétate d'éthyle et séchée sur sulfate de magnésium puis le solvant est évaporé sous pression réduite (2 kPa). On obtient 1,95 g d'une huile jaune pâle.

**CCM** rf = 0.65 (silicagel éluant AcOEt-MeOH-TEA = 90-5-5)

$\alpha_d$ = -3 (1% $CHCl_3$)

**1H-RMN (CDCl3) :** δ 1,09 (t, 3H, $CH_2$-$CH_3$); 1,35 (s, 9H, tBu); 1,75 (m, 6H, $CH_2$-CH-$CH_2$ et $CH_2$-$CH_2$-$CH_2$-NH); 2,44 (m, 2H, $CH_2$-$CH_3$); 2,60 (t, 2H, $CH_2$-$CH_2$-$CH_2$-NH); 2,81 (m, 2H, $CH_2$-CH-$CH_2$ et NH-$CH_2$-CH-NH); 3,23 (m, 2H, $CH_2$-$CH_2$-$CH_2$-NH); 3,41 (m, 4H, $CH_2$-$CH_2$-N-$CH_2$-$CH_2$); 3,42 et 3,57 (2m, 2H, NH-$CH_2$-CH-NH); 6,25 (m, 2H, $CH_2$-$CH_2$-$CH_2$-NH et NH-$CH_2$-CH-NH); 6,30 et 7,04 (2d, 2H, H naphthyridine); 8,08 ppm (s, 1H, N=CH-N).

**IR (CHCl3)** 3439 (NH); 1726 (C=O); 1580 - 1502 (heterocycle)

**HPLC / SM** $482^+$ $[MH]^+$; $426^+$ $[MH^+ - tBu]^+$; $339^+$ $[MH^+ - CH_2CH(NH_2)CO_2tBu]^+$; $241.7^+$ $[M+2H]^2$

## Exemples 21 à 31

### Exemple 28

### Etape a)

Synthèse du 3-[[5-éthyl-6-[4-(1,2,3,4-tétrahydro-1,8-naphthyridin-7-yl)-1-pipéridinyl]-4-pyrimidinyl]amino]-N-(4-méthoxy-benzènesulfonyl)alaninate de (1,1-diméthyléthyle).

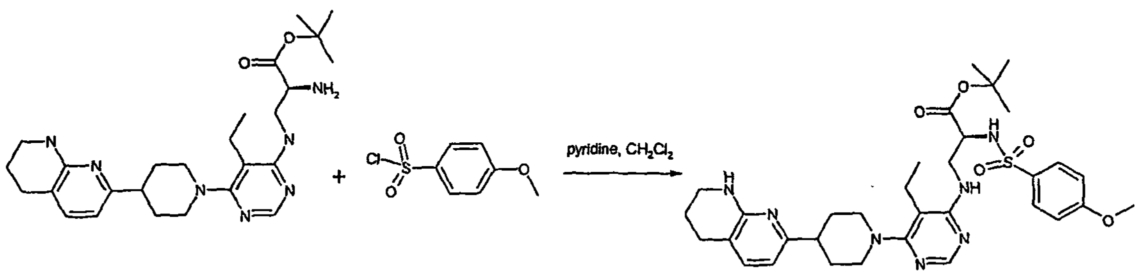


A un mélange de 150 mg (0,31 mmoles) de 3-[5-éthyl-6-[4-(1,2,3,4-tétrahydro-1,8-naphtiridin-7-yl)-1-pipéridinyl]-4-pyrimidinyl]amino]alaninate de (1,1-diméthyléthyle) en solution dans 6 ml de dichlorométhane et 650 µl de pyridine, on additionne 64,3 mg (0,31 mmoles)de chlorure de 4-méthoxybenzènesulfonyle en solution dans 3 ml de dichlorométhane. Le mélange réactionnel est agité à température ambiante pendant 5 heures. Puis, le solvant est évaporé sous pression réduite (2 kPa) et le résidu est chromatographié sur silicagel avec l'éluant suivant : acétate d'éthyledichlorométhane/méthanol(95/5) 50-50 à acétate d'éthyle-méthanol 90-10. On obtient 55,8 mg (Rdt = 28 %) de produit attendu.

**CCM :** Rf = 0,63 (silicagel, éluant : dichlorométhane-méthanol-eau-acide acétique 85-15-2-2)

**1H-RMN ($CDCl_3$) :** δ 1,21 (t, 3H, CH2-CH3); 1,30 (s, 9H, tBu); 1,92 et 2,02 (2m, 6H, N-CH2-CH2-CH-CH2-CH2, CH2-CH2-CH2-NH); 2,48 (q, 2H, CH2-CH3); 2,72 (m, 3H, N-CH2-CH2-CH-CH2-CH2, CH2-CH2-CH2-NH); 2,97 et 3,61 (2m, 4H, N-CH2-CH2-CH-CH2-CH2); 3,45 (m, 2H, CH2-CH2-CH2-NH); 3,74 et 3,86 et 3,95 (3m, 6H, NH-CH2-CH-NH, OCH3); 4,95 (t, 1H, NH mobile); 5,92 (dl, 1H, NH mobile); 6.43 et 7,20 (2d, 2H, CH=CH naphtiridine); 6,95 et 7,79 (2d, 4H, CH=CH benzène); 8,29 (s, 1H, N=CH-N).

**SM :** 652 (MH+) .

**Etape b)**

Synthèse du 3-[[5-éthyl-6-[4-(1,2,3,4-tétrahydro-1,8-naphthyridin-7-yl)-1-pipéridinyl]-4-pyrimidinyl]amino]-N-(4-méthoxy-benzènesulfonyl)alanine.

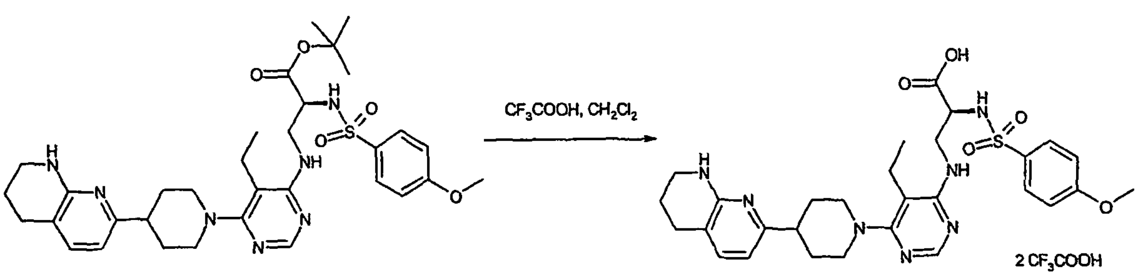


On agite 55,8 mg (0,086 mmoles) de 3-[[5-éthyl-6-[4-(1,2,3,4-tétrahydro-1,8-naphthyridin-7-yl)-1-pipéridinyl]-4-pyrimidinyl]amino]-N-(4-méthoxy-benzènesulfonyl)alaninate de (1,1-diméthyléthyle) dans 5 ml de dichlorométhane avec 0,5 ml d'acide trifluoroacétique à température ambiante jusqu'à disparition du produit de départ en CCM (silicagel, éluant : CH2Cl2-MeOH-H2O-AcOH 85-15-2-2). On rajoute du toluène et on évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 59,8 mg (Rdt = 85 % exprimé en ditrifluoroacétate) de produit attendu.

**CCM :** Rf = 0,33 (silicagel, éluant : dichlorométhane-méthanol-eau-acide acétique 85-15-2-2).

**1H-RMN (MeOD) :** δ 1,22 (t, 3H, CH2-CH3); 1,96 et 2,10 (2m, 6H, N-CH2-CH2-CH-CH2-CH2, CH2-CH2-CH2-NH); 2,55 (q, 2H, CH2-CH3); 2,85 (t, 2H, CH2-CH2-CH2-NH); 2,95 (m, 1H, N-CH2-CH2-CH-CH2-CH2); 3,20 et 3,67 et 3,92 (3m, 6H, N-CH2-CH2-CH-CH2-CH2, NH-CH2-CH-NH); 3,51 (m, 2H, CH2-CH2-CH2-NH); 4,24 (m, 1H, NH-CH2-CH-NH); 6.71 et 7,64 (2d, 2H, CH=CH naphtiridine); 6,99 et 7,74 (2d, 4H, CH=CH benzène); 8,27 (s, 1H, N=CH-N).

**SM :** 596 (MH+) .

Mode opératoire général de préparation des sulfonamides

**Etape a)**

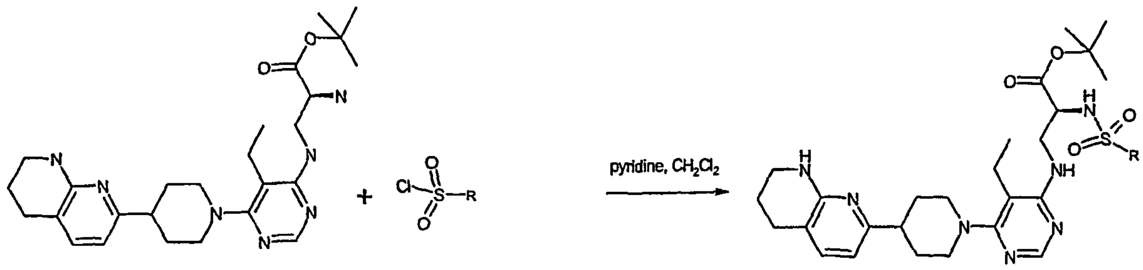


A un mélange de 150 mg (0,31 mmoles) de 3-[5-éthyl-6-[4-(1,2,3,4-tétrahydro-1,8-naphtiridin-7-yl)-1-pipéridinyl]-4-pyrimidinyl]amino]alaninate de (1,1-diméthyléthyle) en solution dans 6 ml de dichlorométhane et 650 µl de pyridine, on additionne 0,31 mmoles (masse $m_x$) de chlorure de sulfonyle en solution dans 3 ml de dichlorométhane. Le mélange réactionnel est agité à température ambiante pendant 5 heures. Puis, le solvant est évaporé sous pression réduite (2 kPa) et le résidu est chromatographié sur silicagel avec l'éluant suivant : acétate d'éthyle-dichlorométhane/méthanol (95/5) 50-50 à acétate d'éthyle-méthanol 90-10. On obtient une masse $m_y$ de produit attendu.

CCM : Rf (éluant : dichlorométhane-méthanol-eau-acide acétique 85-15-2-2).

**Etape b)**

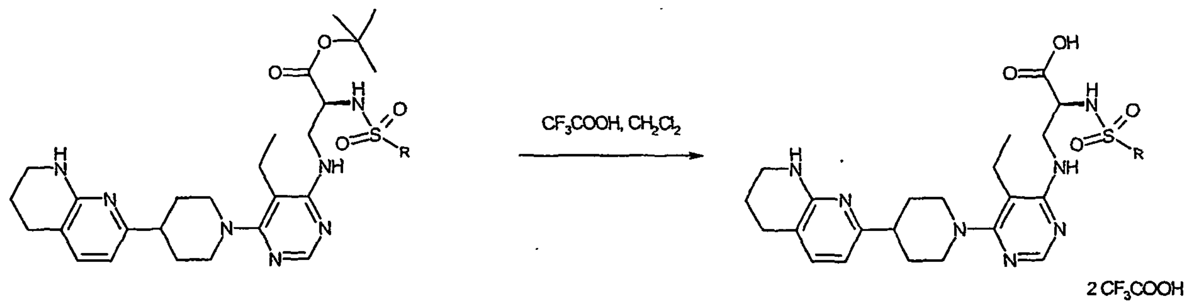


On agite une masse $m_y$ de 3-[[5-éthyl-6-[4-(1,2,3,4-tétrahydro-1,8-naphthyridin-7-yl)-1-pipéridinyl]-4-pyrimidinyl]amino]-N-(R-sulfonyl)alaninate de (1,1-diméthyléthyle) dans 5 ml de dichlorométhane avec 0,5 ml d'acide trifluoroacétique à température ambiante jusqu'à disparition du produit de départ en CCM (silicagel, éluant : CH2Cl2-MeOH-H2O-AcOH 85-15-2-2). On rajoute du toluène et on évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient une masse $m_z$ de produit attendu.

CCM : Rf (éluant : dichlorométhane-méthanol-eau-acide acétique 85-15-2-2).

| | Chlorure de sulfonyle | $m_x$ (mg) | Ester formé | $m_y$ (mg) | Rdt | SM (MH+) | Rf |
|---|---|---|---|---|---|---|---|
| Exemple 21 | 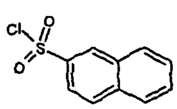 | 47,1 | 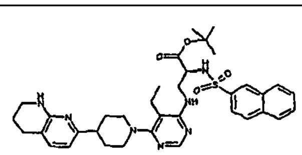 | 30 | 22 | 672 | 0,61 |

(suite)

| | Chlorure de sulfonyle | $m_x$ (mg) | Ester formé | $m_y$ (mg) | Rdt | SM (MH+) | Rf |
|---|---|---|---|---|---|---|---|
| Exemple 22 | 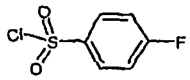 | 40,4 | 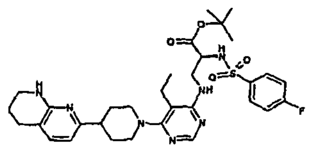 | 38,7 | 29 | 640 | 0,63 |
| Exemple 23 | 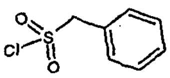 | 59,3 | 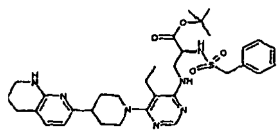 | 48 | 24 | 636 | 0,69 |
| Exemple 24 | 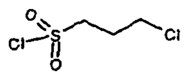 | 55,1 | 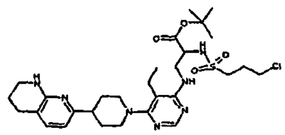 | 18,4 | 10 | 622-624 | 0,65 |
| Exemple 25 | 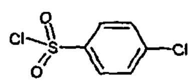 | 65,6 | 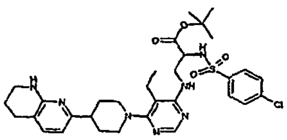 | 57,6 | 28 | 656-658 | 0,67 |
| Exemple 26 | 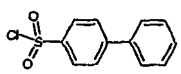 | 78,6 | 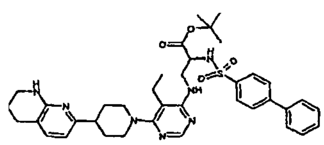 | 70 | 32 | 698 | 0,67 |
| Exemple 27 | 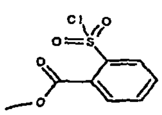 | 73,0 | 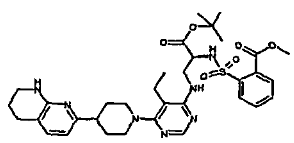 | 49,3 | 23 | 680 | 0,67 |
| Exemple 29 | 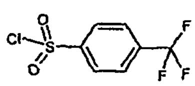 | 76,1 | 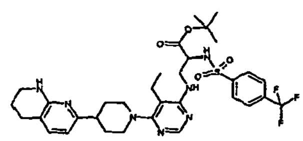 | 63,4 | 30 | 690 | 0,63 |
| Exemple 30 | 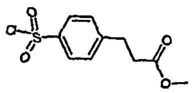 | 81,7 | 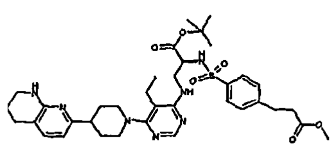 | 21,4 | 10 | 708 | 0,63 |
| Exemple 31 | 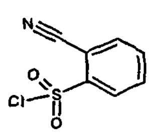 | 62,7 | 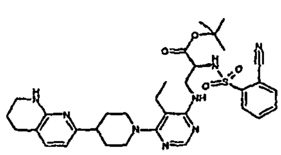 | 56,3 | 28 | 647 | 0,65 |

$M_x$ : masse de chlorure de sulfonyle introduite.
$M_y$ : masse d'ester obtenue.

| | Acide obtenu | FW (base libre) | base libre + 2TFA | $m_z$ (mg) | SM (MH+) | Rf |
|---|---|---|---|---|---|---|
| Exemple 21 | 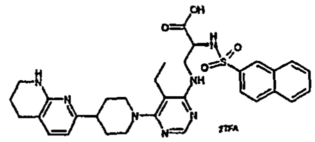 | 615,76 | 843,76 | 20,8 | 616 | 0,4 |
| Exemple 22 | 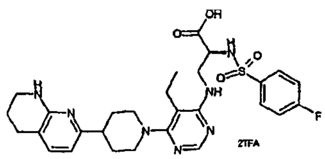 | 583,69 | 811,69 | 31,2 | 584 | 0,33 |
| Exemple 23 | 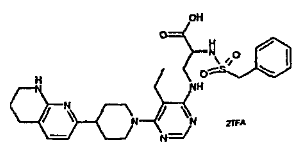 | 579,73 | 807,73 | 45,4 | 580 | 0,3 |
| Exemple 24 | 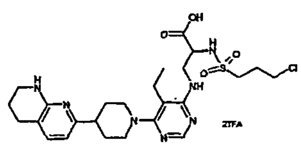 | 566,13 | 794,13 | 11,6 | 566-568 | 0,26 |
| Exemple 25 | 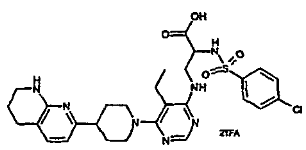 | 600,14 | 828,14 | 58,3 | 600-601 | 0,3 |
| Exemple 26 | 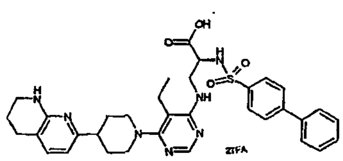 | 641,8 | 869,8 | 56,6 | 642 | 0,37 |
| Exemple 27 | 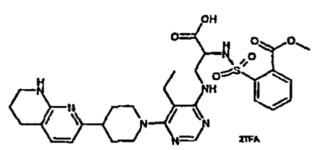 | 623,74 | 851,74 | 41,2 | 624 | 0,37 |
| Exemple 29 | 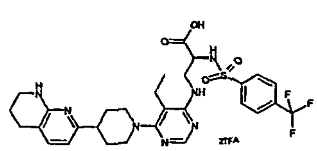 | 633,7 | 861,7 | 59,8 | 634 | 0,33 |
| Exemple 30 | 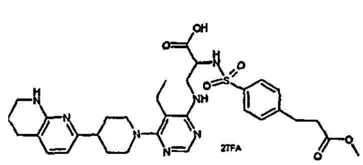 | 651,79 | 879,79 | 48,5 | 652 | 0,35 |

(suite)

| | Acide obtenu | FW (base libre) | base libre + 2TFA | $m_z$ (mg) | SM (MH+) | Rf |
|---|---|---|---|---|---|---|
| Exemple 31 | 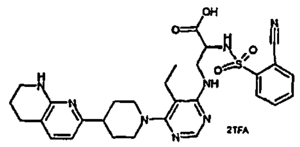 | 590,71 | 818,71 | 28 | 591 | 0,26 |
| $M_z$ : masse d'acide obtenue. | | | | | | |

**Exemple 32**

Synthèsedu3-[[5-éthyl-6-[4-(1,2,3,4-tétrahydro-1,8-naphthyridin-7-yl)-1-pipéridinyl]-4-pyrimidinyl]amino]-N-(1-naphtalènesulfonyl)alaninate de (1,1-diméthyléthyle).

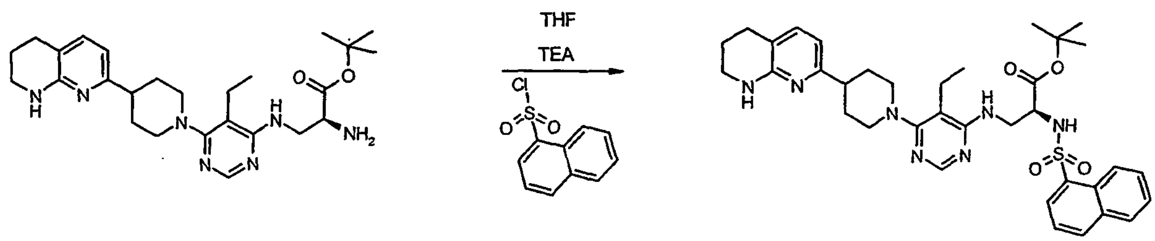


Un mélange de 239 mg (0,49 mmoles) de 3-[5-éthyl-6-[4-(1,2,3,4-tétrahydro-1,8-naphthyridin-7-yl)-1-pipéridinyl]-4-pyrimidinyl]amino] alaninate de (1,1-diméthyléthyle), 113 mg (0,5 mmoles) de chlorure de 1-naphtalènesulfonyle et de 0,104 ml (0,75 mmoles) de triéthylamine dans 50 ml de tétrahydrofurane est agité durant 5 heures à température ambiante. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un gradient d'heptane-acétate d'éthyle 75-25 à 0-100. On obtient 220 mg (Rdt = 67 %) de produit attendu sous forme d'un solide blanc.

**CCM :** Rf = 0,10 (silicagel, éluant : chlorure de méthylène-méthanol-acide acétique-eau 90-10-1-1).

**IR (CHCl3) :** 3440 (NH); 1728 (C=O); 1583, 1555, 1503 cm-1 (Hétérocycle+aromatique)

**1H-RMN (CDCl3) :** δ 1,02 (t, 3H, $CH_2CH_3$); 1,15 (s, 9H, tBu); 1,81 à 2,03(m, 6H, $CH_2$-CH-$CH_2$ et $CH_2$-$CH_2$-$CH_2$-NH); 2,23 et 2,31 (2q, 2H, $CH_2$-$CH_3$); 2,67 (t, 1H, $CH_2$-CH-$CH_2$); 2,72 (t, 2H, $CH_2$-$CH_2$-$CH_2$-NH); 2,93 et 3,56 (tl et dl, 4H, $CH_2$-$CH_2$-N-$CH_2$-$CH_2$); 3,43 (m, 2H, $CH_2$-$CH_2$-$CH_2$-NH); 3,66 et 3,76 (2m, 2H, NH-$CH_2$-CH-NH); 3,97 (m, 1H, NH-$CH_2$-CH-NH); 4,73 (t, 1H, t, 1H, NH-$CH_2$-CH-NH); 6,55 (s,1H, $CH_2$-$CH_2$-$CH_2$-NH); 6,42 et 7,05(2d, 2H, H naphthyridine); 7,51 (t, 1H, H3 1-SO2naphtyl); 7,57 et 7,62 (2t, 2H, H6 et H7 1-SO2naphtyl); 7,90 (d, 1H, H2 1-SO2naphtyl); 8,02 (d, 1H, H4 1-SO2naphtyl); 8,22 (m, 2H, N=CH-N et H5 1-SO2naphtyl); 8,61 ppm (m, 1H, H8 1-SO2naphtyl).

**SM :** 672 (MH+); 426 (616-SOOnapht+); 670- (M-H).

**$[\alpha]_D$ :** +3,3 (1% CHCl3).

Synthèse du 3-[[5-éthyl-6-[4-(1,2,3,4-tétrahydro-1,8-naphthyridin-7-yl)-1-pipéridinyl]-4-pyrimidinyl]amino]- N-(1-naphtalènesulfonyl)alanine

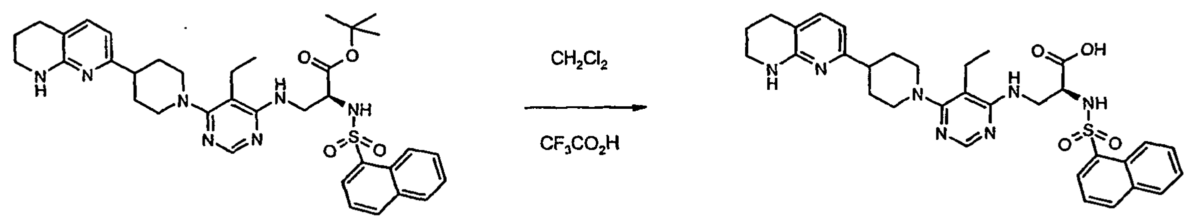


On agite 220 mg (0,33 mmoles) de 3-[[5-éthyl-6-[4-(1,2,3,4-tétrahydro-1,8-naphthyridin-7-yl)-1-pipéridinyl]-4-pyrimidinyl]amino]-N-(1-naphtalènesulfonyl)alaninate de (1,1-diméthyléthyle) dans 20 ml de dichlorométhane avec 2 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 230 mg (Rdt = 82 % exprimé en ditrifluoroacétate) de produit attendu sous forme d'un solide blanc.

**CCM :** Rf = 0,10 (silicagel, éluant : dichlorométhane-méthanol-eau-acide acétique 90-10-1-1)

**IR ($CHCl_3$)** : 3412-3257(OH, NH); 1668(C=O); 1628, 1582, 1505 cm-1(C=C, C=N, aromatique)

**1H-RMN (DMSO-d6) :** $\delta$ 0,87 (t, 3H, $CH_2$-$CH_3$); 1,77 (m, 6H, $CH_2$-CH-$CH_2$, $CH_2$-$CH_2$-$CH_2$-NH); 2,05 (q, 1H, $CH_2$-$CH_3$); 2,16 (q, 1H, $CH_2$-$CH_3$); 2,62 (t, 2H, $CH_2$-$CH_2$-$CH_2$-NH); 2,73 et 3,50 (2m, 4H, $CH_2$-$CH_2$-N-$CH_2$-$CH_2$); 2,80 (m, 1H, $CH_2$-CH-$CH_2$); 3,20 (m, 2H, $CH_2$-$CH_2$-$CH_2$-NH); 3,50 (m, 2H, CH-$CH_2$-$CH_2$-N); 3,66 et 3,76 (2m, 2H, NH-$CH_2$-CH-NH); 3,97 (t, 1H, NH-$CH_2$-CH-NH); 6,26 (s, 1H, $CH_2$-$CH_2$-$CH_2$-NH); 6,31 et 7,06 (2d, 2H, H naphthyridine); 7,55 (t, 1H, H3 1-SO2naphtyl); 7,62 (m, 2H, H6 et H7 1-SO2naphtyl); 7,96 ppm (s,N=CH-N); 8,02 (m, 1H, H5 1-SO2naphtyl); 8,08 (d, 1H, H2 1-SO2naphtyl); 8,14 (d, 1H, H4 1-SO2naphtyl); 8,58 (m, 1H, H8 1-SO2naphtyl).

**SM :** 616 (MH+); 426 (MH-SOOnapht+); 1231 (2MH+); 308,8 (M2H++); 614- (M-H-)

**[$\alpha_D$] :** (0,4 % CH3OH) : +7,0

**Exemple 33**

Synthèse du 3-[[5-éthyl-6-[4-(1,2,3,4-tétrahydro-1,8-naphthyridin-7-yl)-1-pipéridinyl]-4-pyrimidinyl]amino]-N-(2-pyridinyl)alaninate de (1,1-diméthyléthyle).

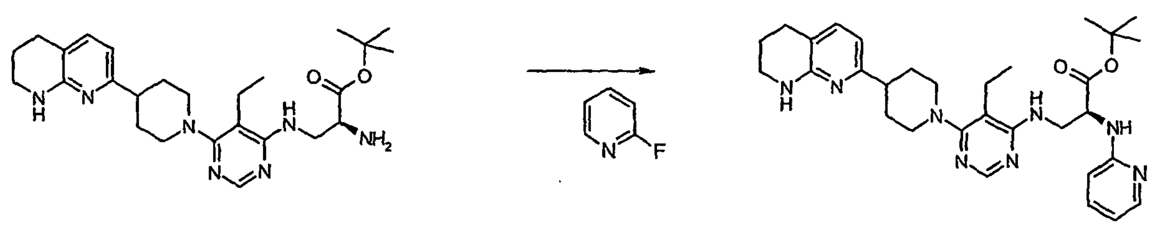

Un mélange de 240 mg (0,50 mmole) de 3-[5-éthyl-6-[4-(1,2,3,4-tétrahydro-1,8-naphthyridin-7-yl)-1-pipéridinyl]-4-pyrimidinyl]amino] alaninate de (1,1-diméthyléthyle) et de 3 ml de 2-fluoropyridine est agité durant 24 heures au reflux. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un mélange d'acétate d'éthyle-chlorure de méthylène-méthanol 80-16-4. On obtient 13 mg (Rdt = 05 %) de produit attendu sous forme d'huile.

**CCM :** Rf = 0,40 (silicagel, éluant : chlorure de méthylène-méthanol 90-10).

**SM :** 559 (MH+).

Synthèse du 3-[[5-éthyl-6-[4-(1,2,3,4-tétrahydro-1,8-naphthyridin-7-yl)-1-pipéridinyl]-4-pyrimidinyl]amino]- N-(2-pyridinyl)alanine

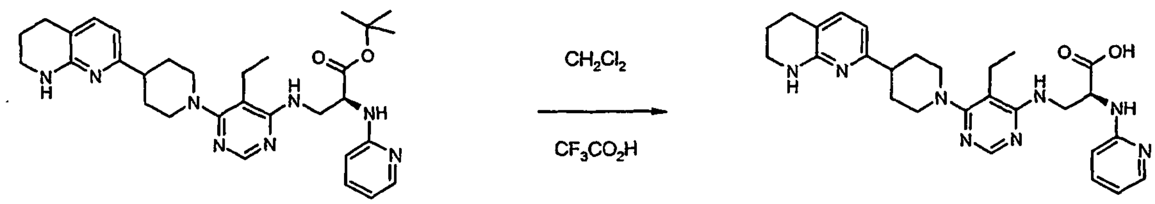


On agite 13 mg (0,023 mmole) de 3-[[5-éthyl-6-[4-(1,2,3,4-tétrahydro-1,8-naphthyridin-7-yl)-1-pipéridinyl]-4-pyrimidinyl]amino]-N-(2-pyridinyl)alaninate de (1,1-diméthyléthyle) dans 2 ml de dichlorométhane avec 0,2 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane puis versé sur de l'éther diisopropylique. Le précipité est filtré. On obtient 13 mg (Rdt = 76 % exprimé en ditrifluoroacétate) de produit attendu sous forme d'un solide beige.

**CCM :** Rf = 0,35 (silicagel, éluant : dichlorométhane-méthanol-eau-acide acétique 85-15-2-2)

**SM :** 503 (MH+); 501- (M-H-)

## Exemple 34

Synthèse du 3-[[5-éthyl-6-[4-(1,2,3,4-tétrahydro-1,8-naphthyridin-7-yl)-1-pipéridinyl]-4-pyrimidinyl]amino]-N-(2-benzothiazolyl)alaninate de (1,1-diméthyléthyle).

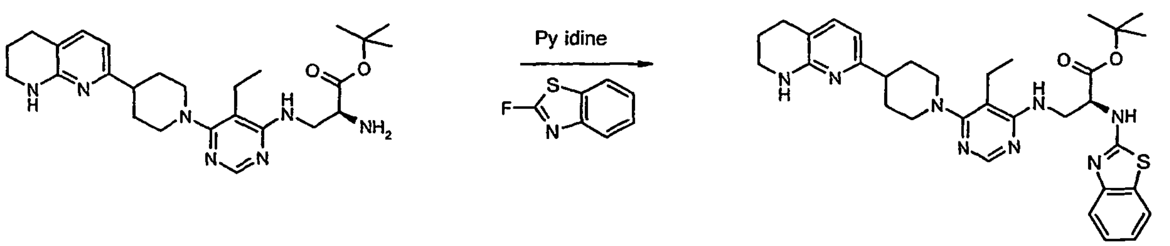


Un mélange de 200 mg (0,42 mmole) de 3-[5-éthyl-6-[4-(1,2,3,4-tétrahydro-1,8-naphthyridin-7-yl)-1-pipéridinyl]-4-pyrimidinyl]amino] alaninate de (1,1-diméthyléthyle) et de 90mg (0,6 mmole) de 2-fluorobenzothiazol dans 5 ml de pyridine est agité durant 2 heures à 100°C. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié une première fois sur silicagel en éluant avec un gradient de chlorure de méthylène-acétate d'éthyle-méthanol 100-0-0 à 0-100-0 puis 0-95-5; puis une deuxième fois sur phase inverse RP8 en éluant avec un gradient de méthanol-eau 80-20 à 100-0. On obtient 50 mg (Rdt=19 %) de produit attendu sous forme d'un solide rosé.

**CCM :** Rf= 0,20 (silicagel, éluant : acétate d'éthyleméthanol 98-2).

**1H-RMN** (CDCl3) : δ 1,06 (t, 3H, $CH_2\underline{CH_3}$); 1,49 (s, 9H, tBu); 1,77 à 2,01(m, 6H, $\underline{CH_2}$-CH-$\underline{CH_2}$ et $CH_2$-$\underline{CH_2}$-$CH_2$-NH); 2,41 (q, 2H, $\underline{CH_2}$-$CH_3$); 2,61 (t, 1H, $CH_2$-$\underline{CH}$-$CH_2$); 2,72 (t, 2H, $\underline{CH_2}$-$CH_2$-$CH_2$-NH); 2,92 et 3,51 (ql et tl, 4H, $CH_2$-$\underline{CH_2}$-N-$\underline{CH_2}$-$CH_2$); 3,42 (m, 2H, $CH_2$-$CH_2$-$\underline{CH_2}$-NH); 3,95 et 4,09 (2m, 2H, NH-$\underline{CH_2}$-CH-NH); 4,88 (m, 1H, NH-$CH_2$-$\underline{CH}$-NH); 4,98 et 5,63 (m et t, 2H, $\underline{NH}$-$CH_2$-CH-NH et NH-$CH_2$-CH-$\underline{NH}$); 6,67 (sl, 1H, $CH_2$-$CH_2$-$CH_2$-$\underline{NH}$); 6,41 et 7,13(2d, 2H, H naphthyridine); 7,12, 7,31 et 7,57 (t masqué, t et tl 4H, H benzothiazol); 8,32ppm (s, 1H, N=$\underline{CH}$-N).

**SM :** 615 (MH+); 559 (MH-tBu+); 613- (M-H-).

Synthèse du 3-[[5-éthyl-6-[4-(1,2,3,4-tétrahydro-1,8-naphthyridin-7-yl)-1-pipéridinyl]-4-pyrimidinyl]amino]- N-(2-benzothiazolyl)alanine

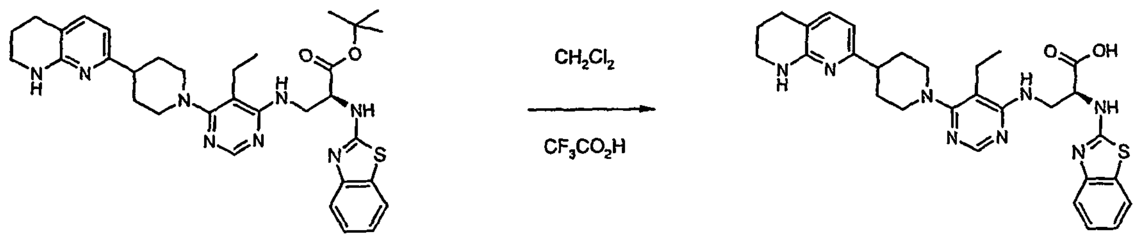


On agite 45 mg (0,073 mmole) de 3-[[5-éthyl-6-[4-(1,2,3,4-tétrahydro-1,8-naphthyridin-7-yl)-1-pipéridinyl]-4-pyrimidinyl]amino]-N-(2-benzothiazolyl)alaninate de (1,1-diméthyléthyle) dans 10 ml de dichlorométhane avec 1 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 60 mg (Rdt = 97 % exprimé en ditrifluoroacétate) de produit attendu sous forme d'un solide blanc.

**CCM :** Rf = 0,45 (silicagel, éluant : dichlorométhane-méthanol-eau-acide acétique 85-15-2-2)

**1H-RMN (CDCl3) :** δ 1,15 (t, 3H, $CH_2$-$\underline{CH_3}$); 1,75 à 2,07(m, 6H, $\underline{CH_2}$-CH-$\underline{CH_2}$, $CH_2$-$\underline{CH_2}$-$CH_2$-NH); 2,51 (q, 1H, $\underline{CH_2}$-$CH_3$); 2,76 (tl, 2H, $\underline{CH_2}$-$CH_2$-$CH_2$-NH); 2,96 (m, 1H, $CH_2$-$\underline{CH}$-$CH_2$); 3,20 et 3,73 (2m, 4H, $CH_2$-$\underline{CH_2}$-N-$\underline{CH_2}$-$CH_2$); 3,51 (m, 2H, $CH_2$-$CH_2$-$\underline{CH_2}$-NH); 4,23 (m, 2H, NH-$\underline{CH_2}$-CH-NH); 4,67 (m, 1H, NH-$CH_2$-$\underline{CH}$-NH); 6,38 et 7,35(2d, 2H, H naphthyridine); 7,20, 7,35, 7,47 et 7,54 (t, t masqué, dl et dl 4H, Hbenzothiazol); 7,73 et 10,05 (2m, 2H, Hmobiles); 8,36 ppm (s, 1H, N=$\underline{CH}$-N).

**SM :** 559 (MH+); 557- (M-H-)

## Exemple 35

En opérant par analogie avec l'exemple 34, à partir de 3-[5-éthyl-6-[4-(1,2,3,4-tétrahydro-1,8-naphthyridin-7-yl)-1-pipéridinyl]-4-pyrimidinyl]amino] alaninate de (1,1-diméthyléthyle), on prépare la 3-[[5-éthyl-6-[4-(1,2,3,4-tétrahydro-1,8-naphthyridin-7-yl)-1-pipéridinyl]-4-pyrimidinyl]amino]-N-[2-(4-methoxybenzymidazolyl)]alanine sous forme d'un solide beige.

**CCM :** Rf = 0,40 (silicagel, éluant : dichlorométhane-méthanol-eau-acide acétique 85-15-2-2)

**1H-RMN (CDCl3) :** δ 1,12 (m, 3H, $CH_2\underline{CH_3}$); 1,65 à 2,07 (m, 6H, $\underline{CH_2}$-CH-$\underline{CH_2}$ et _$CH_2$-$\underline{CH_2}$-$CH_2$-NH); 2,47 (m, 2H, $\underline{CH_2}$-$CH_3$); 2,74 (m, 2H, $\underline{CH_2}$-$CH_2$-$CH_2$-NH); 2,92 (m, 1H, $CH_2$-$\underline{CH}$-$CH_2$); 3,17 et 3,73 (2m, 4H, $CH_2$-$\underline{CH_2}$-N-$\underline{CH_2}$-$CH_2$); 3,52 (m, 2H, $CH_2$-$CH_2$-$\underline{CH_2}$-NH); 3,79 (s, 1H, O$\underline{CH3}$); 4,18 (m, 2H, NH-$\underline{CH_2}$-CH-NH); 4,81 (m, 1H, NH-$CH_2$-$\underline{CH}$-NH); 6,35 et 7,36c (2d, 2H, H naphthyridine); 6,72 et 7,18 (2d, 2H, NC(C)$\underline{CHCH}$C(CH)OCH3); 6,87 (s, 1H,NC(C)$\underline{CH}$C(CH)OCH3); 6,37, 7,70 et 9,84 (3Hmobiles); 8,32 ppm (s, 1H, N=$\underline{CH}$-N).

**SM :** 572 (MH+); 570- (M-H-)

## Exemple 36

Synthèse du 2-(2-benzyloxycarbonylamino-3-{5-methyl-6-[4-(5,6,7,8-tétrahydro-[1,8] naphtyridin-2-yl)-pipéridin-1-yl]-pyrimidin-4-ylamino}-propinylamino)-propionate de terbutyle.

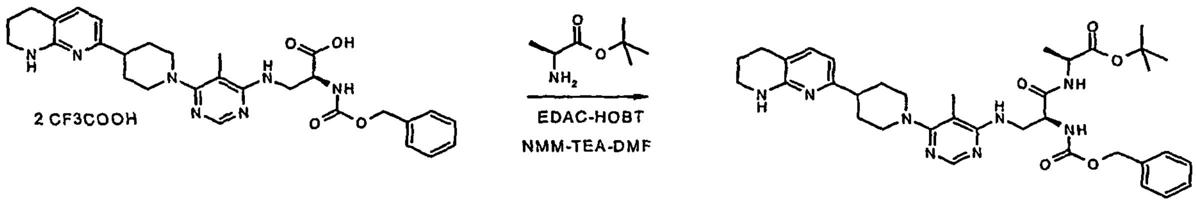


On agite 170 mg (0,30 mmole) de 3-[[5-méthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, 150 mg (1,0 mmole) de L-alaninate de terbutyle, 66 mg (0,48 mmole) de 1-hydoxy benzotriazole, 90 mg (0,48 mmole) de chlorhydrate de 1-[3-(diméthylamino) propyl]-3-ethyl carbodiimide, 0,015 ml (0,135 mmole) de N-methhylmorpholine et de 0,210 ml (1,50 mmoles) de triéthylamine dans 10 ml de diméthylformamide pendant 24 heures à température ambiante. Le mélange réactionnel est évaporé à sec sous pression réduite (2 kPa) et le résidu est repris par de l'acétate d'éthyle et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un mélange d'acétate d'éthyle-chlorure de méthylène-méthanol de 50-50-10. On obtient 120 mg (Rdt=60 %) de produit attendu sous forme d'un solide blanc.

**CCM :** Rf=0,40 (silicagel, éluant : dichlorométhane-méthanol-eau-acide acétique 90-10-1-1)

**SM :** 673 (MH+).

Synthèse de l'acide 2-(2-benzyloxycarbonylamino-3-{5-methyl-6-[4-(5,6,7,8-tétrahydro-[1,8] naphtyridin-2-yl)-pipéridin-1-yl]-pyrimidin-4-ylamino}-propinylamino)-propionique.

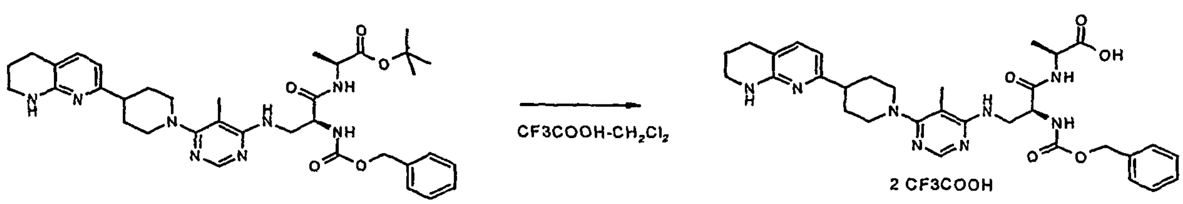


On agite 120 mg (0,179 mmoles) de 2-(2-benzyloxycarbonylamino-3-{5-methyl-6-[4-(5,6,7,8-tétrahydro-[1,8] naphtyridin-2-yl)-pipéridin-1-y1]-pyrimidin-4-ylamino}-propinylamino)-propionate de terbutyle dans 5 ml de dichlorométhane avec 1 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 95 mg (Rdt = 63 % exprimé en ditrifluoroacétate) de produit attendu sous forme d'un solide blanc.

**CCM :** Rf = 0,45 (silicagel, éluant : dichlorométhane-méthanol-eau-acide acétique 85-15-2-2)

**SM :** 616 (MH+).

**Exemple 37**

Synthèse du 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-propionate de terbutyle.

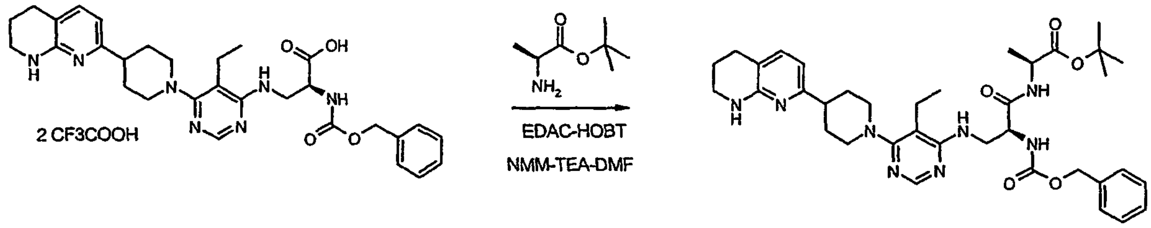


On agite 80 mg (0,15 mmole) de 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, 32 mg (0,20 mmole) de L-alaninate de terbutyle, 22 mg (0,16 mmole) de 1-hydoxy benzotriazole, 30 mg (0,16 mmole) de chlorhydrate de 1-[3-(diméthylamino) propyl]-3-ethyl carbodiimide, 0,050 ml (0,45 mmole) de N-methhylmorpholine et de 0,070 ml (0,50 mmole) de triéthylamine dans 5 ml de diméthylformamide pendant 24 heures à température ambiante. Le mélange réactionnel est évaporé à sec sous pression réduite (2 kPa) et le résidu est repris par de l'acétate d'éthyle et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'acétate d'éthyle-chlorure de méthylène-méthanol de 50-50-0 à 50-45-5. On obtient 55 mg (Rdt = 53 %) de produit attendu sous forme d'un solide blanc.

**CCM** : Rf = 0,30 (silicagel, éluant : dichlorométhane-méthanol-eau-acide acétique 90-10-1-1)

**1H-RMN (CDCl3) :** δ 0,91 (t, 3H, CH2-CH3); 1,38 (d, 3H, CONH-CH(CH3)-CO); 1,48 (s, 9H, tBu); 1,79 à 2,07 (m, 6H, $CH_2$-$CH_2$-$CH_2$-NH, $CH_2$-CH-$CH_2$); 2,49 (m, 2H, $CH_2$-$CH_3$); 2,66 (m, 1H, $CH_2$-CH-$CH_2$); 2,73 (t, 2H, $CH_2$-$CH_2$-$CH_2$-NH); 2,97 et 3,63 (2m, 4H, $CH_2$-$CH_2$-N-$CH_2$-$CH_2$); 3,42 (m, 2H, $CH_2$-$CH_2$-$CH_2$-NH); 3,73 et 4,14 (2m, 2H, NH-$CH_2$-CH-NH); 4,31 (m, 1H, NH-$CH_2$-CH-NH); 4,44 (q, 1H, CONH-CH(CH3)-CO); 5,14 (m, 2H, $CH_2$-Ph); 5,35, 6,92 et 7,89 (3H, Hmobiles); 6,42 et 7,14 (2d, 2H, H naphthyridine); 7,36 (m, 5H, Ph); 8,29 ppm (s, 1H, N=CH-N).

**SM :** 687 (MH+).

Synthèse de l'acide 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-propionique.

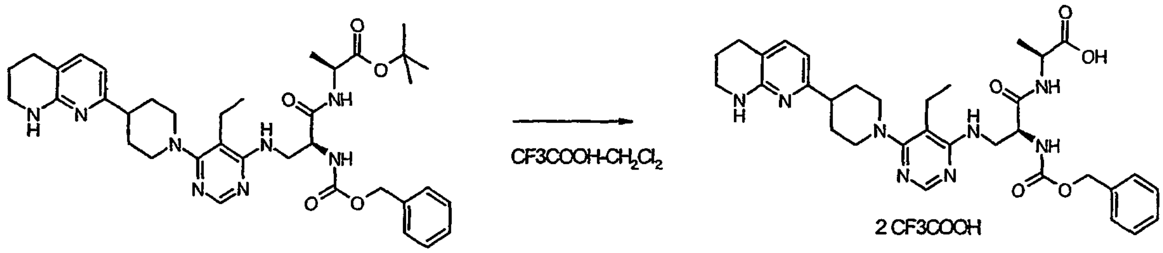


On agite 50 mg (0,073 mmoles) de 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-propionate de terbutyle dans 5 ml de dichlorométhane avec 0,5 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 53 mg (Rdt = 85 % exprimé en ditrifluoroacétate) de produit attendu sous forme d'un solide blanc.

**CCM :** Rf = 0,12 (silicagel, éluant : dichlorométhane-méthanol-eau-acide acétique 90-10-1-1)

**1H-RMN (CDCl3) :** δ 1,16 (t, 3H, CH2-CH3); 1,41 (d, 3H, CONH-CH(CH3)-CO); 1,80 à 2,10 (m, 6H, $CH_2$-$CH_2$-$CH_2$-NH, $CH_2$-CH-$CH_2$); 2,48 (m, 2H, $CH_2$-$CH_3$); 2,79 (m, 2H, $CH_2$-$CH_2$-$CH_2$-NH); 3,00 (tl, 1H, $CH_2$-CH-$CH_2$); 3,28 et 3,84 (2m, 4H, $CH_2$-$CH_2$-N-$CH_2$-$CH_2$); 3,52 (m, 2H, $CH_2$-$CH_2$-$CH_2$-NH); 3,78 et 4,13 (2m, 2H, NH-$CH_2$-CH-NH); 4,46 (m, 1H, NH-$CH_2$-CH-NH); 4,59 (m, 1H, CONH-CH(CH3)-CO); 5,05 (m, 2H, $CH_2$-Ph); 6,41 et 7,38 (2d, 2H, H naphthyridine); 6,68, 7,20, 7,95 et 9,68 (4H, Hmobiles); 7,31 (m, 5H, Ph); 8,28 ppm (s, 1H, N=CH-N).

**SM :** 631 (MH+).

**Exemple 38**

1°) Synthèse du tert-butyl 4-hydroxy-1-pipéridinecarboxylate 1 :

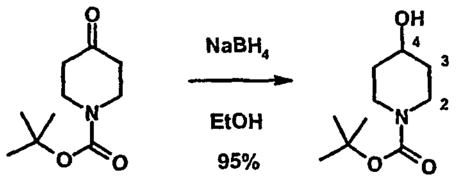


commercial 1

On dissout 1 g (5 mmol) de tert-butyl 4-oxo-1-pipéridinecarboxylate (commercialisée par Aldrich) dans 5 ml d'éthanol. On refroidit cette solution à 0°C à l'aide d'un bain de glace et on additionne par portion 200 mg (7,56 mmol) de tetraborohydrure de sodium et on agite 4 heures à température ambiante. On additionne sur le mélange réactionnel une solution aqueuse saturée en chlorure d'ammonium. On évapore l'éthanol sous pression réduite (2 kPa) puis on reprend le mélange réactionnel avec de l'acétate d'éthyle. On sépare la phase organique de la phase aqueuse. On répète une fois cette extraction et on rassemble ensuite les phases organiques que l'on sèche sur sulfate de magnésium. On concentre sous pression réduite (2 kPa) et l'on récupère ainsi 1,05 g (Rdt = 100 %) d'une huile incolore.

**CCM :** Rf = 0,5 (silicagel, éluant : CH2C12/ MeOH 90:10

**1H-RMN (CDCl3) :** δ 1,47 (s, 9H, tBu) et (m, 2H; -CH**H**-CH2-N-CH2-C**H**H-); 1,87 (m, 2H, -CH**H**-CH2-N-CH2-CH**H**- ); 3,04 (m, 2H, - CH**H**-N-C**H**H-); 3,85 (m, 2H, -C**H**H-N-CH**H**-) et (m,1H, -C**H**-OH)

2°) Synthèse du tert-butyl 4-iodo-1-pipéridinecarboxylate 2 :

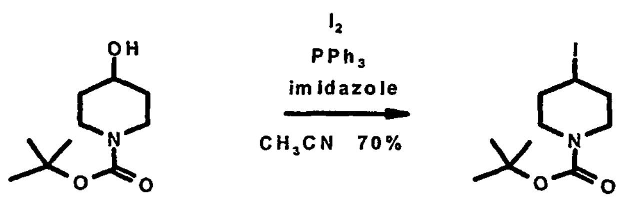


2

On dissout 2,15 g de triphénylphosphine (8,2 mmole) et 2,08 g d'iode (8,2 mmole) dans 30 ml d'acétonitrile.

On laisse agite pendant 10 min à température ambiante puis on ajoute 918 mg d'imidazole (13.5 mmol) et on laisse agiter encore 10 min à température ambiante. On ajoute ensuite 1 g (5 mmol) de tert-butyl 4-hydroxy-1-pipéridinecarboxylate du stade précédent et on laisse agiter 24h à température ambiante. On traite la réaction en ajoutant une solution aqueuse de thiosulfate de sodium et on évapore l'acétonitrile sous pression réduite (2 kPa). On reprend à l'acétate d'éthyle, on extrait et on lave avec une solution aqueuse de thiosulfate de sodium. On sèche les phases organiques sur MgSO4, on filtre et évapore l'acétate d'éthyle sous pression réduite (2 kPa). On chromatographie sur Silicagel en éluant au dichloromethane puis dichloromethane/méthanol 90:10.

On récupère 1,1 g (rdt = 70 %) d'huile incolore.

**CCM :** Rf = 0,8 (silicagel, éluant : CH2C12/ MeOH 90:10

**1H-RMN (CDCl3) :** δ 1,47 (s, 9H, tBu); 2.03 (m, 4H, -C**H2-**CHI-C**H2**-); 3,30 et 3,60 (2m, 4H, -C**H2**-N-C**H2**-); 4,46 (m, 1H - C**H**I-)

3°) Synthèse de la 2-bromo-6(2,5-diméthyl-pyrol-1-yl)-pyridine 3 :

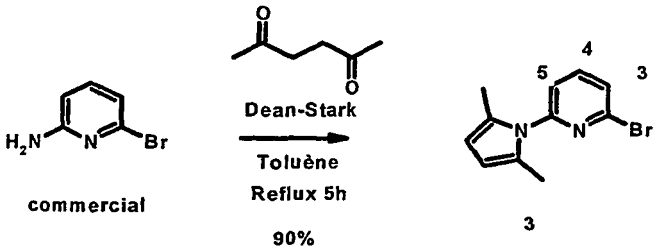


commercial

3

Dans un ballon de 100 ml surmonté d'un montage de Dean Stark on place 1 g (5,78 mmol) de 2-amino-6-bromopyridine commercialisée par Aldrich dans 30 ml de toluène. Ajoute 0.3 ml d'acide acétique et 0,8 ml (6,78 mmol) d'acétonylacétone commercialisée par Aldrich. On chauffe au reflux du toluène pendant 5 heures. On laisse revenir à température ambiante et on évapore le toluène sous pression réduite (2 kPa). On ajoute de l'eau et on extrait à l'acétate d'éthyle. On rassemble les phases organiques que l'on sèche sur sulfate de magnésium. On évapore l'acétate d'éthyle sous pression réduite(2kPa) et le résidu brut est purifié par chromatographie sur silica gel en éluant au dichloromethane.

On récupère 1 g (Rdt = 90 %) de poudre jaune.

**CCM :** Rf = 0,7 (silicagel, éluant : CH2Cl2)

**1H-RMN (CDCl3)** : δ 2,20 (s, 6H, -C**H3**C=CH-CH=CC**H3**-); 5,90 (s, 2H, -CH3C=C**H**-C**H**=CCH3-); 7,08 (d, 1H, H3 ou H5 ); 7,16 (d, 1H, H3 ou H5); 7,29( t, 1H, H4)

4°) Synthèse du 6-(2,5-Dimethyl-pyrrol-1-yl)-3',4',5',6'-tetrahydro-2'H-[2,4']bipyridinyl-1'-carboxylic acid tert-butyl ester 4 :

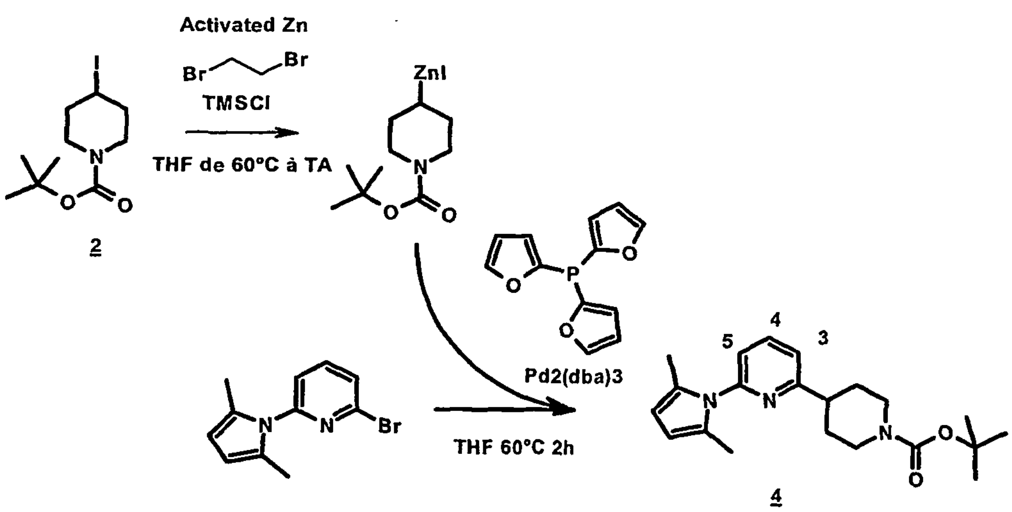


4

Sous atmosphère d'argon, on met en suspension 284 mg (4,34mmol)de zinc électrolytique auxquels on ajoute 0,033 ml de 1-2 dibromoéthane et 1 ml de tétrahydrofurane.

On agite 3 min à 60°C et on laisse revenir à température ambiante. On ajoute 0,047 ml de chlorure de triméthylsilyl et on agite 30 min à température ambiante. On additionne 1 g (3.2 mmol) de 2 solubilisé au préalable dans 2ml de tétrahydrofurane. Ongite ce mélange réactionnel 45 min à température ambiante et on l'additionne à une solution contenant 30 mg (0,032 mmol)de tris(dibenzylideneacétone) dipalladium commercialisé par Aldrich et 30 mg (0,13 mmol) de tris(2-furyl)phosphine commercialisée par LANCASTER.On ajoute ensuite 1 g (4 mmol) de 3 solubilisé au préalable dans 10ml de tétrahydrofurane.Le mélange réactionnel est laissé sous agitation magnétique à 60°C pendant 2h. On laisse revenir à température ambiante, on filtre sur clarcel et on extrait entre acétate d'éthyle et une solution aqueuse saturée en bicarbonate de sodium. On extrait 2 fois la phase aqueuse à l'acétate d'éthyle, puis on rassemble les phases organiques que l'on sèche sur sulfate de magnésium. On évapore l'acétate d'éthyle sous pression réduite (2 kPa) et le résidu brut est purifié par chromatographie sur silicagel en éluant avec un mélange heptane/ acétate d'éthyle 4:1. On récupère 350 mg (rdt = 30 %) de produit attendu sous forme d'huile jaune.

**CCM :** Rf = 0,2 (silicagel, éluant : Heptane/ Acétate d'éthyle 90:10)

**1H-RMN (CDCl3)** : δ 1.50 (s, 9H, tBu); 1.78 et 1.97 (m, 4H, -C**H2**-CH2-N-CH2-C**H2**-); 2.18 (s, 6H, -C**H3**C=CH-CH=CC**H3**-); 2.85 et 2.95 (m, 3H, C**H**-CH2-C**H**H-N-C**H**H-CH2-); 4.28 (m, 2H, -CH2-CH**H**-N-CH**H**-CH2-); 5.92 (s, 2H, -CH3C=C**H**-C**H**=CCH3-); 7.08 (d, 1H, H3 ou H5); 7.16 (d, 1H, H3 ou H5 ); 7.29( t, 1H, H4)

5°) Synthèse du 6-(2,5-diméthyl-pyrrol-1-yl)-1',2',3',4',5',6'-hexahydro-[2,4']bipyridinyl

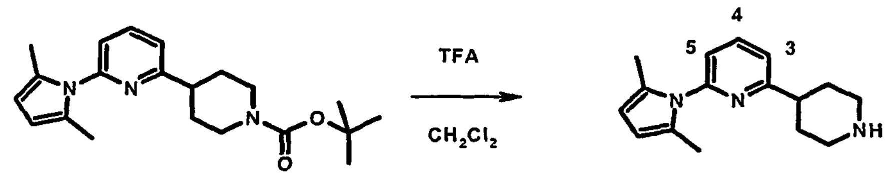

92%

4 5

On met en solution 330 mg (0,928 mmol) de 4 dans 3 ml de dichlorométhane auxquels on ajoute 0,3 ml d'acide trifluoroacétique. On agite pendant 2 heures à température ambiante. On évapore 1e dichloromethane sous pression réduite (2 kPa). On reprend le résidu obtenu à l'eau, on basifie jusqu'à pH = 10 avec de l'ammoniaque concentré et on extrait le produit au dichloromethane. La phase organique est séchée sur sulfate de magnésium et on évapore le dichloromethane sous pression réduite (2 kPa). On récupère 220 mg (Rdt = 92 %) d'huile jaune.

**CCM :** Rf = 0,3 (silicagel, éluant : CH2Cl2/ MeOH 90:10)

**1H-RMN (CDCl3) :** δ 1.90 et 2.08 (m, 4H, -C**H2**-CH2-N-CH2-C**H2**-); 2.18 (s, 6H, -C**H3**C=CH-CH=CC**H3**-); 2.88 et 3.34 (m, 4H, -CH2-C**H2-**N-C**H2**-CH2-); 2.95,(m, 1H, C**H**-CH2-CH2-N-CH2-); 4.10 (m, 1H, NH); 5.92 (s, 2H, -CH3C=C**H**-C**H**=CCH3-); 7.08 (d, 1H, H3 ou H5); 7.16 (d, 1H, H3 ou H5); 7.29(t, 1H, H4)

**SM :** 256 (MH+)

6°) Synthèse du 1'-(6-chloro-5-méthyl-pyrimidin-4-yl)-6-(2,5-dimethyl-pyrrol-1-yl)-1',2',3',4',5',6'-hexahydro-[2,4'] bipyridinyl :

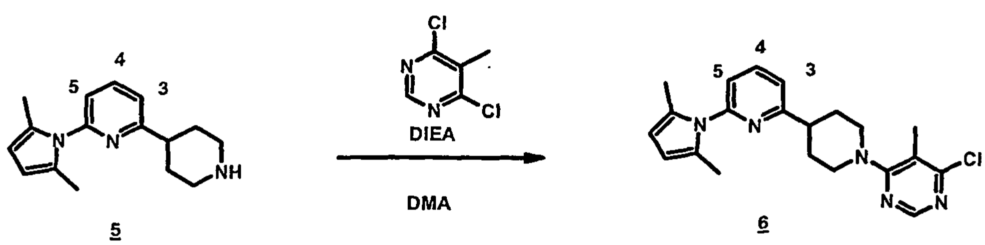


5 6

On dissout 220 mg (0,860 mmol) de 5 dans 2 ml de dimethylacétamide auxquels on ajoute 140 mg (0,860 mmol) de 4,6-dichloro-5-methyl-pyrimidine commercialisée par SPECS et 0,2 ml de diisopropylethylamine. Le mélange est chauffé à 110°C sous agitation magnétique pendant une heure. On laisse revenir le mélange à température ambiante et on évapore la diméthylacétamide sous pression réduite (0,2 kPa). Reprend le résidu brut à l'acétate d'éthyle et lave à l'eau. On extrait la phase aqueuse 2 fois à l'acétate d'éthyle, les phases organiques sont rassemblées et séchées sur sulfate de magnésium. On évapore l'acétate d'éthyle sous pression réduite (0,2 kPa) et on récupère 330 mg de résine marron qui sont mis en jeu pour le stade suivant sans purification.

**CCM** : Rf = 0,4 (silicagel, éluant : CH2Cl2)

**1H-RMN (CDCl3) :** δ 2,02 et 2,10 (m, 4H, -C**H2**-CH2-N-CH2-C**H2**-); 2,18 (s, 6H, -C**H3**C=CH-CH=CC**H3**-); 2,30(s, 3H, CH3); 3.08 et 4,01 (m, 4H, -CH2-C**H2**-N-C**H2**-CH2-); 3,02, (m, 1H, C**H**-CH2-CH2-N-CH2-); 5,92 (s, 2H, -CH3C=C**H**-C**H**=CCH3-); 7,09 (d, 1H, H3 ou H5); 7,20 (d, 1H, H3 ou H5 ); 7,29(t, 1H, H4); 8,41 (s, 1H, =N-CH=N)

7°) Synthèse du 2-Benzyloxycarbonylamino-3-{6-[6-(2,5-dimethyl-pyrrol-1-yl)-3',4',5',6'-tetrahydro-2'H-[2,4']bipyridinyl-1'-yl]-5-méthyl-pyrimidin-4-ylamino}-propionic acid tert-butyl ester 7 :

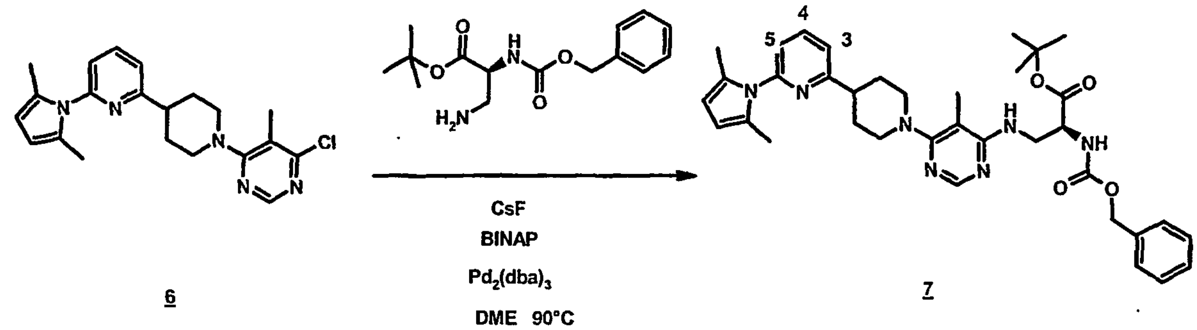


Les 330 mg (0,866 mmol) du stade précédent sont solubilisés dans 5 ml de dimethoxyethane. On ajoute successivement 286 mg (1 mmol) de 3-amino-N-[(phénylméthoxy)carbonyl] alaninate de (1,1-diméthyléthyle) (préparé selon J. Med. Chem. (2001), 44(8), 1158-1176), 184 mg (1.21 mmol) de fluorure de Césium, 54 mg (10 % mol) de rac-2,2'-bis(diphenylphosphino)-1,1'-Binaphtyl commercialisé par Aldrich, et 40mg(5%mol) de tris(dibenzylideneacétone) dipalladium commercialisé par Aldrich. Ce mélange est chauffé à 100°C pendant 18 heures sous agitation magnétique. On ajoute de nouveau 54 mg (10 % mol) de rac-2,2'-Bis(diphenylphosphino)-1,1'-Binaphtyl,40 mg (5 % mol) de tris (dibenzylideneacétone)dipalladium et on chauffe 2h supplémentaires à 100°C. On laisse revenir à température ambiante et on évapore le diméthoxyéthane sous pression réduite(2 kPa).On reprend le résidu obtenu à l'acétate d'éthyle et on lave avec une solution aqueuse saturée en bicarbonate de sodium. On extrait la phase aqueuse à l'acétate d'éthyle, rassemble les phases organiques que l'on sèche sur sulfate de magnésium. On évapore l'acétate d'éthyle sous pression réduite (2 kPa). Le résidu obtenu est purifié par chromatographie sur silicagel en éluant avec un mélange heptane/acétate d'éthyle 1:1. On récupère 200 mg de solide jaune.

**CCM :** Rf = 0,2 (silicagel, éluant : Heptane/ acétate d'éthyle 1:1)

**1H-RMN (CDCl3) :** δ 1,50 (s,9H, tBu); 1,97(s,3H, CH3); 2,02 à 2,10(m, 4H, -C**H2**-CH2-N-CH2-C**H2**- ); 2,18 (s, 6H, -C**H3**C=CH-CH=CC**H3**-); 3,20 et 3,78( m, 4H, -CH2-C**H2**-N-C**H2**-CH2- ); 3,00(m, 1H, C**H**-CH2-CH2-N-CH2-); de 3,85 à 4,00 (m,2H, NH-**CH2-**CHCOOtBuNH); 4,47(m,1H,NH-CH2C**H**-COOtBuNH); 5,12(2H,-O-**CH2-**Phényl); 5,92 (s, 2H, -CH3C=C**H**-C**H**=CCH3-); 6,12(m,1H mobile); 7,09(d,1H, H3 ou H5); 7,21(d,1H,H3 ou H5); 7,78(t, 1H,H4); 7,45 (m,5H aromatiques); 8,32(s, 1H, =N-CH=N)

**SM** : 641 (MH+),584(MH-tBu+)

8°) Synthèse du 3-[6-(6-Amino-3',4',5',6'-tetrahydro-2'H-[2,4']bipyridinyl-1'-yl)-5-methyl-pyrimidin-4-ylamino]-2-benzyloxycarbonylamino-propionic acid tert-butyl ester 8 :

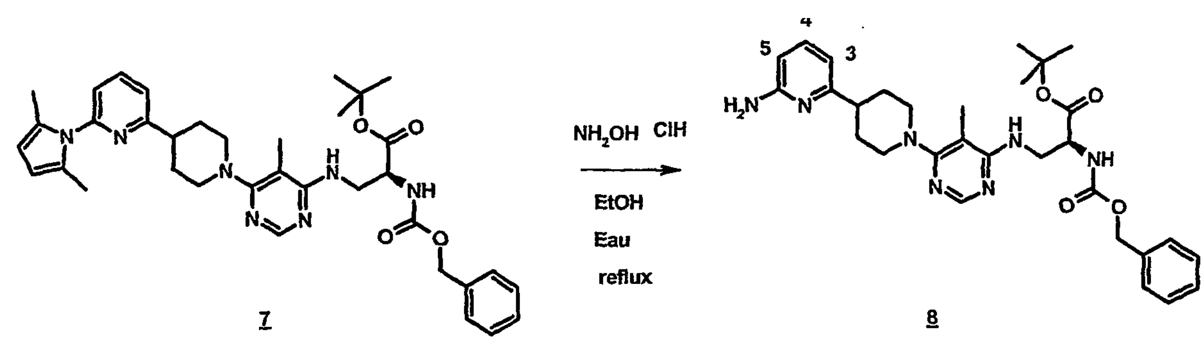


On dissous 100 mg (0,15 mmol) de 7 dans 3 ml d'éthanol et 0.3 ml d'eau. On ajoute 50 mg (0.75 mmol) de chlorhydrate d'hydroxylamine commercialisé ACROS et on chauffe 18 h à 90°C. On évapore les solvants sous pression réduite (2 kPa) et on purifie le résidu brut par chromatographie sur silicagel en éluant avec un mélange CH2Cl2/MeOH

90:10. On obtient 30 mg (36 %) de produit attendu sous forme de résine incolore.

**CCM :** Rf = 0,5 (silicagel, éluant : CH2Cl2/ MeOH 90:10)

**1H-RMN (CDCl3) :** δ 1,50 (s,9H, tBu); 1,97(s,3H, CH3); 2.02 à 2,10(m, 4H, -C**H2**-CH2-N-CH2-C**H2**- ); 2,18 (s, 6H, -C**H3**C=CH-CH=CC**H3**-); 3,20 et 3,78( m, 4H, -CH2-C**H2**-N-C**H2**-CH2- ); 3,00(m, 1H, C**H**-CH2-CH2-N-CH2-); de 3,85 à 4,00(m,2H, NH-**CH2-**CHCOOtBuNH);4,47(m,1H,NH-CH2C**H**-COOtBuNH); 5,12(2H,-O-**CH2-**Phényl); 5,92 (s, 2H, -CH3C=C**H**-C**H**=CCH3-); 6,18, 6,57,6.62(3d,H3 et H5 +1H mobile); 7,45(m,5H aromatiques); 7,60(t, 1H, H4); 8,32(s, 1H, =N-CH=N).

**SM :** 562 (MH+),372(MH-tBu et -CO-O-benzyl+)

9°) Synthèse du 3-[6-(6-Amino-3',4',5',6'-tetrahydro-2'H-[2,4']bipyridinyl-1'-yl)-5-methyl-pyrimidin-4-ylamino]-2-benzyloxycarbonylamino-propionic acid 9 :

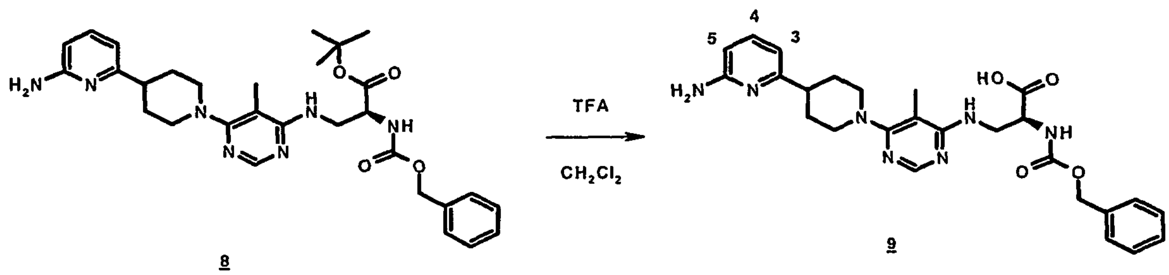


8 9

30 mg (0,053 mmol) de 8 préparé précédemment sont solubilisés dans 2 ml de dichlorométhane et 0,2 ml de d'acide trifluoroacétique. On laisse agiter pendant 9 h à température ambiante. On additionne du toluène et on évapore à sec sous pression réduite(2 kPA). Le produit est purifié par chromatographie sur silicagel en éluant avec un mélange CH2Cl2/MeOH/acide acétique 90:10:1.

On récupère 10 mg (Rdt = 37 %) de produit attendu concrétisé sous forme de solide beige dans un mélange CH2Cl2/éther isopropylique.

**CCM :** Rf = 0,2 (silicagel, éluant : CH2C12/ MeOH/ acide acétique 90:10:1

**1H-RMN (DMSO) :** δ 1,90(s,3H, CH3); 1,80 à 1. 85 (m, 4H, -C**H2-**CH2-N-CH2-C**H2**-); partiellement masqués par eau du DMSO :(4H, - CH2-C**H2**-N-C**H2**-CH2-) et (2H, NH-**CH2**-CHCOOtBuNH); 2,80,(m, 1H, C**H**-CH2-CH2-N-CH2-); 4,15(m,1H,CH2-C**H**-COOtBuNH-); 5.02(2H,-O-**CH2**-Phényl);6,27 (d, 1H, H3 ou H5 ); 6,40 ( d, 1H, H3 ou H5 ); 6.52( m, 1H, H4); 7.5(m,5H aromatiques); 8,10(s, 1H, =N-CH=N).

**SM :** 506 (MH+), 372 (MH-CO-O-Benzyl+).

**Exemple 39**

3-[5-éthyl-6-[4-(aminométhyl-2-pyridinyl)-1-pipéridinyl]-4-pyrimidinylamino]-N-[(phénylméthoxy)carbonyl]alanine, bis (trifluoroacetate).

**Etape a)**

Synthèse de l'acide(4-aminométhylpipéridine)-1-carboxylate de(1,1-diméthyléthyle).

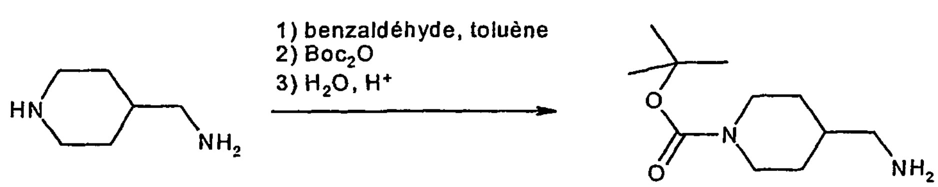


A une solution de 10 g (87.5 mmoles) de 4-aminométhylpipéridine (commercialisée par Aldrich) dans 110 ml de toluène placée sous flux d'azote, on additionne 9 mL de benzaldéhyde (87,5 mmoles), puis on chauffe ce mélange

à 120°C (présence d'un dean-stark) pendant 8 heures. On laisse le mélange réactionnel revenir à température ambiante puis on additionne 21 g (96,25 mmoles) de Di-tert-butyl dicarbonate à 0°C sur ¼ d'heure.. On laisse agiter en présence d'azote pendant une nuit. Le lendemain, on concentre le mélange réactionnel à sec sous pression réduite (2 kPa) et l'huile est reprise par 115 mL d'une solution aqueuse de $KHSO_4$ à 1N et on agite violemment, sous azote pendant 7 heures. On additionne de l'acétate d'éthyle au mélange réactionnel et la phase aqueuse est extraite deux fois à l'acétate d'éthyle. Ensuite les eaux-mères sont basifiées par ajout de pastilles de NaOH puis la phase aqueuse est extraite 3 fois par du chloroforme en relargant au NaCl. Les phases organiques regroupées sont séchées sur sulfate de magnésium puis le solvant évaporé sous pression réduite (2 kPa). On obtient 17,3 g (Rdt=92 %) de produit attendu sous forme d'une huile jaune.

**CCM :** Rf = 0,5 (alumine, éluant : dichlorométhane-méthanol (90-10)

**1H-RMN (CDCl3) :** δ 1,05 et 1,67 (2m, 4H, N-CH2-CH2-CH-CH2-CH2); 1,17(s, 2H, NH2); 1,42 (s, 10H,tBu, CH2-CH-CH2); 2,55 (d, 2H, CH2-NH2); 2,65 et 4,07 (2m, 4H, N-CH2-CH2-CH-CH2-CH2).

**SM :** 215 (MH+); 159 (MH-tBu+).

**Etape b)**

Synthèse de l'acide(4-(2-aminométhylpyridinyl)-pipéridine)-1-carboxylate de (1,1-diméthyléthyle).

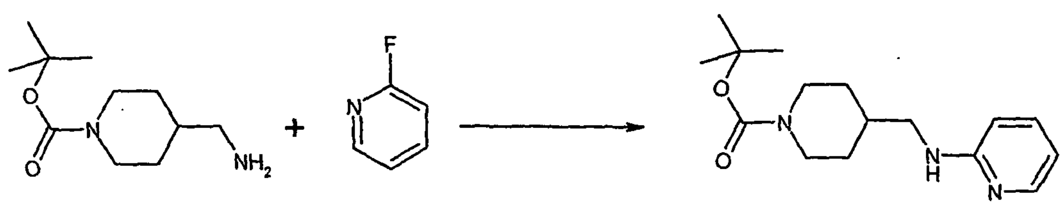

Un mélange de 2,18 g (10,19 mmoles) de l'acide(4-aminométhylpipéridine)-1-carboxylate de(1,1-diméthyléthyle) dans 5 ml de 2-fluoropyridine est porté au reflux pendant 6 heures. Après retour à température ambiante, le solvant est évaporé sous pression réduite (2 kPa) et le résidu solide jaune est repris par un mélange d'eau, d'acétate d'éthyle et d'une solution saturée de bicarbonate de sodium. La phase organique est décantée, lavée avec une solution saturée de chlorure de sodium puis séchée sur sulfate de magnésium et évaporée à sec sous pression réduite (1,3 kPa). Le résidu est chromatographié sur silicagel avec l'éluant suivant : acétate d'éthyle-cyclohexane 70-30. On obtient 1,19 g (Rdt = 40 %) de produit attendu.

**CCM :** Rf = 0,4 (silicagel, éluant : acétate d'éthylecyclohexane 70-30)

**1H-RMN ($CDCl_3$)** : δ 1,20 et 1,80 (2m, 5H, N-CH2-CH2-CH-CH2-CH2 ); 1,47 (s, 9H, tBu); 2,71 et 4,15 (2m, 4H, N-CH2-CH2-CH-CH2-CH2); 3,21 (m, 2H, CH-CH2-NH); 4,80 (m, 1H, CH-CH2-NH); 6,41 (d, 1H, CH=CH pyridine); 6,60 (m, 1H, CH=CH pyridine); 7,45 (m, 1H, CH=CH pyridine); 8,07 (m, 1H, N-CH=CH).

**SM** : 292 (MH+); 192 (MH-tBu+).

**Etape c)**

Synthèse de la 2-(4-methylpipéridinyl)aminopyridine.

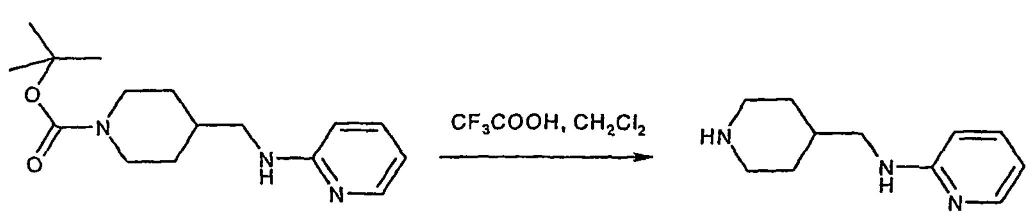


On agite 1,09 g (3,74 mmoles) de l'acide(4-(2-aminométhylpyridinyl)-pipéridine)-1-carboxylate de (1,1-diméthyléthyle) dans 25 ml de dichlorométhane avec 6 ml d'acide trifluoroacétique à température ambiante jusqu'à disparition du produit de départ en CCM (silicagel, éluant : acétate d'éthyle 100). On additionne alors 20 ml de toluène et le mélange est évaporé à sec sous pression réduite (2 kPa). Le résidu est repris avec un mélange de chlorure de méthylène et une solution aqueuse d'hydroxyde de sodium 2N. La phase organique est décantée, lavée avec une solution saturée en chlorure de sodium, séchée sur sulfate de magnésium et évaporée à sec sous pression réduite (2 kPa). L'huile obtenue est reprise avec un peu d'éther diisopropylique et de pentane puis condensée à sec sous pression réduite (2 kPa). On obtient 590 mg (Rdt = 82 %) de produit attendu sous forme d'un solide jaune.

**CCM :** Rf = 0,2 (silicagel, éluant : dichlorométhane-méthanol-eau-acide acétique 70-30-6-3).

**1H-RMN (CDCl3) :** δ 1,25 et 1,8 (2m, 4H, NH-CH2-CH2-CH-CH2-CH2); 1.72 (m, 1H, CH2-CH-CH2); 2,62 et 3,15 (2m, 6H, NH-CH2-CH2-CH-CH2-CH2 et CH-CH2-NH); 4,62 (s, 1H, NH mobile); 6,37 (d, 1H, CH=CH pyridine); 6,56 (m, 1H, CH=CH pyridine); 7,41 (m, 1H, CH=CH pyridine); 8,07 (m, 1H, N-CH=CH).

**SM :** 192 (MH+).

**Etape d)**

Synthèse de la 4-bromo-5-éthyl-6-[4-(aminométhyl-2-pyridinyl)-1-pipéridinyl]-pyrimidine.

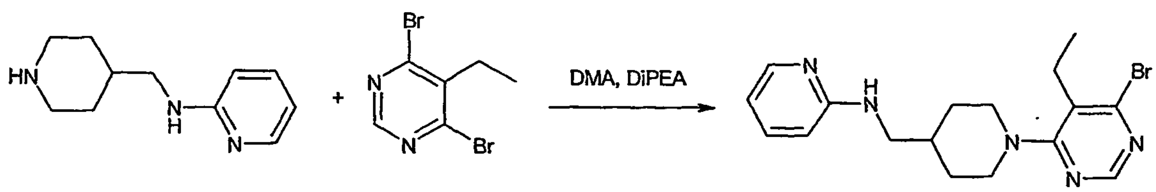


A un mélange de 657 mg (2,47 mmoles) de 4,6-dibromo-5-éthyl-pyrimidine et de 450 mg (2,35 mmoles) de la 2-(4-methylpipéridinyl)aminopyridine dans 55 ml de N,N-diméthylacétamide, on additionne 1,8 ml de diisopropyléthylamine. On chauffe ce mélange à 110°C pendant 4 heures. Puis on élimine le solvant sous pression réduite (0,2 kPa) et on reprend le résidu par un mélange d'acétate d'éthyle, d'eau et d'une solution saturée de bicarbonate de sodium. La phase organique est décantée, séchée sur sulfate de magnésium puis condensée sous pression réduite (2 kPa). L'huile marron obtenue est chromatographiée sur alumine en éluant avec le gradient suivant : heptane-acétate d'éthyle 80-20 à 70-30. On obtient 747 mg (Rdt = 84 %) de produit attendu sous forme d'un solide beige.

**CCM :** Rf = 0,5 (alumine, éluant : heptane-acétate d'éthyle 50-50).

**1H-RMN ($CDCl_3$) :** δ 1,29 (t, 3H, CH2-CH3); 1,42 et 1,9 (2m, 5H, N-CH2-CH2-CH-CH2-CH2); 2,70 (q, 2H, CH2-CH3); 2,95 et 3,88 (2m, 4H, N-CH2-CH2-CH-CH2-CH2); 3,27 (m, 2H, CH-CH2-NH); 4,81 (m, 1H, NH mobile); 6.42 (m, 1H, CH=CH pyridine); 6.60 (m, 1H, CH=CH pyridine); 7,46 (m, 1H, CH=CH pyridine); 8,09 (m, 1H, N-CH=CH); 8,31 (s, 1H, N=CH-N).

**SM :** 378 (MH+).

**Etape e)**

Synthèse du 3-[5-éthyl-6-[4-(aminométhyl-2-pyridinyl)-1-pipéridinyl]-4-pyrimidinylamino]-N-[(phénylméthoxy)carbonyl] alaninate de (1,1-diméthyléthyle).

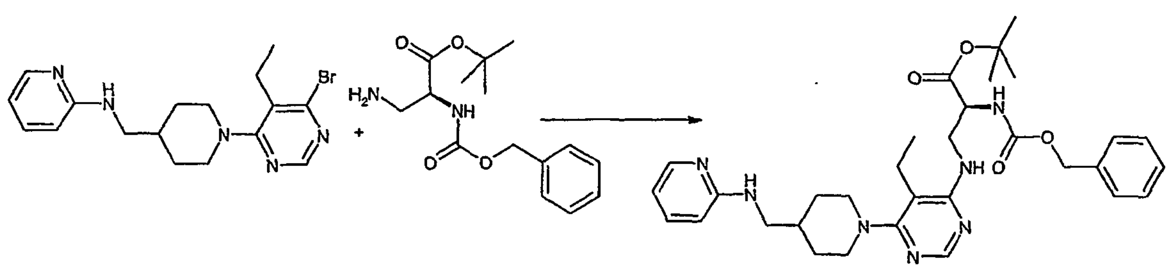

Un mélange de 300 mg (0,79 mmoles) de la 4-bromo-5-éthyl-6-[4-(aminométhyl-2-pyridinyl)-1-pipéridinyl]-pyrimidine, de 282,5 mg (0,96 mmoles) de 3-amino-N-[(phénylméthoxy)carbonyl]alaninate de (1,1-diméthyléthyle), de 168,6 mg (1,11 mmoles) de fluorure de césium, de 49,8 mg (0,08 mmoles) de tris(dibenzylidèneacétone)dipalladium (0) et de 49,8 mg (0,08 mmoles) de rac-2,2'-Bis(diphenyl-phosphino)-1,1'-binaphtyle dans 8 ml de 1,4-dioxane est porté au reflux pendant 7 heures. Le mélange réactionnel est ensuite ramené à température ambiante pour l'ajout de 49,8 mg (0,08 mmoles)de tris(dibenzilidèneacétone)dipalladium(0), puis on porte de nouveau au reflux pendant 4 heures. Après refroidissement, le mélange est concentré sous pression réduite (2 kPa) puis le résidu obtenu est repris par un mélange d'acétate d'éthyle, d'eau et d'une solution saturée de bicarbonate de sodium. La phase organique est décantée, séchée sur sulfate de magnésium puis concentrée sous pression réduite (2 kPa).

Le résidu est chromatographié une première fois sur alumine en éluant avec le gradient suivant : éther diisopropylique-acétate d'éthyle 80-20 à 100 % acétate d'éthyle. La deuxième chromatographie s'effectue sur silicagel avec l'acétate d'éthyle pour éluant.

On obtient 272 mg (Rdt = 58 %) de produit attendu.

**CCM :** Rf = 0,4 (silicagel, éluant : acétate d'éthyle).

**1H-RMN ($CDCl_3$)** : δ 1,15 (t, 3H, CH2-CH3); 1,47 et 1,90 (2m, 13H, tBu, N-CH2-CH2-CH-CH2-CH2); 1,81 (m, 1H, N-CH2-CH2-CH-CH2-CH2 ); 2,41 (q, 2H, CH2-CH3); 2,85 et 3,50(2m, 4H, N-CH2-CH2-CH-CH2-CH2); 3,27 (m, 2H, CH-CH2-NH); 3,90 (m, 2H, NH-CH2-CH-NH); 4,45 (m, 1H, NH-CH2-CH-NH); 4,85 (m, 1H, NH mobile); 5,05 (m, 1H, NH mobile); 5,12 (s, 2H, O-CH2-Ph); 6,16 (dl, 1H,

NH mobile); 6.42 (d, 1H, CH=CH pyridine); 6.60 (m, 1H, CH=CH pyridine); 7,35 (m, 5H, Ph); 7,45 (m, 1H, CH=CH pyridine); 8,07 (dl, 1H, N-CH=CH); 8,28 (s, 1H, N=CH-N).

**SM :** 590 (MH+); 534 (MH-tBu+). **[α]$_D$ ($CHCl_3$) =** +2,9

**Etape f)**

Synthèse de la 3-[5-éthyl-6-[4-(aminométhyl-2-pyridinyl)-1-pipéridinyl]-4-pyrimidinylamino]-N-[(phénylméthoxy)carbonyl]alanine, bis(trifluoroacétate).

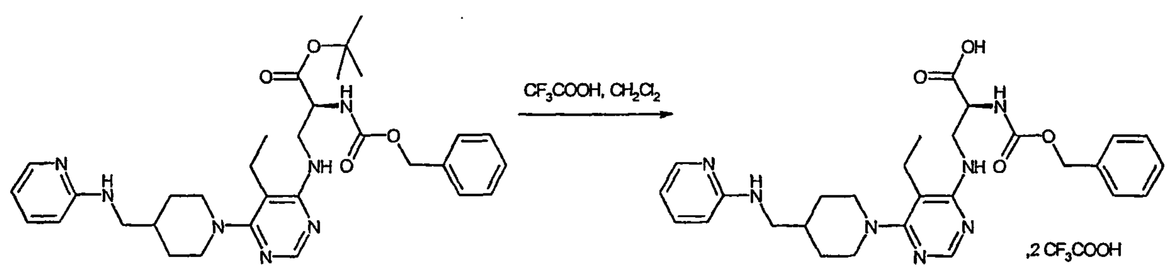


On agite 229 mg (0,39 mmoles) de 3-[5-éthyl-6-4-(aminométhyl-2-pyridinyl)-1-pipéridinyl]-4-pyrimidinylamino]-N-[(phénylméthoxy)carbonyl]alaninate de (1,1-diméthyléthyle) dans 7,5 ml de dichlorométhane avec 0,9 ml d'acide trifluoroacétique à température ambiante jusqu'à disparition du produit de départ en CCM (silicagel, éluant : dichlorométhane-méthanol-eau-acide acétique 90-10-1-1). A la fin de la réaction, on ajoute du toluène au mélange réactionnel puis on évapore à sec sous pression réduite (2 kPa).Le résidu est solubilisé dans le minimum de dichlorométhane avec un peu de méthanol puis versé sur de l'éther éthylique. Le précipité est filtré. On obtient 97,4 mg (Rdt = 38 %) de produit attendu sous forme d'un solide amorphe.

**CCM :** Rf = 0,1 (silicagel, éluant : dichlorométhane-méthanol-eau-acide acétique 90-10-1-1).

**1H-RMN ($CDCl_3$ + 2 gouttes MeOD) :** δ 1,12 (t, 3H, CH2-CH3); 1,43 et 1,95 (2m, 5H, N-CH2-CH2-CH-CH2-CH2); 2,41 (q, 2H, CH2-CH3); 3,10 et 3,65(2m, 4H, N-CH2-CH2-CH-CH2-CH2); 3,26 (d, 2H, CH-CH2-NH); 3,80 et 4,02 (2m, 2H, NH-CH2-CH-NH); 4,41 (m, 1H, NH-CH2-CH-NH); 5,10 (s, 2H, O-CH2-Ph); 6,74 (t, 1H, CH=CH pyridine); 6,87 (d, 1H, CH=CH pyridine); 7,32 (m, 5H, Ph); 7,81 (m, 2H, CH=CH pyridine et N-CH=CH); 8,23 (s, 1H, N=CH-N).

**SM :** 534 (MH+).

**[α]$_D$ ($CHCl_3$)=**+1,88

## Exemple 40

### Etape a)

Synthèse de l'acide 4-[3-(4-méthoxy-2-nitro-phenyl)-thioureidométhyl]-pipéridine-1-carboxylate de (1,1-diméthyléthyle).

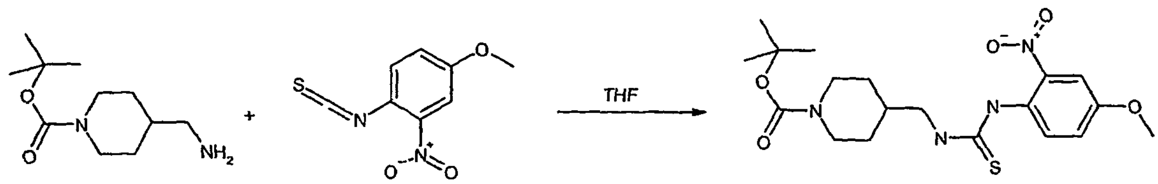


Un mélange de 1 g (4,67 mmoles) de l'acide 4-aminométhylpipéridine)-1-carboxylate de (1,1-diméthyléthyle) et de 1,08 g (6,06 mmoles) de 4-méthoxy-2-nitrophényl isothiocyanate dans 70 ml de tétrahydrofurane est agité à température ambiante, sous flux d'azote, pendant 5 heures. Puis, le solvant est évaporé sous pression réduite (2 kPa) et le résidu est chromatographié sur silicagel avec l'éluant suivant : acétate d'éthyle-heptane de 20-80 à 30-70. On obtient 1,66 g (Rdt = 84 %) de produit attendu.

**CCM** : Rf = 0,4 (silicagel, éluant : acétate d'éthyle-heptane 50-50)

**1H-RMN ($CDCl_3$) :** δ 1,25 et 1,76 (2m, 4H, N-CH2-CH2-CH-CH2-CH2 ); 1,47 (s, 9H, tBu); 1,91 (m, 1H, N-CH2-CH2-CH-CH2-CH2); 2,73 et 4,15 (2m, 4H, N-CH2-CH2-CH-CH2-CH2); 3.51 (m, 2H, CH-CH2-NH); 3,90 (s, 3H, O-CH3); 6,66 (m, 1H, CH=CH aromatique); 7,22 (m, 1H, CH=CH aromatique); 7,59 (m, 1H, CH=CH aromatique).

**SM :** 425(MH+); 325(MH-COOtBu+).

### Etape b)

Synthèse du 4-[(6-méthoxy-2-aminométhylbenzimidazole)]-pipéridine-1-carboxylate de (1,1-diméthyléthyle).

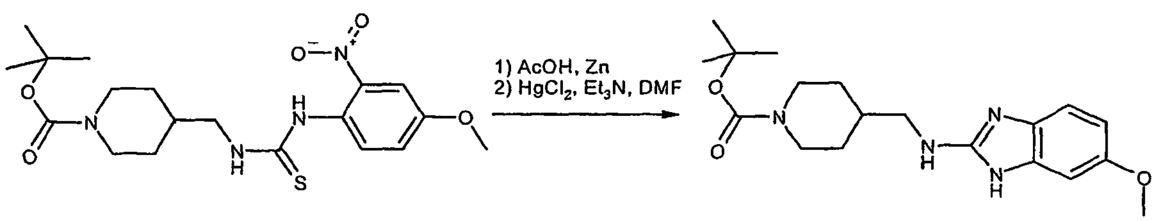


A une solution de 1,12 g (2,64 mmoles) de l'acide 4-[3-(4-méthoxy-2-nitro-phenyl)-thioureidométhyl]-pipéridine-1-carboxylate de (1,1-diméthyléthyle) dans 100 ml d'acide acétique est additionné un mélange de 2 g de zinc activé dans un peu d'acide acétique (le zinc est activé par chauffage au pistolet chauffant). On laisse sous agitation pendant 5 heures puis on additionne du clarcel à la solution, le mélange est filtré sur clarcel et le solvant est évaporé sous pression réduite (2 kPa). On obtient 1,8 g du produit attendu.

Ce brut est ensuite mis en solution dans 100 ml de diméthylformamide et on y ajoute 10 ml de triéthylamine et 500 mg de dichlorure de mercure. Le mélange réactionnel est placé sous agitation à température ambiante pendant 15 heures. Puis on évapore le solvant sous pression réduite (0,2 kPa) et le résidu est repris avec un mélange d'eau, d'une solution saturée de bicarbonate de sodium et de l'acétate d'éthyle. La phase organique est décantée, séchée sur sulfate de magnésium et le solvant éliminé par évaporation sous pression réduite (2 kPa). Le résidu est chromatographié sur alumine en éluant avec le gradient suivant : acétate d'éthyle-dichlorométhane/méthanol 50-50(100% CH2Cl2) à 50-50(95/5 CH2Cl2/MeOH). On obtient 740 mg (Rdt=77% sur 2 étapes) de produit attendu.

**CCM :** Rf = 0,4 (alumine, éluant : acétate d'éthyle-dichlorométhane/méthanol 50-50(95/5)).

**1H-RMN ($CDCL_3$) :** δ 1,07 et 1,64 (2m, 4H, N-CH2-CH2-CH-CH2-CH2); 1,46 (s, 9H, tBu); 1,76 (m, 1H, N-CH2-CH2-CH-CH2-CH2); 2,61 et 4,05 (2m, 4H, NH-CH2-CH2-CH-CH2-CH2); 3,29 (m, 2H, CH-CH2-NH); 3,81 (s, 3H, O-CH3); 6,66 et 7,12 (2d, 2H, CH=CH benzimidazole); 6,88 (s, 1H, NH-C=CH-C-OCH3).

**SM :** 361 (MH+); 305 (MH-tBu+); 261 (MH-COOtBu+).

**Etape c)**

Synthèse de la 4-(6-méthoxy-2-aminométhylbenzimidazole)pipéridine, tri(trifluoroacétate).

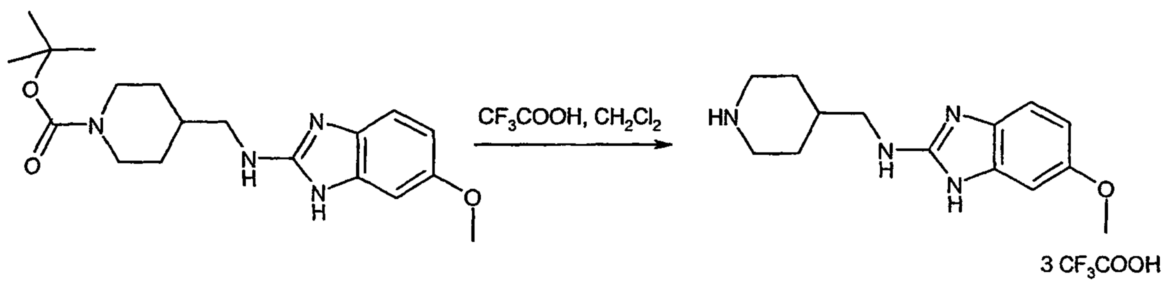


On agite 918 mg (2,55 mmoles) de l'acide 4-[(6-méthoxy-2-aminométhylbenzimidazole)]-pipéridine-1-carboxylate de (1,1-diméthyléthyle) dans 35 ml de dichlorométhane avec 6 ml d'acide trifluoroacétique à température ambiante jusqu'à disparition du produit de départ en CCM (silicagel, éluant : dichlorométhane-méthanol-eau-acide acétique 90-10-1-1). On additionne alors du toluène et évapore le mélange sous pression réduite (2 kPa). On obtient 1,15 g de produit attendu sous forme de sel de trifluoroacétate.

**CCM :** Rf = 0,15 (silicagel, éluant : dichlorométhane-méthanol-eau-acide acétique 90-10-1-1).

**1H-RMN (MeOD) :** δ 1,50 et 2,05 (2m, 5H, NH-CH2-CH2-CH-CH2-CH2); 3,00 et 3,43 (2m, 4H, NH-CH2-CH2-CH-CH2-CH2); 3,35 (d, 2H, CH-CH2-NH); 3,81 (s, 3H, O-CH3); 6,85 et 7,25 (2m, 2H, CH=CH benzimidazole); 6,91 (s, 1H, NH-C=CH-C-OCH3).

**SM :** 261 (MH+).

**Etape d)**

Synthèse de la 4-bromo-5-éthyl-6-[4-(6-méthoxy-2-aminométhylbenzimidazole)-1-pipéridinyl]pyrimidine.

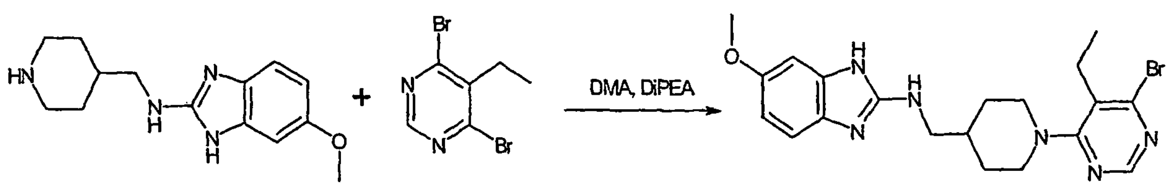


Dans un monocol contenant 356 mg (1,37 mmoles) de la 4-(6-méthoxy-2-aminométhylbenzimidazole)pipéridine, libérée de son sel, on ajoute 35 ml de N,N-diméthylacétamide, 381,84 mg (1,43 mmoles) de 4,6-dibromo-5-éthylpyrimidine mis en solution, puis on aditionne 1,2 ml de diisopropyléthylamine. Ce mélange est chauffé à 110°C pendant 3 heures puis concentré sous pression réduite (0,2 kPa). Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium, filtrées puis le solvant évaporé sous pression réduite (2 kPa).Le produit obtenu est chromatographié sur silicagel en éluant avec un gradient de dichlorométhane-méthanol 95-5 puis dichlorométhane-méthanol 90-10).

On obtient 383 mg (Rdt = 63 %) de produit attendu.

Préparation de la 4-(6-méthoxy-2-aminométhyl benzimidazole)pipéridine sous forme d'amine libre :

700 mg de 4-(6-méthoxy-2-aminométhylbenzimidazole)pipéridine est déplacée de son sel par 6 équivalents massiques de résine basique amberlyst A21 (résine de type R-$NMe_2$) dans un mélange $CH_2Cl_2$-MeOH-AcOEt 1-1-1 sous agitation pendant 30 minutes. On lave préalablement la résine et on la laisse gonfler 20 minutes dans ce mélange de solvant. Cette opération doit être répétée 3 fois pour que le déplacement du sel soit total. Après filtration de la résine

et évaporation des solvants, on obtient 356 mg (1,37 mmoles) de 4-(6-méthoxy-2-aminométhylbenzimidazole)pipéridine libre.

**CCM :** Rf = 0,23 (silicagel, éluant : dichlorométhane-méthanol 90-10).

**1H-RMN ($CDCL_3$) :** δ 1,27 (t, 3H, CH2-CH3); 1,32 et 1,80 (2m, 4H, N-CH2-CH2-CH-CH2-CH2); 1,89 (m, 1H, N-CH2-CH2-CH-CH2-CH2 ); 2,63 (q, 2H, CH2-CH3); 2,85 et 3,82 (2m, 7H, N-CH2-CH2-CH-CH2-CH2, O-CH3); 3,37 (m, 2H, CH-CH2-NH); 6,67 et 7,15 (2d, 2H, CH=CH benzimidazole); 6.90 (s, 1H, NH-C=CH-C-OCH3); 8,27 (s, 1H, N=CH-N).

**SM :** 446 (MH+).

**Etape e)**

Synthèse de la 4-bromo-5-éthyl-6-[4-(6-méthoxy-2-aminométhyl-1-carboxylate de (1,1-diméthyléthyle)benzimidazole)-1-pipéridinyl]pyrimidine.

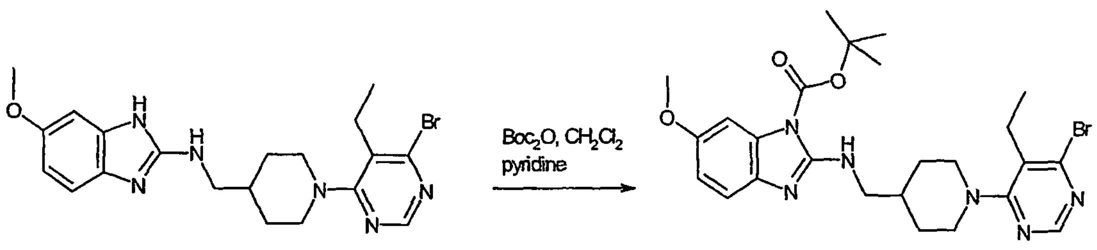


Au mélange de 159,7 mg (0,36 mmoles) de 4-Bromo-5-éthyl-6-[4-(6-méthoxy-2-aminométhylbenzimidazole)-1-pipéridinyljpyrimidine dans 15 ml de dichlorométhane, on additionne 1,5 ml de pyridine puis 471 mg (2,16 mmoles) de Di-tert-butyldicarbonate. On laisse sous agitation à température ambiante pendant une nuit. On additionne un peu de toluène au mélange réactionnel puis on évapore le solvant sous pression réduite (2 kPa). Le résidu est repris avec un mélange d'eau, d'acétate d'éthyle et d'une solution saturée de bicarbonate de sodium. La phase organique est décantée, séchée sur sulfate de magnésium puis condensée sous pression réduite (2 kPa). Le brut obtenu est chromatographié sur silicagel en éluant avec le gradient suivant : heptane-acétate d'éthyle 60-40 à 50-50. On obtient 136 mg (Rdt = 69 %) de produit attendu (sous forme de 2 régioisomères distingués par RMN avec un rapport de 50/50).

**CCM :** Rf = 0,5 (silicagel, éluant : dichlorométhane-méthanol 90-10).

**1H-RMN ($CDCL_3$) :** δ 1,30 (t, 6H, CH2-CH3); 1,47 et 1,96 (2m, 8H, N-CH2-CH2-CH-CH2-CH2); 1,72 (2sl, 20H, tBu, N-CH2-CH2-CH-CH2-CH2 ); 2,70 (q, 4H, CH2-CH3); 2,97 et 3,89(2m, 8H, N-CH2-CH2-CH-CH2-CH2); 3,52 (m, 4H, CH-CH2-NH); 3.82 (s, 6H, OCH3); 6,62 et 6,82 et 7,31 et 7,46 (4d, 4H, CH=CH benzimidazole); 7,00 et 7,26 (2s, 2H, NH-C=CH-C-OCH3); 8,31 (s, 2H, N=CH-N).

**SM :** 545/547 (MH+).

**Etape f)**

Synthèse du 3-[5-éthyl-6-[4-(6-méthoxy-2-aminométhylbenzimidazole-1-carboxylate de (1,1-diméthyléthyle))-1-pipéridinyl]-4-pyrimidinylamino]-N-[(phénylméthoxy)carbonyl]alaninate de (1,1-diméthyléthyle).

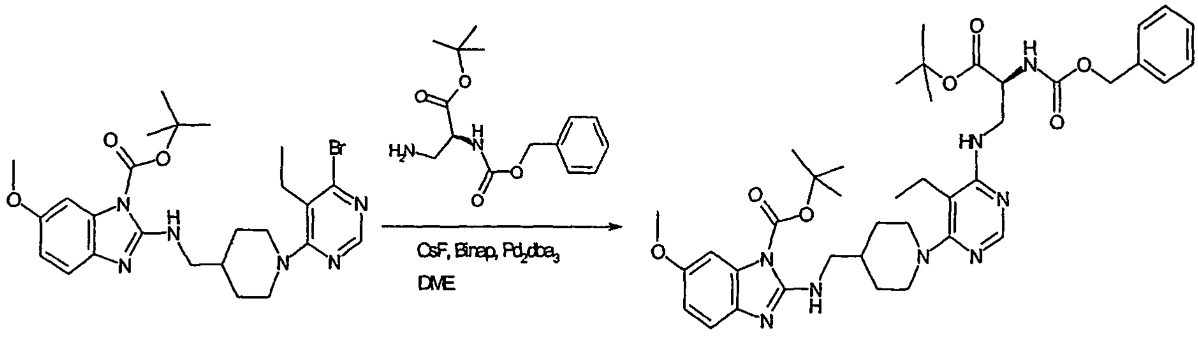


Un mélange de 136 mg (0,25 mmoles) de la 4-Bromo-5-éthyl-6-[4-(6-méthoxy-2-aminométhyl-1-carboxylate de (1,1-diméthyléthyle)benzimidazole)-1-pipéridinyl]pyrimidine, de 88,5 mg (0,30 mmoles) de 3-amino-N-[(phénylméthoxy)carbonyl]alaninate de (1,1-diméthyléthyle), de 53,2 mg (0,35 mmoles) de fluorure de césium, de 11,4 mg (0,012 mmoles) de tris(dibenzylidèneacétone)dipalladium(0) et de 15,6 mg (0,025 mmoles) de rac-2,2'-Bis(diphenyl-phosphino)-1,1'-binaphtyle dans 6 ml de 1,2-diméthoxyéthane est porté au reflux pendant 24 heures. Au cours de la réaction, on additionne 11.4 mg de tris(dibenzilidèneacétone)dipalladium(0). Après refroidissement, le mélange est concentré sous pression réduite (2 kPa) puis le résidu obtenu est repris par un mélange d'acétate d'éthyle, d'eau et d'une solution saturée de bicarbonate de sodium. La phase organique est décantée, séchée sur sulfate de magnésium puis concentrée sous pression réduite (2 kPa). Le résidu est chromatographié une première fois sur silicagel en éluant avec le gradient suivant : acétate d'éthyle-heptane 40-60 à 100 % acétate d'éthyle. La deuxième chromatographie s'effectue sur alumine en éluant avec le gradient suivant : acétate d'éthyle-dichlorométhane-méthanol 50-50-0 à 50-50-2 %.

On obtient 58 mg (Rdt = 30 %) de produit attendu (sous forme de 2 régioisomères distingués par RMN avec un rapport de 50/50).

**CCM :** Rf = 0,4 (silicagel, éluant : acétate d'éthyle).

**1H-RMN ($CDCl_3$) :** δ 1,15 (t, 6H, CH2-CH3); 1,46 et 1,91 (2m, 26H, tBu, N-CH2-CH2-CH-CH2-CH2); 1,72 (m, 20H, tBu, N-CH2-CH2-CH-CH2-CH2 ); 2,41 (q, 4H, CH2-CH3); 2,87 et 3,51(2m, 12H, N-CH2-CH2-CH-CH2-CH2, CH-CH2-NH); 3,82 (s, 6H, OCH3); 3,90 (m, 4H, NH-CH2-CH-NH); 4,43 (m, 2H, NH-CH2-CH-NH); 5,12 (s, 4H, O-CH2-Ph); 6,12 (dl, 2H, NH mobile); 6.61 (d, 1H, CH=CH benzimidazole); 6.82 (m, 1H, CH=CH benzimidazole); 7,00 (sl, 1H, NH-C=CH-C-OCH3); 7,35 (m, 12H, Ph, CH=CH benzimidazole); 7,45 (d, 1H, CH=CH benzimidazole); 8,29 (s, 2H, N=CH-N).

**HPLC/SM :** 759 (MH+); 659 (MH-COOtBu+); 603 (MH-COOtBu-tBu+).

**Etape g)**

Synthèse de la 3-[5-éthyl-6-[4-(6-méthoxy-2-aminométhyl benzimidazole)-1-pipéridinyl]-4-pyrimidinylamino]-N-[(phénylméthoxy)carbonyl]alanine, bis (trifluoroacétate).

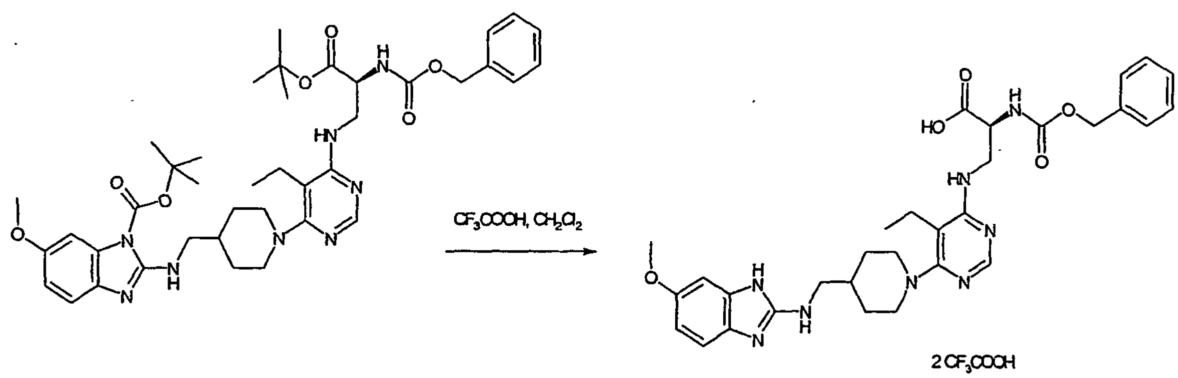


On agite 58 mg (0,08 mmoles) de 3-[5-éthyl-6-[4-(6-méthoxy-2-aminométhylbenzimidazole-1-carboxylate de (1,1-diméthyléthyle))-1-pipéridinyl]-4-pyrimidinylamino]-N-[(phénylméthoxy)carbonyl]alaninate de (1,1-diméthyléthyle)

dans 2 ml de dichlorométhane avec 0,2 ml d'acide trifluoroacétique à température ambiante jusqu'à disparition du produit de départ en CCM (silicagel, éluant : dichlorométhane-méthanol-eau-acide acétique 90-10-1-1). A la fin de la réaction, on ajoute du toluène au mélange réactionnel puis on évapore à sec sous pression réduite (2 kPa).Le résidu est solubilisé dans le minimum de dichlorométhane avec un peu de méthanol puis versé sur de l'éther diisopropylique. Le précipité est filtré. On obtient 16 mg (Rdt = 35 %) de produit attendu sous forme d'un solide amorphe.

**CCM :** Rf = 0,3 (silicagel, éluant : dichlorométhane-méthanol-eau-acide acétique 90-10-1-1).

**1H-RMN (MeOD) :** δ 1,12 ( t, 3H, CH2-CH3); 1,49 et 1,95 (2m, 5H, N-CH2-CH2-CH-CH2-CH2); 2,51 (q, 2H, CH2-CH3); 3,00 et 3,52 (2m, 4H, N-CH2-CH2-CH-CH2-CH2); 3,39 (d, 2H, CH-CH2-NH); 3,77 et 4,01 (2m, 2H, NH-CH2-CH-NH) ;3,86 (s, 3H, OCH3); 4,56 (m, 1H, NH-CH2-CH-NH); 5,07 (m, 2H, O-CH2-Ph); 6,91 (d, 1H, CH=CH benzimidazole); 6,96 (s, 1H, NH-C=CH-C-OCH3); 7,32 (m, 6H, Ph, CH=CH benzimidazole); 8,20 (s, 1H, N=CH-N).

**HPLC/SM :** 603 (MH+);

**Exemple 41**

Synthèse de la 2,5-diméthyl-4,6-dihydroxy-pyrimidine :

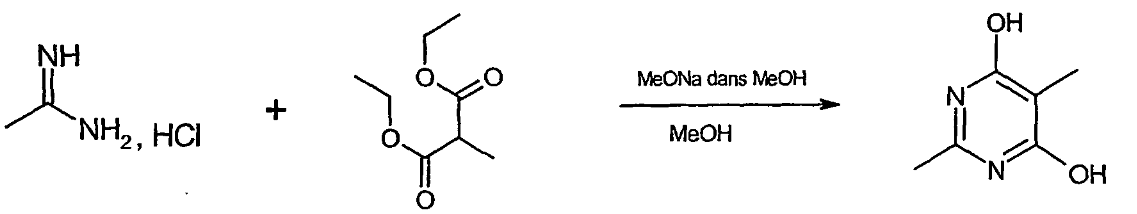


Un monocol contenant 40 ml de méthanol, placé sous atmosphère d'azote, est refroidit à 0°C par un bain de glace, on additionne 9,72 g de méthylate de sodium (soit une solution de concentration c=3 $mol.l^{-1}$) au mélange réactionnel puis on ajoute à 0°C et par petites quantités 5 g (53 mmoles)de chlorhydrate de d'acétamidine. On laisse agiter à température ambiante pendant une vingtaine de minutes, puis on ajoute goutte à goutte 8,3 ml de méthylmalonate de diéthyle. On maintient l'agitation pendant 3 heures. Puis le méthanol est condensée sous pression réduite (2 kPa). Le brut obtenu est repris avec un minimum d'eau, refroidit à 0°C puis acidifié avec de l'acide acétique pur jusqu'à un pH entre 4 et 5. Le précipité blanc formé est filtré, rincé à l'eau, éther éthylique et pentane. Puis le produit blanc est séché sur $P_2O_5$ sous pression réduite (0,2 kPa). On obtient 3,3 g (Rdt = 49 %) de produit attendu.

**CCM :** Rf = 0,2 (Silicagel, éluant : dichlorométhane-méthanol-eau-acide acétique 85-15-2-2).

**1H-RMN (DMSOd6) :** δ 1,68 (s, 3H, OH-CH=C-CH3); 2,18 (s, 3H, N=C-CH3).

Synthèse de la 2,5-diméthyl-4,6-dichloro pyrimidine :

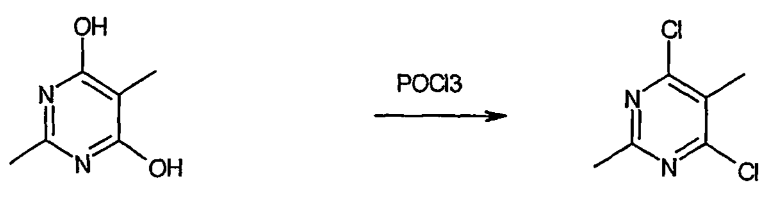


Un mélange de 3,3 g (23,5 mmoles) de 2,5-diméthyl-4,6-dihydroxy-pyrimidine et 15 ml d'oxychlorure de phosphore, est porté au reflux pendant 8 heures. Après retour à température ambiante,le milieu réactionnel est versé lentement sur un mélange de glace et d'eau. Cette phase aqueuse est extraite à l'acétate d'éthyle. La phase organique est lavée avec une solution saturée de bicarbonate de sodium puis séchée sur du sulfate de magnésium et évaporée à sec sous pression réduite (2 kPa). On obtient 3,39 g (Rdt = 81 %) de produit attendu.

**CCM :** Rf = 0,9 (Silicagel, éluant : acétate d'éthyle 100 %)

**1H-RMN (CDCl3)** : δ 2,46 (s, 3H, Cl-CH=C-CH3); 2,68 (s, 3H, N=C-CH3)
**SM :** 177/179 (MH+).

Synthèse de la 6-[4-(1,2,3,4-tetrahydro-1,8-naphthyridin-7-yl)-1-pipéridinyl]-2,5-diméthyl-4-chloro-pyrimidine :

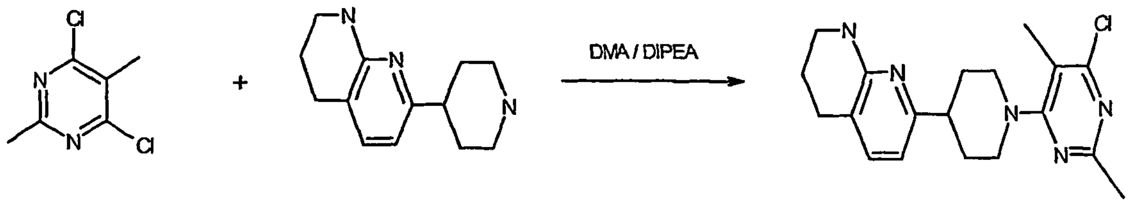


Dans un monocol contenant 2,95 g (13,5 mmoles) de 4-(1,2,3,4-tetrahydro-1,8-naphthyridin-7-yl)-1-pipéridine libérée de son sel, on ajoute 2 g (11,3 mmoles) de 4,6-dichloro-2,5-diméthyl-pyrimidine mis en solution dans 25 ml de diméthylacétamide et 5 ml de diisopropyléthylamine. Ce mélange est chauffé à 130°C pendant 4 heures puis concentré à sec sous vide. Le résidu obtenu est repris par un mélange d'eau, d'acétate d'éthyle et d'une solution de bicarbonate de sodium saturée. La phase organique est séparée et la phase acqueuse réextraite par de l'acétate d'éthyle. Les phases organiques regroupées sont séchées sur sulfate de magnésium puis le solvant évaporé sous pression réduite (2 kPa). Le résidu est chromatographié sur silicagel en éluant avec un gradient d'acétate d'éthyle (100 %) puis d'acétate d'éthyle-méthanol (95-5). On obtient 2,2 g (Rdt = 55 %) de produit attendu.
Préparation de la naphtyridine sous forme d'amine libre :
8,3 g de naphtyridine est déplacée de son sel par 6 équivalents massiques de résine basique amberlyst A21 (résine de type R-$NMe_2$ ) dans un mélange $CH_2Cl_2$ / MeOH /AcOEt 1/1/1 sous agitation pendant 30 minutes. On lave préalablement la résine et on la laisse gonfler 20 minutes dans ce mélange de solvant. Cette opération doit être répétée 3 fois pour que le déplacement du sel soit total.
Après filtration de la résine et évaporation des solvants, on obtient 2,95 g (13,5 mmoles) de naphtyridine libre.
**CCM** : Rf = 0,15 [silicagel, éluant : dichlorométhane-méthanol 95-5]
**1H-RMN (CDCl3) :** δ 1,90 et 2,01 (2m, 6H, NH-CH2-CH2-CH2, N-CH2-CH2-CH-CH2-CH2) ; 2,26 (s, 3H, CH3) ; 2,51 (s, 3H, N=C-CH3); 2,72 (m, 3H, NH-CH2-CH2-CH2, N-CH2-CH2-CH-CH2-CH2); 2,97 et 3,97 (2m, 4H, CH2-CH2-N-CH2-CH2); 3,42 (m, 2H, NH-CH2-CH2-CH2); 6,41 et 7,16 (2d, 2H, CH=CH naphthyridine).
**SM :** 358(MH+).

Synthèse du 3-[[6-[4-(1,2,3,4-tetrahydro-1,8-naphthyridin-7-yl)-1-pipéridinyl]-2,5-diméthyl-4-pyrimidinyl]amino]-N-[(phénylméthoxy)carbonyl]alaninate de (1,1-diméthyléthyle).

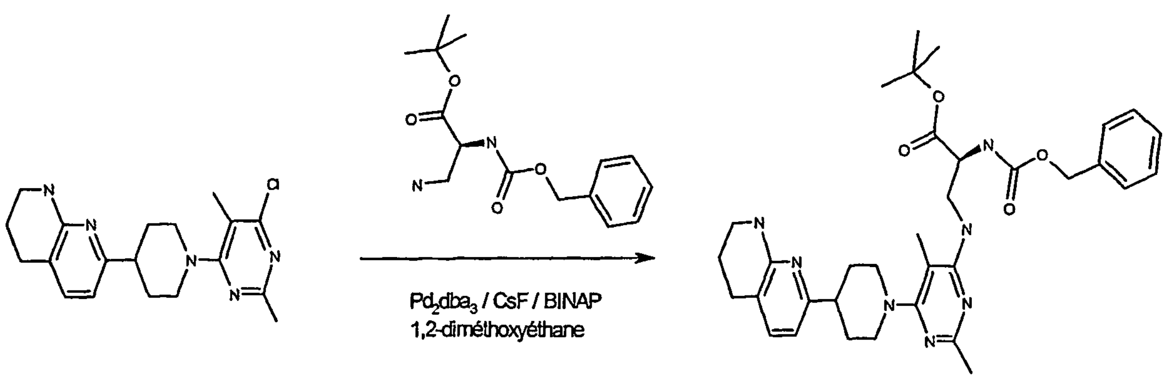


Un mélange de 2,2 g (6,15 mmoles) de 6-[4-(1,2,3,4-tetrahydro-1,8-naphthyridin-7-yl)-1-pipéridinyl]-2,5-diméthyl-4-chloro-pyrimidine et de 2,17 g (7,38.mmoles) de 3-amino-N-[(phénylméthoxy)carbonyl] alaninate de (1,1-diméthyléthyle) (préparé selon J. Med. Chem. (2001), 44(8), 1158-1176), en présence de 1,31 g (8,61 mmoles) de fluorure de cesium, de 383 mg (0,615 mmoles) de (2,2'-bis(diphénylphosphino)-1,1'-binaphthyl et de 281 mg (0,307 mmoles) de tris-dibenzylidèneacétone)dipalladium(o), dans 55 ml de 1,2-diméthoxyéthane est chauffé au reflux pendant 24 heures.

Le mélange réactionnel est ensuite ramené à température ambiante, on ajoute alors 281 mg (0,307 mmoles) de tris-dibenzylidèneacétone)dipalladium(o), puis le mélange réactionnel est porté au reflux pendant encore 24 heures. Après refroidissement la solution est concentrée à sec sous pression réduite (2 kPa) puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous pression réduite (2 kPa). Le résidu est chromatographié sur alumine avec un gradient d'éther isopropylique/acétate d'éthyle (50/50)-dichlorométhane (50-50). Les fractions contenant le produit attendu sont rassemblées pour une seconde purification sur silicagel avec un gradient acétate d'éthyle-heptane-méthanol 50-50-0 à 90-0-10. On obtient 550 mg (Rdt = 15 %) de produit attendu.

**CCM :** Rf = 0.3 (silicagel, éluant : dichlorométhane-méthanol 90-10)

**1H-RMN (CDCl3) :** δ 1,46 (s, 9H, tBu); 1,92 (m, 9H, NH-CH2-<u>CH2</u>-CH2, N-CH2-<u>CH2</u>-CH-<u>CH2</u>-CH2, C=C-<u>CH3</u>); 2.41 (s, 3H, N=C-<u>CH3</u>); 2.70 (m, 3H, NH-CH2-CH2-<u>CH2</u>, N-CH2-CH2-<u>CH</u>-CH2-CH2); 2.91 et 3.66 (2m, 4H, CH2-<u>CH2</u>-N-CH2-CH2); 3.44 (m, 2H, NH-<u>CH2</u>-CH2-CH2); 3,90 (m, 2H, NH-<u>CH2</u>-CH-NH); 4.38 (m, 1H, NH-CH2-<u>CH</u>-NH); 5,13 (s, 2H, O-<u>CH2</u>-Ph); 6.42 et 7,16 (2d, 2H, <u>CH</u>=<u>CH</u> naphthyridine); 7,35 (m, 5H, Ph).

**SM** : 616(MH+)

Synthèse de l'acide correspondant :

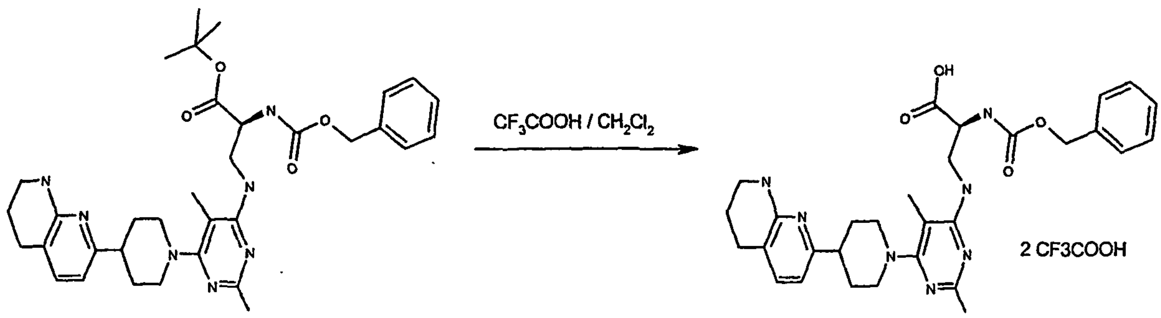


On agite à température ambiante un mélange de 500 mg (0,81 mmoles) de la 3-[[6-[4-(1,2,3,4-tetrahydro-1,8-naphthyridin-7-yl)-1-pipéridinyl]-2,5-diméthyl-4-pyrimidinyl]amino]-N-[(phénylméthoxy)carbonyl]alaninate de (1,1-diméthyléthyle) et de 5 ml d'acide trifluoroacétique dans 30 ml de dichlorométhane pendant 24 heures. On additionne alors du toluène et évapore à sec le mélange. Le résidu est solubilisé dans le minimum de dichlorométhane avec un peu de méthanol puis versé sur de l'éther diisopropylique. Le précipité est filtré. On obtient 485 mg (Rdt = 76 % exprimé en ditrifluoroacétate) de produit attendu.

**CCM :** Rf = 0,3 (silicagel, éluant : dichlorométhane-méthanol-eau-acide acétique 90-10-1-1).

**1H-RMN (CDCl3) :** δ 1,97 (m, 9H, NH-CH2-<u>CH2</u>-CH2, N-CH2-<u>CH2</u>-CH-<u>CH2</u>-CH2, C=C-<u>CH3</u>); 2,54 (s, 3H, N=C-<u>CH3</u>); 2,78 (m, 2H, NH-CH2-CH2-<u>CH2</u>); 2,98 (m, 1H, N-CH2-CH2-<u>CH</u>-CH2-CH2); 3,22 et 3.80 à 4,07 (2m, 6H, N-<u>CH2</u>-CH2-CH-CH2-<u>CH2</u>, NH-<u>CH2</u>-CH-NH); 3,51 (m, 2H, NH-<u>CH2</u>-CH2-CH2); 4,45 (m, 1H, NH-CH2-<u>CH</u>-NH); 5,10 (s, 2H, O-<u>CH2</u>-Ph); 6,42 et 7,37 (2m, 3H, <u>CH</u>=<u>CH</u> naphthyridine, NH mobile); 7,32 (m, 5H, Ph).

**SM :** 560 (MH+) ; 426 [MH+ - (COOCH2Ph)]+.

**<u>Test pharmacologique</u> : Test ELISA Kistrine/Récepteur Vitronectine ($\alpha_v\beta_3$)**

Protocole :

Des plaques 96 puits MaxiSorp sont coatées une nuit à 40°C avec 100 μl de Kistrine à 1 μg/ml (dilution en tampon de coating : carbonate 0,05 M/NaOH pH 9,6). Le lendemain, les puits sont vidés et les ligands (kistrine) sont ensuite fixés (tampons de fixation : PBS contenant 0,5 % de BSA (pH = 7,4)) pendant 1 heure à température ambiante avec une agitation douce de 125 rpm. Les puits sont lavés six fois (tampon de lavage : PBS contenant 0,05 % de Tween 20 (pH 7,7) puis on ajoute par puits et dans cet ordre :

- 40 μl de tampon d'incubation
- 10 μl de la dilution du produit à tester (les produits sont dilués dans un mélange 50:50 DMSO/Eau)
- 50 μl de récepteur $\alpha_v\beta_3$ humain (cf Pytella et al. Methods Enzymol. (1987) 144 (Dilution en tampon d'incubation, à

adapter suivant le lot de récepteur et selon le ligand). Le ligand, le récepteur $\alpha_v\beta_3$ et les produits à étudier sont co-incubés pendant 3 heures à température ambiante avec une agitation douce de 125 rpm.

Les puits sont à nouveau lavés six fois, puis incubés pendant 2 heures à température ambiante avec une agitation douce de 125 rpm, en présence de 100 $\mu$l d'anticorps anti-récepteur couplé à une peroxydase (L'anticorps 4B12-HRP est dilué en tampon d'incubation (50 mM TRIS pH 7,4; 0,5 % BSA; 0,05 % Tween 20; 1 mM $MnCl_2$; 50 $\mu$M $CaCl_2$; 50 $\mu$M $MgCl_2$; 100 mM NaCl). La dilution est à adapter suivant le lot de récepteur.

Les puits sont ensuite lavés six fois avant la mesure de la liaison ligand-récepteur faite par l'intermédiaire d'un kit révélateur de peroxydase (TBM Microwell Peroxidase Substrate System Kirkegaard; Ref cat 50-76-00).

Ce kit contient un flacon A de substrat (3,3',5,5'-tétraméthylebenzidine à 0,4 g/l) et un flacon B ($H_2O_2$ à 0,02 % en tampon Citrate/Acide citrique). Extemporanément, un volume de A est mélangé à un volume de B, puis le mélange réactionnel est distribué à raison de 100 $\mu$l/puits.

La réaction enzymatique se développe entre 6 à 10 minutes pour Kistrine/$\alpha_v\beta_3$ puis son évolution est stoppée par l'addition de 100 $\mu$l d'acide phosphorique 1M. La densité optique est déterminée à 450 nm.

## Expression des résultats

On trace la courbe suivante : le pourcentage de liaison en fonction du logarithme de chaque concentration du produit testé.

Pour chaque produit, on détermine l'IC50 suivant la formule suivante : IC50 = (BO+ Bmin)/2

BO = Maximum de liaison en l'absence de tout produit

Bmin = Minimum de liaison en présence de la concentration la plus élevée de produit.

| EXEMPLE | K/VnR $IC_{50}$ (nM) |
|---|---|
| 1 | 3 |
| 2 | 160 |
| 3 | 3,1 |
| 4 | 5,1 |

## Activité in vivo

### Hypercalcémie induite par l'hormone parathyroïdienne (PTH) dans un modèle de rat thyroparathyroïdectomisés (TPXT)

La stimulation de la résorption osseuse est induite chez des rats TPXT par perfusion de PTH et les variations de la résorption osseuse sont suivies par la concentration en calcium dans le sérum.

Des rats mâles Sprague Dawley pesant 150-200 g sont thyroparathyroïdectomisés. Les rats sont soumis à un régime standard contenant 7 g Ca/kg (UAR) et de l'eau de Volvic. L'efficacité de la thyroparathyroïdectomie est testée en mesurant les concentrations en Ca dans le sérum 8 jours après l'opération chez des animaux à jeun depuis la veille. Les rats sont considérés comme thyroparathyroïdectomisés lorsque les taux de Ca dans le sérum sont inférieurs à 80 mg/l. La PTH (1-34)de rat (Bachem) est dissoute dans 0,15M de NaCl Cys.HCl 2 % et délivré par des minipompes osmotiques (ALZET 2001D) à la dose de 200 pmol/kg/h. Les minipompes sont introduites dans les cavités intrapéritonéales sous anesthésie (kétamine - 75 mg/kg et acépromazine - 2,5 mg/kg) chez des rats TPXT à jeun depuis la veille. Les rats TPXT contrôles reçoivent les minipompes remplies avec le véhicule de la PTH.

Soit le produit à tester soit le véhicule (contrôles et rats traités par la PTH) sont administrés 2 fois par voie sous-cutanée (2 ml/kg de poids corporel) aux temps 0 et 3 h après le début de l'infusion de PTH. Le test est poursuivi pendant les 6 heures suivantes. A la fin du traitement, la totalité du sang est collectée après décapitation. Les échantillons de sang sont centrifugés à 3000 tpm pendant 15 mn (CR422 Jouan) pour obtenir le sérum.

Les concentrations totales de Ca dans le sérum sont mesurées par colorimétrie (Ciba-Corning) en utilisant un système de lecture de microplaques IEMS Labsystems, à 540 nm.

La différence entre les valeurs moyennes de calcémie des rats traités et des groupes contrôles est analysée en variance et par le test de Dunnett.

L'activité d'un produit est calculée par la formule suivante :

$$\text{effet \%} = \frac{\text{Calcémie (produit) - calcémie (PTH)}}{\text{Calcémie (PTH) - calcémie (contrôle)}} \times 100$$

Les produits des exemples 6, 9, 13 et 15 à 19 testés dans la méthode décrite ci-avant se sont montrés actifs à des doses allant de 2 fois 1 mg/kg à 2 fois 10 mg/kg par voie sous cutanée chez le rat.

## Revendications

1. Les composés de formule (I) :

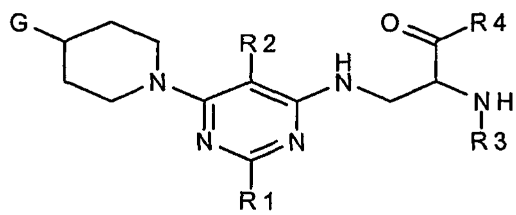

sous toutes leurs formes isomères, seules ou en mélange, ainsi que leurs sels d'addition physiologiquement acceptables et ainsi que leurs solvates, dans laquelle :

- **G** représente :
$R^7R^8N-C(=NR^6)-NH-CO-$
Het-NH-CO-;
Het-NH-$CH_2$-,
Het-,
Het représentant un système monocyclique ou polycyclique, chaque cycle étant constitué de 4 à 10 chaînons aromatiques ou non aromatiques, le cycle ou au moins l'un des cycles renfermant de 1 à 4 atome d'azote, substitué ou non substitué par un ou plusieurs groupements $R_9$
- **R¹** représente un atome d'hydrogène; un groupement ($C_5$-$C_{14}$)-aryle; ($C_5$-$C_{14}$)-aryl-($C_1$-$C_4$)-alkyle-; un radical amino non substitué ou monosubstitué ou disubstitué par un radical alkyle et/ou un radical acyle renfermant de 1 à 4 atomes de carbone;
- **R²** représente un atome d'hydrogène; un atome d'halogène; un groupement nitro; un radical alkyle renfermant de 1 à 4 atomes de carbones; un radical amino non substitué ou monosubstitué ou disubstitué par un radical alkyle et/ou un radical acyle renfermant de 1 à 4 atomes de carbone; un groupement $-(CH_2)_{0-2}-CO_2R^5$; ou un groupement $-(CH_2)_{0-2}-OR^5$;
- **R³** représente :
- un atome d'hydrogène
- un radical $-CO_2R^5$,
- un radical $-SO_2R^5$ ou
- un système monocyclique ou polycyclique, chaque cycle étant constitué de 4 à 10 chaînons aromatiques ou non aromatiques, le cycle ou au moins l'un des cycles renfermant de 1 à 4 hétéroatomes choisis parmi N, O ou S, substitué ou non substitué par un ou plusieurs radicaux $R_9$,
- **R⁴** représente OH; ($C_1$-$C_8$)-alkoxy-; ($C_5$-$C_{14}$)-aryl-($C_1$-$C_4$)-alkyloxy-; ($C_5$-$C_{14}$)-aryloxy-; ($C_3$-$C_{12}$)-cycloalkyloxy; ($C_3$-$C_{12}$)-cycloalkyl-($C_1$-$C_4$)-alkyloxy-; ($C_1$-$C_8$)-alkylcarbonyloxy-($C_1$-$C_4$)-alkyloxy-; ($C_5$-$C_{14}$)-aryl-($C_1$-$C_4$)-alkylcarbonyloxy-($C_1$-$C_4$)alkyloxy-($C_1$-$C_8$)dialkylaminocarbonylméthyloxy-($C_5$-$C_{14}$)-aryl-($C_1$-$C_4$)-dialkylaminocarbonylméthyloxy-; un radical amino non substitué ou monosubstitué ou disubstitué par un radical ($C_1$-$C_4$)-alkyle et/ou ($C_5$-$C_{14}$)-aryle et/ou ($C_5$-$C_{14}$)-aryl-($C_1$-$C_4$)-alkyle- et/ou un radical ($C_1$-$C_5$)-acyle; ou bien le reste d'un aminoacide D ou L;
- **R⁵** représente ($C_1$-$C_8$)-alkyle; ($C_5$-$C_{14}$)-aryle; ($C_5$-$C_{14}$)-aryl-($C_1$-$C_4$) -alkyle-; ($C_3$-$C_{12}$)-cycloalkyle ou ($C_3$-$C_{12}$)-cycloalkyl-($C_1$-$C_4$)-alkyle-, bicycloalkyle-($C_1$-$C_4$)-alkyle-, tricycloalkyle-($C_1$-$C_4$)-alkyle-, les dits radicaux aryles, alkyles, cycloalkyles, bicycloalkyles et tricycloalkyles étant non substitués ou substitués par un ou

plusieurs groupements choisis $R^9$;

- $R^6$ représente un atome d'hydrogène; un groupement hydroxyle; nitro, $(C_1-C_6)$ -alkyl-O-CO-; ou $(C_1-C_6)$ -alkyl-O-CO-O-;
- **$R^7$** et **$R^8$,** indépendamment l'un de l'autre représentent un atome d'hydrogène ou un radical $(C_1-C_6)$-alkyle non substitué ou substitué par $R_9$;
- **$R^9$** représente halogène; amino; nitro; hydroxyle, $(C_1-C_4)$-alkyloxy-; $(C_1-C_4)$ -alkylthio-; carboxy; $(C_1-C_4)$-alkyloxycarbonyle-; $(C_1-C_8)$-alkyle non substitué ou substitué par un ou plusieurs atomes d'halogène, $(C_5-C_{14})$-aryle , $(C_5-C_{14})$-aryl-$(C_1-C_4)$-alkyle-

étant entendu que les radicaux aryles peuvent être non substitués ou substitués par un ou plusieurs radicaux identiques ou différents choisis parmi $(C_1-C_8)$-alkyle, en particulier $(C_1-C_4)$-alkyle, hydroxyle, $(C_1-C_8)$-alkyloxy, $(C_1-C_8)$-alkylthio, halogène choisi parmi fluor, chlore et brome, nitro, amino, $(C_1-C_4)$-alkylamino, di-$(C_1-C_4)$-alkylamino, trifluorométhyle, méthylènedioxy, cyano, aminocarbonyle, $(C_1-C_4)$-alkylaminocarbonyle, di-$(C_1-C_4)$-alkylaminocarbonyle, carboxy, $(C_1-C_4)$-alkoxycarbonyle, phényle, phénoxy, benzyle et benzyloxy ou

- G représente 1,2,3,4-tétrahydro-1,8-naphtyridin-7-yle,
- $R^1$ représente méthyle,
- $R^2$ représente méthyle,
- $R^3$ représente benzyloxycarbonyle et
- $R^4$ représente OH ou t.butoxy.

**2.** Les composés de formule (I) selon la revendication 1 dans laquelle G représente un groupement Het, Het-NHCO-, ou Het-NH-$CH_2$- avec Het représentant les hétérocycles suivants :

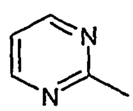
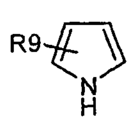
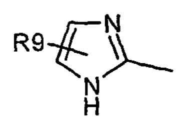

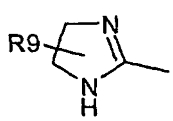
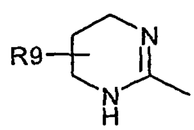
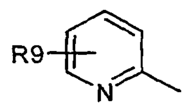

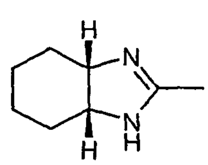
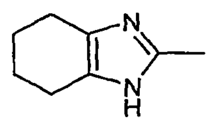
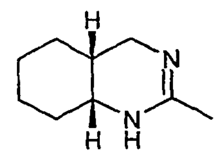

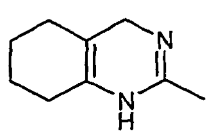
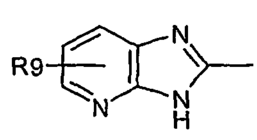
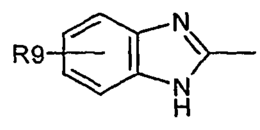

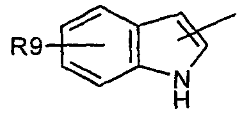 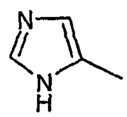 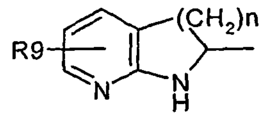

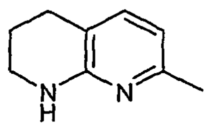 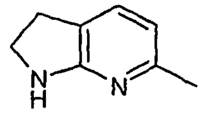 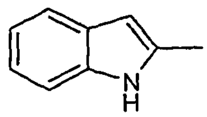 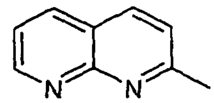

**3.** Les composés de formule (I) selon l'une quelconque des revendications 1 à 2 dans laquelle $R^3$ est :

- un hétérocycle choisi parmi

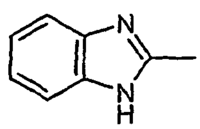 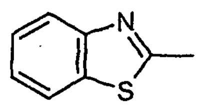 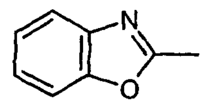

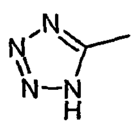 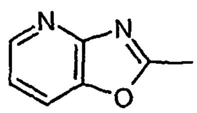 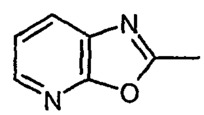

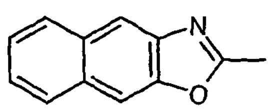 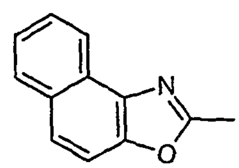

ainsi que leurs sels d'addition pharmaceutiquement acceptables.

**4.** Les composés de formule (I) selon l'une quelconque des revendications 1 à 2 dans laquelle $R^3$ est un groupement benzyloxycarbonyle, ainsi que leurs sels d'addition pharmaceutiquement acceptables.

**5.** Les composés de formule (I) selon l'une quelconque des revendications 1 à 4 dans laquelle $R_2$ est un hydrogène, un radical alkyle renfermant de 1 à 4 atomes de carbones ou un atome de fluor ainsi que leurs sels d'addition pharmaceutiquement acceptables.

**6.** Les composés de formule (I) selon la revendication 5 dans laquelle $R^2$ est un méthyle ou éthyle

**7.** Les composés de formule (I) selon l'une quelconque des revendications 1 à 6 dans laquelle :

. G représente

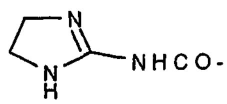 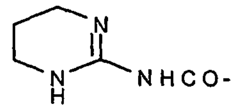 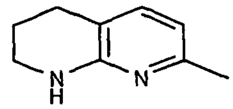

ainsi que leurs sels d'addition pharmaceutiquement acceptables.

**8.** Les composés de formule (I) selon l'une quelconque des revendications 1 à 7 dans laquelle :

G représente

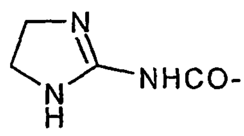 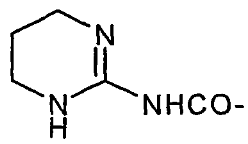 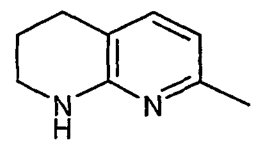

$R^1$ représente un atome d'hydrogène
$R^2$ représente un atome d'hydrogène, un atome de fluor, un radical méthyle ou un radical éthyle,
$R^3$ représente un groupement benzyloxycarbonyloxy
$R^4$ représente un groupement hydroxy
ainsi que les sels d'addition pharmaceutiquement acceptables

**9.** Les composés de formule (I) selon l'une quelconque des revendications 1 à 8 dont les noms suivent :

3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine,
3-[[5-éthyl- 6-[4-[(1,2,3,4,5,6- hexahydro- 2- pyrimidinyl) iminocarbonyl]- 1- pipéridinyl]- 4- pyrimidinyl] amino]-N-[(phénylméthoxy)carbonyl] alanine
3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alanine
3-[[6-[4-[(1,2,3,4,5,6- hexahydro- 2- pyrimidinyl) iminocarbonyl]- 1- pipéridinyl]- 5- méthyl- 4- pyrimidinyl] amino]-N-[(phénylméthoxy)carbonyl]alanine
3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate d'éthyle
3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alaninate d'isopropyle
3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle)
3-[[5-méthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle)
3-[[-6-[4-(1,2,3,4-tétrahydro-1,8-naphthyridin-7-yl)-1-pipéridinyl]-2,5-diméthyl-4-pyrimidinyl]amino]-N-[(phénylméthoxy)carbonyl]alaninate de (1,1-diméthyléthyle)
3-[[5-éthyl-6-[4-(1,2,3,4-tétrahydro-1,8-naphthyridin-7-yl)-1-pipéridinyl]-4-pyrimidinyl]amino]-N-(1-naphtalènesulfonyl)alaninate de (1,1-diméthyléthyle)
sous la configuration (R) ou la configuration (S) ou leurs mélanges, ainsi que leurs sels d'addition.

**10.** Un procédé de préparation des composés de formule (I) dans lequel :

a) on fait réagir un composé de formule (II)

(II) 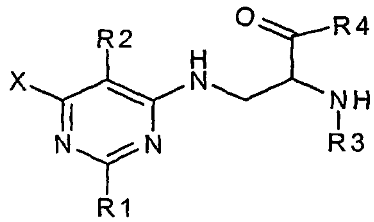

dans laquelle $R^1$, $R^2$, $R^3$ et $R^4$ sont tels que définis à la revendication 1, avec un composé de formule (III)

(III) 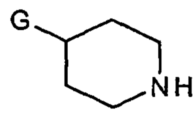

dans laquelle G est telle que définie à la revendiction 1 en présence d'une base ou un réactif de couplage de métal de transition

b) le composé de formule (I) ainsi obtenu est soumis, éventuellement au clivage de la fonction $R^3$-NH- pour régénérer l'amine libre, suivi de la condensation de radicaux $R^3$ de structure $-CO_2-R^5$ ou $-SO_2-R^5$, et/ou le cas échéant, à l'hydrolyse et éventuellement à l'estérification ou à l'amidification et/ou à la salification.

**11.** Un procédé de préparation des composés de formule (I) dans lequel :

a) on fait réagir un composé de formule (II) telle que définie à la revendication 10 avec un composé de formule (IIIa)

(IIIa) 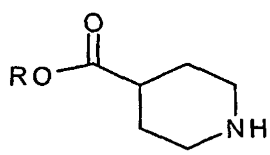

afin d'obtenir le composé intermédiaire de formule (IV)

(IV) 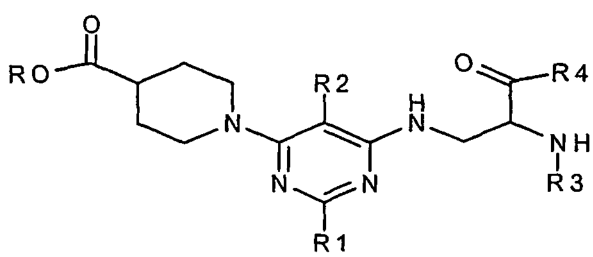

b) on fait réagir le composé de formule (IV) avec un composé de formule $Het-NH_2$, afin d'obtenir les composés de formule (I) avec G représentant un groupement Het-NHCO-,

c) le composé de formule (I) obtenu est soumis, éventuellement au clivage de la fonction $R^3$-NH- pour régénérer l'amine libre, suivi de la condensation de radicaux $R^3$ de structure $-CO_2-R^5$ ou $-SO_2-R^5$, et/ou le cas échéant, à l'estérification ou à l'amidification et/ou à la salification.

**12.** Un procédé de préparation des composés de formule (I) selon l'une des revendications 1 à 9, **caractérisé en ce que**

a) l'on fait réagir un produit de formule générale (IIa)

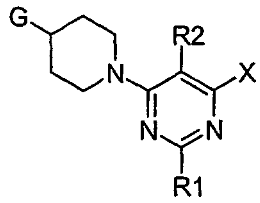

(IIa)

dans laquelle $R^1$, $R^2$, G et X sont tels que définis précédemment,
avec un produit de formule (VI)

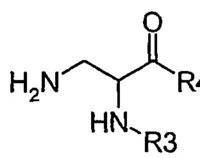

(VI)

dans laquelle $R^3$ et $R^4$ sont définis comme précédemment, soit en présence d'une base forte, soit par catalyse au palladium,
b) puis le produit de formule (I) est soumis , éventuellement au clivage de la fonction $R^3$-NH- pour régénérer l'amine libre, suivi de la condensation de radicaux $R^3$ de structure $-CO_2-R^5$ ou $-SO_2-R^5$, et/ou le cas échéant à l'hydrolyse et éventuellement à l'estérification ou à l'amidification et/ou à la salification.

**13.** A titre de médicament, les composés de formule (I) et/ou leurs sels physiologiquement acceptables tels que définis selon l'une quelconque des revendications 1 à 9.

**14.** Une composition pharmaceutique comprenant un médicament tel que défini à la revendication 13 ainsi qu'un ou plusieurs excipients.

**15.** A titre de médicament, selon la revendication 13, ayant une activité antagoniste du récepteur de la vitronectine, un composé de formule (I) et/ou ses sels physiologiquement acceptables tel que défini selon l'une quelconque des revendications 1 à 9.

**16.** A titre de médicament, selon la revendication 13, ayant une activité inhibitrice de la résorption osseuse ou pour le traitement ou la prévention de l'ostéoporose, un composé de formule (I) et/ou ses sels physiologiquement acceptables tel que défini selon l'une quelconque des revendications 1 à 9.

**17.** A titre de médicament, selon la revendication 13, ayant une activité inhibitrice de la croissance tumorale ou des métastases cancéreuses, un composé de formule (I) et/ou ses sels physiologiquement acceptables tel que défini selon l'une quelconque des revendications 1 à 9.

**18.** A titre de médicament, selon la revendication 13, ayant une activité anti-inflammatoire ou pour le traitement ou la prévention des désordres cardiovasculaires, la resténose, l'artériosclérose, les néphropathies ou rétinopathies, un composé de formule (I) et/ou ses sels physiologiquement acceptables et/ou ses prodrugs tel que défini selon l'une quelconque des revendications 1 à 9.

**19.** Utilisation des composés de formule (I) et/ou leurs sels physiologiquement acceptables tels que définis selon l'une quelconque des revendications 1 à 9 pour la préparation de médicaments destinés à la prévention ou au traitement de l'ostéoporose.

**20.** Utilisation des composés de formule (I) et/ou leurs sels physiologiquement acceptables tels que définis selon l'une quelconque des revendications 1 à 9 pour la préparation de médicaments destinés à inhiber la croissance tumorale

ou les métastases cancéreuses.

**21.** Utilisation des composés de formule (I) et/ou leurs sels physiologiquement acceptables tels que définis selon l'une quelconque des revendications 1 à 9 pour la préparation de médicaments destinés à la prévention ou au traitement des désordres cardiovasculaires, de la resténose, de l'artériosclérose, des néphropathies ou des rétinopathies.

## Claims

**1.** The compounds of formula (I)

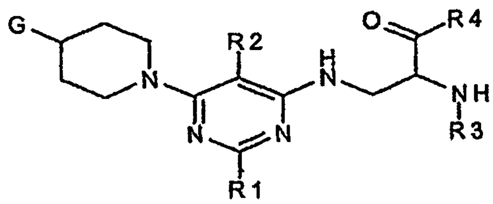

in all their isomeric forms, alone or in mixture, as well as their physiologically acceptable addition salts, and their solvates, in which
G represents
$R^7R^8N$-C (=$NR^6$)-NH-CO-
Het-NH-CO-;
Het-NH-$CH_2$-;
Het-,
Het representing a monocyclic or polycyclic system, each ring being constituted by 4 to 10 aromatic or non aromatic members, the ring or at least one of the rings containing 1 to 4 nitrogen atoms, substituted or non substituted by one or more $R^9$ groups
$R^1$ represents a hydrogen atom; a ($C_5$-$C_{14}$)-aryl; ($C_5$-$C_{14}$)-aryl-($C_1$-$C_4$)-alkyl-group; an amino radical non substituted or monosubstituted or disubstituted by an alkyl radical and/or an acyl radical containing 1 to 4 carbon atoms;
$R^2$ represents a hydrogen atom; a halogen atom; a nitro group; an alkyl radical containing 1 to 4 carbon atoms; an amino radical non substituted or monosubstituted or disubstituted by an alkyl radical and/or an acyl radical containing 1 to 4 carbon atoms; a $-(CH_2)_{0-2}-CO_2R^5$ group; or a $-(CH_2)_{0-2}OR^5$ group;
$R^3$ represents
a hydrogen atom
a $-CO_2R^5$ radical,
a $-SO_2R^5$ radical or
a monocyclic or polycyclic system, each ring being constituted by 4 to 10 aromatic or non aromatic members, the ring or at least one of the rings containing 1 to 4 heteroatoms chosen from N, O or S, substituted or non substituted by one or more $R^9$ radicals,
$R^4$ represents OH; ($C_1$-$C_8$)-alkoxy-; ($C_5$-$C_{14}$)-aryl-($C_1$-$C_4$)-alkyloxy-; ($C_5$-$C_{14}$)-aryloxy-; ($C_3$-$C_{12}$)-cycloalkyloxy; ($C_3$-$C_{12}$)-cycloalkyl-($C_1$-$C_4$)-alkyloxy-; ($C_1$-$C_8$)-alkylcarbonyloxy-($C_1$-$C_4$)-alkyloxy-; ($C_5$-$C_{14}$)-aryl-($C_1$-$C_4$)-alkylcarbonyloxy-($C_1$-$C_4$)-alkyloxy-; ($C_1$-$C_8$)-dialkylaminocarbonylmethyloxy; ($C_5$-$C_{14}$)-aryl-($C_1$-$C_4$)-dialkylaminocarbonylmethyloxy-; an amino radical non substituted or monosubstituted or disubstituted by a ($C_1$-$C_4$)-alkyl and/or ($C_5$-$C_{14}$)-aryl and/or ($C_5$-$C_{14}$)-aryl-($C_1$-$C_4$)-alkyl- and/or a ($C_1$-$C_5$)-acyl radical; or the remainder of an amino acid D or L;
$R^5$ represents ($C_1$-$C_8$)-alkyl-; ($C_5$-$C_{14}$)-aryl-; ($C_5$-$C_{14}$)-aryl-($C_1$-$C_4$)-alkyl-; ($C_3$-$C_{12}$)-cycloalkyl or ($C_3$-$C_{12}$)-cycloalkyl-($C_1$-$C_4$)-alkyl-, bicycloalkyl-($C_1$-$C_4$)-alkyl-, tricycloalkyl-($C_1$-$C_4$)-alkyl-, the said aryl, alkyl, cycloalkyl, bicycloalkyl and tricycloalkyl radicals being non substituted or substituted by one or more chosen $R^9$ groups;
$R^6$ represents a hydrogen atom; a hydroxyl; nitro, ($C_1$-$C_6$)-alkyl-O-CO-; or ($C_1$-$C_6$)-alkyl-O-CO-O-group:
and
$R^7$ and $R^8$, independently of one another represent a hydrogen atom or a ($C_1$-$C_6$)-alkyl radical non substituted or substituted by $R^9$;
$R^9$ represents halogen; amino; nitro; hydroxyl, ($C_1$-$C_4$)-alkyloxy-; ($C_1$-$C_4$)-alkylthio-; carboxy; ($C_1$-$C_4$)-alkyloxycarbonyl-; ($C_1$-$C_8$)-alkyl non substituted or substituted by one or more halogen atoms, ($C_5$-$C_{14}$)-aryl, ($C_5$-$C_{14}$)-aryl-($C_1$-$C_4$)-alkyl;
it being understood that the aryl radicals can be non substituted or substituted by one or more identical or different

radicals chosen from ($C_1$-$C_8$)-alkyl, in particular ($C_1$-$C_4$)-alkyl, hydroxyl, ($C_1$-$C_8$)-alkylox, ($C_1$-$C_8$)-alkylthio, halogen chosen from fluorine, chlorine and bromine, nitro, amino, ($C_1$-$C_4$)-alkylamino, di-($C_1$-$C_4$)-alkylamino, trifluoromethyl, methylenedioxy, cyano, aminocarbonyl, ($C_1$-$C_4$)-alkylaminocarbonyl, di-($C_1$-$C_4$)-alkylaminocarbonyl, carboxy, ($C_1$-$C_4$)-alkoxycarbonyl, phenyl, phenoxy, benzyl and benzyloxy.

**2.** The compounds of formula (I) according to claim 1 in which G represents a Het, Het-NHCO-, or Het-NH-$CH_2$-group with Het representing the following heterocycles:

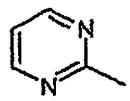
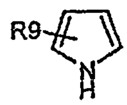
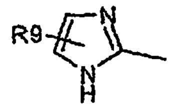

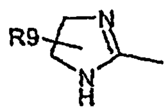
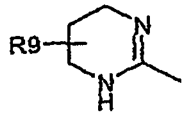
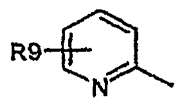

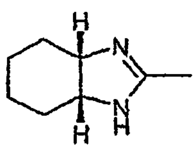
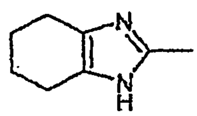
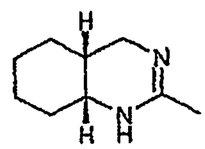

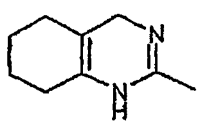
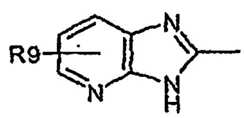
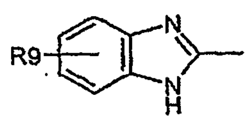

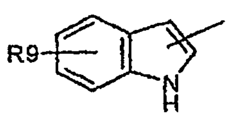
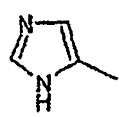
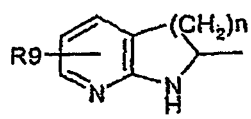

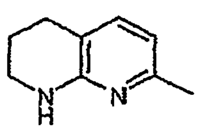
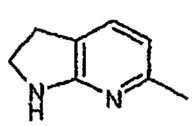
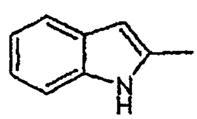
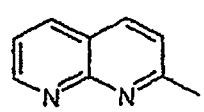

**3.** The compounds of formula (I) according to any one of claims I to 2 in which $R^3$ is:

a heterocycle chosen from

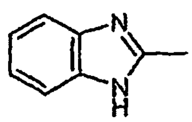 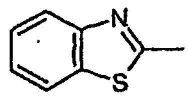 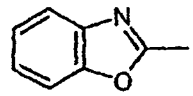

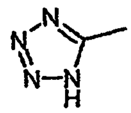 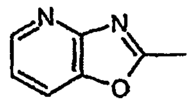 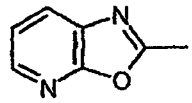

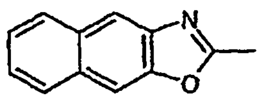 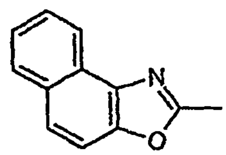

as well as their pharmaceutically acceptable addition salts.

**4.** The compounds of formula (I) according to any one of claims 1 to 2 in which $R^3$ is a benzyloxycarbonyl group, as well as their pharmaceutically acceptable addition salts.

**5.** The compounds of formula (I) according to any one of claims 1 to 4 in which $R^2$ is a hydrogen, an alkyl radical containing 1 to 4 carbon atoms or a fluorine atom as well as their pharmaceutically acceptable addition salts.

**6.** The compounds of formula (I) according to claim 5 in which $R^2$ is a methyl or ethyl.

**7.** The compounds of formula (I) according to any one of claims 1 to 6 in which:

G represents

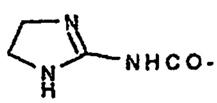 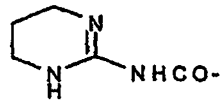 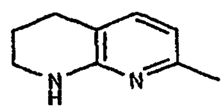

as well as their pharmaceutically acceptable addition salts.

**8.** The compounds of formula (I) according to any one of claims 1 to 7 in which:

G represents

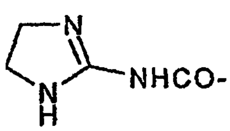 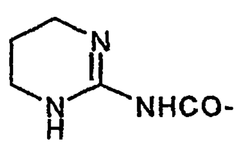 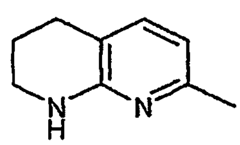

$R^1$ represents a hydrogen atom
$R^2$ represents a hydrogen atom, a fluorine atom, a methyl radical or an ethyl radical,
$R^3$ represents a benzyloxycarbonyloxy group
$R^4$ represents a hydroxy group

as well as the pharmaceutically acceptable addition salts

**9.** The compounds of formula (I) according to any one of claims 1 to 8 the names of which follow:

3-[[5-ethyl-6-[4-(1,2,3,4-tetrahydro-1,8-naphthyridin-7-yl)-1-piperidinyl]-4-pyrimidinyl]amino]-N-[(phenylmethoxy)carbonyl]alanine,
3-[[5- ethyl- 6-[4-[(1,2,3,4,5,6- hexahydro- 2- pyrimidinyl) iminocarbonyl]- 1- piperidinyl]- 4- pyrimidinyl] amino]-N-[(phenylmethoxy)carbonyl]alanine,
3-[[6-[4-(1,2,3,4-tetrahydro-1,8-naphthyridin-7-yl)-1-piperidinyl]-5-methyl-4-pyrimidinyl]amino]-N-[(phenylmethoxy)carbonyl]alanine,
3-[[6-[4-[(1,2,3,4,5,6- hexahydro- 2- pyrimidinyl) iminocarbonyl]- 1- piperidinyl]- 5- methyl- 4- pyrimidinyl] amino]-N-[(phenylmethoxy)carbonyl]alanine,
ethyl 3-[[5-ethyl-6-[4-(1,2,3,4-tetrahydro-1,8-naphthyridin-7-yl)-1-piperidinyl]-4-pyrimidinyl]amino]-N-[(phenylmethoxy)carbonyl]alaninate
isopropyl 3-[[6-[4-(1,2,3,4-tetrahydro-1,8-naphthyridin-7-yl)-1-piperidinyl]-5-methyl-4-pyrimidinyl]amino]-N-[(phenylmethoxy)carbonyl]alaninate
(1,1-dimethylethyl)-3-[[5-ethyl-6-[4-(1,2,3,4-tetrahydro-1,8-naphthyridin-7-yl)-1-piperidinyl]-4-pyrimidinyl]amino]-N-[(phenylmethoxy)carbonyl]alaninate
(1,1-dimethylethyl)-3-[[5-methyl-6-[4-(1,2,3,4-tetrahydro-1,8-naphthyridin-7-yl)-1-piperidinyl]-4-pyrimidinyl] amino]-N-[(phenylmethoxy)carbonyl]alaninate
(1,1-dimethylethyl)-3-[[6-[4-1,2,3,4-tetrahydro-1,8-naphthyridin-7-yl)-1-piperidinyl]-2,5-dimethyl-4-pyrimidinyl] amino]-N-[phenylmethoxy)carbonyl] alaninate
(1,1-dimethylethyl)-3-[[5-ethyl-6-[4-(1,2,3,4-tetrahydro-1,8-naphthyridin-7-yl)-1-piperidinyl]-4-pyrimidinyl]amino]-N-(1-naphtalenesulfonyl)alaninate
in (R) configuration or (S) configuration or their mixtures, as well as their addition salts.

**10.** A process for the preparation of the compounds of formula (I) in which

a) a compound of formula (II)

(II) 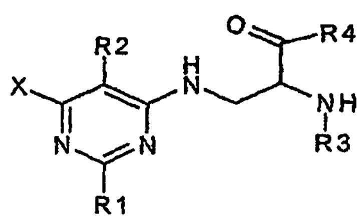

in which $R^1$, $R^2$, $R^3$ and $R^4$ are as defined in claim 1 is reacted,
with a compound of formula (III)

(III) 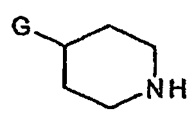

in which G is as defmed in claim 1 in the presence of a base or a transition metal coupling reagent
b) the compound of formula (I) thus obtained is subjected, optionally to cleavage of the $R^3$-NH-function in order to regenerate the free amine, followed by condensation of the $R^3$ radicals of $-CO_2-R^5$ or $-SO_2-R^5$ structure, and/or if appropriate to hydrolysis and optionally to esterification or to amidification and/or to salification.

**11.** A process for the preparation of the compounds of formula (I) in which

a) a compound of formula (II) as defined in claim 10 is reacted with a compound of formula (IIIa)

(IIIa) 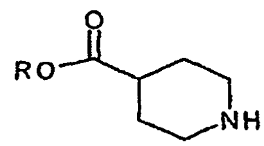

in order to obtain the intermediate compound of formula (IV)

(IV) 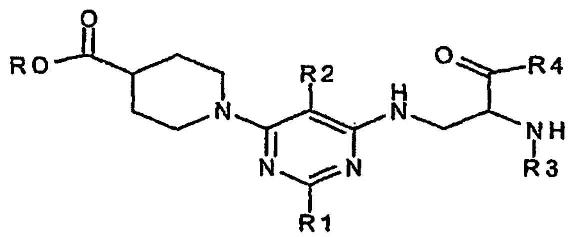

b) the compound of formula (IV) is reacted with a formula of Het-$NH_2$ in order to obtain the compounds of formula (I) with G representing a Het-NHCO-group,
c) the compound of formula (I) obtained is subjected, optionally to cleavage of the function $R^3$-NH- in order to regenerate the free amine, followed by condensation of the $R^3$ radicals of -$CO_2$-$R^5$ or - $SO_2$-$R^5$ structure, and/or if appropriate to hydrolysis and optionally to esterification or to amidification and/or to salification.

**12.** A process for the preparation of the compounds of formula (I) according to one of claims 1 to 9, **characterized in that**

a) a product of general formula (IIa)

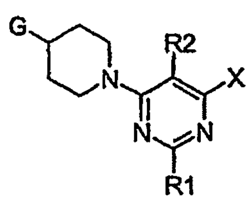
(IIa)

in which
$R^1$, $R^2$, G and X are as defined previously, is reacted with a product of formula (VI)

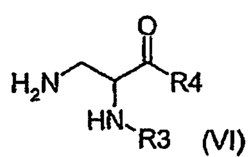
(VI)

in which $R^3$ and $R^4$ are as defined previously, either in the presence of a strong base, or by catalysis with palladium,
b) then the product of formula (I) is subjected, optionally to cleavage of the function $R^3$-NH- in order to regenerate the free amine, followed by condensation of the $R^3$ radicals of -$CO_2$-$R^5$ or -$SO_2$-$R^5$ structure, and/or if appropriate to hydrolysis and optionally to esterification or to amidification and/or to salification.

**13.** As a medicament, the compounds of formula (I) and/or their physiologically acceptable salts as defined according to any one of claims 1 to 9.

**14.** A pharmaceutical composition comprising a medicament as defined in claim 13 as well as one or more excipients.

**15.** As a medicament, according to claim 13, having a antagonist activity on the vitronectin receptor, a compound of formula (I) and/or its physiologically acceptable salts as defined according to any one of claims 1 to 9.

**16.** As a medicament, according to claim 13, having an inhibitory activity on bone resorption or for the treatment or prevention of osteoporosis, a compound of formula (I) and/or its physiologically acceptable salts as defined according to any one of claims 1 to 9.

**17.** As a medicament, according to claim 13, having an inhibitory activity on tumorous growth or cancerous metastases, a compound of formula (I) and/or its physiologically acceptable salts as defined according to any one of claims 1 to 9.

**18.** As a medicament, according to claim 13, having an anti-inflammatory activity or for the treatment or prevention of cardiovascular disorders, restenosis, arteriosclerosis, nephropathies or retinopathies, a compound of formula (I) and/or its physiologically acceptable salts and/or its prodrugs as defined according to any one of claims 1 to 9.

**19.** Use of the compounds of formula (I) and/or their physiologically acceptable salts as defined according to any one of claims 1 to 9 for the preparation of medicaments intended for the prevention or treatment of osteoporosis.

**20.** Use of the compounds of formula (I) and/or their physiologically acceptable salts as defined according to any one of claims 1 to 9 for the preparation of medicaments intended to inhibit tumorous growth or cancerous metastases.

**21.** Use of the compounds of formula (I) and/or their physiologically acceptable salts as defined according to any one of claims 1 to 9 for the preparation of medicaments for the prevention or treatment of cardiovascular disorders, restenosis, arteriosclerosis, nephropathies or retinopathies.

**Patentansprüche**

**1.** Verbindungen mit der Formel (I)

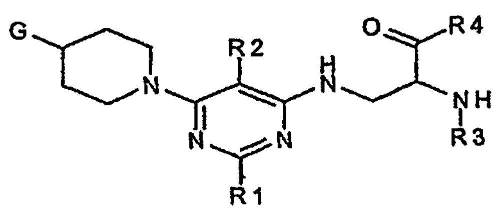

in allen ihren isomeren Formen, allein oder im Gemisch, sowie ihre physiologisch akzeptierbaren Additionssalze und ihre Solvate, wobei
G darstellt:
$R^7R^8N-C(=NR^6)-NH-CO-$
Het-NH-CO-;
Het-NH-$CH_2$-,
Het-,
wobei Het ein monocyclisches oder polycyclisches System darstellt, wobei jeder Ring durch 4 bis 10 aromatische oder nichtaromatische Glieder gebildet wird, wobei der Ring oder mindestens einer der Ringe 1 bis 4 Stickstoffatome enthält, die durch eine oder mehrere $R_9$-Gruppen substituiert oder nicht substituiert sind,
$R^1$ ein Wasserstoffatom; ein ($C_5$-$C_{14}$)-Aryl; eine ($C_5$-$C_{14}$)-Aryl-($C_1$-$C_4$)-Alkyl-Gruppe, ein nicht substituiertes oder durch ein Alkykadikal und/oder ein 1 bis 4 Kohlenstoffatome enthaltendes Acylradikal einfach oder zweifach substituiertes Aminoradikal darstellt;
$R^2$ ein Wasserstoffatom; ein Halogenatom; eine Nitrogruppe, ein 1 bis 4 Kohlenstoffatome enthaltendes Alkylradikal; ein nicht substituiertes oder durch ein Alkylradikal und/oder ein 1 bis 4 Kohlenstoffatome enthaltendes Acylradikal einfach oder zweifach substituiertes Aminoradikal; eine $-(CH_2)_{0-2}-CO_2R^5$-Gruppe oder eine $-(CH_2)_{0-2}-OR^5$-Gruppe darstellt;
$R^3$ darstellt:

ein Wasserstoffatom;
ein $-CO_2R^5$-Radikal,

ein $-SO_2R^5$-Radikal oder
ein monocyclisches oder polycyclisches System, wobei jeder Ring durch 4 bis 10 aromatische oder nichtaromatische Glieder gebildet wird, wobei der Ring oder mindestens einer der Ringe 1 bis 4 unter N, O oder S ausgewählte Heteroatome enthält, die durch ein oder mehrere $R_9$-Radikale substituiert oder nicht substituiert sind,
$R^4$ OH, $(C_1-C_8)$-Alkoxy-; $(C_5-C_{14})$-Aryl-$(C_1-C_4)$-Alkyloxy-; $(C_5-C_{14})$-Aryloxy-; $(C_3-C_{12})$-Cycloalkyloxy-; $(C_3-C_{12})$-Cycloalkyl-$(C_1-C_4)$-alkyloxy-;$(C_1-C_8)$-Alkylcarbonyloxy-$(C_1-C_4)$-Alkyloxy-;$(C_5-C_{14})$-Aryl-$(C_1-C_4)$-Alkylcarbonyloxy-$(C_1-C_4)$-Alkyloxy-;$(C_1-C_8)$-Dialkylaminocarbonylmethyloxy-;$(C_5-C_{14})$-Aryl-$(C_1-C_4)$-Dialkylaminocarbonylmethyloxy-; ein nicht substituiertes oder durch ein $(C_1-C_4)$-Alkyl und/oder $(C_5-C_{14})$-Aryl und/oder $(C_5-C_{14})$-Aryl-$(C_1-C_4)$-Aklradikal und/oder ein $(C_1-C_5)$-Acylradikal einfach oder zweifach substituiertes Aminoradikal oder den Rest einer Aminosäure D oder L darstellt;
$R^5$ ein $(C_1-C_8)$-Alkyl; $(C_5-C_{14})$-Aryl; $(C_5-C_{14})$-Aryl-$(C_1-C_4)$-Alkyl-; $(C_3-C_{12})$-Cycloalkyl oder $(C_3-C_{12})$- Cycloalkyl-$(C_1-C_4)$-Alkyl-, Bicycloalkyl-$(C_1-C_4)$-Alkyl-, Tricycloalkyl-$(C_1-C_4)$-Alkyl- darstellt, wobei die Aryl-, Alkyl-, Cycloalkyl-, Bicycloalkyl- und Tricycloalkylradikale nicht substituiert oder durch eine oder mehrere ausgewählte $R^9$-Gruppen substituiert sind;
$R^6$ ein Wasserstoffatom; eine Hydroxyl-; Nitro-, $(C_1-C_6)$-Alkyl-O-CO- oder $(C_1-C_6)$-Alkyl-O-CO-O-Gruppe darstellt;
$R^7$ und $R^8$ unabhängig voneinander ein Wasserstoffatom oder ein nicht substituiertes oder durch $R_9$ substituiertes $(C_1-C_6)$-Alkylradikal darstellen;
$R^9$ ein Halogen; eine Amino-; Nitro-; Hydroxyl-, $(C_1-C_4)$-Alkyloxy-; $(C_1-C_4)$-Alkylthio-; Carboxy-; $(C_1-C_4)$-Alkyloxycarbonyl-; $(C_1-C_8)$-Alkyl darstellen, das nicht substituiert oder durch ein oder mehrere Halogenatome, $(C_5-C_{14})$-Aryl, -$(C_5-C_{14})$-Aryl-$(C_1-C_4)$-Alkyl- substituiert ist;

wobei es sich versteht, daß die Arylradikale nicht substituiert oder durch ein oder mehrere identische oder verschiedene Radikale substituiert sein können, die unter $(C_1-C_8)$-Alkyl, insbesondere $(C_1-C_4)$-Alkyl, Hydroxyl, $(C_1-C_8)$-Alkyloxy-, $(C_1-C_8)$-Alkylthio-, einem unter Fluor, Chlor und Brom ausgewählten Halogen, Nitro-, Amino-, $(C_1-C_4)$-Alkylamino-, Di-$(C_1-C_4)$-Alkylamino-, Trifluormethyl-, Methylendioxy-, Cyano-, Aminocarbonyl-, $(C_1-C_4)$-Alkylaminocarbonyl-, Di-$(C_1-C_4)$-Alkylaminocarbonyl-, Carboxy-, $(C_1-C_4)$-Alkoxycarbonyl-, Phenyl-, Phenoxy-, Benzyl- und Benzyloxy-Radikalen ausgewählt sind.

**2.** Verbindungen mit der Formel (I) nach Anspruch 1, wobei G eine Het-, Het-NHCO- oder Het-NH-$CH_2$-Gruppe darstellt, wobei Het die folgenden Heterocyclen darstellt:

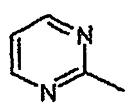
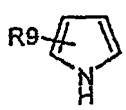
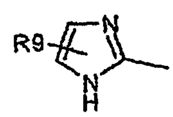

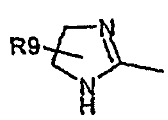
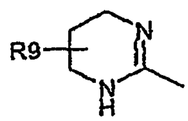
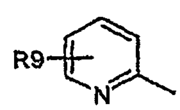

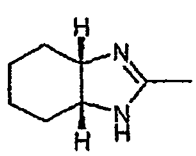
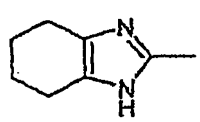
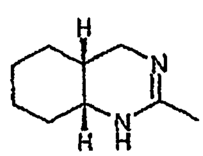

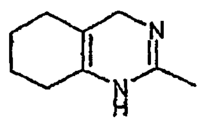 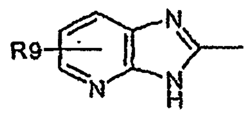 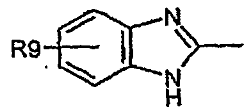

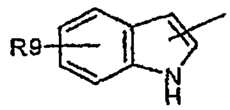 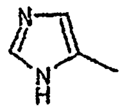 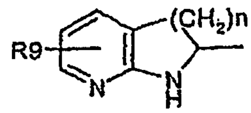

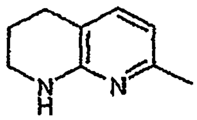 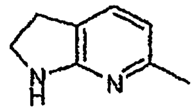 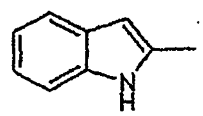 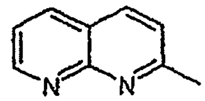

**3.** Verbindungen mit der Formel (I) nach einem der Ansprüche 1 bis 2, wobei $R^3$ einen Heterocyclus darstellt, der unter

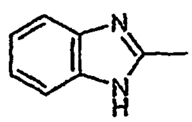 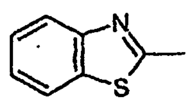 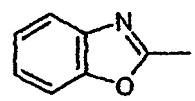

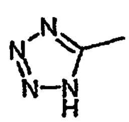 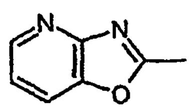 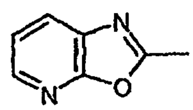

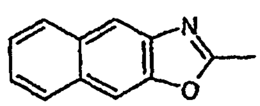 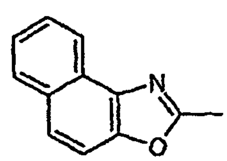

ausgewählt ist, sowie ihre pharmazeutisch akzeptierbaren Additionssalze.

**4.** Verbindungen mit der Formel (I) nach einem der Ansprüche 1 bis 2, wobei $R^3$ eine Benzyloxycarbonylgruppe ist; sowie ihre pharmazeutisch akzeptierbaren Additionssalze.

**5.** Verbindungen mit der Formel (I) nach einem der Ansprüche 1 bis 4, wobei $R_2$ Wasserstoff, ein 1 bis 4 Kohlenstoffatome enthaltendes Alkylradikal oder ein Fluoratom ist, sowie ihre pharmazeutisch akzeptierbaren Additionssalze.

**6.** Verbindungen mit der Formel (I) nach Anspruch 5, wobei $R^2$ ein Methyl oder Ethyl ist.

**7.** Verbindungen mit der Formel (I) nach einem der Ansprüche 1 bis 6, wobei:

G darstellt:

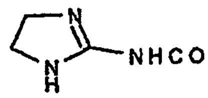 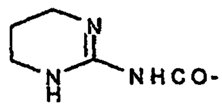 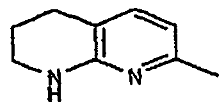

sowie ihre pharmazeutisch akzeptierbaren Additionssalze.

**8.** Verbindungen mit der Formel (I) nach einem der Ansprüche 1 bis 7, wobei:

G darstellt:

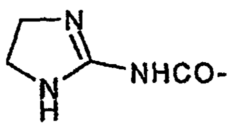 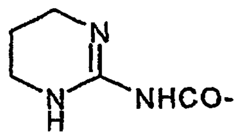 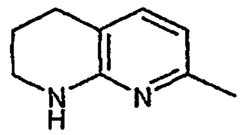

$R^1$ ein Wasserstoffatom darstellt;
$R^2$ ein Wasserstoffatom, ein Fluoratom, ein Methylradikal oder ein Ethylradikal darstellt;
$R^3$ eine Benzyloxycarbonyloxy-Gruppe darstellt;
$R^4$ eine Hydroxylgruppe darstellt;
sowie die pharmazeutisch akzeptierbaren Additionssalze.

**9.** Verbindungen mit der Formel (I) nach einem der Ansprüche 1 bis 8, mit den folgenden Namen:

3-[[5-Ethyl-6-[4-(1,2,3,4-tetrahydro-1,8-naphthyridin-7-yl)-1-piperidinyl]-4-pyrimidinyl]amino]-N-[(phenylinethoxy)carbonyl]alanin,
3-[[5- Ethyl- 6-[4-(1,2,3,4,5,6- hexahydro- 2- pyrimidinyl) iminocarbonyl]- 1- piperidinyl]- 4- pyrimidinyl] amino]-N-[(phenylmethoxy) carbonyl]alanin,
3-[[6-[4-(1,2,3,4-Tetrahydro-1,8-naphthyridin-7-yl)-1-piperidinyl]-5-methyl-4-pyrimidinyl] amino]-N-[(phenylmethoxy)carbonyl]alanin,
3-[[6-[4-[(1,2,3,4,5,6- Hexahydro- 2- pyrimidinyl) iminocarbonyl]- 1- piperidinyl]- 5- methyl- 4- pyrimidinyl] amino]-N-[(phenylmethoxy)carbonyl]alanin,
Ethyl-3-[[5-ethyl-6-[4-(1,2,3,4-tetrahydro-1,8-naphthyridin-7-yl)-1-piperidinyl]-4-pyrimidinyl]amino]-N-[(phenylmethoxy)carbonyl]alaninat,
Isopropyl- 3-[[6-[4-(1,2,3,4- tetrahydro- 1,8- naphthyridin- 7- yl)- 1- piperidinyl]- 5- methyl- 4- pyrimidinyl] amino]-N-[(phenylmethoxy)carbonyl]alaninat,
(1,1-Dimethylethyl)-3-[[5-ethyl-6-[4-(1,2,3,4-tetrahydro-1,8-naphthyridin-7-yl)-1-piperidinyl]-4-pyrimidinyl]amino]-N-[(phenylmethoxy)carbonyl]alaninat,
(1,1-Dimethylethyl)-3-[[5-methyl-6-[4-(1,2,3,4-tetrahydro-1,8-naphthyridin-7-yl)-1-piperidinyl]-4-pyrimidinyl] amino]-N-[(phenylmethoxy)carbonyl]alaninat,
(1,1-Dimethylethyl)-3-[[-6-[4-(1,2,3,4-tetrahydro-1,8-naphthyridin-7-yl)-1-piperidinyl]-2,5-dimethyl-4-pyrimidinyl]amino]-N-[phenylmethoxy)carbonyl]alaninat,
(1,1-Dimethylethyl)-3-[[5-ethyl-6-[4-(1,2,3,4-tetrahydro-1,8-naphthyridin-7-yl)-1-piperidinyl]-4-pyrimidinyl]amino]-N-(1-naphthalinsulfonyl)alaninat,
in (R)-Konfiguration oder (S)-Konfiguration, oder ihre Gemische sowie ihre Additionssalze.

**10.** Verfahren zur Herstellung der Verbindungen mit der Formel (I), wobei

a) eine Verbindung mit der Formel (II)

(II) 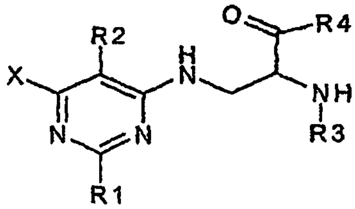

wobei $R^1$, $R^2$, $R^3$ und $R^4$ den Definitionen in Anspruch 1 entsprechen, mit einer Verbindung mit der Formel (III)

(III) 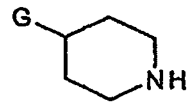

wobei G wie in Anspruch 1 definiert ist, in Gegenwart einer Base oder eines Übergangsmetall-Haftvermittlers zur Reaktion gebracht wird;
b) die so erhaltene Verbindung mit der Formel (I) wahlweise einer Abspaltung der Funktion $R^3$-NH- zur Regenerierung des freien Amins, gefolgt von einer Kondensation der $R^3$-Radikale mit der Struktur $-CO_2-R^5$ oder $-SO_2-R^5$, und/oder gegebenenfalls einer Hydrolyse und wahlweise einer Veresterung oder Amidisierung und/oder Salzbildung ausgesetzt wird.

**11.** Verfahren zur Herstellung der Verbindungen mit der Formel (I), wobei

a) eine Verbindung mit der Formel (II), wie in Anspruch 10 definiert, mit einer Verbindung mit der Formel (IIIa) zur Reaktion gebracht wird:

(IIIa) 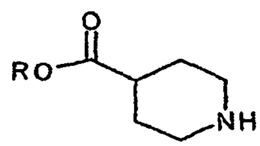

um die Zwischenverbindung mit der Formel (IV) zu erhalten:

(IV) 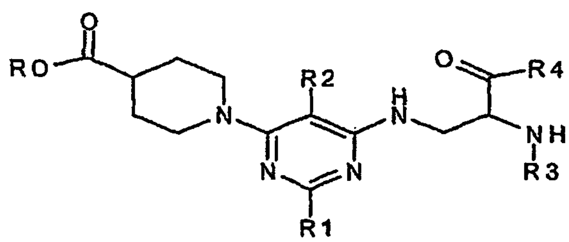

b) die Verbindung mit der Formel (IV) mit einer Verbindung mit der Formel Het-$NH_2$ zur Reaktion gebracht wird, um die Verbindungen mit der Formel (I) zu erhalten, wobei G eine Het-NHCO-Gruppe darstellt,
c) die erhaltene Verbindung mit der Formel (I) wahlweise einer Abspaltung der Funktion $R^3$-NH- zur Regenerierung des freien Amins, gefolgt von einer Kondensation von $R^3$-Radikalen mit der Struktur $-CO_2-R^5$ oder $-SO_2R^5$, und/oder gegebenenfalls einer Veresterung oder Amidisierung und/oder Salzbildung ausgesetzt wird.

**12.** Verfahren zur Herstellung der Verbindungen mit der Formel (I) nach einem der Ansprüche 1 bis 9,
**dadurch gekennzeichnet, daß**

a) ein Produkt mit der allgemeinen Formel (IIa)

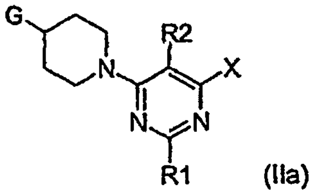

(IIa)

wobei $R^1$, $R^2$, G und X den weiter oben gegebenen Definitionen entsprechen,
mit einem Produkt mit der Formel (VI)

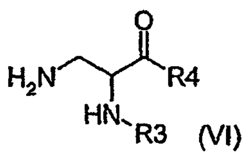

(VI)

wobei $R^3$ und $R^4$ den weiter oben gegebenen Definitionen entsprechen, entweder in Gegenwart einer starken Base oder durch Katalyse mit Palladium zur Reaktion gebracht wird,
b) dann das Produkt mit der Formel (I) wahlweise einer Abspaltung der Funktion $R^3$-NH-zur Regenerierung des freien Amins, gefolgt von einer Kondensation von $R^3$-Radikalen mit der Struktur $-CO_2-R^5$ oder $-SO_2-R^5$, und/oder gegebenenfalls einer Hydrolyse und wahlweise einer Veresterung oder Amidisierung und/oder Salzbildung ausgesetzt wird.

**13.** Als Medikament, die Verbindungen mit der Formel (I) und/oder ihre physiologisch akzeptierbaren Salze nach einem der Ansprüche 1 bis 9.

**14.** Pharmazeutische Zusammensetzung, die ein Medikament nach Anspruch 13 sowie ein oder mehrere Trägerstoffe aufweist.

**15.** Als Medikament nach Anspruch 13 mit antagonistischer Wirkung auf den Vitronectin-Rezeptor, eine Verbindung mit der Formel (I) und/oder ihre physiologisch akzeptierbaren Salze nach einem der Ansprüche 1 bis 9.

**16.** Als Medikament nach Anspruch 13 mit Hemmwirkung auf Knochenresorption oder für die Behandlung oder Verhütung von Osteoporose, eine Verbindung mit der Formel (I) und/oder ihre physiologisch akzeptierbaren Salze nach einem der Ansprüche 1 bis 9.

**17.** Als Medikament nach Anspruch 13 mit Hemmwirkung auf Tumorwachstum oder Krebsmetastasen, eine Verbindung mit der Formel (I) und/oder ihre physiologisch akzeptierbaren Salze nach einem der Ansprüche 1 bis 9.

**18.** Als Medikament nach Anspruch 13 mit entzündungshemmender Wirkung oder für die Behandlung oder Verhütung von Herz-Kreislauf-Störungen, Restenose, Arteriosklerose, Nephropathien oder Retinopathien, eine Verbindung mit der Formel (I) und/oder ihre physiologisch akzeptierbaren Salze und/oder ihre Prodrugs nach einem der Ansprüche 1 bis 9.

**19.** Verwendung der Verbindungen mit der Formel (I) und/oder ihrer physiologisch akzeptierbaren Salzen nach einem der Ansprüche 1 bis 9 für die Herstellung von Medikamenten, die für die Verhütung oder Behandlung von Osteoporose vorgesehen sind.

**20.** Verwendung der Verbindungen mit der Formel (I) und/oder ihrer physiologisch akzeptierbaren Salzen nach einem der Ansprüche 1 bis 9 für die Herstellung von Medikamenten, die für die Hemmung von Tumorwachstum oder Krebsmetastasen vorgesehen sind.

**21.** Verwendung der Verbindungen mit der Formel (I) und/oder ihrer physiologisch akzeptierbaren Salzen nach einem

der Ansprüche 1 bis 9 für die Verhütung oder Behandlung von Herz-Kreislauf-Störungen, Restenose, Arteriosklerose, Nephropathien oder Retinopathien.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9412181 A
- WO 9408577 A
- EP 528586 A
- EP 528587 A
- WO 9532710 A
- WO 9600574 A
- WO 9600730 A
- WO 9800395 A
- WO 9932457 A
- WO 9937621 A
- EP 0820991 A
- EP 1065207 A
- WO 0078317 A


### Littérature non-brevet citée dans la description


- **HORTON et al.** *Exp. Cell. Res.,* 1991, vol. 195, 368
- **SATO et al.** *J. Cell. Biol.,* 1990, vol. 111, 1713
- **FISHER et al.** *Endocrinology,* 1993, vol. 132, 1411
- **BROWN et al.** *cardiovascular Res.,* 1994, vol. 28, 1815
- **BROOK et al.** *Cell,* 1994, vol. 79, 1157
- **CHERESH et al.** *Science,* 1995, vol. 270, 1500
- **GREENE.** Wuts protective Group in Organic Synthesis. Wiley, 1991
- **TOTU; KÔNIG et al.** Proc. 21st Europ. Peptide Symp. 1990, 243
- *Dermot Cox DN§P,* Mai 1995, vol. 8 (4), 197-205
- Drug targeting, voir Targeted Drug Delivery. **R. C. JULIANO et al.** Handbook of Experimental Pharmacology. Springer Verlag, vol. 100
- *J. Med. Chem.,* 2001, vol. 44 (8), 1158-1176
- **R. F. EVANS.** *J. Chem. Soc.,* 1964, 2450-2455
- **PYTELLA et al.** *Methods Enzymol.,* 1987, 144